# EUROPEAN PATENT APPLICATION

(11) **EP 1 580 195 A1**
(43) Date of publication of application: **28.09.2005**
(21) Application number: 04290818.6
(22) Date of filing: 26.03.2004
(51) Int. Cl.: C07K 14/245, A61K 39/108, C07K 16/12

(54) **Compositions of polypeptides specific to pathogenic ExPEC E. coli strains and their use as vaccines and in immunotherapy**

(71) Applicant: Mutabilis SA, 75730 Paris Cedex 15 (FR)
(72) Inventor: Escaich, Sonia, 75012 Paris (FR)
(74) Representative: Peaucelle, Chantal

(57) **Abstract**

The invention relates to compositions of polypeptides specific to pathogenic strains comprising at least one polypeptide of a first group, having a sequence selected in the group comprising the sequences of SEQ ID N°1 to N°66 or 133-145 and at least one peptide of a second group, having SEQ ID N° 159, or homologous sequences of polypeptides of the first group and/or the second group with a minimum of 25% of identity with the whole sequences of said polypeptides.

Application for the preparation of vaccine compositions specific to *E. coli* extra-intestinal infections.

## Description

The invention relates to new compositions of polypeptides specific to pathogenic strains, particularly to extra-intestinal *E. coli* strains.

It more particularly relates to combinations of antigenic polypeptides and combinations of antibodies directed against said polypeptides and to their use as vaccines and in immunotherapy, respectively.

Although *Escherichia coli* is probably the best known bacterial species and is one of the most common isolated in clinical microbiology laboratories, misconceptions abound regarding the various types of *E*. *coli* and the infections they cause.

*E. coli* strains of biological significance to humans can be broadly classified in 3 major groups:
1. Commensal strains, which are part of the normal flora.
2. Intestinal pathogenic strains, which are not part of the normal flora. This group contains various pathotypes (EPEC, EHEC, ETEC, EIEC) not including *Shigella.*
3. Extra-intestinal strains (ExPEC) which are responsible for infections outside the gastro-intestinal (GI) tract, but can also be part of the normal flora. All hosts, either immunocompromised or not are susceptible to these infections.

ExPEC strains are responsible for the majority of the urinary tract infections (UTI) particularly cystitis, pyelonephritis, and cathether associated infections.

They are also responsible for abdominal infections, nosocomial pneumoniae, neonatal meningitidis, soft tissue infections, and bone infections. Each one of these localizations can lead to bacteremia with a risk of sepsis in case of organ failure. ExPEC strains are indeed the most common Gram negative bacilli isolated from blood cultures.

750 000 cases of bacterial sepsis occur each year in the US, and are responsible for 225 000 deaths. In a recent study on 1690 cases of sepsis, it was shown that the main bacteria species identified is ExPEC (16% of the cases) and then *S.aureus* (14% of the cases).

These numbers demonstrate the importance of ExPEC strains in both hospital and community acquired infections.

ExPEC strains correspond to a homogenous subset of *E. coli* strains. Analysis of phylogenetic relationships among *E. coli* strains by MLEE has revealed that *E. coli* belong to 4 main phylogenetic groups designated A, B1, B2 and D.

The pathogenesis of ExPEC strains is that of extra-cellular microorganisms, i.e., they are well adapted to growth in the extra-cellular fluids and efficiently resist phagocytosis by polymorphonuclear. Initial studies have shown that virulence factors known to be important for the extra-cellular growth are mainly found in B2/D *E. coli*., thus suggesting that B2/D subgroups contain most of the ExPEC strains. This was reinforced by experiments performed on animals showing that B2/D strains are more virulent than A and B1 strains. Subsequent epidemiological studies have indeed confirmed these hypotheses. B2/D isolates are those predominantly responsible for neonatal meningitidis (87%) and community or nosocomial acquired urosepsis, (93 % and 85%, respectively).

Similar results have been reported for cystitis (70% are due to the sole B2 *E. coli*), thus demonstrating that the importance of ExPEC strains.

These recent findings demonstrate that the B2/D subgroup of strains is the E. *coli* core genome the best adapted to growth in extra-cellular fluids.

In addition to this core genome, ExPEC strains have various pathogenicity islands which encode virulence factors associated with the different pathogenesis of extra-intestinal *E. coli* infections (UTI, urosepsis, neonatal meningitidis...). Among the main virulence factors are the capsule, which is well-known to be important for extra-cellular growth, and the iron chelation systems (aerobactin and enterochelin, for example). In addition, depending on the pathogenesis, these strains can produce toxins (CNF, hemolysin...), adhesins (pap, sfa...) and other iron chelation systems.

The notion that B2/D *E. coli* correspond to a distinct subset of pathogenic E. *coli* strains is reinforced by the fact that B2/D *E. coli* are not broadly isolated from the stools of humans. They were recovered from only 11% of individuals, whereas A and B1 subgroups are present in the stools of 74% of the individuals of a human population.

As mentioned above the pathogenesis of ExPEC strains relies on their ability to multiply in the extra-cellular fluids and to resist bactericidal activity of the complement and phagocytosis by polymorphonuclear. Therefore, as for other extra-cellular pathogens (*Haemophilus influenzae, Streptococcus pneumonieae* and *Neisseria meningitidis*) a protective antigen against ExPEC has to induce antibodies that promote opsonisation and/or the bactericidal activity of serum.

Considering the above statements, an efficient antigen has to be largely represented among the population of B2/D *E. coli.*

Similarly to other extra-cellular pathogens, the capsular polysaccharide would be an ideal antigen, however most pathogenic B2 strains express the K1 polysaccharide. The latter has a structure identical to that of group B meningococcus, which is non-immunogenic and shares common antigens with the brain. Another possible target may be the lipopolysaccharide (LPS). However there are a large number of different LPS serotypes that are shared by various subgroups.

The inventors have now found that some specific compositions of polypeptides coded by the B2/D genome, but absent from A and B1 *E. coli* strains, are particularly useful as antigens and can specifically prevent the pathologies due to ExPEC strains. Homologs of these antigenic components can be found in other pathogenic bacterial species and therefore are useful to prevent the pathologies caused by these bacteria. Accordingly, any reference to products specific to ExPEC strains and to their uses will encompass components in these species.

For example homologuous antigens could be present in the following species and be as such used for prevention of disease due to the bacteria:
*Pseudomonas aeruginosa, Escherichia coli* O157:H7, Yersinia *pestis, Vibrio cholerae, Legionella pneumophila, Salmonella enterica, Salmonella typhimurium, Haemophilus influenzae, Neisseria meningitidis, Neisseria gonorrhoeae,* *Bacillus anthracis, Burkholderia cepacia, Campylobacter jejuni, Chlamydia pneumoniae, Chlamydia trachomatis, Clostridium botulinum, Clostridium difficile, Cryptococcus neoformans, Enterobacter* cloacae, *Enterococcus faecalis, Helicobacter pylori, Klebsiella pneumoniae, Mycobacterium Ieprae, Mycobacterium tuberculosis, Pseudomonas aeruginosa, Salmonella paratyphi, Salmonella typhi, Staphylococcus aureus, Klebsiella pneumoniae, Listeria monocytogenes, Moxarella catarrhalis, Shigella dysenteriae, Shigella flexneri, Shigella sonnei, Staphylococcus epidermidis, Streptococcus pneumoniae,* and any species falling within the genera of any of the above species.

It is then an object of the invention to provide new combinations of isolated antigenic polypeptides, and new combinations of isolated polynucleotides belonging to the core B2/D genome and not present in commensal *E. coli.*

Another object of the invention is to provide new combinations of antibodies raised against the antigenic polypeptides of said combinations, or peptidic fragments thereof.

It is still another object of the invention to provide vectors and host cells containing said polynucleotides.

Another object of the invention is to provide vaccine compositions specific to extra intestinal infections caused by ExPEC and pathologies caused by other pathogenic strains expressing antigenic polypeptides homologous to the ExPEC antigenic polypeptides.

The invention also relates to means for detecting and treating a development of E. coli in a human or animal compartment which is extra-intestinal (systemic and non-diarrhoeal infections, such as septicaemia, pyelonephritis, or meningitis in the newborn).

The combinations of isolated antigenic polypeptides used according to the invention are selected among polypeptides specific to B2/D *E. coli* strains and not present in A and B1 isolates of *E. coli.* They are encoded by genes belonging to the core B2/D genome and are not present in commensal *E. coli.*

They comprise at least one polypeptide of a first group, having a sequence selected in the group comprising the sequences of SEQ ID N°1 to N°66 or 133-145 and at least one peptide of a second group, having SEQ ID N° 159, or homologous sequences of polypeptides of the first group and/or the second group with a minimum of 25% of identity with the whole sequences of said polypeptides.

Preferred compositions comprise combinations with the polypeptide of the second group having SEQ ID N° 159.

Others preferred compositions comprise combinations wherein the polypeptides of the first group have a sequence selected in the group comprising SEQ ID N° 14, 15, 17, 21, 22, 23, 28, 29, 30, 32, 36, 38, 39, 41-44, 46, 49, 50, 52 to 55, 58, 60, 63, 133-138.

The above-mentioned polypeptides of the first group and the polynucleotides coding for said polypeptides are disclosed in WO 03/074553 in the name of Mutabilis SA.

The polypeptide of SEQ ID N° 159 and the polynucleotides having SEQ ID N°160 coding for said polypeptides are disclosed in WO 0121636 in the name of New-York University.

The invention also relates to combinations wherein said homologous isolated antigenic polypeptides of the first group have at least 25% identity to a polypeptide having a sequence SEQ ID N° as above defined, more particularly having SEQ ID N°14, 15, 17, 21, 22, 23, 28, 29, 30, 32, 36, 38, 39, 41-44, 46, 49, 50, 52 to 55, 58, 60, 63, 133-138, or at least 25% identity to a fragment comprising at least 5, at least 10, at least 20, at least 30, at least 40, at least 50, at least 60 or more than 60 consecutive amino acids of a polypeptide having a sequence corresponding to said SEQ ID N°s, as determined using BLASTP or BLASTX with the default parameters.

The invention also relates to combinations comprising homologous isolated antigenic peptides of second group having at least 25% identity to a polypeptide having SEQ ID N°159.

The invention also relates to the use in combination of isolated polynucleotides coding for a polypeptide of the first group and of isolated polynucleotides coding for polypeptides a polypeptide of the second group such as above defined according to the universal genetic code and taking into account the degeneracy of this code. The term "polynucleotide" emcompasses any nucleotidic sequence such as DNA, including cDNA, RNA, including mRNA.

The polynucleotides coding for the polypeptides of the first group have preferably sequences corresponding to SEQ ID N° 67 to SEQ ID N° 132 or 146 to 158.

More preferably, said polynucleotides have sequences corresponding to SEQ ID N° 80, 81, 83, 87, 88, 89, 94, 95, 96, 98, 102, 104, 105, 107-110, 112, 115, 116, 118, 119, 126, 127, 130, 132, 135, 146-151.

The polynucleotides coding for the polypeptides of the second group have preferably sequence SEQ ID N° 160 of homologs to said polynucleotides. Said homologs may have at least 25% identity to a polynucleotide having said sequences, or at least 25% identity to a fragment comprising at least 15, at least 30, at least 60, at least 90, at least 120, at least 150, at least 180 or more than 180 consecutive nucleotide of a polynucleotide having one of said SEQ ID N°s, as determined using BLASTN with the default parameters, and are encompassed by the invention inasmuch as they are capable of coding for a polypeptide having the antigenic properties of those according to the invention.

The present application is also aimed towards any expression vector comprising at least one isolated polynucleotides coding for a polypeptide of said first group and at least one polynucleotide coding for a polypeptide of said second group according to the universal genetic code and taking into account the degeneracy of this code. The term "polynucleotide" encompasses any nucleotidic sequence such as DNA, including cDNA, RNA, including mRNA.

Preferred vectors comprise polynucleotides coding for the polypeptides of the first group having preferably sequences corresponding to SEQ ID N°77 to SEQ ID N°132 or 146 to 158.

More preferred vectors comprise, polynucleotides having sequences corresponding to SEQ ID N° 80, 81, 83, 87, 88, 89, 94, 95, 96, 98, 102, 104, 105, 107-110, 112, 115, 116, 118, 119, 126, 127, 130, 132, 135, 146-151.

More preferred vectors further comprise polynucleotides coding for the polypeptides of the second group having sequence SEQ ID N° 160.

Said vectors may also comprise homologs to said polynucleotides. Said homologs may have at least 25% identity to a polynucleotide having said sequences, or at least 25% identity to a fragment comprising at least 15, at least 30, at least 60, at least 90, at least 120, at least 150, at least 180 or more than 180 consecutive nucleotide of a polynucleotide having one of said SEQ ID N°s, as determined using BLASTN with the default parameters, and are encompassed by the invention inasmuch as they are capable of coding for a polypeptide having the antigenic properties of those according to the invention.

The invention also relates to any cell transformed by genetic engineering, characterized in that it comprises, by transfection, at least one of polynucleotides coding for a polypeptide of said first group and at least one polynucleotide coding for a polypeptide of said second group and/or at least one vector according to the invention, and/or in that said transformation induces the production by this cell of said polypeptides.

The combinations of said antigenic polypeptides are capable of inducing an antibody response for prevention of infections due to ExPEC strains regardless of the pathogenesis and of the infection site (UTI, pyelonephritis, sepsis, bacteremia, neonatal meningitis).

The invention thus relates to vaccine compositions specific to *E. coli* extra-intestinal infections, comprising an effective amount of at least one antigenic polypeptide or fragment thereof of said first group and at least one antigenic polypeptide or fragment thereof of the second group, with a carrier, particularly at least one polypeptide of SEQ ID N°1 to SEQ ID N°66 and 133-145 and the homologous polypeptides, and at least one polypeptide of SEQ ID N° 159.

Such vaccine compositions are particularly useful for preventing urinary system infections, pyelonephritis, sepsis, bacteremia, neonatal meningitis.

The vaccine compositions of the invention are indicated for :
- Immunodepressed patients, ideally before the start of the immunosuppressive therapy: patients suffering from cancer, diabetes, leukaemia, transplant patients, patients receiving long-term steroids therapy.
- Patients before surgery where there is a high risk of *E. coli* infections (abdominal surgery).
- In all these cases, the *E. coli* vaccine of the invention could be administered in association with a *Staphylococcus aureus* vaccine or a group B *Streptococcus* vaccine,
- Patients with recurrent UTI, especially after one episode of pyelonephritis,
- The prevention of neonatal infections will require vaccination of the mother, implying vaccination long before pregnancy to avoid potential problem. Ideally such a vaccine should be associated with a Group B *Streptococcus* polysaccharide vaccine in order to also prevent late onset neonatal infections. It should be pointed out that the induction of a level of antibodies against B2/D *E. coli* in pregnant women would also prevent UTI, which are always a risk in the context of a pregnancy.

The formulation and the dose of said vaccine compositions can be developed and adjusted by those skilled in the art as a function of the indication targeted, of the method of administration desired, and of the patient under consideration (age, weight).

These compositions comprise one or more physiologically inert vehicles, and in particular any excipient suitable for the formulation and/or for the method of administration desired.

For example the vaccine could be a suspension of the purified polypeptide in sterile water with aluminium based miniral salt as adjuvant and be administered subcutanously with a first and boosting injection.

The combinations of antibodies respectively raised against at least one polypeptides of said first group and at least one polypeptide of said second group are also part of the invention.

They are capable of binding to said polypeptides in physiological-type conditions (in vivo or mimicking *in vivo)* when administered to a human or animal organism, and ELISA-type conditions when said binding product is intended to be used in assays and methods *in vitro.* Such combinations of antibodies advantageously inhibit the extra-intestinal growth of ExPEX strains in human or animal.

The invention thus relates to pharmaceutical compositions comprising an effective amount of a combination of antibodies such as above defined.

Such pharmaceutical compositions are particularly useful for immunotherapy applications for treatment and prevention of severe infections in at risk populations such as neonates or patients undergoing surgical procedures, or having urinary tract infections to prevent septicemia. For these applications specific human monoclonal antibody (Mab) will be derived from said peptides or polypeptides.

Such pharmaceutical compositions comprising an effective amount of a combination of antibodies such as above defined are also useful for treating neonatal infections, in association with antibodies against *Staphylococcus aureus* and/or antibodies against group B *Streptococcus.*

The methods for manufacturing such antibodies using the polypeptides of the combinations according to the invention are available to those skilled in the art. They are conventional methods which comprise, in particular, the immunization of animals such as rabbits and the harvesting of the serum produced, followed optionally by the purification of the serum obtained. A technique suitable for the production of monoclonal antibodies is that of Köhler and Milstein (Nature 1975, 256:495-497) .

Said antibodies do not recognize the cells of the human or animal to which it is intended.

The antibodies or fragments thereof are advantageously humanized when intended for a human administration.

Alternatively, humanized Mab could be derived from murine or rat Mab specific of the antigen. These fully humanized Mab are constructed using conventional molecular techniques to graft complementarity-determining regions from the parent murine or rat antibacterial antibody into human IgG1 kappa heavy and light-chain frameworks.

The present invention is also aimed towards the use, of said combinations of at least one polypeptide of the first group, particularly having SEQ ID N°14, 15, 17, 21, 22, 23, 28, 29, 30, 32, 36, 38, 39, 41-44, 46, 49, 50, 52 to 55, 58, 60, 63, 133-138, and one polypeptide of the second group, particularly having SEQ ID N° 159, said antibodies raised against said polypeptides or polynucleotides coding for said polypeptides for the diagnosis of the presence or absence of undesirable extra-intestinal *E. coli*, and/or for the diagnosis of an extra-intestinal *E. coli* infection.

The detection of the presence or absence of such compounds can in particular be carried out by nucleotide hybridization, by PCR amplification or by detection of their polypeptide products. Detection of the presence of such compounds makes it possible to conclude that a B2/D *E. coli* strain is present.

The invention also relates to pharmaceutical compositions for alleviating and/or preventing and/or treating an undesirable growth of E. *coli* comprising an effective amount of at least one polypeptide of said each group particularly having SEQ ID N°1-66 to 133-145, for the first group, and SEQ ID N° 159 for the second group, in combination with a pharmaceutically acceptable carrier.

Preferred pharmaceutical compositions comprise at least one polypeptide having SEQ ID N°14, 15, 17, 21, 22, 23, 28, 29, 30, 32, 36, 38, 39, 41-44, 46, 49, 50, 52 to 55, 58, 60, 63, 133-138, and at least one polypeptide having SEQ ID N° 159.

The present application is also aimed towards any use of said combination of polypeptides such as above defined for the manufacture of a composition, in particular of a pharmaceutical composition, intended to alleviate and/or to prevent and/or to treat an undesirable growth of *E. coli,* such as an *E. coli* infection, (for example systemic and non-diarrhoeal infections), the presence of extra-intestinal E. *coli* or a sanitary contamination.

The present invention is illustrated by the examples, which follow and which are given in a non limiting capacity.

Another example of vaccination to demonstrate immunogenicity of polypeptides:

### Preparation of antigenic peptidic combinations

A polypeptide having SEQ ID N° 28 is purified from an E.coli strain or from an host cell containing a recombine.

Polypeptides having SEQ ID N° 28 and SEQ ID N° 159, respectively, are purified and conjugated with a toxin.

A physiologically inert carrier is added to the preparation, which is sterilized and can be injected parenterally, subcutaneously or intramuscularly.

Said composition can also be sprayed *onto mucosa* with the aid of a spray.

Said combination of polypeptides may be added to a child hood vaccine.

### Protecting effect of said combination in mice infected by E. coli

A total of 100 µg of said purified combination of polypeptides was administered to Balb C mice according to usual procedure of immunization.

A decrease of mortality in immunized animals was observed compared to non-immunized animals.

## Claims

1. Compositions of polypeptides specific to pathogenic strains comprising at least one polypeptide of a first group, having a sequence selected in the group comprising the sequences of SEQ ID N°1 to N°66 or 133-145 and at least one peptide of a second group, having SEQ ID N° 159, or homologous sequences of polypeptides of the first group and/or the second group with a minimum of 25% of identity with the whole sequences of said polypeptides.

2. The compositions according to claim 1, wherein the polypeptides of the second group have SEQ ID N°159.

3. The compositions of claim 1 or 2, wherein the polypeptides of the first group have SEQ ID N° 14, 15, 17, 21, 22, 23, 28, 29, 30, 32, 36, 38, 39, 41-44, 46, 49, 50, 52 to 55, 58, 60, 63 or 133-138

4. The compositions according to anyone of claims 1 to 3, wherein said homologues isolated antigenic polypeptides of the first group have at least 25% identity to a polypeptide having a sequence such as above defined in claim 1, more particularly having SEQ ID N°14, 15, 17, 21, 22, 23, 28, 29, 30, 32, 36, 38, 39, 41-44, 46, 49, 50, 52 to 55, 58, 60, 63, 133-138, or at least 25% identity to a fragment comprising at least 5, at least 10, at least 20, at least 30, at least 40, at least 50, at least 60 or more than 60 consecutive amino acids of a polypeptide having a sequence corresponding to said SEQ ID N°s, as determined using BLASTP or BLASTX with the default parameters.

5. The compositions according to anyone of claims 1 to 4, wherein said homologous isolated antigenic polypeptides of the second group have at least 25% identity to a polypeptide having SEQ ID N° 159.

6. An expression vector comprising at least one isolated polynucleotides coding for a polypeptide of said first group and at least one a polypeptide of said second group according to the universal genetic code and taking into account the degeneracy of this code.

7. An expression vector according to claim 6, wherein the polynucleotides coding for the polypeptides of the first group have sequences corresponding to SEQ ID N° 67 to SEQ ID N° 132 or 146 to 158.

8. An expression vector according to claim 7, wherein said polynucleotides have sequences corresponding to SEQ ID N° 80, 81, 83, 87, 88, 89, 94, 95, 96, 98, 102, 104, 105, 107-110, 112, 115, 116, 118, 119, 126, 127, 130, 132, 135, 146-151.

9. An expression vector according to anyone of claims 6 to 8, wherein the polynucleotide coding for the polypeptide of the second group has SEQ ID N° 160.

10. An expression vector according to anyone of claims 6 to 9, comprising an homolog to said polynucleotides, said homologs having at least 25% identity to a fragment comprising at least 15, at least 30, at least 60, at least 90, at least 120, at least 150, at least 180 or more than 180 consecutive nucleotide of a polynucleotide having one of said SEQ ID N°s, as determined using BLASTN with the default parameters, and are encompassed by the invention inasmuch as they are capable of coding for a polypeptide having the antigenic properties of those according to the invention.

11. A host cell comprising an expression vector according to anyone of claims 6 to 10.

12. Vaccine compositions specific to E. *coli* extra-intestinal infections, comprising an effective amount of at least one antigenic polypeptide or fragment thereof of said first group and at least one antigenic polypeptide or fragment thereof of the second group, with a carrier, particularly at least one polypeptide of SEQ ID N°1 to SEQ ID. N°66 and 133-145 and homologous polypeptides, and at least one polypeptide of SEQ ID N° 159 and homologous peptides.

13. The vaccine compositions of claim 12 for preventing urinary system infections, pyelonephritis, sepsis, bacteremia, neonatal meningitidis.

14. The vaccine composition of claim 12 or 13, adapted to specific indication in combination with components directed against other bacteria, such as *S. Aureus* or group *B Streptococcus,* or other bacteria implicated in systemic infections.

15. Compositions of antibodies specific to polypeptidesic antigens of pathogenic strains particularly to extra-intestinal E. Coli strains, comprising combinations of antibodies directed against at least one polypeptide of said first group and antibodies directed against at least one polypeptide of the second group such as defined in anyone of claims 1 to 5.

16. Compositions according to claim 15, wherein said antibodies are monoclonal antibodies.

17. Pharmaceutical compositions comprising a combination of antibodies according to claim 15 or 16.

18. Pharmaceutical compositions according to claim 17 comprising an effective amount of a combination of antibodies according to claim 15 or 16, for treating neonatal infections, in association with antibodies against *Staphylococcus aureus* and/or antibodies against group B *Streptococcus.*

19. The use of a pharmaceutical composition according to claim 17 or 18 for treatment or prevention of severe infection due to Expec in neonates and patients at risk for such infections.

20. Pharmaceutical compositions for alleviating and/or preventing and/or treating an undesirable growth of E. Coli comprising an effective amount of at least a composition according to anyone of claims 1 to 5, in combination with a pharmaceutically acceptable carrier.


SEQUENCE LISTING

<110>  MUTABILIS

<120>  Comprising of polypeptides specific to pathogenic strains and their use
as vaccines and in immunotherapy

<130>  2013

<160>  160

<170>  PatentIn version 3.1

<210>  1

<211>  163

<212>  PRT

<213>  Escherichia coli

<400>  1

Met Lys Leu Lys Ala Ile Ile Leu Ala Thr Gly Leu Ile Asn Cys Ile
1               5                   10                  15

Val Phe Ser Ala Gln Ala Val Asp Thr Thr Ile Thr Val Thr Gly Asn
            20                  25                  30

Val Leu Gln Arg Thr Cys Asn Val Pro Gly Asn Val Asp Val Ser Leu
        35                  40                  45

Gly Asn Leu Tyr Val Ser Asp Phe Pro Asn Ala Gly Ser Gly Ser Pro
    50                  55                  60

Trp Val Asn Phe Asp Leu Ser Leu Thr Gly Cys Gln Asn Met Asn Thr
65                  70                  75                  80

Val Arg Ala Thr Phe Ser Gly Thr Ala Asp Gly Gln Thr Tyr Tyr Ala
                85                  90                  95

Asn Thr Gly Asn Ala Gly Gly Ile Lys Ile Glu Ile Gln Asp Arg Asp
            100                 105                 110

Gly Ser Asn Ala Ser Tyr His Asn Gly Met Phe Lys Thr Leu Asn Val
        115                 120                 125

Gln Asn Asn Asn Ala Thr Phe Asn Leu Lys Ala Arg Ala Val Ser Lys
    130                 135                 140

Gly Gln Val Thr Pro Gly Asn Ile Ser Ser Val Ile Thr Val Thr Tyr
145                 150                 155                 160

Thr Tyr Ala

<210>  2

<211>  673

<212>  PRT

<213>  Escherichia coli

<400>  2

Met Lys Met Thr Arg Leu Tyr Pro Leu Ala Leu Gly Gly Leu Leu Leu
1               5                   10                  15

Pro Ala Ile Ala Asn Ala Gln Thr Ser Gln Gln Asp Glu Ser Thr Leu
            20                  25                  30

Val Val Thr Ala Ser Lys Gln Ser Ser Arg Ser Ala Ser Ala Asn Asn
        35                  40                  45

Val Ser Ser Thr Val Val Ser Ala Pro Glu Leu Ser Asp Ala Gly Val
    50                  55                  60

Thr Ala Ser Asp Lys Leu Pro Arg Val Leu Pro Gly Leu Asn Ile Glu
65                  70                  75                  80

Asn Ser Gly Asn Met Leu Phe Ser Thr Ile Ser Leu Arg Gly Val Ser
                85                  90                  95

Ser Ala Gln Asp Phe Tyr Asn Pro Ala Val Thr Leu Tyr Val Asp Gly
            100                 105                 110

Val Pro Gln Leu Ser Thr Asn Thr Ile Gln Ala Leu Thr Asp Val Gln
        115                 120                 125

Ser Val Glu Leu Leu Arg Gly Pro Gln Gly Thr Leu Tyr Gly Lys Ser
    130                 135                 140

Ala Gln Gly Gly Ile Ile Asn Ile Val Thr Gln Gln Pro Asp Ser Thr
145                 150                 155                 160

Pro Arg Gly Tyr Ile Glu Gly Gly Val Ser Ser Arg Asp Ser Tyr Arg
                165                 170                 175

Ser Lys Phe Asn Leu Ser Gly Pro Ile Gln Asp Gly Leu Leu Tyr Gly
            180                 185                 190

Ser Val Thr Leu Leu Arg Gln Val Asp Asp Gly Asp Met Ile Asn Pro
        195                 200                 205

Ala Thr Gly Ser Asp Asp Leu Gly Gly Thr Arg Ala Ser Ile Gly Asn
    210                 215                 220

Val Lys Leu Arg Leu Ala Pro Asp Asp Gln Pro Trp Glu Met Gly Phe
225                 230                 235                 240

Ala Ala Ser Arg Glu Cys Thr Arg Ala Thr Gln Asp Ala Tyr Val Gly
                245                 250                 255

Trp Asn Asp Ile Lys Gly Arg Lys Leu Ser Ile Ser Asp Gly Ser Pro
            260                 265                 270

Asp Pro Tyr Met Arg Arg Cys Thr Asp Ser Gln Thr Leu Ser Gly Lys
        275                 280                 285

Tyr Thr Thr Asp Asp Trp Val Phe Asn Leu Ile Ser Ala Trp Gln Gln
    290                 295                 300

Gln His Tyr Ser Arg Thr Phe Pro Ser Gly Ser Leu Ile Val Asn Met
305                 310                 315                 320

Ser Gln Arg Trp Asn Gln Asp Val Gln Glu Leu Arg Ala Ala Thr Leu
                325                 330                 335

Gly Asp Ala Arg Thr Val Asp Met Val Phe Gly Leu Tyr Arg Gln Asn
            340                 345                 350

Thr Arg Glu Lys Leu Asn Ser Ala Tyr Asp Met Pro Thr Met Pro Tyr
        355                 360                 365

Leu Ser Ser Thr Gly Tyr Thr Thr Ala Glu Thr Leu Ala Ala Tyr Ser
    370                 375                 380

Asp Leu Thr Trp His Leu Thr Asp Arg Phe Asp Ile Gly Gly Gly Val
385                 390                 395                 400

Arg Phe Ser His Asp Lys Ser Ser Thr Gln Tyr His Gly Ser Met Leu
                405                 410                 415

Gly Asn Pro Phe Gly Asp Gln Gly Lys Ser Asn Asp Asp Gln Val Leu
            420                 425                 430

Gly Gln Leu Ser Ala Gly Tyr Met Leu Thr Asp Asp Trp Arg Val Tyr
        435                 440                 445

Thr Arg Val Ala Gln Gly Tyr Lys Pro Ser Gly Tyr Asn Ile Val Pro
    450                 455                 460

Thr Ala Gly Leu Asp Ala Lys Pro Phe Val Ala Glu Lys Ser Ile Asn
465                 470                 475                 480

Tyr Glu Leu Gly Thr Arg Tyr Glu Thr Ala Asp Val Thr Leu Gln Ala
                485                 490                 495

Ala Thr Phe Tyr Thr His Thr Lys Asp Met Gln Leu Tyr Ser Gly Pro
            500                 505                 510

Val Gly Met Gln Thr Leu Ser Asn Ala Gly Lys Ala Asp Ala Thr Gly
        515                 520                 525

Val Glu Leu Glu Ala Lys Trp Arg Phe Ala Pro Gly Trp Ser Trp Asp
    530                 535                 540

Ile Asn Gly Asn Val Ile Arg Ser Glu Phe Thr Asn Asp Ser Glu Leu
545                 550                 555                 560

Tyr His Gly Asn Arg Val Pro Phe Val Pro Arg Tyr Gly Ala Gly Ser
                565                 570                 575

Ser Val Asn Gly Val Ile Asp Thr Arg Tyr Gly Ala Leu Met Pro Arg
            580                 585                 590

Leu Ala Val Asn Leu Val Gly Pro His Tyr Phe Asp Gly Asp Asn Gln
        595                 600                 605

Leu Arg Gln Gly Thr Tyr Ala Thr Leu Asp Ser Ser Leu Gly Trp Gln
    610                 615                 620

Ala Thr Glu Arg Met Asn Ile Ser Val Tyr Val Asp Asn Leu Phe Asp
625                 630                 635                 640

Arg Arg Tyr Arg Thr Tyr Gly Tyr Met Asn Gly Ser Ser Ala Val Ala
                645                 650                 655

Gln Val Asn Met Gly Arg Thr Val Gly Ile Asn Thr Arg Ile Asp Phe
            660                 665                 670

Phe

<210>  3

<211>  246

<212>  PRT

<213>  Escherichia coli

<400>  3

Met Asn Lys Val Phe Val Val Ser Val Val Ala Ala Ala Cys Val Phe
1               5                   10                  15

Ala Val Asn Ala Gly Ala Lys Glu Gly Lys Ser Gly Phe Tyr Leu Thr
            20                  25                  30

Gly Lys Ala Gly Ala Ser Val Met Ser Leu Ser Asp Gln Arg Phe Leu
        35                  40                  45

Ser Gly Asp Glu Glu Glu Thr Ser Lys Tyr Lys Gly Gly Asp Asp His
    50                  55                  60

Asp Thr Val Phe Ser Gly Gly Ile Ala Val Gly Tyr Asp Phe Tyr Pro
65                  70                  75                  80

Gln Phe Ser Ile Pro Val Arg Thr Glu Leu Glu Phe Tyr Ala Arg Gly
                85                  90                  95

Lys Ala Asp Ser Lys Tyr Asn Val Asp Lys Asp Ser Trp Ser Gly Gly
            100                 105                 110

Tyr Trp Arg Asp Asp Leu Lys Asn Glu Val Ser Val Asn Thr Leu Met
        115                 120                 125

Leu Asn Ala Tyr Tyr Asp Phe Arg Asn Asp Ser Ala Phe Thr Pro Trp
    130                 135                 140

Val Ser Ala Gly Ile Gly Tyr Ala Arg Ile His Gln Lys Thr Thr Gly
145                 150                 155                 160

Ile Ser Thr Trp Asp Tyr Glu Tyr Gly Ser Ser Gly Arg Glu Ser Leu
                165                 170                 175

Ser Arg Ser Gly Ser Ala Asp Asn Phe Ala Trp Ser Leu Gly Ala Gly
            180                 185                 190

Val Arg Tyr Asp Val Thr Pro Asp Ile Ala Leu Asp Leu Ser Tyr Arg
        195                 200                 205

Tyr Leu Asp Ala Gly Asp Ser Ser Val Ser Tyr Lys Asp Glu Trp Gly
    210                 215                 220

Asp Lys Tyr Lys Ser Glu Val Asp Val Lys Ser His Asp Ile Met Leu
225                 230                 235                 240

Gly Met Thr Tyr Asn Phe
                245

<210>  4

<211>  166

<212>  PRT

<213>  Escherichia coli

<400>  4

Met Lys Leu Lys Ala Ile Ile Leu Ala Thr Gly Leu Ile Asn Cys Ile
1               5                   10                  15

Ala Phe Ser Ala Gln Ala Val Asp Thr Thr Ile Thr Val Thr Gly Arg
            20                  25                  30

Val Leu Pro Arg Thr Cys Thr Ile Gly Asn Gly Gly Asn Pro Asn Ala
        35                  40                  45

Thr Val Val Leu Asp Asn Ala Tyr Thr Ser Asp Leu Ile Ala Ala Asn
    50                  55                  60

Ser Thr Ser Gln Trp Lys Asn Phe Ser Leu Thr Leu Thr Asn Cys Gln
65                  70                  75                  80

Asn Val Asn Asn Val Thr Ser Phe Gly Gly Thr Ala Glu Asn Thr Asn
                85                  90                  95

Tyr Tyr Arg Asn Thr Gly Asp Ala Thr Asn Ile Met Val Glu Leu Gln
            100                 105                 110

Glu Gln Gly Asn Gly Asn Thr Pro Leu Lys Val Gly Ser Thr Lys Val
        115                 120                 125

Val Thr Val Ser Asn Gly Gln Ala Thr Phe Asn Leu Lys Val Arg Ala
    130                 135                 140

Val Ser Lys Gly Asn Ala Gly Ala Gly Ser Ile Asn Ser Gln Ile Thr
145                 150                 155                 160

Val Thr Tyr Thr Tyr Ala
                165

<210>  5

<211>  1295

<212>  PRT

<213>  Escherichia coli

<400>  5

Met Asn Lys Ile Tyr Ser Leu Lys Tyr Ser Ala Ala Thr Gly Gly Leu
1               5                   10                  15

Ile Ala Val Ser Glu Leu Ala Lys Arg Val Ser Gly Lys Thr Asn Arg
            20                  25                  30

Lys Leu Val Ala Thr Met Leu Ser Leu Ala Val Ala Gly Thr Val Asn
        35                  40                  45

Ala Ala Asn Ile Asp Ile Ser Asn Val Trp Ala Arg Asp Tyr Leu Asp
    50                  55                  60

Leu Ala Gln Asn Lys Gly Ile Phe Gln Pro Gly Ala Thr Asp Val Thr
65                  70                  75                  80

Ile Thr Leu Lys Asn Gly Asp Lys Phe Ser Phe His Asn Leu Ser Ile
                85                  90                  95

Pro Asp Phe Ser Gly Ala Ala Ala Ser Gly Ala Ala Thr Ala Ile Gly
            100                 105                 110

Gly Ser Tyr Ser Val Thr Val Ala His Asn Lys Lys Asn Pro Gln Ala
        115                 120                 125

Ala Glu Thr Gln Val Tyr Ala Gln Ser Ser Tyr Arg Val Val Asp Arg
    130                 135                 140

Arg Asn Ser Asn Asp Phe Glu Ile Gln Arg Leu Asn Lys Phe Val Val
145                 150                 155                 160

Glu Thr Val Gly Ala Thr Pro Ala Glu Thr Asn Pro Thr Thr Tyr Ser
                165                 170                 175

Asp Ala Leu Glu Arg Tyr Gly Ile Val Thr Ser Asp Gly Ser Lys Lys
            180                 185                 190

Ile Ile Gly Phe Arg Ala Gly Ser Gly Gly Thr Ser Phe Ile Asn Gly
        195                 200                 205

Glu Ser Lys Ile Ser Thr Asn Ser Ala Tyr Ser His Asp Leu Leu Ser
    210                 215                 220

Ala Ser Leu Phe Glu Val Thr Gln Trp Asp Ser Tyr Gly Met Met Ile
225                 230                 235                 240

Tyr Lys Asn Asp Lys Thr Phe Arg Asn Leu Glu Ile Phe Gly Asp Ser
                245                 250                 255

Gly Ser Gly Ala Tyr Leu Tyr Asp Asn Lys Leu Glu Lys Trp Val Leu
            260                 265                 270

Val Gly Thr Thr His Gly Ile Ala Ser Val Asn Gly Asp Gln Leu Thr
        275                 280                 285

Trp Ile Thr Lys Tyr Asn Asp Lys Leu Val Ser Glu Leu Lys Asp Thr
    290                 295                 300

Tyr Ser His Lys Ile Asn Leu Asn Gly Asn Asn Val Thr Ile Lys Asn
305                 310                 315                 320

Thr Asp Ile Thr Leu His Gln Asn Asn Ala Asp Thr Thr Gly Thr Gln
                325                 330                 335

Glu Lys Ile Thr Lys Asp Lys Asp Ile Val Phe Thr Asn Gly Gly Asp
            340                 345                 350

Val Leu Phe Lys Asp Asn Leu Asp Phe Gly Ser Gly Gly Ile Ile Phe
        355                 360                 365

Asp Glu Gly His Glu Tyr Asn Ile Asn Gly Gln Gly Phe Thr Phe Lys
    370                 375                 380

Gly Ala Gly Ile Asp Ile Gly Lys Glu Ser Ile Val Asn Trp Asn Ala
385                 390                 395                 400

Leu Tyr Ser Ser Asp Asp Val Leu His Lys Ile Gly Pro Gly Thr Leu
                405                 410                 415

Asn Val Gln Lys Lys Gln Gly Ala Asn Ile Lys Ile Gly Glu Gly Asn
            420                 425                 430

Val Ile Leu Asn Glu Glu Gly Thr Phe Asn Asn Ile Tyr Leu Ala Ser
        435                 440                 445

Gly Asn Gly Lys Val Ile Leu Asn Lys Asp Asn Ser Leu Gly Asn Asp
    450                 455                 460

Gln Tyr Ala Gly Ile Phe Phe Thr Lys Arg Gly Gly Thr Leu Asp Leu
465                 470                 475                 480

Asn Gly His Asn Gln Thr Phe Thr Arg Ile Ala Ala Thr Asp Asp Gly
                485                 490                 495

Thr Thr Ile Thr Asn Ser Asp Thr Thr Lys Glu Ala Val Leu Ala Ile
            500                 505                 510

Asn Asn Glu Asp Ser Tyr Ile Tyr His Gly Asn Ile Asn Gly Asn Ile
        515                 520                 525

Lys Leu Thr His Asn Ile Asn Ser Gln Asp Lys Lys Thr Asn Ala Lys
    530                 535                 540

Leu Ile Leu Asp Gly Ser Val Asn Thr Lys Asn Asp Val Glu Val Ser
545                 550                 555                 560

Asn Ala Ser Leu Thr Met Gln Gly His Ala Thr Glu His Ala Ile Phe
                565                 570                 575

Arg Ser Ser Ala Asn His Cys Ser Leu Val Phe Leu Cys Gly Thr Asp
            580                 585                 590

Trp Val Thr Val Leu Lys Glu Thr Glu Ser Ser Tyr Asn Lys Lys Phe
        595                 600                 605

Asn Ser Asp Tyr Lys Ser Asn Asn Gln Gln Thr Ser Phe Asp Gln Pro
    610                 615                 620

Asp Trp Lys Thr Gly Val Phe Lys Phe Asp Thr Leu His Leu Asn Asn
625                 630                 635                 640

Ala Asp Phe Ser Ile Ser Arg Asn Ala Asn Val Glu Gly Asn Ile Ser
                645                 650                 655

Ala Asn Lys Ser Ala Ile Thr Ile Gly Asp Lys Asn Val Tyr Ile Asp
            660                 665                 670

Asn Leu Ala Gly Lys Asn Ile Thr Asn Asn Gly Phe Asp Phe Lys Gln
        675                 680                 685

Thr Ile Ser Thr Asn Leu Ser Ile Gly Glu Thr Lys Phe Thr Gly Gly
    690                 695                 700

Ile Thr Ala His Asn Ser Gln Ile Ala Ile Gly Asp Gln Ala Val Val
705                 710                 715                 720

Thr Leu Asn Gly Ala Thr Phe Leu Asp Asn Thr Pro Ile Ser Ile Asp
                725                 730                 735

Lys Gly Ala Lys Val Ile Ala Gln Asn Ser Met Phe Thr Thr Lys Gly
            740                 745                 750

Ile Asp Ile Ser Gly Glu Leu Thr Met Met Gly Ile Pro Glu Gln Asn
        755                 760                 765

Ser Lys Thr Val Thr Pro Gly Leu His Tyr Ala Ala Asp Gly Phe Arg
    770                 775                 780

Leu Ser Gly Gly Asn Ala Asn Phe Ile Ala Arg Asn Met Ala Ser Val
785                 790                 795                 800

Thr Gly Asn Ile Tyr Ala Asp Asp Ala Ala Thr Ile Thr Leu Gly Gln
                805                 810                 815

Pro Glu Thr Glu Thr Pro Thr Ile Ser Ser Ala Tyr Gln Ala Trp Ala
            820                 825                 830

Glu Thr Leu Leu Tyr Gly Phe Asp Thr Ala Tyr Arg Gly Ala Ile Thr
        835                 840                 845

Ala Pro Lys Ala Thr Val Ser Met Asn Asn Ala Ile Trp His Leu Asn
    850                 855                 860

Ser Gln Ser Ser Ile Asn Arg Leu Glu Thr Lys Asp Ser Met Val Arg
865                 870                 875                 880

Phe Thr Gly Asp Asn Gly Lys Phe Thr Thr Leu Thr Val Asn Asn Leu
                885                 890                 895

Thr Ile Asp Asp Ser Ala Phe Val Leu Arg Ala Asn Leu Ala Gln Ala
            900                 905                 910

Asp Gln Leu Val Val Asn Lys Ser Leu Ser Gly Lys Asn Asn Leu Leu
        915                 920                 925

Leu Val Asp Phe Ile Glu Lys Asn Gly Asn Ser Asn Gly Leu Asn Ile
    930                 935                 940

Asp Leu Val Ser Ala Pro Lys Gly Thr Ala Val Asp Val Phe Lys Ala
945                 950                 955                 960

Thr Thr Arg Ser Ile Gly Phe Ser Asp Val Thr Pro Val Ile Glu Gln
                965                 970                 975

Lys Asn Asp Thr Asp Lys Ala Thr Trp Thr Leu Ile Gly Tyr Lys Ser
            980                 985                 990

Val Ala Asn Ala Asp Ala Ala Lys Lys Ala Thr Leu Leu Met Ser Gly
        995                 1000                1005

Gly Tyr Lys Ala Phe Leu Ala Glu Val Asn Asn Leu Asn Lys Arg
    1010                1015                1020

Met Gly Asp Leu Arg Asp Ile Asn Gly Glu Ser Gly Ala Trp Ala
    1025                1030                1035

Arg Ile Ile Ser Gly Thr Gly Ser Ala Gly Gly Gly Phe Ser Asp
    1040                1045                1050

Asn Tyr Thr His Val Gln Val Gly Ala Asp Asn Lys His Glu Leu
    1055                1060                1065

Asp Gly Leu Asp Leu Phe Thr Gly Val Thr Met Thr Tyr Thr Asp
    1070                1075                1080

Ser His Ala Gly Ser Asp Ala Phe Ser Gly Glu Thr Lys Ser Val
    1085                1090                1095

Gly Ala Gly Leu Tyr Ala Ser Ala Met Phe Glu Ser Gly Ala Tyr
    1100                1105                1110

Ile Asp Leu Ile Gly Lys Tyr Val His His Asp Asn Glu Tyr Thr
    1115                1120                1125

Ala Thr Phe Ala Gly Leu Gly Thr Arg Asp Tyr Ser Ser His Ser
    1130                1135                1140

Trp Tyr Ala Gly Ala Glu Val Gly Tyr Arg Tyr His Val Thr Asp
    1145                1150                1155

Ser Ala Trp Ile Glu Pro Gln Ala Glu Leu Val Tyr Gly Ala Val
    1160                1165                1170

Ser Gly Lys Gln Phe Ser Trp Lys Asp Gln Gly Met Asn Leu Thr
    1175                1180                1185

Met Lys Asp Lys Asp Phe Asn Pro Leu Ile Gly Arg Thr Gly Val
    1190                1195                1200

Asp Val Gly Lys Ser Phe Ser Gly Lys Asp Trp Lys Val Thr Ala
    1205                1210                1215

Arg Ala Gly Leu Gly Tyr Gln Phe Asp Leu Phe Ala Asn Gly Glu
    1220                1225                1230

Thr Val Leu Arg Asp Ala Ser Gly Glu Lys Arg Ile Lys Gly Glu
    1235                1240                1245

Lys Asp Gly Arg Met Leu Met Asn Val Gly Leu Asn Ala Glu Ile
    1250                1255                1260

Arg Asp Asn Leu Arg Phe Gly Leu Glu Phe Glu Lys Ser Ala Phe
    1265                1270                1275

Gly Lys Tyr Asn Val Asp Asn Ala Ile Asn Ala Asn Phe Arg Tyr
    1280                1285                1290

Ser Phe
    1295

<210>  6

<211>  142

<212>  PRT

<213>  Escherichia coli

<400>  6

Met Ile Asn Ile Pro Ser Pro Thr Ala Val Val Met Ala Leu Val Ala
1               5                   10                  15

Ile Ser Thr Leu Pro Ser Pro Ser Arg Val Lys Leu Met Pro Tyr Pro
            20                  25                  30

Pro Arg Ala His Asn Thr Thr Gly Leu Leu Pro Val Arg Glu Ile Cys
        35                  40                  45

Phe Pro His His Gly Asp Asp Gly Arg Asn Ser Ile Glu Pro Ser Ile
    50                  55                  60

Ser Arg Ala Ala His Thr Asp Arg Leu Arg Phe Val Cys Met Thr Arg
65                  70                  75                  80

Thr Gly Ser Thr Thr Ser Arg Pro Phe Cys Pro Ile Pro Arg Ser Pro
                85                  90                  95

Ala Leu Asn Ala Ser Gly Gln Gln Asp Ser Gly Phe Trp Gly Val Ser
            100                 105                 110

Ser Ile Pro Gly Asp Ile Leu Met Phe Gln Leu His Val Leu Ile Val
        115                 120                 125

Phe Ile Cys Lys Ile Asn Leu Ser Asp Asn Asn Ile Ser Tyr
    130                 135                 140

<210>  7

<211>  318

<212>  PRT

<213>  Escherichia coli

<400>  7

Met Tyr Ala Arg Glu Tyr Arg Ser Thr Arg Pro His Lys Ala Ile Phe
1               5                   10                  15

Phe His Leu Ser Cys Leu Thr Leu Ile Cys Ser Ala Gln Val Tyr Ala
            20                  25                  30

Lys Pro Asp Met Arg Pro Leu Gly Pro Asn Ile Ala Asp Lys Gly Ser
        35                  40                  45

Val Phe Tyr His Phe Ser Ala Thr Ser Phe Asp Ser Val Asp Gly Thr
    50                  55                  60

Arg His Tyr Arg Val Trp Thr Ala Val Pro Asn Thr Thr Ala Pro Ala
65                  70                  75                  80

Ser Gly Tyr Pro Ile Leu Tyr Met Leu Asp Gly Asn Ala Val Met Asp
                85                  90                  95

Arg Leu Asp Asp Glu Leu Leu Lys Gln Leu Ser Glu Lys Thr Pro Pro
            100                 105                 110

Val Ile Val Ala Val Gly Tyr Gln Thr Asn Leu Pro Phe Asp Leu Asn
        115                 120                 125

Ser Arg Ala Tyr Asp Tyr Thr Pro Ala Ala Glu Ser Arg Lys Thr Asp
    130                 135                 140

Leu His Ser Gly Arg Phe Ser Arg Lys Ser Gly Gly Ser Asn Asn Phe
145                 150                 155                 160

Arg Gln Leu Leu Glu Thr Arg Ile Ala Pro Lys Val Glu Gln Gly Leu
                165                 170                 175

Asn Ile Asp Arg Gln Arg Arg Gly Leu Trp Gly His Ser Tyr Gly Gly
            180                 185                 190

Leu Phe Val Leu Asp Ser Trp Leu Ser Ser Ser Tyr Phe Arg Ser Tyr
        195                 200                 205

Tyr Ser Ala Ser Pro Ser Leu Gly Arg Gly Tyr Asp Ala Leu Leu Ser
    210                 215                 220

Arg Val Thr Ala Val Glu Pro Leu Gln Phe Cys Thr Lys His Leu Ala
225                 230                 235                 240

Ile Met Glu Gly Ser Ala Thr Gln Gly Asp Asn Arg Glu Thr His Ala
                245                 250                 255

Val Gly Val Leu Ser Lys Ile His Thr Thr Leu Thr Ile Leu Lys Asp
            260                 265                 270

Lys Gly Val Asn Ala Val Phe Trp Asp Phe Pro Asn Leu Gly His Gly
        275                 280                 285

Pro Met Phe Asn Ala Ser Phe Arg Gln Ala Leu Leu Asp Ile Ser Gly
    290                 295                 300

Glu Asn Ala Asn Tyr Thr Ala Gly Cys His Glu Leu Ser His
305                 310                 315

<210>  8

<211>  725

<212>  PRT

<213>  Escherichia coli


<400>  8

Met Arg Ile Asn Lys Ile Leu Trp Ser Leu Thr Val Leu Leu Val Gly
1               5                   10                  15

Leu Asn Ser Gln Val Ser Val Ala Lys Tyr Ser Asp Asp Asp Asn Asp
            20                  25                  30

Glu Thr Leu Val Val Glu Ala Thr Ala Glu Gln Val Leu Lys Gln Gln
        35                  40                  45

Pro Gly Val Ser Val Ile Thr Ser Glu Asp Ile Lys Lys Thr Pro Pro
    50                  55                  60

Val Asn Asp Leu Ser Asp Ile Ile Arg Lys Met Pro Gly Val Asn Leu
65                  70                  75                  80

Thr Gly Asn Ser Ala Ser Gly Thr Arg Gly Asn Asn Arg Gln Ile Asp
                85                  90                  95

Ile Arg Gly Met Gly Pro Glu Asn Thr Leu Ile Leu Ile Asp Gly Val
            100                 105                 110

Pro Val Thr Ser Arg Asn Ser Val Arg Tyr Ser Trp Arg Gly Glu Arg
        115                 120                 125

Asp Thr Arg Gly Asp Thr Asn Trp Val Pro Pro Glu Gln Val Glu Arg
    130                 135                 140

Ile Glu Val Ile Arg Gly Pro Ala Ala Ala Arg Tyr Gly Ser Gly Ala
145                 150                 155                 160

Ala Gly Gly Val Val Asn Ile Ile Thr Lys Arg Pro Thr Asn Asp Trp
                165                 170                 175

His Gly Ser Leu Ser Leu Tyr Thr Asn Gln Pro Glu Ser Ser Glu Glu
            180                 185                 190

Gly Ala Thr Arg Arg Ala Asn Phe Ser Leu Ser Gly Pro Leu Ala Gly
        195                 200                 205

Asp Ala Leu Thr Thr Arg Leu Tyr Gly Asn Leu Asn Lys Thr Asp Ala
    210                 215                 220

Asp Ser Trp Asp Ile Asn Ser Pro Val Gly Thr Lys Asn Ala Ala Gly
225                 230                 235                 240

His Glu Gly Val Arg Asn Lys Asp Ile Asn Gly Val Val Ser Trp Lys
                245                 250                 255

Leu Asn Pro Gln Gln Ile Leu Asp Phe Glu Val Gly Tyr Ser Arg Gln
            260                 265                 270

Gly Asn Ile Tyr Ala Gly Asp Thr Gln Asn Ser Ser Ser Ser Ala Val
        275                 280                 285

Thr Glu Ser Leu Ala Lys Ser Gly Lys Glu Thr Asn Arg Leu Tyr Arg
    290                 295                 300

Gln Asn Tyr Gly Ile Thr His Asn Gly Ile Trp Asp Trp Gly Gln Ser
305                 310                 315                 320

Arg Phe Gly Val Tyr Tyr Glu Lys Thr Asn Asn Thr Arg Met Asn Glu
                325                 330                 335

Gly Leu Ser Gly Gly Gly Glu Gly Arg Ile Leu Ala Gly Glu Lys Phe
            340                 345                 350

Thr Thr Asn Arg Leu Ser Ser Trp Arg Thr Ser Gly Glu Leu Asn Ile
        355                 360                 365

Pro Leu Asn Val Met Val Asp Gln Thr Leu Thr Val Gly Ala Glu Trp
    370                 375                 380

Asn Arg Asp Lys Leu Asp Asp Pro Ser Ser Thr Ser Leu Thr Val Asn
385                 390                 395                 400

Asp Arg Asp Ile Ser Gly Ile Ser Gly Ser Ala Ala Asp Arg Ser Ser
                405                 410                 415

Lys Asn His Ser Gln Ile Ser Ala Leu Tyr Ile Glu Asp Asn Ile Glu
            420                 425                 430

Pro Val Pro Gly Thr Asn Ile Ile Pro Gly Leu Arg Phe Asp Tyr Leu
        435                 440                 445

Ser Asp Ser Gly Gly Asn Phe Ser Pro Ser Leu Asn Leu Ser Gln Glu
    450                 455                 460

Leu Gly Asp Tyr Phe Lys Val Lys Ala Gly Val Ala Arg Thr Phe Lys
465                 470                 475                 480

Ala Pro Asn Leu Tyr Gln Ser Ser Glu Gly Tyr Leu Leu Tyr Ser Lys
                485                 490                 495

Gly Asn Gly Cys Pro Lys Asp Ile Thr Ser Gly Gly Cys Tyr Leu Ile
            500                 505                 510

Gly Asn Lys Asp Leu Asp Pro Glu Ile Ser Val Asn Lys Glu Ile Gly
        515                 520                 525

Leu Glu Phe Thr Trp Glu Asp Tyr His Ala Ser Val Thr Tyr Phe Arg
    530                 535                 540

Asn Asp Tyr Gln Asn Lys Ile Val Ala Gly Asp Asn Val Ile Gly Gln
545                 550                 555                 560

Thr Ala Ser Gly Ala Tyr Ile Leu Lys Trp Gln Asn Gly Gly Lys Ala
                565                 570                 575

Leu Val Asp Gly Ile Glu Ala Ser Met Ser Phe Pro Leu Val Lys Glu
            580                 585                 590

Arg Leu Asn Trp Asn Thr Asn Ala Thr Trp Met Ile Thr Ser Glu Gln
        595                 600                 605

Lys Asp Thr Gly Asn Pro Leu Ser Val Ile Pro Lys Tyr Thr Ile Asn
    610                 615                 620

Asn Ser Leu Asn Trp Thr Ile Thr Gln Ala Phe Ser Ala Ser Phe Asn
625                 630                 635                 640

Trp Thr Leu Tyr Gly Arg Gln Lys Pro Arg Thr His Ala Glu Thr Arg
                645                 650                 655

Ser Glu Asp Thr Gly Gly Leu Ser Gly Lys Glu Leu Gly Ala Tyr Ser
            660                 665                 670

Leu Val Gly Thr Asn Phe Asn Tyr Asp Ile Asn Lys Asn Leu Arg Leu
        675                 680                 685

Asn Val Gly Val Ser Asn Ile Leu Asn Lys Gln Ile Phe Arg Ser Ser
    690                 695                 700

Glu Gly Ala Asn Thr Tyr Asn Glu Pro Gly Arg Ala Tyr Tyr Ala Gly
705                 710                 715                 720

Val Thr Ala Ser Phe
                725

<210>  9

<211>  1014

<212>  PRT

<213>  Escherichia coli

<400>  9

Met Gly Asn Gln Trp Gln Gln Lys Tyr Leu Leu Glu Tyr Asn Glu Leu
1               5                   10                  15

Val Ser Asn Phe Pro Ser Pro Glu Arg Val Val Ser Asp Tyr Ile Lys
            20                  25                  30

Asn Cys Phe Lys Thr Asp Leu Pro Trp Phe Ser Arg Ile Asp Pro Asp
        35                  40                  45

Asn Ala Tyr Phe Ile Cys Phe Ser Gln Asn Arg Ser Asn Ser Arg Ser
    50                  55                  60

Tyr Thr Gly Trp Asp His Leu Gly Lys Tyr Lys Thr Glu Val Leu Thr
65                  70                  75                  80

Leu Thr Gln Ala Ala Leu Ile Asn Ile Gly Tyr Arg Phe Asp Val Phe
                85                  90                  95

Asp Asp Ala Asn Ser Ser Thr Gly Ile Tyr Lys Thr Lys Ser Ala Asp
            100                 105                 110

Val Phe Asn Glu Glu Asn Glu Glu Lys Met Leu Pro Ser Glu Tyr Leu
        115                 120                 125

His Phe Leu Gln Lys Cys Asp Phe Ala Gly Val Tyr Gly Lys Thr Leu
    130                 135                 140

Ser Asp Tyr Trp Ser Lys Tyr Tyr Asp Lys Phe Lys Leu Leu Leu Lys
145                 150                 155                 160

Asn Tyr Tyr Ile Ser Ser Ala Leu Tyr Leu Tyr Lys Asn Gly Glu Leu
                165                 170                 175

Asp Glu Arg Glu Tyr Asn Phe Ser Met Asn Ala Leu Asn Arg Ser Asp
            180                 185                 190

Asn Ile Ser Leu Leu Phe Phe Asp Ile Tyr Gly Tyr Tyr Ala Ser Asp
        195                 200                 205

Ile Phe Val Ala Lys Asn Asn Asp Lys Val Met Leu Phe Ile Pro Gly
    210                 215                 220

Ala Lys Lys Pro Phe Leu Phe Lys Lys Asn Ile Ala Asp Leu Arg Leu
225                 230                 235                 240

Thr Leu Lys Glu Leu Ile Lys Asp Ser Asp Asn Lys Gln Leu Leu Ser
                245                 250                 255

Gln His Phe Ser Leu Tyr Ser Arg Gln Asp Gly Val Ser Tyr Ala Gly
            260                 265                 270

Val Asn Ser Val Leu His Ala Ile Glu Asn Asp Gly Asn Phe Asn Glu
        275                 280                 285

Ser Tyr Phe Leu Tyr Ser Asn Lys Thr Leu Ser Asn Lys Asp Val Phe
    290                 295                 300

Asp Ala Ile Ala Ile Ser Val Lys Lys Arg Ser Phe Ser Asp Gly Asp
305                 310                 315                 320

Ile Val Ile Lys Ser Asn Ser Glu Ala Gln Arg Asp Tyr Ala Leu Thr
                325                 330                 335

Ile Leu Gln Thr Ile Leu Ser Met Thr Pro Ile Phe Asp Ile Val Val
            340                 345                 350

Pro Glu Val Ser Val Pro Leu Gly Leu Gly Ile Ile Thr Ser Ser Met
        355                 360                 365

Gly Ile Ser Phe Asp Gln Leu Ile Asn Gly Asp Thr Tyr Glu Glu Arg
    370                 375                 380

Arg Ser Ala Ile Pro Gly Leu Ala Thr Asn Ala Val Leu Leu Gly Leu
385                 390                 395                 400

Ser Phe Ala Ile Pro Leu Leu Ile Ser Lys Ala Gly Ile Asn Gln Glu
                405                 410                 415

Val Leu Ser Ser Val Ile Asn Asn Glu Gly Arg Thr Leu Asn Glu Thr
            420                 425                 430

Asn Ile Asp Ile Phe Leu Lys Glu Tyr Gly Ile Ala Glu Asp Ser Ile
        435                 440                 445

Ser Ser Thr Asn Leu Leu Asp Val Lys Leu Lys Ser Ser Gly Gln His
    450                 455                 460

Val Asn Ile Val Lys Leu Ser Asp Glu Asp Asn Gln Ile Val Ala Val
465                 470                 475                 480

Lys Gly Ser Ser Leu Ser Gly Ile Tyr Tyr Glu Val Asp Ile Glu Thr
                485                 490                 495

Gly Tyr Glu Ile Leu Ser Arg Arg Ile Tyr Arg Thr Glu Tyr Asn Asn
            500                 505                 510

Glu Ile Leu Trp Thr Arg Gly Gly Gly Leu Lys Gly Gly Gln Pro Phe
        515                 520                 525

Asp Phe Glu Ser Leu Asn Ile Pro Val Phe Phe Lys Asp Glu Pro Tyr
    530                 535                 540

Ser Ala Val Thr Gly Ser Pro Leu Ser Phe Ile Asn Asp Asp Ser Ser
545                 550                 555                 560

Leu Leu Tyr Pro Asp Thr Asn Pro Lys Leu Pro Gln Pro Thr Ser Glu
                565                 570                 575

Met Asp Ile Val Asn Tyr Val Lys Gly Ser Gly Ser Phe Gly Asp Arg
            580                 585                 590

Phe Val Thr Leu Met Arg Gly Ala Thr Glu Glu Glu Ala Trp Asn Ile
        595                 600                 605

Ala Ser Tyr His Thr Ala Gly Gly Ser Thr Glu Glu Leu His Glu Ile
    610                 615                 620

Leu Leu Gly Gln Gly Pro Gln Ser Ser Leu Gly Phe Thr Glu Tyr Thr
625                 630                 635                 640

Ser Asn Val Asn Ser Ala Asp Ala Ala Ser Arg Arg His Phe Leu Val
                645                 650                 655

Val Ile Lys Val His Val Lys Tyr Ile Thr Asn Asn Asn Val Ser Tyr
            660                 665                 670

Val Asn His Trp Ala Ile Pro Asp Glu Ala Pro Val Glu Val Leu Ala
        675                 680                 685

Val Val Asp Arg Arg Phe Asn Phe Pro Glu Pro Ser Thr Pro Pro Asp
    690                 695                 700

Ile Ser Thr Ile Arg Lys Leu Leu Ser Leu Arg Tyr Phe Lys Glu Ser
705                 710                 715                 720

Ile Glu Ser Thr Ser Lys Ser Asn Phe Gln Lys Leu Ser Arg Gly Asn
                725                 730                 735

Ile Asp Val Leu Lys Gly Arg Gly Ser Ile Ser Ser Thr Arg Gln Arg
            740                 745                 750

Ala Ile Tyr Pro Tyr Phe Glu Ala Ala Asn Ala Asp Glu Gln Gln Pro
        755                 760                 765

Leu Phe Phe Tyr Ile Lys Lys Asp Arg Phe Asp Asn His Gly Tyr Asp
    770                 775                 780

Gln Tyr Phe Tyr Asp Asn Thr Val Gly Leu Asn Gly Ile Pro Thr Leu
785                 790                 795                 800

Asn Thr Tyr Thr Gly Glu Ile Pro Ser Asp Ser Ser Ser Leu Gly Ser
                805                 810                 815

Thr Tyr Trp Lys Lys Tyr Asn Leu Thr Asn Glu Thr Ser Ile Ile Arg
            820                 825                 830

Val Ser Asn Ser Ala Arg Gly Ala Asn Gly Ile Lys Ile Ala Leu Glu
        835                 840                 845

Glu Val Gln Glu Gly Lys Pro Val Ile Ile Thr Ser Gly Asn Leu Ser
    850                 855                 860

Gly Cys Thr Thr Ile Val Ala Arg Lys Glu Gly Tyr Ile Tyr Lys Val
865                 870                 875                 880

His Thr Gly Thr Thr Lys Ser Leu Ala Gly Phe Thr Ser Thr Thr Gly
                885                 890                 895

Val Lys Lys Ala Val Glu Val Leu Glu Leu Leu Thr Lys Glu Pro Ile
            900                 905                 910

Pro Arg Val Glu Gly Ile Met Ser Asn Asp Phe Leu Val Asp Tyr Leu
        915                 920                 925

Ser Glu Asn Phe Glu Asp Ser Leu Ile Thr Tyr Ser Ser Ser Glu Lys
    930                 935                 940

Lys Pro Asp Ser Gln Ile Thr Ile Ile Arg Asp Asn Val Ser Val Phe
945                 950                 955                 960

Pro Tyr Phe Leu Asp Asn Ile Pro Glu His Gly Phe Gly Thr Ser Ala
                965                 970                 975

Thr Val Leu Val Arg Val Asp Gly Asn Val Val Val Arg Ser Leu Ser
            980                 985                 990

Glu Ser Tyr Ser Leu Asn Ala Asp  Ala Ser Glu Ile Ser  Val Leu Lys
        995                 1000                1005

Val Phe  Ser Lys Lys Phe
    1010

<210>  10

<211>  454

<212>  PRT

<213>  Escherichia coli

<400>  10

Met Val Asp Met Ile Asn Glu Ser Ala Arg Gln Thr Pro Val Ile Ala
1               5                   10                  15

Gln Thr Asp Val Leu Val Ile Gly Gly Gly Pro Ala Gly Leu Ser Ala
            20                  25                  30

Ala Ile Ala Ala Gly Arg Leu Gly Ala Arg Thr Met Ile Val Glu Arg
        35                  40                  45

Tyr Gly Ser Leu Gly Gly Val Leu Thr Gln Val Gly Val Glu Ser Phe
    50                  55                  60

Ala Trp Tyr Arg His Pro Gly Thr Glu Asp Cys Glu Gly Ile Cys Arg
65                  70                  75                  80

Glu Tyr Glu Gly Arg Ala Arg Ala Leu Gly Phe Thr Arg Pro Glu Pro
                85                  90                  95

Gln Ser Ile Ser Glu Val Ile Asp Thr Glu Gly Phe Lys Val Val Ala
            100                 105                 110

Asp Gln Met Ile Thr Glu Ser Gly Val Glu Pro Leu Tyr His Ser Trp
        115                 120                 125

Val Val Asp Val Ile Lys Asp Gly Asp Thr Leu Cys Gly Val Ile Val
    130                 135                 140

Glu Asn Lys Ser Gly Arg Gly Ala Ile Leu Ala Lys Arg Ile Val Asp
145                 150                 155                 160

Cys Thr Gly Asp Ala Asp Ile Ala Ala Arg Ala Gly Ala Pro Trp Thr
                165                 170                 175

Lys Arg Ser Lys Asp Gln Leu Met Gly Val Thr Val Met Phe Ser Cys
            180                 185                 190

Ala Gly Val Asp Val Ala Arg Phe Asn Arg Phe Val Ala Glu Glu Leu
        195                 200                 205

Lys Pro Thr Tyr Ala Asp Trp Gly Lys Asn Trp Thr Ile Gln Thr Thr
    210                 215                 220

Gly Lys Glu Asp Pro Met Phe Ser Pro Tyr Met Glu Asp Ile Phe Thr
225                 230                 235                 240

Arg Ala Gln Gln Asp Gly Val Ile Pro Gly Asp Ala Gln Ala Ile Ala
                245                 250                 255

Gly Thr Trp Ser Thr Phe Ser Glu Ser Gly Glu Ala Phe Gln Met Asn
            260                 265                 270

Met Val Tyr Ala Phe Gly Phe Asp Cys Thr Asp Val Phe Asp Leu Thr
        275                 280                 285

Lys Ala Glu Ile Ala Gly Arg Gln Gln Ala Leu Trp Ala Ile Asp Ala
    290                 295                 300

Leu Arg His Tyr Val Pro Gly Phe Glu Asn Val Arg Leu Arg Asn Phe
305                 310                 315                 320

Gly Ala Thr Leu Gly Thr Arg Glu Ser Arg Leu Ile Glu Gly Glu Ile
                325                 330                 335

Arg Ile Ala Asp Asp Tyr Val Leu Asn Gln Gly Arg Cys Ser Asp Ser
            340                 345                 350

Val Gly Ile Phe Pro Glu Phe Ile Asp Gly Ser Gly Tyr Leu Ile Leu
        355                 360                 365

Pro Thr Thr Gly Arg Phe Phe Gln Ile Pro Tyr Gly Cys Leu Val Pro
    370                 375                 380

Gln Lys Val Glu Asn Leu Leu Val Ala Gly Arg Cys Ile Ser Ala Gly
385                 390                 395                 400

Val Val Ala His Thr Ser Met Arg Asn Met Met Cys Cys Ala Val Thr
                405                 410                 415

Gly Glu Ala Ala Gly Thr Ala Ala Val Val Ser Leu Gln Gln Asn Cys
            420                 425                 430

Thr Val Arg Gln Val Ala Ile Pro Asp Leu Gln Asn Thr Leu Gln Gln
        435                 440                 445

Gln Gly Val Arg Leu Ala
    450

<210>  11

<211>  253

<212>  PRT

<213>  Escherichia coli

<400>  11

Met Ser Ala Lys Arg Arg Leu Leu Ile Ala Cys Thr Leu Ile Thr Ala
1               5                   10                  15

Ile Tyr His Phe Pro Ala Tyr Ser Ser Leu Glu Tyr Lys Gly Thr Phe
            20                  25                  30

Gly Ser Ile Asn Ala Gly Tyr Ala Asp Trp Asn Ser Gly Phe Val Asn
        35                  40                  45

Thr His Arg Gly Glu Val Trp Lys Val Thr Ala Asp Phe Gly Val Asn
    50                  55                  60

Phe Lys Glu Ala Glu Phe Tyr Ser Phe Tyr Glu Ser Asn Val Leu Asn
65                  70                  75                  80

His Ala Val Ala Gly Arg Asn His Thr Val Ser Ala Met Thr His Val
                85                  90                  95

Arg Leu Phe Asp Ser Asp Met Thr Phe Phe Gly Lys Ile Tyr Gly Gln
            100                 105                 110

Trp Asp Asn Ser Trp Gly Asp Asp Leu Asp Met Phe Tyr Gly Phe Gly
        115                 120                 125

Tyr Leu Gly Trp Asn Gly Glu Trp Gly Phe Phe Lys Pro Tyr Ile Gly
    130                 135                 140

Leu His Asn Gln Ser Gly Asp Tyr Val Ser Ala Lys Tyr Gly Gln Thr
145                 150                 155                 160

Asn Gly Trp Asn Gly Tyr Val Val Gly Trp Thr Ala Val Leu Pro Phe
                165                 170                 175

Thr Leu Phe Asp Glu Lys Phe Val Leu Ser Asn Trp Asn Glu Ile Glu
            180                 185                 190

Leu Asp Arg Asn Asp Ala Tyr Thr Glu Gln Gln Phe Gly Arg Asn Gly
        195                 200                 205

Leu Asn Gly Gly Leu Thr Ile Ala Trp Lys Phe Tyr Pro Arg Trp Lys
    210                 215                 220

Ala Ser Val Thr Trp Arg Tyr Phe Asp Asn Lys Leu Gly Tyr Asp Gly
225                 230                 235                 240

Phe Gly Asp Gln Met Ile Tyr Met Leu Gly Tyr Asp Phe
                245                 250

<210>  12

<211>  492

<212>  PRT

<213>  Escherichia coli

<400>  12

Met Ala Ser Leu Ile Gly Leu Ala Val Cys Thr Gly Asn Ala Phe Ser
1               5                   10                  15

Pro Ala Leu Ala Ala Glu Ala Lys Gln Pro Asn Leu Val Ile Ile Met
            20                  25                  30

Ala Asp Asp Leu Gly Tyr Gly Asp Leu Ala Thr Tyr Gly His Gln Ile
        35                  40                  45

Val Lys Thr Pro Asn Ile Asp Arg Leu Ala Gln Glu Gly Val Lys Phe
    50                  55                  60

Thr Asp Tyr Tyr Ala Pro Ala Pro Leu Ser Ser Pro Ser Arg Ala Gly
65                  70                  75                  80

Leu Leu Thr Gly Arg Met Pro Phe Arg Thr Gly Ile Arg Ser Trp Ile
                85                  90                  95

Pro Ser Gly Lys Asp Val Ala Leu Gly Arg Asn Glu Leu Thr Ile Ala
            100                 105                 110

Asn Leu Leu Lys Ala Gln Gly Tyr Asp Thr Ala Met Met Gly Lys Leu
        115                 120                 125

His Leu Asn Ala Gly Gly Asp Arg Thr Asp Gln Pro Gln Ala Gln Asp
    130                 135                 140

Met Gly Phe Asp Tyr Ser Leu Ala Asn Thr Ala Gly Phe Val Thr Asp
145                 150                 155                 160

Ala Thr Leu Asp Asn Ala Lys Glu Arg Pro Arg Tyr Gly Met Val Tyr
                165                 170                 175

Pro Thr Gly Trp Leu Arg Asn Gly Gln Pro Thr Pro Arg Ala Asp Lys
            180                 185                 190

Met Ser Gly Glu Tyr Val Ser Ser Glu Val Val Asn Trp Leu Asp Asn
        195                 200                 205

Lys Lys Asp Ser Lys Pro Phe Phe Leu Tyr Val Ala Phe Thr Glu Val
    210                 215                 220

His Ser Pro Leu Ala Ser Pro Lys Lys Tyr Leu Asp Met Tyr Ser Gln
225                 230                 235                 240

Tyr Met Ser Ala Tyr Gln Lys Gln His Pro Asp Leu Phe Tyr Gly Asp
                245                 250                 255

Trp Ala Asp Lys Pro Trp Arg Gly Val Gly Glu Tyr Tyr Ala Asn Ile
            260                 265                 270

Ser Tyr Leu Asp Ala Gln Val Gly Lys Val Leu Asp Lys Ile Lys Ala
        275                 280                 285

Met Gly Glu Glu Asp Asn Thr Ile Val Ile Phe Thr Ser Asp Asn Gly
    290                 295                 300

Pro Val Thr Arg Glu Ala Arg Lys Val Tyr Glu Leu Asn Leu Ala Gly
305                 310                 315                 320

Glu Thr Asp Gly Leu Arg Gly Arg Lys Asp Asn Leu Trp Glu Gly Gly
                325                 330                 335

Ile Arg Val Pro Ala Ile Ile Lys Tyr Gly Lys His Leu Pro Gln Gly
            340                 345                 350

Met Val Ser Asp Thr Pro Val Tyr Gly Leu Asp Trp Met Pro Thr Leu
        355                 360                 365

Ala Lys Met Met Asn Phe Lys Leu Pro Thr Asp Arg Thr Phe Asp Gly
    370                 375                 380

Glu Ser Leu Val Pro Val Leu Glu Gln Lys Ala Leu Lys Arg Glu Lys
385                 390                 395                 400

Pro Leu Ile Phe Gly Ile Asp Met Pro Phe Gln Asp Asp Pro Thr Asp
                405                 410                 415

Glu Trp Ala Ile Arg Asp Gly Asp Trp Lys Met Ile Ile Asp Arg Asn
            420                 425                 430

Asn Lys Pro Lys Tyr Leu Tyr Asn Leu Lys Ser Asp Arg Tyr Glu Thr
        435                 440                 445

Leu Asn Leu Ile Gly Lys Lys Pro Asp Ile Glu Lys Gln Met Tyr Gly
    450                 455                 460

Lys Phe Leu Lys Tyr Lys Thr Asp Ile Asp Asn Asp Ser Leu Met Lys
465                 470                 475                 480

Ala Arg Gly Asp Lys Pro Glu Ala Val Thr Trp Gly
                485                 490

<210> 13

<211> 345

<212> PRT

<213> Escherichia coli

<400> 13

Leu Ile Ser Leu Ser Phe Ile Pro Val Met Ser Ala Leu Pro Gly Pro
1               5                   10                  15

Ile Ala Lys Gly Phe Arg Asn Glu Arg Gly Phe Val Thr Thr Thr Ile
            20                  25                  30

Cys Ala Met Gly Glu Leu Leu Ala Glu Phe Leu Ser Arg Asn Pro His
        35                  40                  45

Gln Lys Phe Thr Gln Pro Gly Glu Phe Ile Gly Pro Phe Pro Ser Gly
    50                  55                  60

Ala Pro Ala Ile Phe Ala Ala Gln Val Ala Lys Leu Ser His Arg Ala
65                  70                  75                  80

Ile Phe Phe Gly Cys Val Gly Asn Asp Asp Phe Ala Arg Leu Ile Ile
                85                  90                  95

Glu Arg Leu Arg His Glu Gly Val Ile Thr Asp Gly Ile His Val Met
            100                 105                 110

Asn Asn Ala Val Thr Gly Thr Ala Phe Val Ser Tyr Gln Asn Pro Gln
        115                 120                 125

Gln Arg Asp Phe Val Phe Asn Ile Pro Asn Ser Ala Cys Gly Leu Phe
    130                 135                 140

Thr Ala Glu His Ile Asp Lys Asp Leu Leu Lys Gln Cys Asn His Leu
145                 150                 155                 160

His Ile Val Gly Ser Ser Leu Phe Ser Phe Arg Met Ile Asp Val Met
                165                 170                 175

Arg Lys Ala Ile Thr Thr Ile Lys Ser Ala Gly Gly Thr Val Ser Phe
            180                 185                 190

Asp Pro Asn Ile Arg Lys Glu Met Leu Ser Ile Pro Glu Met Ala Gln
        195                 200                 205

Ala Leu Asp Tyr Leu Ile Glu Tyr Thr Asp Ile Phe Ile Pro Ser Glu
    210                 215                 220

Ser Glu Leu Pro Phe Phe Ala Arg His Lys Asn Leu Ser Glu Glu Gln
225                 230                 235                 240

Ile Val Ser Asp Leu Leu His Gly Gly Val Lys His Val Ala Ile Lys
                245                 250                 255

Arg Ala Gln Arg Gly Ala Ser Tyr Tyr Lys Leu Lys Asn Gly Thr Leu
            260                 265                 270

His Ala Gln His Val Ala Gly His Asp Ile Glu Ile Ile Asp Pro Thr
        275                 280                 285

Gly Ala Gly Asp Cys Phe Gly Ala Thr Phe Ile Thr Leu Phe Leu Ser
    290                 295                 300

Gly Phe Pro Ala His Lys Ala Leu Gln Tyr Ala Asn Ala Ser Gly Ala
305                 310                 315                 320

Leu Ala Val Met Arg Gln Gly Pro Met Glu Gly Ile Ser Ser Leu Ala
                325                 330                 335

Asp Ile Glu Asp Phe Leu Gln Gln His
            340                 345

<210>  14

<211>  192

<212>  PRT

<213>  Escherichia coli

<400> 14

Met Tyr Met Pro Gly Lys Gln Met Leu Cys Cys Ile Leu Ile Ser Ile
1               5                   10                  15

Ile Ser Glu Gly Asp Met Lys Ile Phe Ile Ser Leu Phe Leu Phe Ile
            20                  25                  30

Ile Ser Thr Asn Ser Phe Ala Asp Asp Ile Thr His Ala Gly Val Val
        35                  40                  45

Arg Ile Glu Gly Leu Ile Thr Glu Lys Thr Cys Ile Ile Ser Asp Glu
    50                  55                  60

Ser Lys Asn Phe Thr Val Asn Met Pro Asp Val Pro Ser Ser Ser Val
65                  70                  75                  80

Arg Ser Ala Gly Asp Val Thr Glu Lys Val Tyr Phe Ser Ile Thr Leu
                85                  90                  95

Thr Arg Cys Gly Ser Asp Val Gly Asn Ala Tyr Ile Lys Phe Thr Gly
            100                 105                 110

Asn Thr Val Ser Glu Asp Ala Ser Leu Tyr Lys Leu Glu Asp Gly Ser
        115                 120                 125

Val Glu Gly Leu Ala Leu Thr Ile Phe Asp Lys Asn Lys Gly Ser Ile
    130                 135                 140

Ser Asn Asp Val Lys Ser Met Val Phe Ser Leu Thr Ser Ser Val Asp
145                 150                 155                 160

Asn Ile Leu His Phe Phe Ala Ala Tyr Lys Ala Leu Lys Asn Asn Val
                165                 170                 175

Gln Pro Gly Asp Ala Asn Ala Ser Val Ser Phe Ile Val Thr Tyr Asp
            180                 185                 190

<210>  15

<211>  201

<212>  PRT

<213>  Escherichia coli


<400>  15

Met Ile Lys Phe Arg Leu Tyr Ile Pro Pro Val Ile Leu Gly Phe Val
1               5                   10                  15

Ile Val Pro Leu Leu Val Trp Pro Thr Val Ile Ala Leu Ala Val Leu
            20                  25                  30

Ile Phe Thr Leu Thr Phe Leu Ala Glu Ile Ile Phe Ser Phe Pro Leu
        35                  40                  45

Leu Val Val Arg Ile Ser Leu Gln Glu Leu Gln Leu Glu Leu Leu Val
    50                  55                  60

Val Tyr Ala Leu Phe Phe Ser Val Met Gly Gly Ile Gly Trp Gln Phe
65                  70                  75                  80

Ser Arg Arg Thr Pro Pro Glu Leu Lys Asn Arg Leu His Cys Trp Leu
                85                  90                  95

Val Phe Ser Pro Val Tyr Phe Trp Leu Ile Leu Ser Asn Phe Ile Leu
            100                 105                 110

Tyr Ile Ser Pro Glu Lys Ser Ala Leu Leu Glu Asn Ile Arg Asn Phe
        115                 120                 125

Phe Leu Thr Phe Val Trp Leu Pro Leu Asn Phe Ser Pro Phe Trp Pro
    130                 135                 140

Gln Pro Trp Thr Asp Phe Val Gly Pro Ile Ser Ala Gln Leu Gly Phe
145                 150                 155                 160

Ala Leu Gly Tyr Tyr Cys Gln Trp Arg Ser Lys Asn Arg Ser His Arg
                165                 170                 175

Lys Lys Trp Gly Asp Trp Val Thr Cys Leu Ser Leu Ala Ile Leu Ala
            180                 185                 190

Leu Gly Pro Leu Phe Asn Tyr Leu Gln
        195                 200

<210>  16

<211>  234

<212>  PRT

<213>  Escherichia coli

<400>  16

Met Lys Phe Asn Leu Ser Asn Leu Ser Ala Val Leu Leu Ala Ser Gly
1               5                   10                  15

Met Leu Met Ser Thr Ala Val Thr Ala Ala Pro Gly Asp Ala Thr Gln
            20                  25                  30

Phe Gly Gly Ala Asp Thr Asp Trp Ser Thr Val Asp Tyr Pro Arg Leu
        35                  40                  45

Thr Asp Met Asp Asp Asn Val Asp Ser Met Gly Gly Lys Ile Arg Phe
    50                  55                  60

Thr Gly Arg Val Val Lys Ala Thr Cys Lys Val Ala Thr Asp Ser Lys
65                  70                  75                  80

Gln Ile Glu Val Val Leu Pro Val Val Pro Ser Asn Leu Phe Thr Gly
                85                  90                  95

Ile Asp Val Glu Ala Gln Gly Ala Ser Asn Gln Thr Asp Phe Asn Ile
            100                 105                 110

Asn Leu Thr Glu Cys Ser Asn Thr Asp Asp Gln Lys Ile Glu Phe Arg
        115                 120                 125

Phe Thr Gly Thr Ala Asp Ser Ala Asn Lys Thr Leu Ala Asn Glu Val
    130                 135                 140

Glu Gly Ser Thr Asp Ala Asp Asn Ser Gly Asn Ala Gly Ala Thr Gly
145                 150                 155                 160

Val Gly Ile Arg Ile Tyr Ser Lys Gly Thr Thr Asn Asn Gly Leu Ile
                165                 170                 175

Asn Leu Asn Thr Thr Ala Ala Glu Gly Ser Ala Ser Thr Ala Ala Tyr
            180                 185                 190

Thr Ile Pro Gly Asn Ala Thr Thr His Asp Phe Ser Ala Ala Phe Thr
        195                 200                 205

Ala Gly Tyr Ala Gln Asn Gly Ser Thr Val Ala Pro Gly Val Val Lys
    210                 215                 220

Ser Thr Ala Ser Phe Val Val Leu Tyr Glu
225                 230

<210>  17

<211>  336

<212>  PRT

<213>  Escherichia coli

<400>  17

Met Arg Ile His Thr Tyr Trp Tyr Arg Arg Tyr Phe Ile Leu Leu Ile
1               5                   10                  15

Ile Ile Phe Ser Asn Val Leu Ser Ser Ile Ala Asn Ala Glu Asp Met
            20                  25                  30

Gly Arg Glu Arg Ala Tyr Cys Tyr Pro Gly Ser Pro Ser Asn Asn Thr
        35                  40                  45

Thr Pro Ala Ser Phe Ser Tyr Asn Phe Gly Thr Ile Val Val Ser Asp
    50                  55                  60

Val Asn Lys Asn Ala Pro Gly Thr Val Leu Pro Ser Gln Ile Trp Lys
65                  70                  75                  80

Val Gly Thr Tyr Lys Ala Tyr Cys Asn Ser Leu Asp Asp Tyr Glu Ile
                85                  90                  95

Tyr Phe Ser Ala Val Ser Gly Ile Asp Pro Ser Gly Ala Ser Gly Asp
            100                 105                 110

His Gln Gly Ser Asp Val Phe Ile Pro Leu Thr His Glu Ile Ser Val
        115                 120                 125

Ser Thr His Ile Lys Leu Tyr Asn Gln Asn Gly Thr Met Thr Asp Lys
    130                 135                 140

Ile Val Pro Phe Glu Asn Tyr Asn Thr Asn Tyr Pro Gly Asp Arg Ser
145                 150                 155                 160

Lys Pro Ser Asn Trp Ala Ser Gly Thr Glu Gly Tyr Ile Lys Ile Arg
                165                 170                 175

Ile Asp Lys Lys Ile Ile Ser Asp Val Ser Leu Ser Asn Val Leu Leu
            180                 185                 190

Val Ser Leu Tyr Val Ser Gln Ile Pro Thr Glu His Gly Pro Ile Pro
        195                 200                 205

Val Phe Asn Ala Tyr Ile Gly Asn Leu Asn Ile Gln Val Pro Gln Gly
    210                 215                 220

Cys Thr Ile Asn Glu Gly Thr Ser Phe Thr Val Asn Met Pro Asp Val
225                 230                 235                 240

Trp Ala Ser Glu Leu Ser Arg Ala Gly Ala Gly Ala Lys Pro Ala Gly
                245                 250                 255

Val Thr Pro Val Ala Thr Thr Ile Pro Ile Asn Cys Thr Asn Lys Asp
            260                 265                 270

Thr Asp Ala Val Met Thr Leu Val Phe Asp Gly Asn Ile Ser Ala Thr
        275                 280                 285

Arg Asp Thr Asn Gly Lys Gln Ser Ile Ile Gln Ala Gln Asp Asn Pro
    290                 295                 300

Asp Val Gly Ile Met Ile Met Asp Ser Gln Gln Asn Ser Val Asp Leu
305                 310                 315                 320

Asn Ala Leu Ala Thr Ser Val Gly Val Pro Phe Arg Leu Val Glu Asn
                325                 330                 335

<210>  18

<211>  864

<212>  PRT

<213>  Escherichia coli

<400>  18

Met Asn Leu Lys Leu Lys Arg Cys Glu Tyr Trp Met Ala Ala Gln Lys
1               5                   10                  15

Gln Met Lys Arg Val Val Pro Leu Leu Leu Val Ile Met Pro Ala Cys
            20                  25                  30

Ser Ile Ala Gly Met Arg Phe Asn Pro Ala Phe Leu Ser Gly Asp Thr
        35                  40                  45

Glu Ala Val Ala Asp Leu Ser Arg Phe Glu Lys Gly Met Thr Tyr Leu
    50                  55                  60

Pro Gly Ser Tyr Glu Val Glu Val Trp Val Asn Asp Ser Pro Leu Leu
65                  70                  75                  80

Ser Arg Thr Val Thr Phe Lys Ala Asp Asp Glu Asn Gln Leu Ile Pro
                85                  90                  95

Cys Leu Ser Leu Ala Asp Leu Leu Ser Leu Gly Ile Asn Lys Asn Ala
                100                 105                 110

Leu Pro Glu Gln Ala Leu Ala Ser Ser Glu Asn Ser Cys Leu Asp Leu
        115                 120                 125

Arg Ile Trp Phe Pro Asp Val His Tyr Met Pro Glu Leu Asp Ala Gln
    130                 135                 140

Arg Leu Lys Leu Thr Phe Pro Gln Ala Ile Ile Lys Arg Asp Ala Arg
145                 150                 155                 160

Gly Tyr Ile Pro Pro Glu Gln Trp Asp Asn Gly Ile Thr Ala Phe Leu
                165                 170                 175

Leu Asn Tyr Asp Phe Ser Gly Asn Asn Asp Arg Gly Asp Tyr Ser Ser
                180                 185                 190

Asn Asn Tyr Tyr Leu Asn Leu Arg Ala Gly Ile Asn Ile Gly Ala Trp
        195                 200                 205

Arg Phe Arg Asp Tyr Ser Thr Trp Ser Arg Gly Ser Asn Ser Ala Gly
    210                 215                 220

Lys Leu Glu His Ile Ser Ser Thr Leu Gln Arg Val Ile Ile Pro Phe
225                 230                 235                 240

Arg Ser Glu Leu Thr Leu Gly Asp Thr Trp Ser Ser Ser Asp Val Phe
                245                 250                 255

Asp Ser Val Ser Ile Arg Gly Ile Lys Leu Glu Ser Asp Glu Asn Met
                260                 265                 270

Leu Pro Asp Ser Gln Ser Gly Phe Ala Pro Thr Val Arg Gly Ile Ala
        275                 280                 285

Lys Ser Arg Ala Gln Val Thr Ile Lys Gln Asn Gly Tyr Val Ile Tyr
    290                 295                 300

Gln Thr Tyr Met Pro Pro Gly Pro Phe Glu Ile Ser Asp Leu Asn Pro
305                 310                 315                 320

Thr Ser Ser Ala Gly Asp Leu Glu Val Thr Ile Lys Glu Ser Asp Asn
                325                 330                 335

Ser Glu Thr Val Tyr Thr Val Pro Tyr Ala Ala Val Pro Ile Leu Gln
                340                 345                 350

Arg Glu Gly His Leu Lys Tyr Ser Thr Thr Val Gly Gln Tyr Arg Ser
        355                 360                 365

Asn Ser Tyr Asn Gln Lys Ser Pro Tyr Val Phe Gln Gly Glu Leu Ile
    370                 375                 380

Trp Gly Leu Pro Trp Asp Ile Thr Ala Tyr Gly Gly Ala Gln Phe Ser
385                 390                 395                 400

Glu Asp Tyr Arg Ala Leu Ala Leu Gly Leu Gly Leu Asn Leu Gly Val
                405                 410                 415

Phe Gly Ala Thr Ser Phe Asp Val Thr Gln Ala Asn Ser Ser Leu Val
            420                 425                 430

Asp Gly Ser Lys His Gln Gly Gln Ser Tyr Arg Phe Leu Tyr Ser Lys
        435                 440                 445

Ser Leu Val Gln Thr Gly Thr Ala Phe His Ile Ile Gly Tyr Arg Tyr
    450                 455                 460

Ser Thr Gln Gly Phe Tyr Thr Leu Ser Asp Thr Thr Tyr Gln Gln Met
465                 470                 475                 480

Ser Gly Thr Val Val Asp Pro Lys Thr Leu Asp Asp Lys Asp Tyr Val
                485                 490                 495

Tyr Asn Trp Asn Asp Phe Tyr Asn Leu Arg Tyr Ser Lys Arg Gly Lys
            500                 505                 510

Phe Gln Ala Ser Val Ser Gln Pro Phe Gly Asn Tyr Gly Ser Met Tyr
        515                 520                 525

Leu Ser Ala Ser Gln Gln Thr Tyr Trp Asn Thr Asp Lys Lys Asp Ser
    530                 535                 540

Leu Tyr Gln Val Gly Tyr Asn Thr Ser Ile Lys Gly Ile Tyr Leu Asn
545                 550                 555                 560

Val Ala Trp Asn Tyr Ser Lys Ser Pro Gly Thr Asn Ala Asp Lys Ile
                565                 570                 575

Val Ser Leu Asn Val Ser Leu Pro Ile Ser Asn Trp Leu Ser Ser Thr
            580                 585                 590

Asn Asp Gly Arg Ser Ser Ser Asn Ala Met Thr Ala Thr Tyr Gly Tyr
        595                 600                 605

Ser Gln Asp Asn His Gly Gln Val Asn Gln Tyr Thr Gly Val Ser Gly
    610                 615                 620

Ser Leu Leu Glu Gln His Asn Leu Ser Tyr Asn Ile Gln His Gly Phe
625                 630                 635                 640

Ala Asn Gln Asp Asn Ser Ser Ser Gly Ser Val Gly Val Asn Tyr Arg
                645                 650                 655

Gly Ala Tyr Gly Ser Leu Asn Ser Ala Tyr Ser Tyr Asp Asn Glu Gly
            660                 665                 670

Asn Gln Gln Ile Asn Tyr Gly Ile Ser Gly Ala Leu Val Val His Glu
        675                 680                 685

Asn Gly Leu Thr Leu Ser Gln Pro Leu Gly Glu Thr Asn Val Leu Ile
    690                 695                 700

Lys Ala Pro Gly Ala Asn Asn Val Asp Val Gln Arg Gly Thr Gly Ile
705                 710                 715                 720

Ser Thr Asp Trp Arg Gly Tyr Ala Val Val Pro Tyr Ala Thr Glu Tyr
                725                 730                 735

Arg Arg Asn Asn Ile Ser Leu Asp Pro Met Ser Met Asn Met His Thr
            740                 745                 750

Glu Leu Asp Ile Thr Ser Thr Glu Val Ile Pro Gly Lys Gly Ala Leu
        755                 760                 765

Val Arg Ala Glu Phe Ala Ala His Ile Gly Ile Arg Gly Leu Phe Thr
    770                 775                 780

Val Arg Tyr Arg Asn Lys Ser Val Pro Phe Gly Ala Thr Ala Ser Ala
785                 790                 795                 800

Gln Ile Lys Asn Ser Ser Gln Ile Thr Gly Ile Val Gly Asp Asn Gly
                805                 810                 815

Gln Leu Tyr Leu Ser Gly Leu Pro Leu Glu Gly Val Ile Asn Ile Gln
            820                 825                 830

Trp Gly Asp Gly Val Gln Gln Lys Cys Gln Ala Asn Tyr Lys Leu Pro
        835                 840                 845

Glu Thr Glu Leu Asp Asn Pro Val Ser Tyr Ala Thr Leu Glu Cys Arg
    850                 855                 860

<210> 19

<211> 169

<212> PRT

<213> Escherichia coli

<400> 19

Met Gly Ala Ile Tyr Val Lys Arg Leu Ile Leu Ser Val Ala Leu Ile
1               5                   10                  15

Ile Pro Ile Ala Ser Asn Ala Ser Asp Ala Leu Asn Gln Pro Ser Ser
            20                  25                  30

Ser Leu Asn Asp Gly Val Glu Thr Phe Phe Ile Ser Cys Phe Asp Met
        35                  40                  45

Pro Gln Glu Thr Thr Thr Asp Met Asp Ala Cys Gln Arg Val Gln Leu
    50                  55                  60

Ala Gln Val Ser Trp Val Lys Asn Lys Tyr Ser Val Ala Ala Leu Asn
65                  70                  75                  80

Arg Leu Lys Gln Asp Asn Lys Asp Asp Pro Gln Arg Leu Gln Glu Leu
                85                  90                  95

Thr Ala Ser Phe Asn Ala Glu Ser Glu Ala Trp Thr Glu Leu Ile Glu
            100                 105                 110

Lys Ala Ser Lys Ser Val Gln Val Asp Tyr Val Gly Gly Thr Ile Ala
        115                 120                 125

Gly Thr Ala Val Ala Ser Arg Gln Ile Gly Leu Leu Glu Leu Gln Ser
    130                 135                 140

His Asp Ile Trp Glu His Trp Leu Arg Ser Arg Gly Leu Asn Ser Ser
145                 150                 155                 160

Ser Phe Ala Arg Thr Lys Val Gln Ile
                165

<210> 20

<211> 713

<212> PRT

<213> Escherichia coli

<400> 20

Met Ala Met Phe Thr Pro Ser Phe Ser Gly Leu Lys Gly Arg Ala Leu
1               5                   10                  15

Phe Ser Leu Leu Phe Ala Ala Pro Met Ile His Ala Thr Asp Ser Val
            20                  25                  30

Thr Thr Lys Asp Gly Glu Thr Ile Thr Val Thr Ala Asp Ala Asn Thr
        35                  40                  45

Ala Thr Glu Ala Thr Asp Gly Tyr Gln Pro Leu Ser Thr Ser Thr Ala
    50                  55                  60

Thr Leu Thr Asp Met Pro Met Leu Asp Ile Pro Gln Val Val Asn Thr
65                  70                  75                  80

Val Ser Asp Gln Val Leu Glu Asn Gln Asn Ala Thr Thr Leu Asp Glu
                85                  90                  95

Ala Leu Tyr Asn Val Ser Asn Val Val Gln Thr Asn Thr Leu Gly Gly
            100                 105                 110

Thr Gln Asp Ala Phe Val Arg Arg Gly Phe Gly Ala Asn Arg Asp Gly
        115                 120                 125

Ser Ile Met Thr Asn Gly Leu Arg Thr Val Leu Pro Arg Ser Phe Asn
    130                 135                 140

Ala Ala Thr Glu Arg Val Glu Val Leu Lys Gly Pro Ala Ser Thr Leu
145                 150                 155                 160

Tyr Gly Ile Leu Asp Pro Gly Gly Leu Ile Asn Val Val Thr Lys Arg
                165                 170                 175

Pro Glu Lys Thr Phe His Gly Ser Val Ser Ala Thr Ser Ser Ser Phe
            180                 185                 190

Gly Gly Gly Thr Gly Gln Leu Asp Ile Thr Gly Pro Ile Glu Gly Thr
        195                 200                 205

Gln Leu Ala Tyr Arg Leu Thr Gly Glu Val Gln Asp Glu Asp Tyr Trp
    210                 215                 220

Arg Asn Phe Gly Lys Glu Arg Ser Thr Phe Ile Ala Pro Ser Leu Thr
225                 230                 235                 240

Trp Phe Gly Asp Asn Ala Thr Val Thr Met Leu Tyr Ser His Arg Asp
                245                 250                 255

Tyr Lys Thr Pro Phe Asp Arg Gly Thr Ile Phe Asp Leu Thr Thr Lys
            260                 265                 270

Gln Pro Val Asn Val Asp Arg Lys Ile Arg Phe Asp Glu Pro Phe Asn
        275                 280                 285

Ile Thr Asp Gly Gln Ser Asp Leu Ala Gln Leu Asn Ala Glu Tyr His
    290                 295                 300

Leu Asn Ser Gln Trp Thr Ala Arg Phe Asp Tyr Ser Tyr Ser Gln Asp
305                 310                 315                 320

Lys Tyr Ser Asp Asn Gln Ala Arg Val Thr Ala Tyr Asp Ala Thr Thr
                325                 330                 335

Gly Thr Leu Thr Arg Arg Val Asp Ala Thr Gln Gly Ser Thr Gln Arg
            340                 345                 350

Met His Ala Thr Arg Ala Asp Leu Gln Gly Asn Val Asp Ile Ala Gly
        355                 360                 365

Phe Tyr Asn Glu Ile Leu Gly Gly Val Ser Tyr Glu Tyr Tyr Asp Leu
    370                 375                 380

Leu Arg Thr Asp Met Ile Arg Cys Lys Lys Ala Lys Asp Phe Asn Ile
385                 390                 395                 400

Tyr Asn Pro Val Tyr Gly Asn Thr Ser Lys Cys Thr Thr Val Ser Ala
                405                 410                 415

Ser Asp Ser Asp Gln Thr Ile Lys Gln Glu Asn Tyr Ser Ala Tyr Ala
            420                 425                 430

Gln Asp Ala Leu Tyr Leu Thr Asp Asn Trp Ile Ala Val Ala Gly Ile
        435                 440                 445

Arg Tyr Gln Tyr Tyr Thr Gln Tyr Ala Gly Lys Gly Arg Pro Phe Asn
    450                 455                 460

Val Asn Thr Asp Ser Arg Asp Glu Gln Trp Thr Pro Lys Leu Gly Leu
465                 470                 475                 480

Val Tyr Lys Leu Thr Pro Ser Val Ser Leu Phe Ala Asn Tyr Ser Gln
                485                 490                 495

Thr Phe Met Pro Gln Ser Ser Ile Ala Ser Tyr Ile Gly Asp Leu Pro
            500                 505                 510

Pro Glu Ser Ser Asn Ala Tyr Glu Val Gly Ala Lys Phe Glu Leu Phe
        515                 520                 525

Asp Gly Ile Thr Ala Asp Ile Ala Leu Phe Asp Ile His Lys Arg Asn
    530                 535                 540

Val Leu Tyr Thr Glu Ser Ile Gly Asp Glu Thr Ile Ala Lys Thr Ala
545                 550                 555                 560

Gly Arg Val Arg Ser Arg Gly Val Glu Val Asp Leu Ala Gly Ala Leu
                565                 570                 575

Thr Glu Asn Ile Asn Ile Ile Ala Ser Tyr Gly Tyr Thr Asp Ala Lys
            580                 585                 590

Val Leu Glu Asp Pro Asp Tyr Ala Gly Lys Pro Leu Pro Asn Val Pro
        595                 600                 605

Arg His Thr Gly Ser Leu Phe Leu Thr Tyr Asp Ile His Asn Met Pro
    610                 615                 620

Gly Asn Asn Thr Leu Thr Phe Gly Gly Gly Gly His Gly Val Ser Arg
625                 630                 635                 640

Arg Ser Ala Thr Asn Gly Ala Asp Tyr Tyr Leu Pro Gly Tyr Phe Val
                645                 650                 655

Ala Asp Ala Phe Ala Ala Tyr Lys Met Lys Leu Gln Tyr Pro Val Thr
            660                 665                 670

Leu Gln Leu Asn Val Lys Asn Leu Phe Asp Lys Thr Tyr Tyr Thr Ser
        675                 680                 685

Ser Ile Ala Thr Asn Asn Leu Gly Asn Gln Ile Gly Asp Pro Arg Glu
    690                 695                 700

Val Gln Phe Thr Val Lys Met Glu Phe
705                 710

<210>  21

<211>  606

<212>  PRT

<213>  Escherichia coli

<400> 21

Met Lys Ile Ser Trp Asn Tyr Ile Phe Lys Asn Lys Trp Arg Phe His
1               5                   10                  15

Ile Thr Ser Ile Ser Leu Phe Leu Ile Met Leu Ala Val Ser Ile Ala
            20                  25                  30

Phe Leu His Leu Arg Phe Asn Thr Leu Ser Ser Thr Asp Lys Met Arg
        35                  40                  45

Leu Glu Met Tyr Lys Ser Thr Leu Tyr Ser Thr Ile Glu Gln Phe Tyr
    50                  55                  60

Val Leu Pro Tyr Met Leu Ser Thr Asp His Ile Ile Arg Gln Ala Val
65                  70                  75                  80

Ile Thr Pro Asp Asp Met Thr Ser Ser Glu Leu Asn Gln Arg Ile Ala
                85                  90                  95

His Phe Asn Thr Gln Leu Lys Thr Ala Ala Ile Phe Ile Leu Asp Thr
            100                 105                 110

Gln Gly Lys Ala Ile Ala Ser Ser Asn Trp Gln Asp Pro Gly Ser Tyr
        115                 120                 125

Val Gly Gln Asn Tyr Ser Tyr Arg Pro Tyr Tyr Lys His Ala Met Ser
    130                 135                 140

Gly Leu Asn Gly Arg Phe Tyr Gly Ile Gly Ser Thr Thr Asn Thr Pro
145                 150                 155                 160

Gly Phe Phe Leu Ser Thr Ser Ile Lys Asp Lys Gly Lys Ile Val Gly
                165                 170                 175

Val Val Val Val Lys Ile Ser Leu Asn Glu Ile Glu Lys Ala Trp Ala
            180                 185                 190

Glu Gly Pro Glu Asn Ile Ile Val Asn Asp Glu His Gly Ile Ile Phe
        195                 200                 205

Leu Ser Ser Lys Ser Pro Trp Arg Met Arg Thr Leu Gln Pro Leu Pro
    210                 215                 220

Val Gln Ala Lys Gln Lys Leu Gln Ser Thr Arg Gln Tyr Ser Leu Asp
225                 230                 235                 240

Asn Leu Leu Pro Ala Asp Tyr Tyr Pro Cys Tyr Thr Val Ser Asn Phe
                245                 250                 255

Thr Phe Leu Lys Asp Lys Lys Glu Gln Leu Cys Leu Phe Pro Gln Tyr
            260                 265                 270

Tyr Thr Gln Gln Ile Ala Ile Pro Glu Phe Asn Trp Lys Met Thr Ile
        275                 280                 285

Met Val Pro Leu Asp Asn Leu Tyr Trp Ser Trp Ala Ile Ser Leu Val
    290                 295                 300

Ile Thr Leu Ile Ile Tyr Leu Leu Phe Leu Leu Phe Ile Lys Tyr Trp
305                 310                 315                 320

Arg Met Arg Ser His Ala Gln Gln Leu Leu Thr Leu Ala Asn Glu Thr
                325                 330                 335

Leu Glu Lys Gln Val Lys Glu Arg Thr Ser Ala Leu Glu Leu Ile Asn
            340                 345                 350

Gln Lys Leu Ile Gln Glu Ile Lys Glu Arg Ser Gln Ala Glu Gln Val
        355                 360                 365

Leu Gln Ile Thr Arg Ser Glu Leu Ala Glu Ser Ser Lys Leu Ala Ala
    370                 375                 380

Leu Gly Gln Met Ala Thr Glu Ile Ala His Glu Gln Asn Gln Pro Leu
385                 390                 395                 400

Ala Ala Ile His Ala Leu Thr Asp Asn Ala Arg Thr Met Leu Lys Lys
                405                 410                 415

Glu Met Tyr Pro Gln Val Glu Gln Asn Leu Lys His Ile Ile Ser Val
            420                 425                 430

Ile Glu Arg Met Thr Gln Leu Ile Ser Glu Leu Lys Ala Phe Ala Ser
        435                 440                 445

Arg His Arg Val Pro Lys Gly Ser Ala Asp Val Ile Lys Val Met Tyr
    450                 455                 460

Ser Ala Val Ala Leu Leu Asn His Ser Met Glu Lys Asn Asn Ile Glu
465                 470                 475                 480

Arg Arg Ile Lys Ala Pro Ser Met Pro Leu Phe Val Asn Cys Asp Glu
                485                 490                 495

Leu Gly Leu Glu Gln Ile Phe Ser Asn Leu Ile Ser Asn Ala Leu Asp
            500                 505                 510

Ser Met Glu Gly Ser Ser Tyr Lys Arg Leu Asp Ile Ala Ile Arg Gln
        515                 520                 525

Ala Asn Asn Lys Val Ile Ile Thr Ile Lys Asp Ser Gly Gly Gly Phe
    530                 535                 540

Ala Pro Glu Val Val Asp Arg Ile Phe Glu Pro Phe Phe Thr Thr Lys
545                 550                 555                 560

Arg Arg Gly Met Gly Leu Gly Leu Ala Ile Val Ser Glu Ile Val Arg
                565                 570                 575

Asn Ser Asn Gly Ala Leu His Ala Ser Asn His Pro Glu Gly Gly Ala
            580                 585                 590

Val Met Thr Leu Thr Trp Pro Glu Trp Gly Glu Glu His Glu
        595                 600                 605

<210>  22

<211>  101

<212>  PRT

<213>  Escherichia coli

<400>  22

Val Leu Thr Pro Gln His Leu Arg Cys Val Leu Thr Cys Ser Asp Leu
1               5                   10                  15

Leu Thr Leu Leu Ser Gly Thr Val Met Ser Gln Met Pro Leu Tyr Phe
            20                  25                  30

Leu Asn Thr Gln Lys Lys Leu Thr Ala His Tyr Glu Trp Leu Gln Ile
        35                  40                  45

Asn Leu Thr Asp Thr Tyr Glu Leu Val Lys Arg Leu Met Pro Ile Pro
    50                  55                  60

Ser Leu Asp Val Val Val Lys Val Gly Lys Leu Val Leu Pro Glu Lys
65                  70                  75                  80

Gly His His Gly Phe Tyr Pro Glu Ala Gly Val Val Tyr Arg Thr Val
                85                  90                  95

Ala Pro Glu Asn Pro
            100

<210>  23

<211>  263

<212>  PRT

<213>  Escherichia coli

<400>  23

Met Met Lys Asn Thr Gly Tyr Ile Leu Ala Leu Cys Leu Thr Ala Ser
1               5                   10                  15

Gly His Val Leu Ala His Asp Val Trp Ile Thr Gly Lys Gln Ala Glu
            20                  25                  30

Asn Asn Val Thr Ala Glu Ile Gly Tyr Gly His Asn Phe Pro Ser Lys
        35                  40                  45

Gly Thr Ile Pro Asp Arg Arg Asp Phe Phe Glu Asn Pro Arg Leu Tyr
    50                  55                  60

Asn Gly Lys Glu Thr Ile Thr Leu Lys Pro Ala Ser Thr Asp Tyr Val
65                  70                  75                  80

Tyr Lys Thr Glu Ser Ala Ser Lys Asp Asn Gly Tyr Val Leu Ser Thr
                85                  90                  95

Tyr Met Lys Pro Gly Tyr Trp Ser Arg Thr Ser Ser Gly Trp Lys Pro
            100                 105                 110

Val Ser Arg Glu Gly Arg Asn Asp Val Ala Tyr Cys Glu Phe Val Thr
        115                 120                 125

Lys Tyr Ala Lys Ser Phe Ile Pro Gly Glu Gln Gln Met Pro Ala Gln
    130                 135                 140

Leu Tyr Gln Ser Pro Thr Gly His Glu Leu Glu Ile Ile Pro Leu Ser
145                 150                 155                 160

Asp Ile Ser Arg Phe Ser Glu Asn Val Lys Leu Lys Val Leu Tyr Lys
                165                 170                 175

Thr Ser Pro Leu Ala Gly Ala Ile Met Glu Leu Asp Ser Val Ser Tyr
            180                 185                 190

Leu Thr Ser Ser Arg His Thr His Ala Val Glu His Lys His Pro Val
        195                 200                 205

His Lys Ala Glu Leu Thr Phe Val Thr Asn Glu Asp Gly Ile Val Thr
    210                 215                 220

Val Pro Ser Leu His Ile Gly Gln Trp Leu Ala Lys Val Gln Asn Lys
225                 230                 235                 240

Lys Ser Phe Gln Asp Lys Ser Leu Cys Asp Glu Thr Val Asp Val Ala
                245                 250                 255

Thr Leu Ser Phe Ser Arg Asn
            260

<210>  24

<211>  378

<212>  PRT

<213>  Escherichia coli

<400>  24

Met Gly Lys Ile Lys Tyr Trp Leu Ile Val Gly Phe Ile Ile Leu Phe
1               5                   10                  15

Ala Ile Phe Tyr Ile Ala Ile Ser Asp Arg Asp Ser Thr Leu Ser Arg
            20                  25                  30

Leu Lys Ser Ala Gly Glu Asn Gly Asp Val Glu Ala Gln Tyr Ala Leu
        35                  40                  45

Gly Leu Met Tyr Leu Tyr Gly Glu Ile Leu Asp Val Asp Tyr Gln Gln
    50                  55                  60

Ala Lys Ile Trp Tyr Glu Lys Ala Ala Asp Gln Asn Asp Pro Arg Ala
65                  70                  75                  80

Gln Ala Lys Leu Gly Val Met Tyr Ala Asn Gly Leu Gly Val Asn Gln
                85                  90                  95

Asp Tyr Gln Gln Ser Lys Leu Trp Tyr Glu Lys Ala Ala Ala Gln Asn
            100                 105                 110

Asp Val Asp Ala Gln Phe Leu Leu Gly Glu Met Tyr Asp Asp Gly Leu
        115                 120                 125

Gly Val Ser Gln Asp Tyr Gln His Ala Lys Met Trp Tyr Glu Lys Ala
    130                 135                 140

Ala Ala Gln Asn Asp Glu Arg Ala Gln Val Asn Leu Ala Val Leu Tyr
145                 150                 155                 160

Ala Lys Gly Asn Gly Val Glu Gln Asp Tyr Arg Gln Ala Lys Ser Trp
                165                 170                 175

Tyr Glu Lys Ala Ala Ala Gln Asn Ser Pro Asp Ala Gln Phe Ala Leu
            180                 185                 190

Gly Ile Leu Tyr Ala Asn Ala Asn Gly Val Glu Gln Asp Tyr Gln Gln
        195                 200                 205

Ala Lys Asp Trp Tyr Glu Lys Ala Ala Glu Gln Asn Phe Ala Asn Ala
    210                 215                 220

Gln Phe Asn Leu Gly Met Leu Tyr Tyr Lys Gly Glu Gly Val Lys Gln
225                 230                 235                 240

Asn Phe Arg Gln Ala Arg Glu Trp Phe Glu Lys Ala Ala Ser Gln Asn
                245                 250                 255

Gln Pro Asn Ala Gln Tyr Asn Leu Gly Gln Ile Tyr Tyr Tyr Gly Gln
            260                 265                 270

Gly Val Thr Gln Ser Tyr Arg Gln Ala Lys Asp Trp Phe Glu Lys Ala
        275                 280                 285

Ala Glu Lys Gly His Val Asp Ala Gln Tyr Asn Leu Gly Val Ile Tyr
    290                 295                 300

Glu Asn Gly Glu Gly Val Ser Gln Asn Tyr Gln Gln Ala Lys Ala Trp
305                 310                 315                 320

Tyr Glu Lys Ala Ala Ser Gln Asn Asp Ala Gln Ala Gln Phe Glu Leu
                325                 330                 335

Gly Val Met Asn Glu Leu Gly Gln Gly Glu Ser Ile Asp Leu Lys Gln
            340                 345                 350

Ala Arg His Tyr Tyr Glu Arg Ser Cys Asn Asn Gly Leu Lys Lys Gly
        355                 360                 365

Cys Glu Arg Leu Lys Glu Leu Leu Tyr Lys
    370                 375

<210> 25

<211> 654

<212> PRT

<213> Escherichia coli

<400> 25

Met Asn Val Ile Arg Thr Val Ile Cys Thr Leu Ile Ile Leu Pro Val
1               5                   10                  15

Gly Leu Gln Ala Ala Thr Ser His Ser Ser Met Val Lys Asp Thr Ile
            20                  25                  30

Thr Ile Val Ala Thr Gly Asn Gln Asn Thr Val Phe Glu Thr Pro Ser
        35                  40                  45

Met Val Ser Val Val Thr Asn Asp Thr Pro Trp Ser Gln Asn Ala Val
    50                  55                  60

Thr Ser Ala Gly Met Leu Lys Gly Val Ala Gly Leu Ser Gln Thr Gly
65                  70                  75                  80

Ala Gly Arg Thr Asn Gly Gln Thr Phe Asn Leu Arg Gly Tyr Asp Lys
                85                  90                  95

Ser Gly Val Leu Val Leu Val Asp Gly Val Arg Gln Leu Ser Asp Met
            100                 105                 110

Ala Lys Ser Ser Gly Thr Tyr Leu Asp Pro Ala Leu Val Lys Arg Ile
        115                 120                 125

Glu Val Val Arg Gly Pro Asn Ser Ser Leu Tyr Gly Ser Gly Gly Leu
    130                 135                 140

Gly Gly Val Val Asp Phe Arg Thr Ala Asp Ala Ala Asp Phe Leu Pro
145                 150                 155                 160

Pro Gly Glu Thr Asn Gly Leu Ser Leu Trp Gly Asn Ile Ala Ser Gly
                165                 170                 175

Asp His Ser Thr Gly Ser Gly Leu Thr Trp Phe Gly Lys Thr Gly Lys
            180                 185                 190

Thr Asp Ala Leu Leu Ser Val Ile Met Arg Lys Arg Gly Asn Ile Tyr
        195                 200                 205

Gln Ser Asp Gly Glu His Ala Pro Asn Lys Glu Lys Pro Ala Ala Leu
    210                 215                 220

Phe Ala Lys Gly Ser Val Gly Ile Thr Asp Ser Asn Lys Ala Gly Ala
225                 230                 235                 240

Ser Leu Arg Leu Tyr Arg Asn Asn Thr Thr Glu Pro Gly Asn Ser Thr
                245                 250                 255

Gln Thr His Gly Asp Ser Gly Leu Arg Asp Arg Lys Thr Val Gln Asn
            260                 265                 270

Asp Val Gln Phe Trp Tyr Gln Tyr Ala Pro Val Asp Asn Ser Leu Ile
        275                 280                 285

Asn Val Lys Ser Thr Leu Tyr Leu Ser Asp Ile Thr Ile Lys Thr Asn
    290                 295                 300

Gly His Asn Lys Thr Ala Glu Trp Arg Asn Asn Arg Thr Ser Gly Val
305                 310                 315                 320

Asn Val Val Asn Arg Ser His Thr Leu Ile Phe Pro Gly Ala His Gln
                325                 330                 335

Leu Ser Tyr Gly Ala Glu Tyr Tyr Arg Gln Gln Gln Lys Pro Glu Gly
            340                 345                 350

Ser Ala Thr Leu Tyr Pro Glu Gly Asn Ile Asp Phe Thr Ser Leu Tyr
        355                 360                 365

Phe Gln Asp Glu Met Thr Met Lys Ser Tyr Pro Val Asn Ile Ile Val
    370                 375                 380

Gly Ser Arg Tyr Asp Arg Tyr Lys Ser Phe Asn Pro Arg Ala Gly Glu
385                 390                 395                 400

Leu Lys Ala Glu Arg Leu Ser Pro Arg Ala Ala Ile Ser Val Ser Pro
                405                 410                 415

Thr Asp Trp Leu Met Met Tyr Gly Ser Ile Ser Ser Ala Phe Arg Ala
            420                 425                 430

Pro Thr Met Ala Glu Met Tyr Arg Asp Asp Val His Phe Tyr Arg Lys
        435                 440                 445

Gly Lys Pro Asn Tyr Trp Val Pro Asn Leu Asn Leu Lys Pro Glu Asn
    450                 455                 460

Asn Ile Thr Arg Glu Ile Gly Ala Gly Ile Gln Leu Asp Gly Leu Leu
465                 470                 475                 480

Thr Asp Asn Asp Arg Leu Gln Leu Lys Gly Gly Tyr Phe Gly Thr Asp
                485                 490                 495

Ala Arg Asn Tyr Ile Ala Thr Arg Val Asp Met Lys Arg Met Arg Ser
            500                 505                 510

Tyr Ser Tyr Asn Val Ser Arg Ala Arg Ile Trp Gly Trp Asp Met Gln
        515                 520                 525

Gly Asn Tyr Gln Ser Asp Tyr Val Asp Trp Met Leu Ser Tyr Asn Arg
    530                 535                 540

Thr Glu Ser Met Asp Ala Ser Ser Arg Glu Trp Leu Gly Ser Gly Asn
545                 550                 555                 560

Pro Asp Thr Leu Ile Ser Asp Ile Ser Ile Pro Val Gly His Arg Gly
                565                 570                 575

Val Tyr Ala Gly Trp Arg Ala Glu Leu Ser Ala Ser Ala Thr His Val
            580                 585                 590

Lys Lys Gly Asp Pro His Gln Ala Gly Tyr Thr Ile His Ser Phe Ser
        595                 600                 605

Leu Ser Tyr Lys Pro Val Ser Val Lys Gly Phe Glu Ala Ser Val Thr
    610                 615                 620

Leu Asp Asn Ala Phe Asn Lys Leu Ala Met Asn Gly Lys Gly Val Pro
625                 630                 635                 640

Leu Ser Gly Arg Thr Val Ser Leu Tyr Thr Arg Tyr Gln Trp
                645                 650

<210>  26

<211>  1376

<212>  PRT

<213>  Escherichia coli

<400>  26

Met Asn Lys Ile Tyr Ala Leu Lys Tyr Cys Tyr Ile Thr Asn Thr Val
1               5                   10                  15

Lys Val Val Ser Glu Leu Ala Arg Arg Val Cys Lys Gly Ser Thr Arg
            20                  25                  30

Arg Gly Lys Arg Leu Ser Val Leu Thr Ser Leu Ala Leu Ser Ala Leu
        35                  40                  45

Leu Pro Thr Val Ala Gly Ala Ser Thr Val Gly Gly Asn Asn Pro Tyr
    50                  55                  60

Gln Thr Tyr Arg Asp Phe Ala Glu Asn Lys Gly Gln Phe Gln Ala Gly
65                  70                  75                  80

Ala Thr Asn Ile Pro Ile Phe Asn Asn Lys Gly Glu Leu Val Gly His
                85                  90                  95

Leu Asp Lys Ala Pro Met Val Asp Phe Ser Ser Val Asn Val Ser Ser
            100                 105                 110

Asn Pro Gly Val Ala Thr Leu Ile Asn Pro Gln Tyr Ile Ala Ser Val
        115                 120                 125

Lys His Asn Lys Gly Tyr Gln Ser Val Ser Phe Gly Asp Gly Gln Asn
    130                 135                 140

Ser Tyr His Ile Val Asp Arg Asn Glu His Ser Ser Ser Asp Leu His
145                 150                 155                 160

Thr Pro Arg Leu Asp Lys Leu Val Thr Glu Val Ala Pro Ala Thr Val
                165                 170                 175

Thr Ser Ser Ser Thr Ala Asp Ile Leu Asn Pro Ser Lys Tyr Ser Ala
            180                 185                 190

Phe Tyr Arg Ala Gly Ser Gly Ser Gln Tyr Ile Gln Asp Ser Gln Gly
        195                 200                 205

Lys Arg His Trp Val Thr Gly Gly Tyr Gly Tyr Leu Thr Gly Gly Ile
    210                 215                 220

Leu Pro Thr Ser Phe Phe Tyr His Gly Ser Asp Gly Ile Gln Leu Tyr
225                 230                 235                 240

Met Gly Gly Asn Ile His Asp His Ser Ile Leu Pro Ser Phe Gly Glu
                245                 250                 255

Ala Gly Asp Ser Gly Ser Pro Leu Phe Gly Trp Asn Thr Ala Lys Gly
            260                 265                 270

Gln Trp Glu Leu Val Gly Val Tyr Ser Gly Val Gly Gly Gly Thr Asn
        275                 280                 285

Leu Ile Tyr Ser Leu Ile Pro Gln Ser Phe Leu Ser Gln Ile Tyr Ser
    290                 295                 300

Glu Asp Asn Asp Ala Pro Val Phe Phe Asn Ala Ser Ser Gly Ala Pro
305                 310                 315                 320

Leu Gln Trp Lys Phe Asp Ser Ser Thr Gly Thr Gly Ser Leu Lys Gln
                325                 330                 335

Gly Ser Asp Glu Tyr Ala Met His Gly Gln Lys Gly Ser Asp Leu Asn
            340                 345                 350

Ala Gly Lys Asn Leu Thr Phe Leu Gly His Asn Gly Gln Ile Asp Leu
        355                 360                 365

Glu Asn Ser Val Thr Gln Gly Ala Gly Ser Leu Thr Phe Thr Asp Asp
    370                 375                 380

Tyr Thr Val Thr Thr Ser Asn Gly Ser Thr Trp Thr Gly Ala Gly Ile
385                 390                 395                 400

Ile Val Asp Lys Asp Ala Ser Val Asn Trp Gln Val Asn Gly Val Lys
                405                 410                 415

Gly Asp Asn Leu His Lys Ile Gly Glu Gly Thr Leu Val Val Gln Gly
            420                 425                 430

Thr Gly Val Asn Glu Gly Gly Leu Lys Val Gly Asp Gly Thr Val Val
        435                 440                 445

Leu Asn Gln Gln Ala Asp Ser Ser Gly His Val Gln Ala Phe Ser Ser
    450                 455                 460

Val Asn Ile Ala Ser Gly Arg Pro Thr Val Val Leu Ala Asp Asn Gln
465                 470                 475                 480

Gln Val Asn Pro Asp Asn Ile Ser Trp Gly Tyr Arg Gly Gly Val Leu
                485                 490                 495

Asp Val Asn Gly Asn Asp Leu Thr Phe His Lys Leu Asn Ala Ala Asp
            500                 505                 510

Tyr Gly Ala Thr Leu Gly Asn Ser Ser Asp Lys Thr Ala Asn Ile Thr
        515                 520                 525

Leu Asp Tyr Gln Thr Arg Pro Ala Asp Val Lys Val Asn Glu Trp Ser
    530                 535                 540

Ser Ser Asn Arg Gly Thr Val Gly Ser Leu Tyr Ile Tyr Asn Asn Pro
545                 550                 555                 560

Tyr Thr His Thr Val Asp Tyr Phe Ile Leu Lys Thr Ser Ser Tyr Gly
                565                 570                 575

Trp Phe Pro Thr Gly Gln Val Ser Asn Glu His Trp Glu Tyr Val Gly
            580                 585                 590

His Asp Gln Asn Ser Ala Gln Ala Leu Leu Ala Asn Arg Ile Asn Asn
        595                 600                 605

Lys Gly Tyr Leu Tyr His Gly Lys Leu Leu Gly Asn Ile Asn Phe Ser
    610                 615                 620

Asn Lys Ala Thr Pro Gly Thr Thr Gly Ala Leu Val Met Asp Gly Ser
625                 630                 635                 640

Ala Asn Met Ser Gly Thr Phe Thr Gln Glu Asn Gly Arg Leu Thr Ile
                645                 650                 655

Gln Gly His Pro Val Ile His Ala Ser Thr Ser Gln Ser Ile Ala Asn
            660                 665                 670

Thr Val Ser Ser Leu Gly Asp Asn Ser Val Leu Thr Gln Pro Thr Ser
        675                 680                 685

Phe Thr Gln Asp Asp Trp Glu Asn Arg Thr Phe Ser Phe Gly Ser Leu
    690                 695                 700

Val Leu Lys Asp Thr Asp Phe Gly Leu Gly Arg Asn Ala Thr Leu Asn
705                 710                 715                 720

Thr Thr Ile Gln Ala Asp Asn Ser Ser Val Thr Leu Gly Asp Ser Arg
                725                 730                 735

Val Phe Ile Asp Lys Lys Asp Gly Gln Gly Thr Ala Phe Thr Leu Glu
            740                 745                 750

Glu Gly Thr Ser Val Ala Thr Lys Asp Ala Asp Lys Ser Val Phe Asn
        755                 760                 765

Gly Thr Val Asn Leu Asp Asn Gln Ser Val Leu Asn Ile Asn Glu Ile
    770                 775                 780

Phe Asn Gly Gly Ile Gln Ala Asn Asn Ser Thr Val Asn Ile Ser Ser
785                 790                 795                 800

Asp Ser Ala Val Leu Glu Asn Ser Thr Leu Thr Ser Thr Ala Leu Asn
                805                 810                 815

Leu Asn Lys Gly Ala Asn Val Leu Ala Ser Gln Ser Phe Val Ser Asp
            820                 825                 830

Gly Pro Val Asn Ile Ser Asp Ala Thr Leu Ser Leu Asn Ser Arg Pro
        835                 840                 845

Asp Glu Val Ser His Thr Leu Leu Pro Val Tyr Asp Tyr Ala Gly Ser
    850                 855                 860

Trp Asn Leu Lys Gly Asp Asp Ala Arg Leu Asn Val Gly Pro Tyr Ser
865                 870                 875                 880

Met Leu Ser Gly Asn Ile Asn Val Gln Asp Lys Gly Thr Val Thr Leu
                885                 890                 895

Gly Gly Glu Gly Glu Leu Ser Pro Asp Leu Thr Leu Gln Asn Gln Met
            900                 905                 910

Leu Tyr Ser Leu Phe Asn Gly Tyr Arg Asn Thr Trp Ser Gly Ser Leu
        915                 920                 925

Asn Ala Pro Asp Ala Thr Val Ser Met Thr Asp Thr Gln Trp Ser Met
    930                 935                 940

Asn Gly Asn Ser Thr Ala Gly Asn Met Lys Leu Asn Arg Thr Ile Val
945                 950                 955                 960

Gly Phe Asn Gly Gly Thr Ser Ser Phe Thr Thr Leu Thr Thr Asp Asn
                965                 970                 975

Leu Asp Ala Val Gln Ser Ala Phe Val Met Arg Thr Asp Leu Asn Lys
            980                 985                 990

Ala Asp Lys Leu Val Ile Asn Lys  Ser Ala Thr Gly His  Asp Asn Ser
        995                 1000                 1005

Ile Trp  Val Asn Phe Leu Lys  Lys Pro Ser Asp Lys  Asp Thr Leu
    1010                 1015                 1020

Asp Ile  Pro Leu Val Ser Ala  Pro Glu Ala Thr Ala  Asp Asn Leu
    1025                 1030                 1035

Phe Arg Ala Ser Thr Arg Val Val Gly Phe Ser Asp Val Thr Pro
    1040                1045                1050

Thr Leu Ser Val Arg Lys Glu Asp Gly Lys Lys Glu Trp Val Leu
    1055                1060                1065

Asp Gly Tyr Gln Val Ala Arg Asn Asp Gly Gln Gly Lys Ala Ala
    1070                1075                1080

Ala Thr Phe Met His Ile Ser Tyr Asn Asn Phe Ile Thr Glu Val
    1085                1090                1095

Asn Asn Leu Asn Lys Arg Met Gly Asp Leu Arg Asp Ile Asn Gly
    1100                1105                1110

Glu Ala Gly Thr Trp Val Arg Leu Leu Asn Gly Ser Gly Ser Ala
    1115                1120                1125

Asp Gly Gly Phe Thr Asp His Tyr Thr Leu Leu Gln Met Gly Ala
    1130                1135                1140

Asp Arg Lys His Glu Leu Gly Ser Met Asp Leu Phe Thr Gly Val
    1145                1150                1155

Met Ala Thr Tyr Thr Asp Thr Asp Ala Ser Ala Gly Leu Tyr Ser
    1160                1165                1170

Gly Lys Thr Lys Ser Trp Gly Gly Gly Phe Tyr Ala Ser Gly Leu
    1175                1180                1185

Phe Arg Ser Gly Ala Tyr Phe Asp Leu Ile Ala Lys Tyr Ile His
    1190                1195                1200

Asn Glu Asn Lys Tyr Asp Leu Asn Phe Ala Gly Ala Gly Lys Gln
    1205                1210                1215

Asn Phe Arg Ser His Ser Leu Tyr Ala Gly Ala Glu Val Gly Tyr
    1220                1225                1230

Arg Tyr His Leu Thr Asp Thr Thr Phe Val Glu Pro Gln Ala Glu
    1235                1240                1245

Leu Val Trp Gly Arg Leu Gln Gly Gln Thr Phe Asn Trp Asn Asp
    1250                1255                1260

Ser Gly Met Asp Val Ser Met Arg Arg Asn Ser Val Asn Pro Leu
    1265                1270                1275

Val Gly Arg Thr Gly Val Val Ser Gly Lys Thr Phe Ser Gly Lys
    1280                1285                1290

Asp Trp Ser Leu Thr Ala Arg Ala Gly Leu His Tyr Glu Phe Asp
    1295                1300                1305

Leu Thr Asp Ser Ala Asp Val His Leu Lys Asp Ala Ala Gly Glu
    1310                1315                1320

His Gln Ile Asn Gly Arg Lys Asp Gly Arg Met Leu Tyr Gly Val
    1325                1330                1335

Gly Leu Asn Ala Arg Phe Gly Asp Asn Thr Arg Leu Gly Leu Glu
    1340                1345                1350

Val Glu Arg Ser Ala Phe Gly Lys Tyr Asn Thr Asp Asp Ala Ile
    1355                1360                1365

Asn Ala Asn Ile Arg Tyr Ser Phe
    1370                1375

<210> 27

<211> 349

<212> PRT

<213> Escherichia coli

<400> 27

Met Ile Thr Leu Phe Arg Leu Leu Ala Ile Leu Cys Leu Phe Phe Asn
1               5                   10                  15

Val Ser Ala Phe Ala Val Asp Cys Tyr Gln Asp Gly Tyr Arg Gly Thr
            20                  25                  30

Thr Leu Ile Asn Gly Asp Leu Pro Thr Phe Lys Ile Pro Glu Asn Ala
        35                  40                  45

Gln Pro Gly Gln Lys Ile Trp Glu Ser Gly Asp Ile Asn Ile Thr Val
    50                  55                  60

Tyr Cys Asp Asn Ala Pro Gly Trp Ser Ser Asn Asn Pro Ser Glu Asn
65                  70                  75                  80

Val Tyr Ala Trp Ile Lys Leu Pro Gln Ile Asn Ser Ala Asp Met Leu
                85                  90                  95

Asn Asn Pro Tyr Leu Thr Phe Gly Val Thr Tyr Asn Gly Val Asp Tyr
            100                 105                 110

Glu Gly Thr Asn Glu Lys Ile Asp Thr His Ala Cys Leu Asp Lys Tyr
        115                 120                 125

Glu Gln Tyr Tyr Asn Gly Tyr Tyr His Asp Pro Val Cys Asn Gly Ser
    130                 135                 140

Thr Leu Gln Lys Asn Val Thr Phe Asn Ala His Phe Arg Val Tyr Val
145                 150                 155                 160

Lys Phe Lys Ser Arg Pro Ala Gly Asp Gln Thr Val Asn Phe Gly Thr
                165                 170                 175

Val Asn Val Leu Gln Phe Asp Gly Glu Gly Gly Ala Asn Met Ala Pro
            180                 185                 190

Asn Ala Lys Asn Leu Arg Tyr Ala Ile Thr Gly Leu Asp Asn Ile Ser
        195                 200                 205

Phe Leu Asp Cys Ser Val Asp Val Arg Ile Ser Pro Glu Ser Gln Ile
    210                 215                 220

Val Asn Phe Gly Gln Ile Ala Ala Asn Ser Ile Ala Thr Phe Pro Pro
225                 230                 235                 240

Lys Ala Ala Phe Ser Val Ser Thr Ile Lys Asp Ile Ala Ser Asp Cys
                245                 250                 255

Thr Glu Gln Phe Asp Val Ala Thr Ser Phe Phe Thr Ser Asp Thr Leu
            260                 265                 270

Tyr Asp Asn Thr His Leu Glu Ile Gly Asn Gly Leu Leu Met Arg Ile
        275                 280                 285

Thr Asp Gln Lys Thr Gln Glu Asp Ile Lys Phe Asn Gln Phe Lys Leu
    290                 295                 300

Phe Ser Thr Tyr Ile Pro Gly Gln Ser Ala Ala Met Ala Thr Arg Asp
305                 310                 315                 320

Tyr Gln Ala Glu Leu Thr Gln Lys Pro Gly Glu Pro Leu Val Tyr Gly
                325                 330                 335

Pro Phe Gln Lys Asp Leu Ile Val Lys Ile Asn Tyr His
            340                 345

<210>  28

<211>  840

<212>  PRT

<213>  Escherichia coli


<400>  28

Met Asn Asn Lys Asn Thr Phe Ser Arg Asp Lys Leu Ser His Ala Ile
1               5                   10                  15

Lys Asn Ala Leu Ser Gly Val Val Cys Ser Leu Leu Phe Val Leu Pro
            20                  25                  30

Val His Ala Val Glu Phe Asn Val Asp Met Ile Asp Ala Glu Asp Arg
        35                  40                  45

Glu Asn Ile Asp Ile Ser Arg Phe Glu Lys Lys Gly Tyr Ile Pro Pro
    50                  55                  60

Gly Arg Tyr Leu Val Arg Val Gln Ile Asn Lys Asn Met Leu Pro Gln
65                  70                  75                  80

Thr Leu Ile Leu Glu Trp Val Lys Ala Asp Asn Glu Ser Gly Ser Leu
                85                  90                  95

Leu Cys Leu Thr Lys Glu Asn Leu Thr Asn Phe Gly Leu Asn Thr Glu
            100                 105                 110

Phe Ile Glu Ser Leu Gln Asn Ile Ala Gly Ser Glu Cys Leu Asp Leu
        115                 120                 125

Ser Gln Arg Gln Glu Leu Thr Thr Arg Leu Asp Lys Ala Thr Met Ile
    130                 135                 140

Leu Ser Leu Ser Val Pro Gln Ala Trp Leu Lys Tyr Gln Ala Thr Asn
145                 150                 155                 160

Trp Thr Pro Pro Glu Phe Trp Asp Thr Gly Ile Thr Gly Phe Ile Leu
                165                 170                 175

Asp Tyr Asn Val Tyr Ala Ser Gln Tyr Ala Pro His His Gly Asp Ser
            180                 185                 190

Thr Gln Asn Val Ser Ser Tyr Gly Thr Leu Gly Phe Asn Leu Gly Ala
        195                 200                 205

Trp Arg Leu Arg Ser Asp Tyr Gln Tyr Asn Gln Asn Phe Ala Asp Gly
    210                 215                 220

Arg Ser Val Asn Arg Asp Ser Glu Phe Ala Arg Thr Tyr Leu Phe Arg
225                 230                 235                 240

Pro Ile Pro Ser Trp Ser Ser Lys Phe Thr Met Gly Gln Tyr Asp Leu
                245                 250                 255

Ser Ser Asn Leu Tyr Asp Thr Phe His Phe Thr Gly Ala Ser Leu Glu
            260                 265                 270

Ser Asp Glu Ser Met Leu Pro Pro Asp Leu Gln Gly Tyr Ala Pro Gln
        275                 280                 285

Ile Thr Gly Ile Ala Gln Thr Asn Ala Lys Val Thr Val Ala Gln Asn
    290                 295                 300

Gly Arg Val Leu Tyr Gln Thr Thr Val Ala Pro Gly Pro Phe Thr Ile
305                 310                 315                 320

Ser Asp Leu Gly Gln Ser Phe Gln Gly Gln Leu Asp Val Thr Val Glu
                325                 330                 335

Glu Glu Asp Gly Arg Thr Ser Thr Phe Gln Val Gly Ser Ala Ser Ile
            340                 345                 350

Pro Tyr Leu Thr Arg Lys Gly Gln Val Arg Tyr Lys Thr Ser Leu Gly
        355                 360                 365

Lys Pro Thr Ser Val Gly His Asn Asp Ile Asn Asn Pro Phe Phe Trp
    370                 375                 380

Thr Ala Glu Ala Ser Trp Gly Trp Leu Asn Asn Val Ser Leu Tyr Gly
385                 390                 395                 400

Gly Gly Met Phe Thr Ala Asp Asp Tyr Gln Ala Ile Thr Thr Gly Ile
                405                 410                 415

Gly Phe Asn Leu Asn Gln Phe Gly Ser Leu Ser Phe Asp Val Thr Gly
            420                 425                 430

Ala Asp Ala Ser Leu Gln Gln Gln Asn Ser Gly Asn Leu Arg Gly Tyr
        435                 440                 445

Ser Tyr Arg Phe Asn Tyr Ala Lys His Phe Glu Ser Thr Gly Ser Gln
    450                 455                 460

Ile Thr Phe Ala Gly Tyr Arg Phe Ser Asp Lys Asp Tyr Val Ser Met
465                 470                 475                 480

Ser Glu Tyr Leu Ser Ser Arg Asn Gly Asp Glu Ser Ile Asp Asn Glu
                485                 490                 495

Lys Glu Ser Tyr Val Ile Ser Leu Asn Gln Tyr Phe Glu Thr Leu Glu
            500                 505                 510

Leu Asn Ser Tyr Leu Asn Val Thr Arg Asn Thr Tyr Trp Asp Ser Ala
        515                 520                 525

Ser Asn Thr Asn Tyr Ser Val Ser Val Ser Lys Asn Phe Asp Ile Gly
    530                 535                 540

Asp Phe Lys Gly Ile Ser Ala Ser Leu Ala Val Ser Arg Ile Arg Trp
545                 550                 555                 560

Asp Asp Asp Glu Glu Asn Gln Tyr Tyr Phe Ser Phe Ser Leu Pro Leu
                565                 570                 575

Gln Gln Asn Arg Asn Ile Ser Tyr Ser Met Gln Arg Thr Gly Ser Ser
            580                 585                 590

Asn Thr Ser Gln Met Ile Ser Trp Tyr Asp Ser Ser Asp Arg Asn Asn
        595                 600                 605

Ile Trp Asn Ile Ser Ala Ser Ala Thr Asp Asp Asn Ile Arg Asp Gly
    610                 615                 620

Glu Pro Thr Leu Arg Gly Ser Tyr Gln His Tyr Ser Pro Trp Gly Arg
625                 630                 635                 640

Leu Asn Ile Asn Gly Ser Val Gln Pro Asn Gln Tyr Asn Ser Val Thr
                645                 650                 655

Ala Gly Trp Tyr Gly Ser Leu Thr Ala Thr Arg His Gly Val Ala Leu
            660                 665                 670

His Asp Tyr Ser Tyr Gly Asp Asn Ala Arg Met Met Val Asp Thr Asp
        675                 680                 685

Gly Ile Ser Gly Ile Glu Ile Asn Ser Asn Arg Thr Val Thr Asn Gly
    690                 695                 700

Leu Gly Ile Ala Val Ile Pro Ser Leu Ser Asn Tyr Thr Thr Ser Met
705                 710                 715                 720

Leu Arg Val Asn Asn Asn Asp Leu Pro Glu Gly Val Asp Val Glu Asn
                725                 730                 735

Ser Val Ile Arg Thr Thr Leu Thr Gln Gly Ala Ile Gly Tyr Ala Lys
            740                 745                 750

Leu Asn Ala Thr Thr Gly Tyr Gln Ile Val Gly Val Ile Arg Gln Glu
        755                 760                 765

Asn Gly Arg Phe Pro Pro Leu Gly Val Asn Val Thr Asp Lys Ala Thr
    770                 775                 780

Gly Lys Asp Val Gly Leu Val Ala Glu Asp Gly Phe Val Tyr Leu Ser
785                 790                 795                 800

Gly Ile Gln Glu Asn Ser Ile Leu His Leu Thr Trp Gly Asp Asn Thr
                805                 810                 815

Cys Glu Val Thr Pro Pro Asn Gln Ser Asn Ile Ser Glu Ser Ala Ile
            820                 825                 830

Ile Leu Pro Cys Lys Thr Val Lys
        835                 840

<210>  29

<211>  169

<212>  PRT

<213>  Escherichia coli

<400>  29

Leu Met Asn Thr Lys Gln Ser Val Ala Gln Leu Ala Val Pro His Arg
1               5                   10                  15

Lys Arg Leu Ser Ser Thr Met Val Val Ala Leu Leu Leu Cys Val Val
            20                  25                  30

Ala Gly Ala Val Met Ile Asn Ala Ala Asp Phe Pro Ala Thr Ala Ile
        35                  40                  45

Glu Thr Asp Pro Gly Ala Ser Ala Phe Pro Thr Phe Tyr Ala Cys Ala
    50                  55                  60

Leu Ile Val Leu Ala Val Leu Leu Val Ile Arg Asp Leu Leu Gln Ala
65                  70                  75                  80

Lys Pro Ala Ser Cys Ala Asn Ala Gln Glu Lys Pro Ala Phe Arg Lys
                85                  90                  95

Thr Ala Thr Gly Ile Ala Ala Thr Ala Phe Tyr Ile Val Ala Met Ser
            100                 105                 110

Tyr Cys Gly Tyr Leu Ile Thr Thr Pro Val Phe Leu Ile Val Ile Met
        115                 120                 125

Thr Leu Met Gly Tyr Arg Arg Trp Val Leu Thr Pro Gly Ile Ala Leu
    130                 135                 140

Leu Leu Thr Ala Ile Leu Trp Leu Leu Phe Val Glu Ala Leu Gln Val
145                 150                 155                 160

Pro Leu Pro Val Gly Thr Phe Phe Glu
                165

<210>  30

<211>  311

<212>  PRT

<213>  Escherichia coli

<400>  30

Met Val Leu Leu Ala Gly Ala Ala Leu Ser Ile Ala Pro Val Gln Ala
1               5                   10                  15

Ala Ser Tyr Pro Thr Lys Gln Ile Glu Leu Val Val Pro Tyr Ala Ala
            20                  25                  30

Gly Gly Gly Thr Asp Leu Val Ala Arg Ala Phe Ala Asp Ala Ala Lys
        35                  40                  45

Asn His Leu Pro Val Ser Ile Gly Val Ile Asn Lys Pro Gly Gly Gly
    50                  55                  60

Gly Ala Ile Gly Leu Ser Glu Ile Ala Ala Ala Arg Pro Asn Gly Tyr
65                  70                  75                  80

Lys Ile Gly Leu Gly Thr Val Glu Leu Thr Thr Leu Pro Ser Leu Gly
                85                  90                  95

Met Val Arg Phe Lys Thr Ser Asp Phe Lys Pro Ile Ala Arg Leu Asn
            100                 105                 110

Ala Asp Pro Ala Ala Ile Thr Val Arg Ala Asp Ala Pro Trp Asn Ser
        115                 120                 125

Tyr Glu Glu Phe Met Ala Tyr Ser Lys Ala Asn Pro Gly Lys Val Arg
    130                 135                 140

Ile Gly Asn Ser Gly Thr Gly Ala Ile Trp His Leu Ala Ala Ala Ala
145                 150                 155                 160

Leu Glu Asp Lys Thr Gly Thr Lys Phe Ser His Val Pro Tyr Asp Gly
                165                 170                 175

Ala Ala Pro Ala Ile Thr Gly Leu Leu Gly Gly His Ile Glu Ala Val
            180                 185                 190

Ser Val Ser Pro Gly Glu Val Ile Asn His Val Asn Gly Gly Lys Leu
        195                 200                 205

Lys Thr Leu Val Val Met Ala Asp Glu Arg Met Lys Thr Met Pro Asp
    210                 215                 220

Val Pro Thr Leu Lys Glu Lys Gly Val Asp Leu Ser Ile Gly Thr Trp
225                 230                 235                 240

Arg Gly Leu Ile Val Ser Gln Lys Thr Pro Gln Asp Val Val Asp Val
                245                 250                 255

Leu Ala Lys Ala Ala Lys Glu Thr Ala Glu Glu Pro Ala Phe Gln Asp
            260                 265                 270

Ala Leu Gln Lys Leu Asn Leu Asn Tyr Ala Trp Leu Asp Ala Ala Ser
        275                 280                 285

Phe Gln Thr Gln Ile Ser Glu Gln Glu Lys Tyr Phe Asp Glu Leu Leu
    290                 295                 300

Thr Arg Leu Gly Leu Lys Lys
305                 310

<210> 31

<211> 722

<212> PRT

<213> Escherichia coli

<400> 31

Met Leu Arg Trp Lys Arg Cys Ile Ile Leu Thr Phe Ile Ser Gly Ala
1               5                   10                  15

Ala Phe Ala Ala Pro Glu Ile Asn Val Lys Gln Asn Glu Ser Leu Pro
            20                  25                  30

Asp Leu Gly Ser Gln Ala Ala Gln Gln Asp Glu Gln Thr Asn Lys Gly
        35                  40                  45

Lys Ser Leu Lys Glu Arg Gly Ala Asp Tyr Val Ile Asn Ser Ala Thr
    50                  55                  60

Gln Gly Phe Glu Asn Leu Thr Pro Glu Ala Leu Glu Ser Gln Ala Arg
65                  70                  75                  80

Ser Tyr Leu Gln Ser Gln Ile Thr Ser Thr Ala Gln Ser Tyr Ile Glu
                85                  90                  95

Asp Thr Leu Ser Pro Tyr Gly Lys Val Arg Leu Asn Leu Ser Ile Gly
            100                 105                 110

Gln Gly Gly Asp Leu Asp Gly Ser Ser Ile Asp Tyr Phe Val Pro Trp
        115                 120                 125

Tyr Asp Asn Gln Thr Thr Val Tyr Phe Ser Gln Phe Ser Ala Gln Arg
    130                 135                 140

Lys Glu Asp Arg Thr Ile Gly Asn Ile Gly Leu Gly Val Arg Tyr Asn
145                 150                 155                 160

Phe Asp Lys Tyr Leu Leu Gly Gly Asn Ile Phe Tyr Asp Tyr Asp Phe
                165                 170                 175

Thr Arg Gly His Arg Arg Leu Gly Leu Gly Ala Glu Ala Trp Thr Asp
            180                 185                 190

Tyr Leu Lys Phe Ser Gly Asn Tyr Tyr His Pro Leu Ser Asp Trp Lys
        195                 200                 205

Asp Ser Glu Asp Phe Asp Phe Tyr Glu Glu Arg Pro Ala Arg Gly Trp
    210                 215                 220

Asp Ile Arg Ala Glu Val Trp Leu Pro Ser Tyr Pro Gln Leu Gly Gly
225                 230                 235                 240

Lys Ile Val Phe Glu Gln Tyr Tyr Gly Asp Glu Val Ala Leu Phe Gly
                245                 250                 255

Thr Asp Asn Leu Glu Lys Asp Pro Tyr Ala Val Thr Leu Gly Leu Asn
            260                 265                 270

Tyr Gln Pro Val Pro Leu Leu Thr Val Gly Thr Asp Tyr Lys Ala Gly
        275                 280                 285

Thr Gly Asp Asn Ser Asp Val Ser Ile Asn Ala Thr Leu Asn Tyr Gln
    290                 295                 300

Phe Gly Val Pro Leu Lys Asp Gln Leu Asp Ser Asp Lys Val Lys Ala
305                 310                 315                 320

Ala His Ser Leu Met Gly Ser Arg Leu Asp Phe Val Glu Arg Asn Asn
                325                 330                 335

Phe Ile Val Leu Glu Tyr Lys Glu Lys Asp Pro Leu Asp Val Thr Leu
            340                 345                 350

Trp Leu Lys Ala Asp Ala Thr Asn Glu His Pro Glu Cys Val Ile Lys
        355                 360                 365

Asp Thr Pro Glu Ala Ala Val Gly Leu Glu Lys Cys Lys Trp Thr Ile
    370                 375                 380

Asn Ala Leu Ile Asn His His Tyr Lys Ile Val Ala Ala Ser Trp Gln
385                 390                 395                 400

Ala Lys Asn Asn Ala Ala Arg Thr Leu Val Met Pro Val Ile Lys Glu
                405                 410                 415

Asn Thr Leu Thr Glu Gly Asn Asn Asn His Trp Asn Leu Val Leu Pro
            420                 425                 430

Ala Trp Gln Tyr Ser Ser Asp Gln Ala Glu Gln Glu Lys Leu Asn Thr
        435                 440                 445

Trp Arg Val Arg Leu Ala Leu Glu Asp Glu Lys Gly Asn Arg Gln Asn
    450                 455                 460

Ser Gly Val Val Glu Ile Thr Val Gln Gln Asp Arg Lys Ile Glu Leu
465                 470                 475                 480

Ile Val Asn Asn Ile Ala Asn Pro Glu Glu Asn Asn His Ser His Glu
                485                 490                 495

Ala Ser Ala Gln Ala Asp Gly Val Asp Gly Val Val Met Asp Leu Asp
            500                 505                 510

Val Thr Asp Ser Phe Gly Asp Asn Thr Asp Arg Asn Gly Asp Ala Leu
        515                 520                 525

Pro Glu Asp Asn Leu Thr Pro Gln Leu Tyr Asp Ala Gln Asp Lys Arg
    530                 535                 540

Val Thr Leu Thr Asn Lys Pro Cys Ser Thr Asp Asn Pro Cys Val Phe
545                 550                 555                 560

Ile Ala Lys Gln Asp Lys Glu Lys Gly Thr Val Thr Leu Ser Ser Thr
                565                 570                 575

Leu Pro Gly Thr Tyr Arg Trp Lys Ala Lys Ala Ala Pro Tyr Asp Asp
            580                 585                 590

Ser Asn Tyr Val Asp Val Thr Phe Leu Gly Ala Glu Ile Gly Gly Leu
        595                 600                 605

Asn Ala Phe Ile Tyr Arg Val Gly Ala Ala Lys Pro Ser Asn Leu Ile
    610                 615                 620

Gly Lys Asp Lys Glu Pro Leu Pro Ser Thr Thr Phe Ile Asp Leu Phe
625                 630                 635                 640

Tyr Gly Ala Thr Thr Ile Lys Thr Val Ser Ser Ser Arg Ser Lys Asn
                645                 650                 655

Leu Thr Lys Arg Trp Cys Ser Thr Thr Thr Ser Gly Asn Leu Pro Ala
            660                 665                 670

Arg Ala Ser Met Val Ser Gly Cys Thr Gly Glu His Ser Asn Glu Asp
        675                 680                 685

Ile Val Ile Pro Ala Thr Asn Arg Glu Ala Ala Gln Thr Tyr Gly Ala
    690                 695                 700

Gln Ala Gly Asp Gly Leu Gln Gly Tyr Gly Leu Arg Val Leu Tyr Thr
705                 710                 715                 720

Lys Lys

<210>  32

<211>  319

<212>  PRT

<213>  Escherichia coli

<400> 32

Met Lys Gln Asp Lys Arg Arg Gly Leu Thr Arg Ile Ala Leu Ala Leu
1               5                   10                  15

Ala Leu Ala Gly Tyr Cys Val Ala Pro Val Ala Leu Ala Glu Asp Ser
            20                  25                  30

Ala Trp Val Asp Ser Gly Glu Thr Asn Ile Phe Gln Gly Thr Ile Pro
        35                  40                  45

Trp Leu Tyr Ser Glu Gly Gly Ser Ala Thr Thr Asp Ala Asp Arg Val
    50                  55                  60

Thr Leu Thr Ser Asp Leu Lys Gly Ala Arg Pro Gln Gly Met Lys Arg
65                  70                  75                  80

Thr Ser Val Phe Thr Arg Val Ile Asn Ile Gly Asp Thr Glu Gly Asp
                85                  90                  95

Val Asp Leu Gly Gly Leu Gly Asp Asn Ala Lys Thr Ile Asp Thr Ile
            100                 105                 110

Arg Trp Met Ser Tyr Lys Asp Ala Gln Gly Gly Asp Pro Lys Glu Leu
        115                 120                 125

Ala Thr Lys Val Thr Ser Tyr Thr Leu Thr Asp Ala Asp Arg Gly Arg
    130                 135                 140

Tyr Ile Gly Ile Glu Ile Thr Pro Thr Thr Gln Thr Gly Thr Pro Asn
145                 150                 155                 160

Val Gly Thr Ala Leu His Leu Tyr Asp Val Ser Thr Ala Ser Gly Gly
                165                 170                 175

Gly Ser Asp Ser Asp Asn Val Ala Pro Gly Pro Val Val Asn Gln Asn
            180                 185                 190

Leu Lys Val Ala Ile Phe Val Asp Gly Thr Ser Ile Asn Leu Ile Asn
        195                 200                 205

Gly Ser Thr Pro Ile Glu Leu Gly Lys Thr Tyr Val Ala Lys Leu Tyr
    210                 215                 220

Ser Asp Glu Asn Lys Asn Gly Lys Phe Asp Ala Gly Thr Asp Ala Asp
225                 230                 235                 240

Val Thr Ala Asn Tyr Asp Phe Arg Trp Val Leu Ser Gly Ser Ser Gln
                245                 250                 255

Gln Leu Gly Thr Ser Gly Gly Ile Val Asn Ser Ser Phe Asp Asn Asn
            260                 265                 270

Asn Leu Val Ile Pro Ala Thr Asn Asp Glu Ala Arg Thr Asn Leu Asn
        275                 280                 285

Gly Pro Ala Arg Asp Gly Lys Glu Ala Leu Ser Ile Pro Thr Asn Gly
    290                 295                 300

Asp Gly Val Gln Gly Tyr Lys Leu His Ile Ile Tyr Lys His Lys
305                 310                 315

<210>  33

<211>  629

<212>  PRT

<213>  Escherichia coli

<400>  33

Met Lys Lys Val Leu Thr Leu Ser Leu Leu Ala Leu Cys Val Ser His
1               5                   10                  15

Ser Ala Val Ala Ala Asn Tyr Thr Phe Asn Asn Asp Asn Ile Ala Leu
            20                  25                  30

Ser Phe Asp Asp Thr Asn Ser Thr Ile Val Leu Lys Asp Arg Arg Thr
        35                  40                  45

Asn His Pro Ile Thr Pro Gln Glu Leu Phe Phe Leu Thr Leu Pro Asp
    50                  55                  60

Glu Thr Lys Ile His Thr Ala Asp Phe Lys Ile Lys His Ile Lys Lys
65                  70                  75                  80

Gln Asp Asn Ala Ile Val Ile Asp Phe Thr Arg Pro Asp Phe Asn Val
                85                  90                  95

Thr Val Gln Leu Asn Leu Val Lys Gly Lys Tyr Ala Ser Ile Asp Tyr
            100                 105                 110

Thr Ile Ala Ala Val Gly Gln Pro Arg Asp Val Ala Lys Ile Thr Phe
        115                 120                 125

Phe Pro Thr Lys Lys Gln Phe Gln Ala Pro Tyr Val Asp Gly Ala Ile
    130                 135                 140

Thr Ser Ser Pro Ile Ile Ala Asp Ser Phe Phe Ile Leu Pro Asn Lys
145                 150                 155                 160

Pro Ile Val Asn Thr Tyr Ala Tyr Glu Ala Thr Thr Asn Leu Asn Val
                165                 170                 175

Glu Leu Lys Thr Pro Ile Gln Pro Glu Thr Pro Val Ser Phe Thr Thr
            180                 185                 190

Trp Phe Gly Thr Phe Pro Glu Thr Ser Gln Leu Arg Arg Ser Val Asn
        195                 200                 205

Gln Phe Ile Asn Ala Val Arg Pro Arg Pro Tyr Lys Pro Tyr Leu His
    210                 215                 220

Tyr Asn Ser Trp Met Asp Ile Gly Phe Phe Thr Pro Tyr Thr Glu Gln
225                 230                 235                 240

Asp Val Leu Gly Arg Met Asp Glu Trp Asn Lys Glu Phe Ile Ser Gly
                245                 250                 255

Arg Gly Val Ala Leu Asp Ala Phe Leu Leu Asp Asp Gly Trp Asp Asp
            260                 265                 270

Leu Thr Gly Arg Trp Leu Phe Gly Pro Ala Phe Ser Asn Gly Phe Ser
        275                 280                 285

Lys Val Arg Glu Lys Ala Asp Ser Leu His Ser Ser Val Gly Leu Trp
    290                 295                 300

Leu Ser Pro Trp Gly Gly Tyr Asn Lys Pro Gln Arg Arg Ser Arg Phe
305                 310                 315                 320

Ala Cys Lys Arg Val Trp Val Arg Asn Arg Gly Arg Gln Ala Gly Ala
                325                 330                 335

Phe Gly Ser Glu Leu Leu Lys Asn Phe Asn Glu Gln Ile Ile Asn Leu
            340                 345                 350

Ile Lys Asn Glu His Ile Thr Ser Phe Lys Leu Asp Gly Met Gly Asn
        355                 360                 365

Ala Ser Ser His Ile Lys Gly Ser Pro Phe Ala Ser Asp Phe Asp Ala
    370                 375                 380

Ser Ile Ala Leu Leu His Asn Met Arg Arg Ala Asn Pro Asn Leu Phe
385                 390                 395                 400

Ile Asn Leu Thr Thr Gly Thr Asn Ala Ser Pro Ser Trp Leu Phe Tyr
                405                 410                 415

Ala Asp Ser Ile Trp Arg Gln Gly Asp Asp Ile Asn Leu Tyr Gly Pro
            420                 425                 430

Gly Thr Pro Val Gln Gln Trp Ile Thr Tyr Arg Asp Ala Glu Thr Tyr
        435                 440                 445

Arg Ser Ile Val Arg Lys Gly Pro Leu Phe Pro Leu Asn Ser Leu Met
    450                 455                 460

Tyr His Gly Ile Val Ser Ala Glu Asn Ala Tyr Tyr Gly Leu Glu Lys
465                 470                 475                 480

Val Gln Thr Asp Ser Asp Phe Ala Asp Gln Val Trp Ser Tyr Phe Ala
                485                 490                 495

Thr Gly Thr Gln Leu Gln Glu Leu Tyr Ile Thr Pro Ser Met Leu Asn
            500                 505                 510

Lys Val Lys Trp Asp Thr Leu Ala Lys Ala Ala Lys Trp Ser Lys Glu
        515                 520                 525

Asn Ala Ser Val Leu Val Asp Thr His Trp Ile Gly Gly Asp Pro Thr
    530                 535                 540

Ala Leu Ala Val Tyr Gly Trp Ala Ser Trp Ser Lys Asp Lys Ala Ile
545                 550                 555                 560

Leu Gly Leu Arg Asn Pro Ser Asp Lys Pro Gln Thr Tyr Tyr Leu Asp
                565                 570                 575

Leu Ala Lys Asp Phe Glu Ile Pro Ala Gly Asn Ala Ala Gln Phe Ser
            580                 585                 590

Leu Lys Ala Val Tyr Gly Ser Asn Lys Thr Val Pro Val Glu Tyr Lys
        595                 600                 605

Asn Ala Thr Val Ile Thr Leu Gln Pro Leu Glu Thr Leu Val Phe Glu
    610                 615                 620

Ala Val Thr Ile Asn
625

<210>  34

<211>  1778

<212>  PRT

<213>  Escherichia coli

<400>  34

Met Asn Lys Ile Phe Lys Val Ile Trp Asn Pro Ala Thr Gly Ser Tyr
1               5                   10                  15

Thr Val Ala Ser Glu Thr Ala Lys Ser Arg Gly Lys Lys Ser Gly Arg
            20                  25                  30

Ser Lys Leu Leu Ile Ser Ala Leu Val Ala Gly Gly Leu Leu Ser Ser
        35                  40                  45

Phe Gly Ala Ser Ala Asp Asn Tyr Thr Gly Gln Pro Thr Asp Tyr Gly
    50                  55                  60

Asp Gly Ser Ala Gly Asp Gly Trp Val Ala Ile Gly Lys Gly Ala Lys
65                  70                  75                  80

Ala Asn Thr Phe Met Asn Thr Ser Gly Ala Ser Thr Ala Leu Gly Tyr
                85                  90                  95

Asp Ala Ile Ala Glu Gly Glu Tyr Ser Ser Ala Ile Gly Ser Lys Thr
            100                 105                 110

Leu Ala Thr Gly Gly Ala Ser Met Ala Phe Gly Val Ser Ala Lys Ala
        115                 120                 125

Met Gly Asp Arg Ser Val Ala Leu Gly Ala Ser Ser Val Ala Asn Gly
    130                 135                 140

Asp Arg Ser Met Ala Phe Gly Arg Tyr Ala Lys Thr Asn Gly Phe Thr
145                 150                 155                 160

Ser Leu Ala Ile Gly Asp Ser Ser Leu Ala Asp Gly Glu Lys Thr Ile
                165                 170                 175

Ala Leu Gly Asn Thr Ala Lys Ala Tyr Glu Ile Met Ser Ile Ala Leu
            180                 185                 190

Gly Asp Asn Ala Asn Ala Ser Lys Glu Tyr Ala Met Ala Leu Gly Ala
        195                 200                 205

Ser Ser Lys Ala Gly Gly Ala Asp Ser Leu Ala Phe Gly Arg Lys Ser
    210                 215                 220

Thr Ala Asn Ser Thr Gly Ser Leu Ala Ile Gly Ala Asp Ser Ser Ser
225                 230                 235                 240

Ser Asn Asp Asn Ala Ile Ala Ile Gly Asn Lys Thr Gln Ala Leu Gly
                245                 250                 255

Val Asn Ser Met Ala Leu Gly Asn Ala Ser Gln Ala Ser Gly Glu Ser
            260                 265                 270

Ser Ile Ala Leu Gly Asn Thr Ser Glu Ala Ser Glu Gln Asn Ala Ile
        275                 280                 285

Ala Leu Gly Gln Gly Ser Ile Ala Ser Lys Val Asn Ser Ile Ala Leu
    290                 295                 300

Gly Ser Asn Ser Leu Ser Ser Gly Glu Asn Ala Ile Ala Leu Gly Glu
305                 310                 315                 320

Gly Ser Ala Ala Gly Gly Ser Asn Ser Leu Ala Phe Gly Ser Gln Ser
                325                 330                 335

Arg Ala Asn Gly Asn Asp Ser Val Ala Ile Gly Val Gly Ala Ala Ala
            340                 345                 350

Ala Thr Asp Asn Ser Val Ala Ile Gly Ala Gly Ser Thr Thr Asp Ala
        355                 360                 365

Ser Asn Thr Val Ser Val Gly Asn Ser Ala Thr Lys Arg Lys Ile Val
    370                 375                 380

Asn Met Ala Ala Gly Ala Ile Ser Asn Thr Ser Thr Asp Ala Ile Asn
385                 390                 395                 400

Gly Ser Gln Leu Tyr Thr Ile Ser Asp Ser Val Ala Lys Arg Leu Gly
                405                 410                 415

Gly Gly Ala Thr Val Gly Ser Asp Gly Thr Val Thr Ala Val Ser Tyr
            420                 425                 430

Ala Leu Arg Ser Gly Thr Tyr Asn Asn Val Gly Asp Ala Leu Ser Gly
        435                 440                 445

Ile Asp Asn Asn Thr Leu Gln Trp Asn Lys Thr Ala Gly Ala Phe Ser
    450                 455                 460

Ala Asn His Gly Ala Asn Ala Thr Asn Lys Ile Thr Asn Val Ala Lys
465                 470                 475                 480

Gly Thr Val Ser Ala Thr Ser Thr Asp Val Val Asn Gly Ser Gln Leu
                485                 490                 495

Tyr Asp Leu Gln Gln Asp Ala Leu Leu Trp Asn Gly Thr Ala Phe Ser
            500                 505                 510

Ala Ala His Gly Thr Glu Ala Thr Ser Lys Ile Thr Asn Val Thr Ala
        515                 520                 525

Gly Asn Leu Thr Ala Gly Ser Thr Asp Ala Val Asn Gly Ser Gln Leu
    530                 535                 540

Lys Thr Thr Asn Asp Asn Val Thr Thr Asn Thr Thr Asn Ile Ala Thr
545                 550                 555                 560

Asn Thr Thr Asn Ile Thr Asn Leu Thr Asp Ala Val Asn Gly Leu Gly
                565                 570                 575

Asp Asp Ser Leu Leu Trp Asn Lys Ala Ala Gly Ala Phe Ser Ala Ala
            580                 585                 590

His Gly Thr Glu Ala Thr Ser Lys Ile Thr Asn Val Thr Ala Gly Asn
        595                 600                 605

Leu Thr Ala Gly Ser Thr Asp Ala Val Asn Gly Ser Gln Leu Lys Thr
    610                 615                 620

Thr Asn Asp Asn Val Thr Thr Asn Thr Thr Asn Ile Ala Thr Asn Thr
625                 630                 635                 640

Thr Asn Ile Thr Asn Leu Thr Asp Ala Val Asn Gly Leu Gly Asp Asp
                645                 650                 655

Ser Leu Leu Trp Asn Lys Thr Ala Gly Ala Phe Ser Ala Ala His Gly
            660                 665                 670

Thr Asp Ala Thr Ser Lys Ile Thr Asn Val Thr Ala Gly Asn Leu Thr
        675                 680                 685

Ala Gly Ser Thr Asp Ala Val Asn Gly Ser Gln Leu Lys Thr Thr Asn
    690                 695                 700

Asp Asn Val Thr Thr Asn Thr Thr Asn Ile Ala Thr Asn Thr Thr Asn
705                 710                 715                 720

Ile Thr Asn Leu Thr Asp Ala Val Asn Gly Leu Gly Asp Asp Ser Leu
                    725                 730                 735

Leu Trp Asn Lys Thr Ala Gly Ala Phe Ser Ala Ala His Gly Thr Asp
                740                 745                 750

Ala Thr Ser Lys Ile Thr Asn Val Lys Ala Gly Asp Leu Thr Ala Gly
        755                 760                 765

Ser Thr Asp Ala Val Asn Gly Ser Gln Leu Lys Thr Thr Asn Asp Asn
    770                 775                 780

Val Ser Thr Asn Thr Thr Asn Ile Thr Asn Leu Thr Asp Ala Val Asn
785                 790                 795                 800

Gly Leu Gly Asp Asp Ser Leu Leu Trp Asn Lys Thr Ala Gly Ala Phe
                805                 810                 815

Ser Ala Ala His Gly Thr Asp Ala Thr Ser Lys Ile Thr Asn Val Lys
            820                 825                 830

Ala Gly Asp Leu Thr Ala Gly Ser Thr Asp Ala Val Asn Gly Ser Gln
        835                 840                 845

Leu Lys Thr Thr Asn Asp Asn Val Ser Thr Asn Thr Thr Asn Ile Thr
    850                 855                 860

Asn Leu Thr Asp Ser Val Gly Asp Leu Lys Asp Asp Ser Leu Leu Trp
865                 870                 875                 880

Asn Lys Ala Ala Gly Ala Phe Ser Ala Ala His Gly Thr Glu Ala Thr
                885                 890                 895

Ser Lys Ile Thr Asn Leu Leu Ala Gly Lys Ile Ser Ser Asn Ser Thr
            900                 905                 910

Asp Ala Ile Asn Gly Ser Gln Leu Tyr Gly Val Ala Asp Ser Phe Thr
        915                 920                 925

Ser Tyr Leu Gly Gly Gly Ala Asp Ile Ser Asp Thr Gly Val Leu Ser
    930                 935                 940

Gly Pro Thr Tyr Thr Ile Gly Gly Thr Asp Tyr Thr Asn Val Gly Asp
945                 950                 955                 960

Ala Leu Ala Ala Ile Asn Thr Ser Phe Ser Thr Ser Leu Gly Asp Ala
                965                 970                 975

Leu Leu Trp Asp Ala Thr Ala Gly Lys Phe Ser Ala Lys His Gly Ile
            980                 985                 990

Asn Asn Ala Pro Ser Val Ile Thr Asp Val Ala Asn Gly Ala Val Ser
        995                 1000                1005

Ser Thr Ser Ser Asp Ala Ile Asn Gly Ser Gln Leu Tyr Gly Val
    1010                1015                1020

Ser Asp Tyr Ile Ala Asp Ala Leu Gly Gly Asn Ala Val Val Asn
    1025                1030                1035

Thr Asp Gly Ser Ile Thr Thr Pro Thr Tyr Ala Ile Ala Gly Gly
    1040                1045                1050

Ser Tyr Asn Asn Val Gly Asp Ala Leu Glu Ala Ile Asp Thr Thr
    1055                1060                1065

Leu Asp Asp Ala Leu Leu Trp Asp Thr Thr Ala Asn Gly Gly Asn
    1070                1075                1080

Gly Ala Phe Ser Ala Ala His Gly Lys Asp Lys Thr Ala Ser Val
    1085                1090                1095

Ile Thr Asn Val Ala Asn Gly Ala Val Ser Ala Thr Ser Asn Asp
    1100                1105                1110

Ala Ile Asn Gly Ser Gln Leu Tyr Ser Thr Asn Lys Tyr Ile Ala
    1115                1120                1125

Asp Ala Leu Gly Gly Asp Ala Glu Val Asn Ala Asp Gly Thr Ile
    1130                1135                1140

Thr Ala Pro Thr Tyr Thr Ile Ala Asn Thr Asp Tyr Asn Asn Val
    1145                1150                1155

Gly Glu Ala Leu Asp Ala Leu Asp Asn Asn Ala Leu Leu Trp Asp
    1160                1165                1170

Glu Asp Ala Gly Ala Tyr Asn Ala Ser His Asp Gly Asn Ala Ser
    1175                1180                1185

Lys Ile Thr Asn Val Ala Ala Gly Asp Leu Ser Thr Thr Ser Thr
    1190                1195                1200

Asp Ala Val Asn Gly Ser Gln Leu Asn Ala Thr Asn Ile Leu Val
    1205                1210                1215

Thr Gln Asn Ser Gln Met Ile Asn Gln Leu Ala Gly Asn Thr Ser
    1220                1225                1230

Glu Thr Tyr Ile Glu Glu Asn Gly Ala Gly Ile Asn Tyr Val Arg
    1235                1240                1245

Thr Asn Asp Ser Gly Leu Ala Phe Asn Asp Ala Ser Ala Ser Gly
    1250                1255                1260

Ile Gly Ala Thr Ala Val Gly Tyr Asn Ala Val Ala Ser His Ala
    1265                1270                1275

Ser Ser Val Ala Ile Gly Gln Asp Ser Ile Ser Glu Val Asp Thr
    1280                1285                1290

Gly Ile Ala Leu Gly Ser Ser Ser Val Ser Ser Arg Val Ile Val
    1295                1300                1305

Lys Gly Thr Arg Asn Thr Ser Val Ser Glu Glu Gly Val Val Ile
    1310                1315                1320

Gly Tyr Asp Thr Thr Asp Gly Glu Leu Leu Gly Ala Leu Ser Ile
    1325                1330                1335

Gly Asp Asp Gly Lys Tyr Arg Gln Ile Ile Asn Val Ala Asp Gly
    1340                1345                1350

Ser Glu Ala His Asp Ala Val Thr Val Arg Gln Leu Gln Asn Ala
    1355                1360                1365

Ile Gly Ala Val Ala Thr Thr Pro Thr Lys Tyr Tyr His Ala Asn
    1370                1375                1380

Ser Thr Ala Glu Asp Ser Leu Ala Val Gly Glu Asp Ser Leu Ala
    1385                1390                1395

Met Gly Ala Lys Thr Ile Val Asn Gly Asn Ala Gly Ile Gly Ile
    1400                1405                1410

Gly Leu Asn Thr Leu Val Leu Ala Asp Ala Ile Asn Gly Ile Ala
    1415                1420                1425

Ile Gly Ser Asn Ala Arg Ala Asn His Ala Asp Ser Ile Ala Met
    1430                1435                1440

Gly Asn Gly Ser Gln Thr Thr Arg Gly Ala Gln Thr Asn Tyr Thr
    1445                1450                1455

Ala Tyr Asn Met Asp Ala Pro Gln Asn Ser Val Gly Glu Phe Ser
    1460                1465                1470

Val Gly Ser Glu Asp Gly Gln Arg Gln Ile Thr Asn Val Ala Ala
    1475                1480                1485

Gly Ser Ala Asp Thr Asp Ala Val Asn Val Gly Gln Leu Lys Val
    1490                1495                1500

Thr Asp Ala Gln Val Ser Gln Asn Thr Gln Ser Ile Thr Asn Leu
    1505                1510                1515

Asn Thr Gln Val Thr Asn Leu Asp Thr Arg Val Thr Asn Ile Glu
    1520                1525                1530

Asn Gly Ile Gly Asp Ile Val Thr Thr Gly Ser Thr Lys Tyr Phe
    1535                1540                1545

Lys Thr Asn Thr Asp Gly Ala Asp Ala Asn Ala Gln Gly Lys Asp
    1550                1555                1560

Ser Val Ala Ile Gly Ser Gly Ser Ile Ala Ala Ala Asp Asn Ser
    1565                1570                1575

Val Ala Leu Gly Thr Gly Ser Val Ala Asp Glu Glu Asn Thr Ile
    1580                1585                1590

Ser Val Gly Ser Ser Thr Asn Gln Arg Arg Ile Thr Asn Val Ala
    1595                1600                1605

Ala Gly Val Asn Ala Thr Asp Ala Val Asn Val Ser Gln Leu Lys
    1610                1615                1620

Ser Ser Glu Ala Gly Gly Val Arg Tyr Asp Thr Lys Ala Asp Gly
    1625                1630                1635

Ser Ile Asp Tyr Ser Asn Ile Thr Leu Gly Gly Gly Asn Ser Gly
    1640                1645                1650

Thr Thr Arg Ile Ser Asn Val Ser Ala Gly Val Asn Asn Asn Asp
    1655                1660                1665

Ala Val Asn Tyr Ala Gln Leu Lys Gln Ser Val Gln Glu Thr Lys
    1670                1675                1680

Gln Tyr Thr Asp Gln Arg Met Val Glu Met Asp Asn Lys Leu Ser
    1685                1690                1695

Lys Thr Glu Ser Lys Leu Ser Gly Gly Ile Ala Ser Ala Met Ala
    1700                1705                1710

Met Thr Gly Leu Pro Gln Ala Tyr Thr Pro Gly Ala Ser Met Ala
    1715                1720                1725

Ser Ile Gly Gly Gly Thr Tyr Asn Gly Glu Ser Ala Val Ala Leu
    1730                1735                1740

Gly Val Ser Met Val Ser Ala Asn Gly Arg Trp Val Tyr Lys Leu
    1745                1750                1755

Gln Gly Ser Thr Asn Ser Gln Gly Glu Tyr Ser Ala Ala Leu Gly
    1760                1765                1770

Ala Gly Ile Gln Trp
    1775

<210> 35

<211> 227

<212> PRT

<213> Escherichia coli

<400> 35

Met Asn Leu Lys Lys Thr Leu Leu Ser Val Leu Met Ile Leu Gln Leu
1               5                   10                  15

Cys Leu Leu Val Gly Cys Asp Tyr Ile Glu Lys Ala Ser Lys Val Asp
            20                  25                  30

Asp Leu Val Thr Gln Gln Glu Leu Gln Lys Ser Lys Ile Glu Ala Leu
        35                  40                  45

Glu Lys Gln Gln Glu Leu Asp Lys Arg Lys Ile Glu His Phe Glu Lys
    50                  55                  60

Gln Gln Thr Thr Ile Ile Asn Ser Thr Lys Thr Leu Ala Gly Val Val
65                  70                  75                  80

Lys Ala Val Lys Asn Lys Gln Asp Glu Phe Val Phe Thr Glu Phe Asn
                85                  90                  95

Pro Ala Gln Thr Gln Tyr Phe Ile Leu Asn Asn Gly Ser Val Gly Leu
            100                 105                 110

Ala Gly Lys Ile Leu Ser Ile Asp Ala Val Glu Asn Gly Ser Val Ile
        115                 120                 125

Arg Ile Ser Leu Val Asn Leu Leu Ser Val Pro Val Ser Asn Met Gly
    130                 135                 140

Phe Tyr Ala Thr Trp Gly Gly Glu Lys Pro Thr Asp Ile Asn Ala Leu
145                 150                 155                 160

Ala Lys Trp Gln Gln Leu Leu Phe Ser Thr Ala Met Asn Ser Ser Leu
                165                 170                 175

Lys Leu Leu Pro Gly Gln Trp Gln Asp Ile Asn Leu Thr Leu Lys Gly
            180                 185                 190

Val Ser Pro Asn Asn Leu Lys Tyr Leu Lys Leu Ala Ile Asn Met Ala
        195                 200                 205

Asn Ile Gln Phe Asp Arg Leu Gln Pro Ala Glu Ser Pro Gln Arg Lys
    210                 215                 220

Asn Lys Lys
225

<210> 36

<211> 1109

<212> PRT

<213> Escherichia coli

<400> 36

Met Lys Arg Val Val Arg Leu Leu Gly Val Gly Leu Leu Leu Leu Val
1               5                   10                  15

Val Leu Leu Leu Ile Leu Phe Val Leu Ala Gln Thr Thr Pro Leu Ile
            20                  25                  30

Ser Ala Gln Asp Glu His Ala Val Trp Leu Arg Leu Leu Ile Thr Ala
        35                  40                  45

Ile Val Ile Cys Leu Leu Ser Met Cys Ile Phe Phe Leu Phe Ser Phe
    50                  55                  60

Arg Gln Asn Glu Ala Ser Thr Ile Ser Leu Tyr Ala Gln Pro Thr Asp
65                  70                  75                  80

Ile Lys Glu Ile Asn Thr Glu Gln Pro Asn Tyr Ala Ser Leu Leu Thr
                85                  90                  95

Ile Tyr Leu Arg Asp Arg Tyr Gly Pro Phe Trp Arg Arg Lys Val Arg
            100                 105                 110

Leu Leu Leu Val Thr Gly Glu Pro Glu Gln Ala Glu Ala Ile Ala Pro
        115                 120                 125

Gly Leu Thr Gly Gln His Trp Leu Glu Gly Asp His Thr Val Leu Ile
    130                 135                 140

Tyr Gly Gly Arg Pro Thr Ala Glu Pro Asp Val Thr Leu Leu Thr Ala
145                 150                 155                 160

Leu Lys Lys Leu Arg Arg Ser Arg Pro Leu Asp Gly Ile Ile Trp Ala
                165                 170                 175

Leu Thr Glu Glu Gln Ser Arg Gln Thr Ala Gln Leu Asp Lys Gly Trp
            180                 185                 190

Arg Gly Leu Ile Asn Gly Gly Lys Arg Leu Gly Phe Gln Ala Pro Leu
        195                 200                 205

Tyr Leu Trp Gln Val Cys Asp Asp Gly Asp Tyr Gln Thr Gly Arg Pro
    210                 215                 220

Leu Gln Ser Val Gly Cys Leu Leu Pro Glu Arg Cys Thr Pro Glu Gln
225                 230                 235                 240

Leu Ala Val Met Leu Glu Ala Ala Ala Asp Gly Thr Gly His Val Ala
                245                 250                 255

Ala Thr Asp Arg Tyr Arg Met Phe Ser Ala Ala Ser Gly Ser Tyr Pro
            260                 265                 270

Cys Arg Ala Gly Tyr Cys Ser Leu Ala Asp Arg Pro Glu Thr Ala Ala
        275                 280                 285

Gly Arg Arg Arg Ile Phe Phe Pro Ala Pro Ala Arg Pro Asp Val Gln
    290                 295                 300

Pro Ala Ala Cys Arg Arg Ala Gly Gly Gln His Leu Met Gln Trp Leu
305                 310                 315                 320

Pro Ser Pro Val Trp Ala Gly Val Thr Val Ile Thr Arg Ala Gly Ala
                325                 330                 335

Arg Trp Val Phe Leu Trp Leu Arg Thr Ala Leu Met Ser Ala Val Cys
            340                 345                 350

Val Leu Val Ile Trp Gly Ala Gly Met Thr Thr Ser Phe Phe Ala Asn
        355                 360                 365

Arg Ala Leu Val Gln Glu Thr Gly Ile Gln Thr Ala Arg Ala Leu Asp
    370                 375                 380

Thr Arg Leu Pro Leu Ala Glu Gln Leu Val Ala Leu His Thr Leu Gln
385                 390                 395                 400

Gly Glu Leu Glu Arg Leu Gln Tyr Arg Ile Arg Glu Gly Ala Pro Trp
                405                 410                 415

Tyr Gln Arg Phe Gly Leu Glu Arg Asn Gln Gln Leu Leu Ala Ala Ala
            420                 425                 430

Phe Pro Gly Tyr Ala Gln Ala Ala Asn Arg Leu Val Arg Asp Val Ala
        435                 440                 445

Val Asp His Leu Gln Gln Gln Leu Asn Ala Phe Val Ala Leu Pro Pro
    450                 455                 460

Asn Ser Pro Gln Arg Thr Ala Thr Gly Glu Gln Arg Tyr Lys Gln Leu
465                 470                 475                 480

Lys Ala Leu Leu Met Thr Ser Arg Pro Glu Lys Ala Asp Ala Ala Phe
                485                 490                 495

Phe Ser Thr Thr Leu Met Ala Asp Gly Leu Arg Tyr Glu Asn Ile Pro
            500                 505                 510

Glu Gly Val Arg Gln Ser Val Leu Pro Ser Leu Leu Thr Phe Trp Thr
        515                 520                 525

Ala Asn Leu Pro Glu His Pro Gln Trp Lys Thr Ser Pro Pro Pro Glu
    530                 535                 540

Leu Thr Gly Ala Val Arg Lys Ile Leu Leu Arg Gln Ile Gly Val Arg
545                 550                 555                 560

Asn Ala Glu Asn Thr Leu Tyr Gln Asn Val Leu Gln Gln Val Ser Arg
                565                 570                 575

Asn Tyr Ala Asp Met Thr Leu Ala Asp Met Thr Gly Asp Thr Leu Thr
            580                 585                 590

Glu Ser Leu Phe Ser Thr Glu Gln Thr Val Pro Gly Met Phe Thr Arg
        595                 600                 605

Gln Ala Trp Glu Gly Gln Val Arg Glu Ala Ile Glu Gln Val Val Thr
    610                 615                 620

Ala Arg Arg Glu Glu Ile Asp Trp Val Leu Ser Asp Arg Gln Gln Asp
625                 630                 635                 640

Thr Ser Ala Asp Ile Ser Pro Asp Thr Leu Arg Asn Arg Leu Thr Ser
                645                 650                 655

Arg Tyr Phe Thr Asp Phe Ala Gly Ser Trp Leu Ala Phe Leu Asn Ser
            660                 665                 670

Ile His Trp Lys Lys Glu Asp Ser Leu Ser Gly Ile Leu Asp Gln Leu
        675                 680                 685

Thr Leu Met Ala Asp Ala Arg Gln Ser Pro Leu Ile Ala Leu Thr Asp
    690                 695                 700

Thr Leu Ala Trp Gln Ala Ala Thr Gly Arg Glu Asn Arg Gly Leu Ser
705                 710                 715                 720

Asp Ser Leu Ala Lys Ser Ala Gln Glu Leu Phe Asn Gly Lys Glu Lys
                725                 730                 735

Thr Pro Gln Gln Ser Arg Glu Gly Asp Asp Val Pro Val Gly Pro Leu
            740                 745                 750

Asp Lys Thr Phe Thr Pro Leu Leu Arg Leu Leu Gly Asp Lys Ala Gly
        755                 760                 765

Gly Gly Asp Ser Gln Leu Ser Leu Gln Thr Tyr Leu Thr Arg Val Thr
    770                 775                 780

Arg Val Arg Leu Lys Leu Gln Gln Val Thr Asn Ala Pro Asp Pro Gln
785                 790                 795                 800

Glu Met Thr Gln Gln Leu Ala Gln Thr Val Leu Gln Gly Lys Thr Val
                805                 810                 815

Asp Leu Thr Asp Thr Arg Asp Tyr Gly Arg Leu Ile Ala Ala Ser Leu
            820                 825                 830

Gly Glu Glu Trp Ser Gly Phe Gly Gln Ala Leu Phe Val Arg Pro Val
        835                 840                 845

Glu Gln Ser Trp Arg Gln Val Leu Thr Pro Ala Ala Asp Ser Leu Asn
    850                 855                 860

Arg Gln Trp Gln Arg Ala Ile Val Ser His Trp Asn Gln Asp Phe Ala
865                 870                 875                 880

Gly Arg Tyr Pro Phe Lys Ala Ser Gln Asn Asp Ala Ser Leu Pro Leu
                885                 890                 895

Leu Ala Gln Tyr Leu Arg Asp Asp Gly Arg Ile Asn Leu Phe Ile Ala
            900                 905                 910

Ala Asn Leu Ser Gly Val Leu Lys Arg Glu Gly Arg Tyr Trp Val Ala
        915                 920                 925

Asp Ala Met Asn Thr Gln Gly Leu Thr Val Asn Pro Asp Phe Ile Arg
    930                 935                 940

Ala Leu Asn Arg Leu Arg Asp Val Ala Asp Thr Ala Phe Ala Ser Gly
945                 950                 955                 960

Asp Ala Gly Ile His Phe Glu Leu Arg Ala Lys Pro Ala Arg Asp Val
                965                 970                 975

Met Lys Thr His Leu Val Ile Asp Gly Gln Glu Leu Glu Tyr Phe Asn
            980                 985                 990

Gln Lys Glu Arg Trp Gln Arg Phe  Asn Trp Pro Asp Glu  Gln Trp Gln
        995                 1000                1005

Pro Gly  Ala Ser Leu Ser Trp  Thr Ser Thr Gln Ala  Met Glu Arg
    1010                1015                1020

Ile Leu  Ala Asp Tyr Arg Gly  Ser Trp Ser Leu Ile  Arg Leu Leu
    1025                1030                1035

Glu Gln  Ala Gln Val Thr Pro  Val Asp Ser Ser Thr  Phe Lys Val
    1040                1045                1050

Val Trp  Lys Ala Gln Asp Gly  Leu Pro Leu Asn Tyr  Leu Leu Arg
    1055                1060                1065

Val Glu  Gln Gly Lys Gly Pro  Leu Ala Leu Leu Glu  Leu Lys Asn
    1070                1075                1080

Phe Arg  Leu Pro Gly Gln Val  Phe Leu Thr Gly Lys  Ser Met Lys
    1085                1090                1095

Asp Val Glu Glu Tyr Gly Glu Asp Ala Asp Glu
    1100                1105

<210> 37

<211> 178

<212> PRT

<213> Escherichia coli

<400> 37

Met Phe Pro Ile Arg Phe Lys Arg Pro Ala Leu Leu Cys Met Ala Met
1               5                   10                  15

Leu Thr Val Val Leu Ser Gly Cys Gly Leu Ile Gln Lys Val Val Asp
            20                  25                  30

Glu Ser Lys Ser Val Ala Ser Ala Val Phe Tyr Lys Gln Ile Lys Ile
        35                  40                  45

Leu His Leu Asp Phe Phe Ser Arg Ser Ala Leu Asn Thr Asp Ala Glu
    50                  55                  60

Asp Thr Pro Leu Ser Thr Met Val His Val Trp Gln Leu Lys Thr Arg
65                  70                  75                  80

Glu Asp Phe Asp Lys Ala Asp Tyr Asp Thr Leu Phe Met Gln Glu Glu
                85                  90                  95

Lys Thr Leu Glu Lys Asp Val Leu Ala Lys His Thr Val Trp Val Lys
            100                 105                 110

Pro Glu Gly Thr Ala Ser Leu Asn Val Pro Leu Asp Lys Glu Thr Gln
        115                 120                 125

Phe Val Ala Ile Ile Gly Gln Phe Tyr His Pro Asp Glu Lys Ser Asp
    130                 135                 140

Ser Trp Arg Leu Val Ile Lys Arg Asp Glu Leu Glu Ala Asp Lys Pro
145                 150                 155                 160

Arg Ser Ile Glu Leu Met Arg Ser Asp Leu Arg Leu Leu Pro Leu Lys
                165                 170                 175

Asp Lys

<210> 38

<211> 280

<212> PRT

<213> Escherichia coli

<400> 38

Met Ile Ser Gly Gly Asn Met Leu Lys Glu Trp Met Ile Phe Thr Cys
1               5                   10                  15

Ser Leu Leu Thr Leu Ala Gly Ala Ser Leu Pro Leu Ser Gly Cys Ile
            20                  25                  30

Ser Arg Gly Gln Glu Ser Ile Ser Glu Gly Ala Ala Phe Gly Ala Gly
        35                  40                  45

Ile Leu Arg Glu Pro Gly Ala Thr Lys Lys Ala Asp Thr Lys Asp Leu
    50                  55                  60

Asn Val Pro Pro Pro Val Tyr Gly Pro Pro Gln Val Ile Phe Arg Ile
65                  70                  75                  80

Asp Asp Asn Arg Tyr Phe Thr Leu Glu Asn Tyr Thr His Cys Glu Asn
                85                  90                  95

Gly Gln Thr Phe Tyr Asn Asn Lys Ala Lys Asn Ile His Val Lys Ile
            100                 105                 110

Leu Asp Ala Ser Gly Tyr Leu Phe Lys Gly Arg Leu Phe Trp Leu Ser
        115                 120                 125

Thr Arg Asp Asp Phe Leu Ala Phe Pro Ala Thr Leu Asn Thr Arg His
    130                 135                 140

Ala Ser Cys Met Gly Ser Asn Lys Gly Cys Met Asn Ala Val Ile Val
145                 150                 155                 160

Thr Thr Asp Gly Gly Lys Arg Arg Ser Gly Val Pro Tyr Gly Ser Tyr
                165                 170                 175

Thr Gln Asn Pro Thr Gly Ala Thr Arg Asp Tyr Asp Met Leu Val Met
            180                 185                 190

Asn Asp Gly Phe Tyr Leu Leu Arg Tyr Arg Gly Gly Gln Gly Arg Phe
        195                 200                 205

Ser Pro Val Ile Leu Arg Trp Ile Leu Ser Thr Glu Asp Ser Ser Gly
    210                 215                 220

Val Val Arg Ser Glu Asp Ala Tyr Glu Leu Phe Arg Pro Gly Glu Glu
225                 230                 235                 240

Val Pro Ser Thr Gly Phe Tyr Lys Ile Asp Leu Ser Arg Phe Tyr Pro
                245                 250                 255

Lys Asn Asn Val Met Glu Met Gln Cys Asp Arg Thr Leu Glu Pro Val
            260                 265                 270

Gln Pro Ser Glu Ser Lys Ile Gln
        275                 280

<210>  39

<211>  501

<212>  PRT

<213>  Escherichia coli

<400>  39

Met Glu His Val Ser Ile Lys Thr Leu Tyr His Leu Leu Cys Cys Met
1               5                   10                  15

Leu Leu Phe Ile Ser Ala Met Cys Ala Leu Ala Gln Glu His Glu Pro
            20                  25                  30

Ile Gly Ala Gln Asp Glu Arg Leu Ser Thr Leu Ile His Gln Arg Met
        35                  40                  45

Gln Glu Ala Lys Val Pro Ala Leu Ser Val Ser Val Thr Ile Lys Gly
    50                  55                  60

Val Arg Gln Arg Phe Val Tyr Gly Val Ala Asp Val Ala Ser Gln Lys
65                  70                  75                  80

Ala Asn Thr Leu Asp Thr Val Tyr Glu Leu Gly Ser Met Ser Lys Ala
                85                  90                  95

Phe Thr Gly Leu Val Val Gln Ile Leu Ile Gln Glu Gly Arg Leu Arg
            100                 105                 110

Gln Gly Asp Asp Ile Ile Thr Tyr Leu Pro Glu Met Arg Leu Asn Tyr
        115                 120                 125

Gln Gly Lys Pro Ala Ser Leu Thr Val Ala Asp Phe Leu Tyr His Thr
    130                 135                 140

Ser Gly Leu Pro Phe Ser Thr Leu Ala Arg Leu Glu Asn Pro Met Pro
145                 150                 155                 160

Gly Ser Ala Val Ala Gln Gln Leu Arg Asn Glu Asn Leu Leu Phe Ala
                165                 170                 175

Pro Gly Ala Lys Phe Ser Tyr Ala Ser Ala Asn Tyr Asp Val Leu Gly
            180                 185                 190

Ala Val Ile Glu Asn Val Thr Gly Lys Thr Phe Thr Glu Val Ile Ala
        195                 200                 205

Glu Arg Leu Thr Gln Pro Leu Gly Met Ser Ala Thr Val Ala Val Lys
    210                 215                 220

Gly Asp Glu Ile Ile Val Asn Lys Ala Ser Gly Tyr Lys Leu Gly Phe
225                 230                 235                 240

Gly Lys Pro Val Leu Phe His Ala Pro Leu Ala Arg Asn His Val Pro
                245                 250                 255

Ala Ala Tyr Ile His Ser Thr Leu Pro Asp Met Glu Ile Trp Ile Asp
            260                 265                 270

Ala Trp Leu His Arg Lys Ala Leu Pro Ala Thr Leu Arg Glu Ala Met
        275                 280                 285

Ser Asn Ser Trp Arg Gly Asn Ser Asp Val Pro Leu Ala Ala Asp Asn
    290                 295                 300

Arg Ile Leu Tyr Ala Ser Gly Trp Phe Ile Asp Gln Asn Gln Gly Pro
305                 310                 315                 320

Tyr Ile Ser His Gly Gly Gln Asn Pro Asn Phe Ser Ser Cys Ile Ala
                325                 330                 335

Leu Arg Pro Asp Gln Gln Ile Gly Ile Val Ala Leu Ala Asn Met Asn
            340                 345                 350

Ser Asn Leu Ile Leu Gln Leu Cys Ala Asp Ile Asp Asn Tyr Leu Arg
        355                 360                 365

Ile Gly Lys Tyr Ala Asp Gly Ala Gly Asp Ala Ile Thr Ala Thr Asp
    370                 375                 380

Thr Leu Phe Val Tyr Leu Thr Leu Leu Leu Cys Phe Trp Gly Ala Val
385                 390                 395                 400

Val Val Val Arg Gly Ala Phe Arg Val Tyr Arg Ala Thr Ala His Gly
                405                 410                 415

Pro Gly Lys Gln Gln Arg Leu Arg Leu Arg Val Arg Asp Tyr Ile Ile
            420                 425                 430

Ala Leu Ala Val Pro Gly Leu Val Ala Ala Met Leu Tyr Val Ala Pro
        435                 440                 445

Gly Ile Leu Ser Pro Gly Leu Asp Trp Arg Phe Ile Leu Val Trp Gly
    450                 455                 460

Pro Ser Ser Val Leu Ala Ile Pro Phe Gly Ile Ile Leu Leu Ala Phe
465                 470                 475                 480

Val Leu Thr Leu Asn His Gln Ile Lys Arg Ile Leu Leu His Asn Lys
                485                 490                 495

Glu Trp Asp Asp Glu
            500

<210>  40

<211>  682

<212>  PRT

<213>  Escherichia coli

<400>  40

Met Lys Asn Lys Tyr Ile Ile Ala Pro Gly Ile Ala Val Met Cys Ser
1               5                   10                  15

Ala Val Ile Ser Ser Gly Tyr Ala Ser Ser Asp Lys Lys Glu Asp Thr
            20                  25                  30

Leu Val Val Thr Ala Ser Gly Phe Thr Gln Gln Leu Arg Asn Ala Pro
        35                  40                  45

Ala Ser Val Ser Val Ile Thr Ser Glu Gln Leu Gln Lys Lys Pro Val
    50                  55                  60

Ser Asp Leu Val Asp Ala Val Lys Asp Val Glu Gly Ile Ser Ile Thr
65                  70                  75                  80

Gly Gly Asn Glu Lys Pro Asp Ile Ser Ile Arg Gly Leu Ser Gly Asp
                85                  90                  95

Tyr Thr Leu Ile Leu Val Asp Gly Arg Arg Gln Ser Gly Arg Glu Ser
            100                 105                 110

Arg Pro Asn Gly Ser Gly Gly Phe Glu Ala Gly Phe Ile Pro Pro Val
        115                 120                 125

Glu Ala Ile Glu Arg Ile Glu Val Ile Arg Gly Pro Met Ser Ser Leu
    130                 135                 140

Tyr Gly Ser Asp Ala Ile Gly Gly Val Ile Asn Ile Ile Thr Lys Pro
145                 150                 155                 160

Val Asn Asn Gln Thr Trp Asp Gly Val Leu Gly Leu Gly Gly Ile Ile
                165                 170                 175

Gln Glu His Gly Lys Phe Gly Asn Ser Thr Thr Asn Asp Phe Tyr Leu
            180                 185                 190

Ser Gly Pro Leu Ile Lys Asp Lys Leu Gly Leu Gln Leu Tyr Gly Gly
        195                 200                 205

Met Asn Tyr Arg Lys Glu Asp Ser Ile Ser Gln Gly Thr Pro Ala Lys
    210                 215                 220

Asp Asn Lys Asn Ile Thr Ala Thr Leu Gln Phe Thr Pro Thr Glu Ser
225                 230                 235                 240

Gln Lys Phe Val Phe Glu Tyr Gly Lys Asn Asn Gln Val His Thr Leu
                245                 250                 255

Thr Pro Gly Glu Ser Leu Asp Ala Trp Thr Met Arg Gly Asn Leu Lys
            260                 265                 270

Gln Pro Asn Ser Lys Arg Glu Thr His Asn Ser Arg Ser His Trp Val
        275                 280                 285

Ala Ala Trp Asn Ala Gln Gly Glu Ile Leu His Pro Glu Ile Ala Val
    290                 295                 300

Tyr Gln Glu Lys Val Ile Arg Glu Val Lys Ser Gly Lys Lys Asp Lys
305                 310                 315                 320

Tyr Asn His Trp Asp Leu Asn Tyr Glu Ser Arg Lys Pro Glu Ile Thr
                325                 330                 335

Asn Thr Ile Ile Asp Ala Lys Val Thr Ala Phe Leu Pro Glu Asn Val
            340                 345                 350

Leu Thr Ile Gly Gly Gln Phe Gln His Ala Glu Leu Arg Asp Asp Ser
        355                 360                 365

Ala Thr Gly Lys Lys Thr Thr Glu Thr Gln Ser Val Ser Ile Lys Gln
    370                 375                 380

Lys Ala Val Phe Ile Glu Asn Glu Tyr Ala Ala Thr Asp Ser Leu Ala
385                 390                 395                 400

Leu Thr Gly Gly Leu Arg Leu Asp Asn His Glu Ile Tyr Gly Ser Tyr
                405                 410                 415

Trp Asn Pro Arg Leu Tyr Ala Val Tyr Asn Leu Thr Asp Asn Leu Thr
            420                 425                 430

Leu Lys Gly Gly Ile Ala Lys Ala Phe Arg Ala Pro Ser Ile Arg Glu
        435                 440                 445

Val Ser Pro Gly Phe Gly Thr Leu Thr Gln Gly Gly Ala Ser Ile Met
    450                 455                 460

Tyr Gly Asn Arg Asp Leu Lys Pro Glu Thr Ser Val Thr Glu Glu Ile
465                 470                 475                 480

Gly Ile Ile Tyr Ser Asn Asp Ser Gly Phe Ser Ala Ser Ala Thr Leu
                485                 490                 495

Phe Asn Thr Asp Phe Lys Asn Lys Leu Thr Ser Tyr Asp Ile Gly Thr
            500                 505                 510

Lys Asp Pro Val Thr Gly Leu Asn Thr Phe Ile Tyr Asp Asn Val Gly
        515                 520                 525

Glu Ala Asn Ile Arg Gly Val Glu Leu Ala Thr Gln Ile Pro Val Tyr
    530                 535                 540

Asp Lys Trp His Val Ser Ala Asn Tyr Thr Phe Thr Asp Ser Arg Arg
545                 550                 555                 560

Lys Ser Asp Asp Glu Ser Leu Asn Gly Lys Ser Leu Lys Gly Glu Pro
                565                 570                 575

Leu Glu Arg Thr Pro Arg His Ala Ala Asn Ala Lys Leu Glu Trp Asp
            580                 585                 590

Tyr Thr Gln Asp Ile Thr Phe Tyr Ser Ser Leu Asn Tyr Thr Gly Lys
        595                 600                 605

Gln Ile Trp Ala Ala Gln Arg Asn Gly Ala Lys Val Pro Arg Val Arg
    610                 615                 620

Asn Gly Phe Thr Ser Met Asp Ile Gly Leu Asn Tyr Gln Ile Leu Pro
625                 630                 635                 640

Asp Thr Leu Ile Asn Phe Ala Val Leu Asn Val Thr Asp Arg Lys Ser
                645                 650                 655

Glu Asp Ile Asp Thr Ile Asp Gly Asn Trp Gln Val Asp Glu Gly Arg
            660                 665                 670

Arg Tyr Trp Ala Asn Val Arg Val Ser Phe
        675                 680

<210> 41

<211> 164

<212> PRT

<213> Escherichia coli

<400> 41

Met Gly Phe Arg Lys Thr Ile Ile Thr Ser Val Gly Leu Ile Phe Ile
1               5                   10                  15

Ser Phe Ser Phe Val Ala Lys Cys Ser Gln Leu Lys Asn Leu Asn Asn
            20                  25                  30

Tyr Ser Val Met Leu Cys Gly Lys Val Ser Asn Asn Ile Leu Asp Asp
        35                  40                  45

Ile Gly Gly Tyr Lys Glu Arg Asn Ile Leu Met Leu Arg Ala Ile Lys
    50                  55                  60

Lys Ile Ile Ile Met Thr Ile Val Asn Ile Ile Phe Phe Tyr Ser Phe
65                  70                  75                  80

Gln Ser Thr Ala Asp Glu Met Val Leu Ile Lys Lys Tyr Gly Phe Gly
                85                  90                  95

Leu Glu Arg Asp Ile Lys Gly Arg Pro Leu Ile Tyr Pro Ile Glu Asn
            100                 105                 110

Tyr Asp Glu Cys Lys Lys Lys Cys Asn His Met Asn Tyr Ile Ala Asp
        115                 120                 125

Val Asn Ala Gln Leu Ala Met Ser Lys Lys Asn Asn Arg Ile Phe Ala
    130                 135                 140

Asn Ile Thr Phe Thr Asn Asn Ser Ser Thr Thr Tyr Phe Phe Leu Asn
145                 150                 155                 160

Ile Ile Tyr Leu

<210> 42

<211> 218

<212> PRT

<213> Escherichia coli

<400> 42

Met Asn Gln Ile Lys Asp Asn Lys Val Ile Met Lys Ile Lys Asn Leu
1               5                   10                  15

Ile Ser Val Ile Leu Leu Ser Gly Gly Ile Met Gly Thr Gly Leu Tyr
            20                  25                  30

Ser Ser Asp Asn His Gln Lys Ile Arg Ser Arg Phe Asn Ile Gln Glu
        35                  40                  45

Ser Tyr Cys Ala Ile Lys Thr Asn Gly Val Leu Gly Phe Ser Asn Arg
    50                  55                  60

Lys Asp Val Leu Arg Glu Asn Gly Asp Ser Thr Gly Thr Thr Ser Ser
65                  70                  75                  80

Ser Thr Asn Ala Met Met Leu Met Glu Asn Gly Glu Asn Glu Ile Ser
                85                  90                  95

Leu Glu Ile Gly Ala Leu Arg Trp Phe Ser Asp Lys Pro Ala Ser Thr
            100                 105                 110

Glu Glu Arg Gly His Phe Ser Gln Lys Ala Gly Cys Ser Leu Asp Leu
        115                 120                 125

Val Arg Phe Val Lys Gln Glu Glu Thr Ile Leu Ser Ser Ile Lys Val
    130                 135                 140

Thr Ile Asn Gln Gln Gly Ile Pro Glu Ala Gln Pro Asp Ser Met His
145                 150                 155                 160

Pro Val Ile Arg Lys Glu Ile Leu Ala Glu Gln Ala Glu Pro Gly Phe
                165                 170                 175

Ile Asp Pro Asp Tyr Phe Asn Glu Thr Tyr Phe Pro Lys Gly Met Lys
            180                 185                 190

Val Tyr Gln Phe Thr Gln Lys Val Ser Val Ala Gly Leu Pro Asp Gly
        195                 200                 205

Pro Gly Arg Ser Thr Pro Phe Thr Gly Ala
    210                 215

<210>  43

<211>  2732

<212>  PRT

<213>  Escherichia coli

<400>  43

Met His Gln Pro Pro Val Arg Phe Thr Tyr Arg Leu Leu Ser Tyr Leu
1               5                   10                  15

Val Ser Ala Ile Ile Ala Gly Gln Pro Leu Leu Pro Ala Val Gly Ala
            20                  25                  30

Val Ile Thr Pro Gln Asn Gly Ala Gly Met Asp Lys Ala Ala Asn Gly
        35                  40                  45

Val Pro Val Val Asn Ile Ala Thr Pro Asn Gly Ala Gly Ile Ser His
    50                  55                  60

Asn Arg Phe Thr Asp Tyr Asn Val Gly Lys Glu Gly Leu Ile Leu Asn
65                  70                  75                  80

Asn Ala Thr Gly Lys Leu Asn Pro Thr Gln Leu Gly Gly Leu Ile Gln
                85                  90                  95

Asn Asn Pro Asn Leu Lys Ala Gly Gly Glu Ala Lys Gly Ile Ile Asn
            100                 105                 110

Glu Val Thr Gly Gly Lys Arg Ser Leu Leu Gln Gly Tyr Thr Glu Val
        115                 120                 125

Ala Gly Lys Ala Ala Asn Val Met Val Ala Asn Pro Tyr Gly Ile Thr
    130                 135                 140

Cys Asp Gly Cys Gly Phe Ile Asn Thr Pro His Ala Thr Leu Thr Thr
145                 150                 155                 160

Gly Lys Pro Val Met Asn Ala Asp Gly Ser Leu Gln Ala Leu Glu Val
                165                 170                 175

Thr Glu Gly Ser Ile Thr Ile Asn Gly Ala Gly Leu Asp Gly Thr Arg
            180                 185                 190

Ser Asp Ala Val Ser Ile Ile Ala Arg Ala Thr Glu Val Asn Ala Ala
        195                 200                 205

Leu His Ala Lys Asp Leu Thr Val Thr Ala Gly Ala Asn Arg Val Thr
    210                 215                 220

Ala Asp Gly Arg Val Arg Ala Leu Lys Gly Glu Gly Asp Val Pro Lys
225                 230                 235                 240

Val Ala Val Asp Thr Gly Ala Leu Gly Gly Met Tyr Ala Arg Arg Ile
                245                 250                 255

His Leu Thr Ser Thr Glu Ser Gly Val Gly Val Asn Leu Gly Asn Leu
            260                 265                 270

Tyr Ala Arg Asp Gly Asp Ile Thr Leu Asp Ala Ser Gly Arg Leu Thr
        275                 280                 285

Val Asn Asn Ser Leu Ala Thr Gly Ala Val Thr Ala Lys Gly Gln Gly
    290                 295                 300

Val Thr Leu Thr Gly Asp His Lys Ala Gly Gly Asn Leu Ser Val Ser
305                 310                 315                 320

Ser Arg Arg Asp Ile Val Leu Ser Asn Gly Thr Leu Asn Ser Asp Lys
                325                 330                 335

Asp Leu Ser Leu Thr Ala Gly Gly Arg Ile Thr Gln Gln Asn Glu Lys
            340                 345                 350

Leu Thr Ala Gly Arg Asp Val Thr Leu Ala Ala Lys Asn Ile Thr Gln
        355                 360                 365

Asp Thr Ala Ser Gln Ile Asn Ala Ala Arg Asp Ile Val Thr Val Ala
    370                 375                 380

Ser Asp Thr Leu Thr Thr Gln Gly Gln Ile Thr Ala Gly Gln Asn Leu
385                 390                 395                 400

Thr Ala Ser Ala Thr Thr Leu Thr Gln Asp Gly Ile Leu Leu Ala Lys
                405                 410                 415

Ser His Ala Gly Leu Asn Ala Gly Thr Leu Asn Asn Ser Gly Ala Val
            420                 425                 430

Gln Gly Ala Thr Leu Thr Leu Gly Ser Thr Thr Leu Ser Asn Ser Gly
        435                 440                 445

Ser Leu Leu Ser Gly Gly Pro Leu Thr Met Asn Thr Arg Asp Phe Thr
    450                 455                 460

Gln Ser Gly Arg Thr Gly Ala Lys Gly Lys Val Asp Ile Met Ala Ser
465                 470                 475                 480

Gly Lys Leu Thr Ser Thr Gly Leu Leu Val Thr Met His Leu Val Leu
                485                 490                 495

Lys Ala Gln Asp Val Thr Gln Asn Gly Val Leu Ser Gly Gly Lys Gly
            500                 505                 510

Leu Thr Val Ser Ala Thr Ser Ser Gly Lys Lys Ser Val Thr His Ser
        515                 520                 525

Asp Ala Ala Met Thr Leu Asn Val Thr Thr Val Ala Leu Asp Gly Glu
    530                 535                 540

Thr Ser Ala Gly Asp Thr Leu Arg Val Gln Ala Asp Lys Leu Ser Thr
545                 550                 555                 560

Ala Ala Gly Ala Gln Leu Gln Ser Gly Lys Asn Leu Ser Ile Asn Ala
                565                 570                 575

Arg Asp Ala Arg Leu Ala Gly Thr Gln Ala Ala Gln Gln Thr Met Val
            580                 585                 590

Val Asn Ala Ser Glu Lys Leu Thr His Ser Gly Lys Ser Ser Ala Pro
        595                 600                 605

Ser Leu Ser Leu Ser Ala Pro Glu Leu Thr Ser Ser Gly Val Leu Val
    610                 615                 620

Gly Ser Ala Leu Asn Thr Gln Ser Gln Thr Leu Thr Asn Ser Gly Leu
625                 630                 635                 640

Leu Gln Gly Glu Ala Ser Leu Thr Val Asn Thr Gln Arg Leu Asp Asn
                645                 650                 655

Gln Gln Asn Gly Thr Leu Tyr Ser Ala Ala Asp Leu Thr Leu Asp Ile
            660                 665                 670

Pro Asp Ile Arg Asn Ser Gly Leu Ile Thr Gly Asp Asn Gly Leu Met
        675                 680                 685

Leu Asn Ala Val Ser Leu Ser Asn Pro Gly Lys Ile Ile Ala Asp Thr
    690                 695                 700

Leu Ser Val Arg Ala Thr Thr Leu Asp Gly Asp Gly Leu Leu Gln Gly
705                 710                 715                 720

Ala Gly Ala Leu Ala Leu Ala Gly Asp Thr Leu Ser Gln Gly Ser His
                725                 730                 735

Gly Arg Trp Leu Thr Ala Asp Asp Leu Ser Leu Arg Gly Lys Thr Leu
            740                 745                 750

Asn Thr Ala Gly Thr Thr Gln Gly Gln Asn Ile Thr Val Gln Ala Asp
        755                 760                 765

Arg Trp Ala Asn Ser Gly Ser Val Leu Ala Thr Gly Asn Leu Thr Ala
    770                 775                 780

Ser Ala Thr Gly Gln Leu Thr Ser Thr Gly Asp Ile Met Ser Gln Gly
785                 790                 795                 800

Asp Thr Thr Leu Lys Ala Ala Thr Thr Asp Asn Arg Gly Ser Leu Leu
                805                 810                 815

Ser Ala Gly Thr Leu Ser Leu Asp Gly Asn Ser Leu Asp Asn Arg Gly
            820                 825                 830

Thr Val Gln Gly Asn His Val Thr Ile Arg Gln Asn Ser Val Thr Asn
        835                 840                 845

Ser Gly Thr Leu Thr Gly Ile Ala Ala Leu Thr Leu Ala Ala Arg Met
    850                 855                 860

Ala Ser Pro Gln Pro Ala Leu Met Asn Asn Gly Gly Ser Leu Leu Thr
865                 870                 875                 880

Ser Gly Asp Leu Thr Ile Thr Ala Gly Ser Ile Thr Ser Ser Gly His
                885                 890                 895

Trp Gln Gly Lys Arg Val Leu Ile Thr Ala Asp Ser Leu Ala Asn Ser
            900                 905                 910

Gly Ala Ile Gln Ala Ala Asp Ser Leu Thr Ala Arg Leu Thr Gly Glu
        915                 920                 925

Leu Val Ser Thr Ala Gly Ser Lys Val Thr Ser Asn Gly Glu Met Ala
    930                 935                 940

Leu Ser Ala Leu Asn Leu Ser Asn Ser Gly Gln Trp Ile Ala Lys Asn
945                 950                 955                 960

Leu Thr Leu Lys Ala Asn Ser Leu Thr Ser Ala Gly Asp Ile Thr Gly
                965                 970                 975

Val Asp Thr Leu Thr Leu Thr Val Asn Gln Thr Leu Asn Asn Gln Ala
            980                 985                 990

Asn Gly Lys Leu Leu Ser Ala Gly  Val Leu Thr Leu Lys  Ala Asp Ser
        995                 1000                 1005

Val Thr  Asn Asp Gly Gln Leu  Gln Gly Asn Val Thr  Thr Ile Thr
    1010                 1015                 1020

Ala Gly  Gln Leu Thr Asn Gly  Gly His Leu Gln Gly  Glu Thr Leu
    1025                 1030                 1035

Thr Leu  Thr Ala Ser Gly Gly  Val Asn Asn Arg Ser  Gly Gly Val
    1040                 1045                 1050

Leu Met  Ser Arg Asn Ala Leu  Asn Val Ser Thr Ala  Thr Leu Ser
    1055                 1060                 1065

Asn Gln  Ser Thr Ile Gln Gly  Gly Gly Gly Val Ser  Leu Asn Ala
    1070                 1075                 1080

Thr Asp  Arg Leu Gln Asn Asp  Gly Lys Ile Leu Ser  Gly Ser Asn
    1085                 1090                 1095

Leu Thr  Leu Thr Ala Gln Val  Leu Ala Asn Thr Gly  Ser Gly Leu
    1100                 1105                 1110

Val Gln  Ala Ala Thr Leu Leu  Leu Asp Val Val Asn  Thr Val Asn
    1115                 1120                 1125

Gly Gly  Arg Val Leu Ala Thr  Gly Ser Asp Val Lys  Gly Thr Thr
    1130                 1135                 1140

Leu Asn Asn Thr Gly Thr Leu Gln Gly Ala Thr Leu Val Asn Tyr
    1145                1150                1155

His Thr Phe Ser Ser Gly Thr Leu Leu Gly Thr Ser Gly Leu Gly
    1160                1165                1170

Val Lys Gly Ser Ser Leu Leu Gln Asn Gly Thr Gly Arg Leu Tyr
    1175                1180                1185

Ser Ala Gly Asn Leu Leu Leu Asp Ala Gln Asp Phe Ser Gly Gln
    1190                1195                1200

Gly Gln Val Val Ala Thr Gly Asp Val Thr Leu Lys Leu Ile Ala
    1205                1210                1215

Ala Leu Thr Asn His Gly Thr Leu Ala Ala Gly Lys Thr Leu Ser
    1220                1225                1230

Val Thr Ser Gln Asn Ala Ile Thr Asn Gly Gly Val Met Gln Gly
    1235                1240                1245

Asp Ala Met Val Leu Gly Ala Gly Glu Ala Phe Thr Asn Asn Gly
    1250                1255                1260

Leu Thr Ala Gly Lys Gly Asn Ser Val Phe Ser Ala Gln Arg Leu
    1265                1270                1275

Phe Leu Asn Ala Pro Gly Ser Leu Gln Gly Gly Gly Asp Val Ser
    1280                1285                1290

Leu Asn Ser Arg Ser Asp Ile Thr Ile Ser Gly Phe Thr Gly Thr
    1295                1300                1305

Ala Gly Ser Leu Thr Met Asn Val Ala Gly Thr Leu Leu Asn Ser
    1310                1315                1320

Ala Leu Ile Tyr Ala Gly Asn Asn Leu Lys Leu Phe Thr Asp Arg
    1325                1330                1335

Leu His Asn Gln His Gly Asp Ile Leu Ala Gly Asn Ser Leu Trp
    1340                1345                1350

Val Gln Lys Asp Ala Ser Gly Gly Ala Asn Thr Glu Ile Ile Asn
    1355                1360                1365

Asn Ser Gly Asn Ile Glu Thr His Gln Gly Asp Ile Val Val Arg
    1370                1375                1380

Thr Gly His Leu Leu Asn Gln Arg Glu Gly Phe Ser Ala Thr Thr
    1385                1390                1395

Thr Thr Arg Thr Asn Pro Ser Ser Ile Gln Gly Met Gly Asn Ala
    1400                1405                1410

Leu Val Asp Ile Pro Leu Ser Leu Leu Pro Asp Gly Ser Tyr Gly
    1415                1420                1425

Tyr Phe Thr Arg Glu Val Glu Asn Gln His Gly Thr Pro Cys Asn
    1430                1435                1440

Gly His Gly Ala Cys Asn Ile Thr Met Asp Thr Leu Tyr Tyr Tyr
    1445                1450                1455

Ala Pro Phe Ala Asp Ser Ala Thr Gln Arg Phe Leu Ser Ser Gln
    1460                1465                1470

Asn Ile Thr Thr Val Thr Gly Ala Asp Asn Pro Ala Gly Arg Ile
    1475                1480                1485

Ala Ser Gly Arg Asn Leu Ser Ala Glu Ala Glu Arg Leu Glu Asn
    1490                1495                1500

Arg Ala Ser Phe Ile Leu Ala Asn Gly Asp Ile Ala Leu Ser Gly
    1505                1510                1515

Arg Glu Leu Ser Asn Gln Ser Trp Gln Thr Gly Thr Glu Asn Glu
    1520                1525                1530

Tyr Leu Val Tyr Arg Tyr Asp Pro Lys Thr Phe Tyr Gly Ser Tyr
    1535                1540                1545

Ala Thr Gly Ser Leu Asp Lys Leu Pro Leu Leu Ser Pro Glu Phe
    1550                1555                1560

Glu Asn Asn Thr Ile Arg Phe Ser Leu Asp Gly Arg Glu Lys Asp
    1565                1570                1575

Tyr Thr Pro Gly Lys Thr Tyr Tyr Ser Val Ile Gln Ala Gly Gly
    1580                1585                1590

Asp Val Lys Thr Arg Phe Thr Ser Ser Ile Asn Asn Gly Thr Thr
    1595                1600                1605

Thr Ala His Ala Gly Ser Val Ser Pro Val Val Ser Ala Pro Val
    1610                1615                1620

Leu Asn Thr Leu Ser Gln Gln Thr Gly Gly Asp Ser Leu Thr Gln
    1625                1630                1635

Thr Ala Leu Gln Gln Tyr Glu Pro Val Val Val Gly Ser Pro Gln
    1640                1645                1650

Trp His Asp Glu Leu Ala Gly Ala Leu Lys Asn Ile Ala Gly Gly
    1655                1660                1665

Ser Pro Leu Thr Gly Gln Thr Gly Ile Ser Asp Asp Trp Pro Leu
    1670                1675                1680

Pro Ser Gly Asn Asn Gly Tyr Leu Val Pro Ser Thr Asp Pro Asp
    1685                1690                1695

Ser Pro Tyr Leu Ile Thr Val Asn Pro Lys Leu Asp Gly Leu Gly
    1700                1705                1710

Gln Val Asp Ser His Leu Phe Ala Gly Leu Tyr Glu Leu Leu Gly
    1715                1720                1725

Ala Lys Pro Gly Gln Ala Pro Arg Glu Thr Ala Pro Ser Tyr Thr
    1730                1735                1740

Asp Glu Lys Gln Phe Leu Gly Ser Ser Tyr Phe Leu Asp Arg Leu
    1745                1750                1755

Gly Leu Lys Pro Glu Lys Asp Tyr Arg Phe Leu Gly Asp Ala Val
    1760                1765                1770

Phe Asp Thr Arg Tyr Val Ser Asn Ala Val Leu Ser Arg Thr Gly
    1775                1780                1785

Ser Arg Tyr Leu Asn Gly Leu Gly Ser Asp Thr Glu Gln Met Arg
    1790                1795                1800

Tyr Leu Met Asp Asn Ala Ala Arg Gln Gln Lys Gly Leu Gly Leu
    1805                1810                1815

Glu Phe Gly Val Ala Leu Thr Ala Glu Gln Ile Ala Gln Leu Asp
    1820                1825                1830

Gly Ser Ile Leu Trp Trp Glu Ser Val Thr Ile Asn Gly Gln Thr
    1835                1840                1845

Val Met Val Pro Lys Leu Tyr Leu Ser Pro Glu Asp Ile Thr Leu
    1850                1855                1860

His Asn Gly Ser Val Ile Ser Gly Asn Asn Val Gln Leu Ala Gly
    1865                1870                1875

Gly Asn Ile Thr Asn Ser Gly Gly Ser Ile Asn Ala Gln Asn Asp
    1880                1885                1890

Leu Ser Leu Asp Ser Ser Gly Tyr Ile Asp Asn Leu Asn Ala Gly
    1895                1900                1905

Leu Ile Ser Ala Gly Gly Ser Leu Asp Leu Ser Ala Ile Gly Asp
    1910                1915                1920

Ile Ser Asn Ile Ser Ser Val Ile Ser Gly Lys Thr Val Gln Leu
    1925                1930                1935

Glu Ser Val Ser Gly Asn Ile Ser Asn Ile Thr Arg Arg Gln Gln
    1940                1945                1950

Trp Asn Ala Gly Ser Asp Ser Gln Tyr Gly Gly Val His Leu Ser
    1955                1960                1965

Gly Thr Asp Thr Gly Pro Val Ala Thr Ile Lys Gly Thr Asp Ser
    1970                1975                1980

Leu Ser Leu Asp Ala Gly Lys Asn Ile Asp Ile Thr Gly Ala Thr
    1985                1990                1995

Val Ser Ser Gly Gly Asp Leu Gly Met Ser Ala Gly Asn Asp Ile
    2000                2005                2010

Asn Ile Ala Ala Asn Leu Ile Ser Gly Ser Lys Ser Gln Ser Gly
    2015                2020                2025

Phe Trp His Thr Asp Asp Asn Ser Ser Ser Ser Thr Thr Ser Gln
    2030                2035                2040

Gly Ser Ser Ile Ser Ala Gly Gly Asn Leu Ala Met Ala Ala Gly
    2045                2050                2055

His Asn Leu Asp Val Thr Ala Ser Ser Val Ser Ala Gly His Ser
    2060                2065                2070

Ala Leu Leu Ser Cys Arg Ser Arg Pro Ser Leu Glu Cys Ser Gln
    2075                2080                2085

Gly Lys Ala Lys Thr Ser Arg Asn Gly Arg Ser Glu Ser His Glu
    2090                2095                2100

Ser His Ala Ala Val Ser Thr Val Thr Ala Gly Asp Asn Phe Leu
    2105                2110                2115

Leu Val Ala Gly Arg Asp Ile Ala Ser Gln Ala Ala Gly Met Ala
    2120                2125                2130

Ala Glu Asn Asn Val Val Ile Arg Gly Gly Arg Asp Val Asn Leu
    2135                2140                2145

Val Ala Glu Ser Ala Gly Ala Gly Asp Ser Tyr Thr Ser Lys Lys
    2150                2155                2160

Lys Lys Glu Ile Asn Glu Thr Val Arg Gln Gln Gly Thr Glu Ile
    2165                2170                2175

Ala Ser Gly Gly Asp Thr Thr Val Asn Ala Gly Arg Asp Ile Thr
    2180                2185                2190

Ala Val Ala Ser Ser Val Thr Ala Thr Gly Asn Ile Ser Val Asn
    2195                2200                2205

Ala Gly Arg Asp Val Ala Leu Thr Thr Ala Thr Glu Ser Asp Tyr
    2210                2215                2220

His Tyr Leu Glu Thr Lys Lys Lys Ser Gly Gly Phe Leu Ser Lys
    2225                2230                2235

Lys Thr Thr Arg Thr Ile Ser Glu Asp Ser Ala Thr Arg Glu Ala
    2240                2245                2250

Gly Ser Leu Leu Ser Gly Asn Arg Val Thr Val Asn Ala Gly Asp
    2255                2260                2265

Asn Leu Thr Val Glu Gly Ser Asp Val Val Ala Asp Arg Asp Val
    2270                2275                2280

Ser Leu Ala Ala Gly Asn His Val Asp Val Leu Ala Ala Thr Ser
    2285                2290                2295

Thr Asp Thr Ser Trp Arg Phe Lys Glu Thr Lys Lys Ser Gly Leu
    2300                2305                2310

Met Gly Thr Gly Gly Ile Gly Phe Thr Ile Gly Ser Ser Lys Thr
    2315                2320                2325

Thr His Asp Arg Arg Glu Ala Gly Thr Thr Gln Ser Gln Ser Ala
    2330                2335                2340

Ser Thr Ile Gly Ser Thr Ala Gly Asn Val Ser Ile Thr Ala Gly
    2345                2350                2355

Lys Gln Ala His Ile Ser Gly Ser Asp Val Ile Ala Asn Arg Asp
    2360                2365                2370

Ile Ser Ile Thr Gly Asp Ser Val Val Val Asp Pro Gly His Asp
    2375                2380                2385

Arg Arg Thr Val Asp Glu Lys Phe Glu Gln Lys Lys Ser Gly Leu
    2390                2395                2400

Thr Val Ala Leu Ser Gly Thr Val Gly Ser Ala Ile Asn Asn Ala
    2405                2410                2415

Val Thr Ser Ala Gln Glu Thr Lys Glu Ser Ser Asp Ser Arg Leu
    2420                2425                2430

Lys Ala Leu Gln Ala Thr Lys Thr Ala Leu Ser Gly Val Gln Ala
    2435                2440                2445

Gly Gln Ala Ala Thr Met Ala Ser Ala Thr Gly Asp Pro Asn Ala
    2450                2455                2460

Gly Val Ser Leu Ser Leu Thr Thr Gln Lys Ser Lys Ser Gln Gln
    2465                2470                2475

His Ser Glu Ser Asp Thr Val Ser Gly Ser Thr Leu Asn Ala Gly
    2480                2485                2490

Asn Asn Leu Ser Val Val Ala Thr Gly Lys Asn Arg Gly Asp Asn
    2495                2500                2505

Arg Gly Asp Ile Val Ile Ala Gly Ser Gln Leu Lys Ala Gly Gly
    2510                2515                2520

Asn Thr Ser Leu Asp Ala Ala Asn Asp Ile Leu Leu Ser Gly Ala
    2525                2530                2535

Ala Asn Thr Gln Lys Thr Thr Gly Arg Asn Ser Ser Ser Gly Gly
    2540                2545                2550

Gly Val Gly Val Ser Ile Gly Ala Gly Lys Gly Ala Gly Ile Ser
    2555                2560                2565

Ala Phe Ala Ser Val Asn Ala Ala Lys Gly Arg Glu Lys Gly Asn
    2570                2575                2580

Gly Thr Thr Thr Asp Lys Thr Val Thr Ile Asn Ser Gly Arg Asp
    2585                2590                2595

Thr Val Leu Asn Gly Ala Gln Val Asn Gly Asn Arg Ile Ile Ala
    2600                2605                2610

Asp Val Gly His Asp Leu Leu Ile Ser Ser Gln Gln Asp Thr Ser
    2615                2620                2625

Lys Tyr Asp Ser Lys Gln Thr Ser Val Ala Ala Gly Gly Ser Phe
    2630                2635                2640

Thr Phe Gly Ser Met Thr Gly Ser Gly Tyr Ile Ala Ala Ser Arg
    2645                2650                2655

Asp Lys Met Lys Ser Arg Phe Asp Ser Val Ala Glu Gln Thr Gly
    2660                2665                2670

Met Phe Ala Arg Val Met Val Ala Ser Thr Ser Gln Trp Val Asn
    2675                2680                2685

Ile Pro Asn Trp Met Val Arg Ser Leu Pro His Cys His Thr Gly
    2690                2695                2700

Glu Lys Pro Pro Gly Tyr Arg Thr Leu Gly Leu Val Thr Leu Gln
    2705                2710                2715

Arg Ser Gly Ile Ile Lys Ser Ser His Arg Trp Asn Gln Ser
    2720                2725                2730

<210> 44

<211> 321

<212> PRT

<213> Escherichia coli

<400> 44

Met Met Leu Lys Lys Thr Ile Phe Ile Leu Thr Leu Phe Ser Gly Asn
1               5                   10                  15

Val Ile Ala Ala Thr Val Glu Leu Gly Phe Glu Asn Glu Gln Tyr Asn
            20                  25                  30

Tyr Ala Tyr Arg Ser Ala Asp Val Phe Met Pro Tyr Ile Lys Ser Asn
        35                  40                  45

Phe Asn Pro Val Thr Asp Ser Ala Leu Asn Val Ser Leu Thr Tyr Met
    50                  55                  60

Tyr Gln Asp Gln Tyr Gly Lys Lys His Lys Lys Thr Ser Glu Asp Arg
65                  70                  75                  80

Phe Lys Thr Asn Arg Asp Arg Ile Glu Leu Tyr Leu Lys Gly Tyr Thr
                85                  90                  95

Leu Asn Arg Gly Ala Tyr Ser Phe Ser Pro Ser Ala Gly Phe Arg Tyr
            100                 105                 110

Glu Ser Trp Asp Val Asn Tyr Asp Asn Pro Lys Lys Gln Asp Lys Trp
        115                 120                 125

Lys Leu Glu Leu Arg Phe Tyr Pro Asn Met Thr Tyr Lys Leu Asn Asp
    130                 135                 140

Gln Leu Ser Leu Tyr Met Asn Gly Phe Val Ala Pro Val Phe Phe Lys
145                 150                 155                 160

Thr Gln Gln Glu Ser Arg Lys Asp Asn Asn Tyr Val Lys Gly Lys Leu
                165                 170                 175

Gly Ala Lys Arg Tyr Asn Asn Asp Tyr Tyr Gln Glu Leu Gln Ile Leu
            180                 185                 190

Gly Val Arg Tyr Lys Phe Asn Asn Asp Asn Thr Leu Trp Ala Ser Val
        195                 200                 205

Tyr Asn Glu Arg Lys Tyr Asn Gln His Ser Ser Lys Tyr Asp Arg Trp
    210                 215                 220

Gln Leu Arg Gly Gly Tyr Asp Phe Lys Val Thr Glu Glu Phe Val Leu
225                 230                 235                 240

Ser Pro Phe Ile Arg Tyr Asp Leu Ser Tyr Arg Glu Lys Asn Leu Glu
                245                 250                 255

Ser Thr Ser Asn Asn Gly Leu Ser Lys Asn Asn Lys Glu Ile Arg Thr
            260                 265                 270

Gly Ala Ser Phe Ser Tyr Lys Ile Ile Pro Ser Val Lys Leu Val Gly
        275                 280                 285

Glu Ile Tyr Arg Gln Thr Thr Asn Ile Glu Asn Tyr Tyr Gly Glu His
    290                 295                 300

Ser Glu Asp Lys Asn Arg Met Phe Tyr Lys Leu Gly Ile Asn Lys Thr
305                 310                 315                 320

Phe

<210>  45

<211>  587

<212>  PRT

<213>  Escherichia coli

<400>  45

Met Gln His Arg Gln Lys Asn Ile Leu Thr Lys Thr Ser Leu Leu Ser
1               5                   10                  15

Arg Ala Leu Ser Val Pro Cys Cys Asp Met Phe Arg Arg Gly Ser Pro
            20                  25                  30

Trp Ile Cys Tyr Leu Ser Leu Ser Val Phe Ser Gly Cys Phe Ile Pro
        35                  40                  45

Ala Phe Ser Ser Pro Ala Ala Met Leu Ser Pro Gly Asp Arg Ser Ala
    50                  55                  60

Ile Gln Gln Gln Gln Gln Gln Leu Leu Asp Glu Asn Gln Arg Gln Arg
65                  70                  75                  80

Asp Ala Leu Glu Arg Pro Leu Thr Ile Thr Pro Ser Pro Glu Thr Ser
                85                  90                  95

Ala Gly Thr Glu Gly Pro Cys Phe Thr Val Ser Ser Ile Val Val Ser
            100                 105                 110

Gly Ala Thr Arg Leu Thr Ser Ala Glu Thr Asp Arg Leu Val Pro Trp
        115                 120                 125

Val Asn Gln Cys Leu Asn Ile Thr Gly Leu Thr Ala Val Thr Asp Ala
    130                 135                 140

Val Thr Asp Gly Tyr Ile Arg Arg Gly Tyr Ile Thr Ser Arg Ala Phe
145                 150                 155                 160

Leu Thr Glu Gln Asp Leu Ser Gly Gly Val Leu His Ile Thr Val Met
                165                 170                 175

Glu Gly Arg Leu Gln Gln Ile Arg Ala Glu Gly Ala Asp Leu Pro Ala
            180                 185                 190

Arg Thr Leu Lys Met Val Phe Pro Gly Met Glu Gly Lys Val Leu Asn
        195                 200                 205

Leu Arg Asp Ile Glu Gln Gly Met Glu Gln Ile Asn Arg Leu Arg Thr
    210                 215                 220

Glu Pro Val Gln Ile Glu Ile Ser Pro Gly Asp Arg Glu Gly Trp Ser
225                 230                 235                 240

Val Val Thr Leu Thr Ala Leu Pro Glu Trp Pro Val Thr Gly Ser Val
                245                 250                 255

Gly Ile Asp Asn Ser Gly Gln Lys Ser Thr Gly Thr Gly Gln Leu Asn
            260                 265                 270

Gly Val Leu Ser Phe Asn Asn Pro Leu Gly Leu Ala Asp Asn Trp Phe
        275                 280                 285

Val Ser Gly Gly Arg Ser Ser Asp Phe Ser Val Ser His Asp Ala Arg
    290                 295                 300

Asn Phe Ala Ala Gly Val Ser Leu Pro Tyr Gly Tyr Thr Leu Val Asp
305                 310                 315                 320

Tyr Thr Tyr Ser Trp Ser Asp Tyr Leu Ser Thr Ile Asp Asn Arg Gly
                325                 330                 335

Trp Arg Trp Arg Ser Thr Gly Asp Leu Gln Thr His Arg Leu Gly Leu
            340                 345                 350

Ser His Val Leu Phe Arg Asn Gly Asp Met Lys Thr Ala Leu Thr Gly
        355                 360                 365

Gly Leu Gln His Arg Ile Ile His Asn Tyr Leu Asp Asp Val Leu Leu
    370                 375                 380

Gln Gly Ser Ser Arg Lys Leu Thr Ser Phe Ser Val Gly Leu Asn His
385                 390                 395                 400

Thr His Lys Phe Leu Gly Gly Val Gly Thr Leu Asn Pro Val Phe Thr
                405                 410                 415

Arg Gly Met Pro Trp Phe Gly Ala Glu Ser Asp His Gly Lys Arg Gly
            420                 425                 430

Asp Leu Pro Val Asn Gln Phe Arg Lys Trp Ser Val Ser Ala Ser Phe
        435                 440                 445

Gln Arg Pro Val Thr Asp Arg Val Trp Trp Leu Thr Ser Ala Tyr Ala
    450                 455                 460

Gln Trp Ser Pro Asp Arg Leu His Gly Val Glu Gln Leu Ser Leu Gly
465                 470                 475                 480

Gly Glu Ser Ser Val Arg Gly Phe Lys Asp Gln Tyr Ile Ser Gly Asn
                485                 490                 495

Asn Gly Gly Tyr Leu Arg Asn Glu Leu Ser Trp Ser Leu Phe Ser Leu
            500                 505                 510

Pro Tyr Val Gly Thr Val Arg Ala Val Ala Ala Leu Asp Gly Gly Trp
        515                 520                 525

Leu His Ser Asp Ser Asp Asp Pro Tyr Ser Ser Gly Thr Leu Trp Gly
    530                 535                 540

Ala Ala Ala Gly Leu Ser Thr Thr Ser Gly His Val Ser Gly Ser Phe
545                 550                 555                 560

Thr Ala Gly Leu Pro Leu Val Tyr Pro Asp Trp Leu Ala Pro Asp His
                565                 570                 575

Leu Thr Val Tyr Trp Arg Val Ala Val Ala Phe
            580                 585

<210>  46

<211>  744

<212>  PRT

<213>  Escherichia coli

<400>  46

Met Asn Lys His Thr Leu Leu Leu Thr Val Leu Phe Leu Asn Leu Ile
1               5                   10                  15

Cys Thr Pro Val Phe Ala Gln Asn Trp Gln Val Ala Thr Phe Gly Gln
            20                  25                  30

Ser Thr Asp Leu Asn Phe Ser Ser Leu Ile Asp Ser Ala Lys Ile Gly
        35                  40                  45

Arg Asn Asn Ala Trp Leu Ala Gly Asn Asn Asn Phe Leu Glu Ala Gly
    50                  55                  60

Lys Phe Tyr Thr Leu Pro Thr Asp Phe Phe Ile Glu Ser Arg Gly Gly
65                  70                  75                  80

Lys Ile Ala Asn Ser His Asp Gly Met Thr Val Phe Tyr Thr Ile Val
                85                  90                  95

Pro Val Thr Gln Thr Phe Arg Leu Glu Ala Asp Leu Thr Leu Glu Gln
            100                 105                 110

Ile Gly Pro Glu Val Asn Gly Lys Ser Pro Ala Gly Gln Glu Gly Ala
        115                 120                 125

Gly Leu Phe Val Arg Asp Ile Ile Gly Pro Gln Arg Gln Glu Pro Gln
    130                 135                 140

Ser Ala Gly Thr Glu Glu Tyr Pro Gln Ala Ser Asn Ile Leu Met Asn
145                 150                 155                 160

Ala Phe Ile Thr Gln Asn Lys Lys Asn Asp Asn Leu Val Gln Ile Thr
                165                 170                 175

Ser Ile Val Arg Glu Gly Val Ile Lys Thr Trp Gly Asn Glu Gly Ile
            180                 185                 190

Thr Ile Lys Lys Gln Pro Ile Ile Glu Asn Ile Asn Phe Thr Gln Lys
        195                 200                 205

Arg Asn Ile His Met Thr Ile Glu Arg Leu Pro Glu Lys Phe Ile Leu
    210                 215                 220

Thr Ala Phe Asp Thr Asp Arg Lys Glu Asn Gln Ser Trp Gln Phe Ser
225                 230                 235                 240

Asp Tyr Ser Gly Phe Met Asn Gln Leu Asp Asn Asn Ser Leu Ala Ile
                245                 250                 255

Gly Phe Phe Ala Ala Arg Asn Ala Lys Leu Arg Val Lys Asn Ala Ser
            260                 265                 270

Phe Lys Pro Gly Lys Pro Leu Val Asp Tyr Lys Gln Leu Thr Ser Arg
        275                 280                 285

Gln Phe Ser Arg Val Arg His Lys Ala Pro Glu Leu Phe Leu Ala Ser
    290                 295                 300

Pro Gln Ser Val Val Arg Asn Ser Thr Thr Leu Gln Phe Leu Ala Asn
305                 310                 315                 320

Gln Ala Gly Ile Val Ser Ile Asp Asn Asp Lys Gln Thr Lys Gln Val
                325                 330                 335

Gln Ala Gly Glu Leu Val Gln Phe Pro Val Thr Leu Gln Lys Lys His
            340                 345                 350

Asn Asp Phe Thr Val Asn Phe Asn Val Asp Gly Asn Ile Ser Lys Lys
        355                 360                 365

Ala Ile Arg Ile Glu Gln Val Lys Ser Asn Leu Thr Asp Pro Tyr Glu
    370                 375                 380

Ile Tyr Val Cys Ser Asp Cys Arg Gln Gly Ala Arg Gly Ser Lys Asn
385                 390                 395                 400

Asp Pro Val Asp Leu Gln Thr Ala Val Lys Phe Val Ala Pro Gly Gly
                405                 410                 415

Asn Ile Tyr Leu Asn Asp Gly Gln Tyr His Gly Ile Thr Leu Asp Arg
            420                 425                 430

Glu Leu Ser Gly Ile Pro Gly Lys Tyr Lys Thr Ile Ser Ala Ile Asn
        435                 440                 445

Pro His Lys Ala Ile Phe Ile Asn Lys Thr Phe Asn Leu Asp Ala Ser
    450                 455                 460

Tyr Trp His Leu Lys Ser Val Val Phe Asp Gly Asn Val Asp Asn Gly
465                 470                 475                 480

Asn Asn Lys Pro Ala Tyr Leu Arg Ile Ala Gly Ser Tyr Asn Ile Ile
                485                 490                 495

Glu His Val Ile Ala Arg Asn Asn Asp Asp Thr Gly Ile Ser Ile Ser
            500                 505                 510

Ala Lys Asp Lys Asn Arg Phe Phe Trp Pro Ala His Asn Leu Val Leu
        515                 520                 525

Asn Ser Asp Ser Tyr Asn Asn Leu Asp Leu Ser Gly Ile Asn Ala Asp
    530                 535                 540

Gly Phe Ala Ala Lys Leu Gly Val Gly Pro Gly Asn Ile Phe Arg Gly
545                 550                 555                 560

Cys Ile Ala His Asn Asn Ala Asp Asp Gly Trp Asp Leu Phe Asn Lys
                565                 570                 575

Ile Glu Asp Gly Pro Asn Ala Ser Val Thr Ile Glu Asn Ser Val Ala
            580                 585                 590

Tyr Glu Asn Gly Leu Pro Tyr Asn Lys Ala Asp Ile Leu Lys Gly Ser
        595                 600                 605

Ile Gly Asn Gly Gly Glu Gly Gln Pro Ser Lys Ser Gln Val Ile Asn
    610                 615                 620

Ser Ile Ala Ile Asn Asn Asn Met Asp Gly Phe Thr Asp Asn Phe Asn
625                 630                 635                 640

Thr Gly Ser Leu Ile Val Arg Asn Asn Ile Ala Met Asn Asn Ala Arg
                645                 650                 655

Tyr Asn Tyr Ile Leu Arg Thr Asn Pro Tyr Lys Phe Pro Ser Ser Ile
            660                 665                 670

Leu Phe Asp Asn Asn Tyr Ser Ile Arg Asp Asp Trp Glu Asn Lys Ile
        675                 680                 685

Lys Asp Phe Leu Gly Asp Thr Val Asn Ser Val Asn Tyr Lys Leu Leu
    690                 695                 700

Val Ser His Glu Thr Gly Pro Val Gln Lys Asp Leu Phe Phe Thr Arg
705                 710                 715                 720

Asp Asp Ser Gly Asn Ile Ile Tyr Pro Asp Phe Phe Leu Asn Ile Ile
                725                 730                 735

Asn Lys Phe Asn Glx Thr Met Pro
            740

<210>  47

<211>  136

<212>  PRT

<213>  Escherichia coli

<400>  47

Met Lys Thr Phe Ile Lys Thr Leu Leu Val Ala Val Thr Ile Leu Phe
1               5                   10                  15

Ser Val Phe Ala Thr Ala Lys Gln Val Lys Leu Pro Asn Asn Ile Lys
            20                  25                  30

Tyr Val Asn Thr Thr Glu Ala Phe Ser Cys Thr Glu Ile Asp Gly Met
        35                  40                  45

Asn Cys Gln Thr Lys Asn Pro Phe Asn Tyr Lys Asp Asn Ser Tyr Val
    50                  55                  60

Phe Val Leu Glu Arg Gly Gly Ala Trp Cys Tyr Asp Tyr Thr Val Ser
65                  70                  75                  80

Val Leu Asn Leu Lys Thr Gly Lys Ala Gln Met Leu Glu Tyr Lys Asp
                85                  90                  95

Asn Gln Leu Cys Ser Gly Ser Asn Lys Pro Phe Phe Glu Ile Lys Asn
            100                 105                 110

Gly Val Pro Thr Val Gly Val Ile Asp Thr Ser Gly Lys Pro Val Val
        115                 120                 125

Val Ala Leu Asp Lys Leu Lys Thr
    130                 135

<210>  48

<211>  225

<212>  PRT

<213>  Escherichia coli

<400>  48

Met Gln Leu Pro Val Lys Leu Leu Met Ser Leu Ile Ser Leu Val Ser
1               5                   10                  15

Val Ile Ala Arg Ala Gly Lys Tyr Lys Asn Tyr Ile Arg Asp Glu Ile
            20                  25                  30

Lys Tyr Trp Arg Tyr Thr Ser Tyr Lys Gly Gly Glu Phe Pro Glu Gly
        35                  40                  45

Phe Thr Asp Glu Lys Phe Ser Ser Ala Ile Tyr Asn Gly Arg Ile Phe
    50                  55                  60

Thr Met Lys Arg Leu His Thr Leu Met Leu Phe Leu Ala Val Leu Phe
65                  70                  75                  80

Thr Gly Phe Asn Val Glu Ala Ala Ser Val Lys Gln Ala Leu Ser Cys
                85                  90                  95

Asp Pro Asn Ala Arg Ala Glu Gln Pro Gly Ala Cys Pro Thr Thr Tyr
            100                 105                 110

Glu Leu Tyr Glu Gly Asp Ala Ala Tyr Lys Ala Ala Leu Asp Lys Ala
        115                 120                 125

Leu Lys Pro Val Gly Leu Ser Gly Met Phe Gly Lys Gly Gly Tyr Met
    130                 135                 140

Asp Gly Pro Gly Gly Asn Val Thr Pro Val Thr Ile Asn Gly Thr Val
145                 150                 155                 160

Trp Leu Gln Gly Asp Gly Cys Lys Ala Asn Thr Cys Gly Trp Asp Phe
                165                 170                 175

Ile Val Thr Leu Tyr Asn Pro Lys Thr His Glu Val Val Gly Tyr Arg
            180                 185                 190

Tyr Phe Gly Leu Asp Asp Pro Ala Tyr Leu Val Trp Phe Gly Glu Ile
        195                 200                 205

Gly Val His Glu Phe Ala Tyr Leu Val Lys Asn Tyr Val Ala Ala Val
    210                 215                 220

Asn
225

<210>  49

<211>  721

<212>  PRT

<213>  Escherichia coli

<400>  49

Met Lys Thr Gln Ile Thr Phe Ala Ala Leu Leu Pro Ala Leu Ala Ser
1               5                   10                  15

Phe Ile Pro Leu His Ala His Ala Ser Ser Thr Ser Glu Asp Glu Met
            20                  25                  30

Ile Val Thr Gly Asn Thr Ala Ala Asp Thr Thr Asp Ser Ala Ala Gly
        35                  40                  45

Ala Gly Phe Lys Thr Asn Asp Ile Asp Val Gly Pro Leu Gly Thr Lys
    50                  55                  60

Ser Trp Ile Glu Thr Pro Tyr Ser Ser Thr Thr Val Thr Lys Glu Met
65                  70                  75                  80

Ile Glu Asn Gln Gln Ala Gln Ser Val Ser Glu Met Leu Lys Tyr Ser
                85                  90                  95

Pro Ser Thr Gln Met Gln Ala Arg Gly Gly Met Asp Val Gly Arg Pro
            100                 105                 110

Gln Ser Arg Gly Met Gln Gly Ser Val Val Ala Asn Ser Arg Leu Asp
        115                 120                 125

Gly Leu Asn Ile Val Ser Thr Thr Ala Phe Pro Val Glu Met Leu Glu
    130                 135                 140

Arg Met Asp Val Leu Asn Ser Leu Thr Gly Ala Leu Tyr Gly Pro Ala
145                 150                 155                 160

Ser Pro Ala Gly Gln Phe Asn Phe Val Ala Lys Arg Pro Thr Glu Glu
                165                 170                 175

Thr Leu Arg Lys Val Thr Leu Gly Tyr Gln Ser Arg Ser Ala Phe Thr
            180                 185                 190

Gly His Ala Asp Leu Gly Gly His Phe Asp Glu Asn Lys Arg Phe Gly
        195                 200                 205

Tyr Arg Val Asn Leu Leu Asp Gln Glu Gly Glu Gly Asn Val Asp Asp
    210                 215                 220

Ser Thr Leu Arg Arg Lys Leu Val Ser Val Ala Leu Asp Trp Asn Ile
225                 230                 235                 240

Gln Pro Gly Thr Gln Leu Gln Leu Asp Ala Ser His Tyr Glu Phe Ile
                245                 250                 255

Gln Lys Gly Tyr Val Gly Ser Phe Asn Tyr Gly Pro Asn Val Lys Leu
            260                 265                 270

Pro Ser Ala Pro Asn Pro Lys Asp Lys Asn Leu Ala Leu Ser Thr Ala
        275                 280                 285

Gly Asn Asp Leu Thr Thr Asp Thr Ile Ser Thr Arg Leu Ile His Tyr
    290                 295                 300

Phe Asn Asp Asp Trp Ser Met Asn Ala Gly Val Gly Trp Gln Gln Ala
305                 310                 315                 320

Asp Arg Ala Met Arg Ser Val Ser Ser Lys Ile Leu Asn Asn Gln Gly
                325                 330                 335

Asp Ile Ser Arg Ser Met Lys Asp Ser Thr Ala Ala Gly Arg Phe Arg
            340                 345                 350

Val Leu Ser Asn Thr Ala Gly Leu Asn Gly His Ile Asp Thr Gly Ser
        355                 360                 365

Ile Gly His Asp Leu Ser Leu Ser Thr Thr Gly Tyr Val Trp Ser Leu
    370                 375                 380

Tyr Ser Ala Lys Gly Thr Gly Ser Ser Tyr Ser Trp Gly Thr Thr Asn
385                 390                 395                 400

Met Tyr His Pro Asp Ala Ile Asp Glu Gln Gly Asp Gly Lys Ile Arg
                405                 410                 415

Thr Gly Gly Pro Arg Tyr Arg Ser Ser Val Asn Thr Gln Gln Ser Val
            420                 425                 430

Thr Leu Gly Asp Thr Val Thr Phe Thr Pro Gln Trp Ser Ala Met Phe
        435                 440                 445

Tyr Leu Ser Gln Ser Trp Leu Gln Thr Lys Asn Tyr Asp Lys His Gly
    450                 455                 460

Asn Gln Thr Asn Gln Val Asp Glu Asn Gly Leu Ser Pro Asn Ala Ala
465                 470                 475                 480

Leu Met Tyr Lys Ile Thr Pro Asn Thr Met Ala Tyr Val Ser Tyr Ala
                485                 490                 495

Asp Ser Leu Glu Gln Gly Gly Thr Ala Pro Thr Asp Glu Ser Val Lys
            500                 505                 510

Asn Ala Gly Gln Thr Leu Asn Pro Tyr Arg Ser Lys Gln Tyr Glu Val
        515                 520                 525

Gly Leu Lys Ser Asp Ile Gly Glu Met Asn Leu Gly Ala Ala Leu Phe
    530                 535                 540

Arg Leu Glu Arg Pro Phe Ala Tyr Leu Asp Thr Asp Asn Val Tyr Lys
545                 550                 555                 560

Glu Gln Gly Asn Gln Val Asn Asn Gly Leu Glu Leu Thr Ala Ala Gly
                565                 570                 575

Asn Val Trp Gln Gly Leu Asn Ile Tyr Ser Gly Val Thr Phe Leu Asp
            580                 585                 590

Pro Lys Leu Lys Asp Thr Ala Asn Ala Ser Thr Ser Asn Lys Gln Val
        595                 600                 605

Val Gly Val Pro Lys Val Gln Ala Asn Leu Leu Ala Glu Tyr Ser Leu
    610                 615                 620

Pro Ser Ile Pro Glu Trp Val Tyr Ser Ala Asn Val His Tyr Thr Gly
625                 630                 635                 640

Lys Arg Ala Ala Asn Asp Thr Asn Thr Ser Tyr Ala Ser Ser Tyr Thr
                645                 650                 655

Thr Trp Asp Leu Gly Thr Arg Tyr Thr Thr Lys Val Ser Asn Val Pro
            660                 665                 670

Thr Thr Phe Arg Val Val Val Asn Asn Val Phe Asp Lys His Tyr Trp
        675                 680                 685

Ala Ser Ile Phe Pro Ser Gly Thr Asp Gly Asp Asn Gly Ser Pro Ser
    690                 695                 700

Ala Phe Ile Gly Gly Gly Arg Glu Val Arg Ala Ser Val Thr Phe Asp
705                 710                 715                 720

Phe

<210> 50

<211> 669

<212> PRT

<213> Escherichia coli

<400> 50

Met Lys Asn Ile Thr Leu Trp Gln Arg Leu Arg Gln Val Ser Ile Ser
1               5                   10                  15

Thr Ser Leu Arg Cys Ala Phe Leu Met Gly Ala Leu Leu Thr Leu Ile
            20                  25                  30

Val Ser Ser Val Ser Leu Tyr Ser Trp His Glu Gln Ser Ser Gln Ile
        35                  40                  45

Arg Tyr Ser Leu Asp Lys Tyr Phe Pro Arg Ile His Ser Ala Phe Leu
    50                  55                  60

Ile Glu Gly Asn Leu Asn Leu Val Val Asp Gln Leu Asn Glu Phe Leu
65                  70                  75                  80

Gln Ala Pro Asn Thr Thr Val Arg Leu Gln Leu Arg Thr Gln Ile Ile
                85                  90                  95

Gln His Leu Asp Thr Ile Glu Arg Leu Ser Arg Gly Leu Ser Ser Arg
            100                 105                 110

Glu Arg Gln Gln Leu Thr Val Ile Leu Gln Asp Ser Arg Ser Leu Leu
        115                 120                 125

Ser Glu Leu Asp Arg Ala Leu Tyr Asn Met Phe Leu Leu Arg Glu Lys
    130                 135                 140

Val Ser Glu Leu Ser Ala Arg Ile Asp Trp Leu His Asp Asp Phe Thr
145                 150                 155                 160

Thr Glu Leu Asn Ser Leu Val Gln Asp Phe Thr Trp Gln Gln Gly Thr
                165                 170                 175

Leu Leu Asp Gln Ile Ala Ser Arg Gln Gly Asp Thr Ala Gln Tyr Leu
            180                 185                 190

Lys Arg Ser Arg Glu Val Gln Asn Glu Gln Gln Gln Val Tyr Thr Leu
        195                 200                 205

Ala Arg Ile Glu Asn Gln Ile Val Asp Asp Leu Arg Asp Arg Leu Asn
    210                 215                 220

Glu Leu Lys Ser Gly Arg Asp Asp Asp Ile Gln Val Glu Thr His Leu
225                 230                 235                 240

Arg Tyr Phe Glu Asn Leu Lys Lys Thr Ala Asp Glu Asn Ile Arg Met
                245                 250                 255

Leu Asp Asp Trp Pro Gly Thr Ile Thr Leu Arg Gln Thr Ile Asp Glu
            260                 265                 270

Leu Leu Asp Met Gly Ile Val Lys Asn Lys Met Pro Asp Thr Met Arg
        275                 280                 285

Glu Tyr Val Ala Ala Gln Lys Ala Leu Glu Asp Ala Ser Arg Thr Arg
    290                 295                 300

Glu Ala Thr Gln Gly Arg Phe Arg Thr Leu Leu Glu Ala Gln Leu Gly
305                 310                 315                 320

Ser Thr His Gln Gln Met Gln Met Phe Asn Gln Arg Met Glu Gln Ile
                325                 330                 335

Val His Val Ser Gly Gly Leu Ile Leu Val Ala Thr Ala Leu Ala Leu
            340                 345                 350

Leu Leu Ala Trp Val Phe Asn His Tyr Phe Ile Arg Ser Arg Leu Val
        355                 360                 365

Lys Arg Phe Thr Leu Leu Asn Gln Ala Val Val Gln Ile Gly Leu Gly
    370                 375                 380

Gly Thr Glu Thr Thr Ile Pro Val Tyr Gly Asn Asp Glu Leu Gly Arg
385                 390                 395                 400

Ile Ala Gly Leu Leu Arg His Thr Leu Gly Gln Leu Asn Val Gln Lys
                405                 410                 415

Gln Gln Leu Glu Gln Glu Ile Thr Asp Arg Lys Val Ile Glu Ala Asp
            420                 425                 430

Leu Arg Ala Thr Gln Asp Glu Leu Ile Gln Thr Ala Lys Leu Ala Val
        435                 440                 445

Val Gly Gln Thr Met Thr Thr Leu Ala His Glu Ile Asn Gln Pro Leu
    450                 455                 460

Asn Ala Leu Ser Met Tyr Leu Phe Thr Ala Arg Arg Ala Ile Glu Gln
465                 470                 475                 480

Thr Gln Lys Glu Gln Ala Ser Met Met Leu Gly Lys Ala Glu Gly Val
                485                 490                 495

Ile Ser Arg Ile Asp Ala Ile Ile Arg Ser Leu Arg Gln Phe Thr Arg
            500                 505                 510

Arg Ala Glu Leu Glu Thr Ser Leu His Ala Val Asp Leu Ala Gln Met
        515                 520                 525

Phe Ser Ala Ala Trp Glu Leu Leu Ala Met Arg His Arg Ser Leu Gln
    530                 535                 540

Ala Thr Leu Val Leu Pro Gln Gly Thr Ala Thr Val Ser Gly Asp Glu
545                 550                 555                 560

Val Arg Thr Gln Gln Val Leu Val Asn Val Leu Ala Asn Ala Leu Asp
                565                 570                 575

Val Cys Gly Gln Gly Ala Val Ile Thr Val Asn Trp Gln Met Gln Gly
            580                 585                 590

Lys Thr Leu Asn Val Phe Ile Gly Asp Asn Gly Pro Gly Trp Pro Glu
        595                 600                 605

Ala Leu Leu Pro Ser Leu Leu Lys Pro Phe Thr Thr Ser Lys Glu Val
    610                 615                 620

Gly Leu Gly Ile Gly Leu Ser Ile Cys Val Ser Leu Met Glu Gln Met
625                 630                 635                 640

Lys Gly Glu Leu Arg Leu Ala Ser Thr Met Thr Arg Asn Ala Cys Val
                645                 650                 655

Val Leu Gln Phe Arg Leu Thr Asp Val Glu Asp Ala Lys
            660                 665

<210>  51

<211>  753

<212>  PRT

<213>  Escherichia coli

<400>  51

Met Asn Val Ile Lys Leu Ala Ile Gly Ser Gly Ile Leu Leu Leu Ser
1               5                   10                  15

Cys Gly Ala Tyr Ser Gln Ser Ile Ser Glu Lys Thr Asn Ser Asp Lys
            20                  25                  30

Lys Gly Ala Ala Glu Phe Ser Pro Leu Ser Val Ser Val Gly Lys Thr
        35                  40                  45

Thr Ser Glu Gln Glu Ala Leu Glu Lys Thr Gly Ala Thr Ser Ser Arg
    50                  55                  60

Thr Thr Asp Lys Asn Leu Gln Ser Leu Asp Ala Thr Val Arg Ser Met
65                  70                  75                  80

Pro Gly Thr Tyr Thr Gln Ile Asp Pro Gly Gln Gly Ala Ile Ser Val
                85                  90                  95

Asn Ile Arg Gly Met Ser Gly Phe Gly Arg Val Asn Thr Met Val Asp
            100                 105                 110

Gly Ile Thr Gln Ser Phe Tyr Gly Thr Ser Thr Ser Gly Thr Thr Thr
        115                 120                 125

His Gly Ser Thr Asn Asn Met Ala Gly Val Leu Ile Asp Pro Asn Leu
    130                 135                 140

Leu Val Ala Val Asp Val Thr Arg Gly Asp Ser Ser Gly Ser Glu Gly
145                 150                 155                 160

Ile Asn Ala Leu Ala Gly Ser Ala Asn Met Arg Thr Ile Gly Val Asp
                165                 170                 175

Asp Val Ile Phe Asn Gly Asn Thr Tyr Gly Leu Arg Ser Arg Phe Ser
            180                 185                 190

Val Gly Ser Asn Gly Leu Gly Arg Ser Gly Met Ile Ala Leu Gly Gly
        195                 200                 205

Lys Ser Asp Ala Phe Thr Asp Thr Gly Ser Ile Gly Val Met Ala Ala
    210                 215                 220

Val Ser Gly Ser Ser Val Tyr Ser Asn Phe Ser Asn Gly Ser Gly Ile
225                 230                 235                 240

Asn Ser Lys Glu Phe Gly Tyr Asp Lys Tyr Met Lys Gln Asn Pro Lys
                245                 250                 255

Ser Gln Leu Tyr Lys Met Asp Ile Arg Pro Asp Glu Phe Asn Ser Phe
            260                 265                 270

Glu Leu Ser Ala Arg Thr Tyr Glu Asn Lys Phe Thr Arg Arg Asp Ile
        275                 280                 285

Thr Ser Asp Asp Tyr Tyr Ile Lys Tyr His Tyr Thr Pro Phe Ser Glu
    290                 295                 300

Leu Ile Asp Phe Asn Val Thr Ala Ser Thr Ser Arg Gly Asn Gln Lys
305                 310                 315                 320

Tyr Arg Asp Gly Ser Leu Tyr Thr Phe Tyr Lys Thr Ser Ala Gln Asn
                325                 330                 335

Arg Ser Asp Ala Leu Asp Ile Asn Asn Thr Ser Arg Phe Thr Val Ala
            340                 345                 350

Asp Asn Asp Leu Glu Phe Met Leu Gly Ser Lys Leu Met Arg Thr Arg
        355                 360                 365

Tyr Asp Arg Thr Ile His Ser Ala Ala Gly Asp Pro Lys Ala Asn Gln
    370                 375                 380

Glu Ser Ile Glu Asn Asn Pro Phe Ala Pro Ser Gly Gln Gln Asp Ile
385                 390                 395                 400

Ser Ala Leu Tyr Thr Gly Leu Lys Val Thr Arg Gly Ile Trp Glu Ala
                405                 410                 415

Asp Phe Asn Leu Asn Tyr Thr Arg Asn Arg Ile Thr Gly Tyr Lys Pro
            420                 425                 430

Ala Cys Asp Ser Arg Val Ile Cys Val Pro Gln Gly Ser Tyr Asp Ile
        435                 440                 445

Asp Asp Lys Glu Gly Gly Phe Asn Pro Ser Val Gln Leu Ser Ala Gln
    450                 455                 460

Val Thr Pro Trp Leu Gln Pro Phe Ile Gly Tyr Ser Lys Ser Met Arg
465                 470                 475                 480

Ala Pro Asn Ile Gln Glu Met Phe Phe Ser Asn Ser Gly Gly Ala Ser
                485                 490                 495

Met Asn Pro Phe Leu Lys Pro Glu Arg Ala Glu Thr Trp Gln Ala Gly
            500                 505                 510

Phe Asn Ile Asp Thr Arg Asp Leu Leu Val Glu Gln Asp Ala Leu Arg
        515                 520                 525

Phe Lys Ala Leu Ala Tyr Arg Ser Arg Ile Gln Asn Tyr Ile Tyr Ser
    530                 535                 540

Glu Ser Tyr Leu Val Cys Ser Gly Gly Arg Lys Cys Ser Leu Pro Glu
545                 550                 555                 560

Val Ile Gly Asn Gly Trp Glu Gly Ile Ser Asp Glu Tyr Ser Asp Asn
                565                 570                 575

Met Tyr Ile Tyr Val Asn Ser Ala Ser Asp Val Ile Ala Lys Gly Phe
            580                 585                 590

Glu Leu Glu Met Asp Tyr Asp Ala Gly Phe Ala Phe Gly Arg Leu Ser
        595                 600                 605

Phe Ser Gln Gln Gln Thr Asp Gln Pro Thr Ser Ile Ala Ser Thr His
    610                 615                 620

Phe Gly Ala Gly Asp Ile Thr Glu Leu Pro Arg Lys Tyr Met Thr Leu
625                 630                 635                 640

Asp Thr Gly Val Arg Phe Phe Asp Asn Ala Leu Thr Leu Gly Thr Ile
                645                 650                 655

Ile Lys Tyr Thr Gly Lys Ala Arg Arg Leu Ser Pro Asp Phe Glu Gln
            660                 665                 670

Asp Glu His Thr Gly Ala Ile Ile Lys Gln Asp Leu Pro Gln Ile Pro
        675                 680                 685

Thr Ile Ile Asp Leu Tyr Gly Thr Tyr Glu Tyr Asn Arg Asn Leu Thr
    690                 695                 700

Leu Lys Leu Ser Val Gln Asn Leu Met Asn Arg Asp Tyr Ser Glu Ala
705                 710                 715                 720

Leu Asn Lys Leu Asn Met Met Pro Gly Leu Gly Asp Glu Thr His Pro
                725                 730                 735

Ala Asn Ser Ala Arg Gly Arg Thr Trp Ile Phe Gly Gly Asp Ile Arg
            740                 745                 750

Phe

<210> 52

<211> 133

<212> PRT

<213> Escherichia coli

<400> 52

Met Ser Ser Lys Thr Lys Cys Trp Leu Trp Met Leu Leu Val Ile Leu
1               5                   10                  15

Ser Glu Thr Ser Ala Thr Ser Thr Leu Lys Met Phe Asp Asn Ser Glu
            20                  25                  30

Gly Met Thr Lys Thr Leu Leu Leu Ala Leu Ile Val Val Leu Tyr Cys
        35                  40                  45

Ile Cys Tyr Tyr Ser Leu Ser Arg Ala Val Lys Asp Ile Pro Val Gly
    50                  55                  60

Leu Ala Tyr Ala Thr Trp Ser Gly Thr Gly Ile Leu Met Val Ser Thr
65                  70                  75                  80

Leu Gly Ile Leu Phe Tyr Gly Gln His Pro Asp Thr Ala Ala Ile Ile
                85                  90                  95

Gly Met Val Ile Ile Ala Ser Gly Ile Ile Ile Met Asn Leu Phe Ser
            100                 105                 110

Lys Met Gly Ser Glu Glu Ala Glu Glu Thr Pro Val Thr Asn Leu Asp
        115                 120                 125

Lys Lys Ile Ala Asn
    130

<210>  53

<211>  286

<212>  PRT

<213>  Escherichia coli

<400>  53

Met Tyr Ile Lys Lys His Trp Ile Ala Leu Ser Ile Leu Leu Ile Pro
1               5                   10                  15

Cys Ile Gly Asn Ala Gln Glu Ile Lys Ile Asp Glu Ser Trp Leu His
            20                  25                  30

Gln Ser Leu Asn Val Ile Gly Arg Thr Asp Ser Arg Phe Gly Pro Arg
        35                  40                  45

Leu Thr Asn Asp Leu Tyr Pro Glu Tyr Thr Val Ala Gly Arg Lys Asp
    50                  55                  60

Trp Phe Asp Phe Tyr Gly Tyr Val Asp Leu Pro Lys Phe Phe Gly Val
65                  70                  75                  80

Gly Ser His Tyr Asp Val Gly Ile Trp Asp Glu Gly Ser Pro Leu Phe
                85                  90                  95

Thr Glu Ile Glu Pro Arg Phe Ser Ile Asp Lys Leu Thr Gly Leu Asn
            100                 105                 110

Leu Ala Phe Gly Pro Phe Lys Glu Trp Phe Ile Ala Asn Asn Tyr Val
        115                 120                 125

Tyr Asp Met Gly Asp Asn Gln Ser Ser Arg Gln Ser Thr Trp Tyr Met
    130                 135                 140

Gly Leu Gly Thr Asp Ile Asp Thr Gly Leu Pro Ile Lys Leu Ser Ala
145                 150                 155                 160

Asn Ile Tyr Ala Lys Tyr Gln Trp Gln Asn Tyr Gly Ala Ala Asn Glu
                165                 170                 175

Asn Glu Trp Asp Gly Tyr Arg Phe Lys Ile Lys Tyr Ser Ile Pro Leu
            180                 185                 190

Thr Asn Leu Phe Gly Gly Arg Leu Val Tyr Asn Ser Phe Thr Asn Phe
        195                 200                 205

Asp Phe Gly Ser Asp Leu Ala Asp Lys Ser His Asn Asn Lys Arg Thr
    210                 215                 220

Ser Asn Ala Ile Ala Ser Ser His Ile Leu Ser Leu Leu Tyr Glu His
225                 230                 235                 240

Trp Lys Phe Ala Phe Thr Leu Arg Tyr Phe His Asn Gly Gly Gln Trp
                245                 250                 255

Asn Ala Gly Glu Lys Val Asn Phe Gly Asp Gly Pro Phe Glu Leu Lys
            260                 265                 270

Asn Thr Gly Trp Gly Thr Tyr Thr Thr Ile Gly Tyr Gln Phe
        275                 280                 285

<210>  54

<211>  172

<212>  PRT

<213>  Escherichia coli

<400>  54

Met Arg Ile Ala Pro Arg Thr Phe Phe Ala Ile Ser Ala Leu Ala Phe
1               5                   10                  15

Ile Val Ala Ser Gly Phe Ser Phe Trp Arg Leu Ser Pro Ala Glu Asn
            20                  25                  30

Thr Gly Ile Met Ser Cys Ser Thr Lys Gly Ile Met Arg Phe Glu Asn
        35                  40                  45

Met Glu Lys Glu Asn Val Asn Gly Asn Ile His Phe Asn Phe Gly Ser
    50                  55                  60

Gln Gly Lys Gly Ser Met Val Leu Glu Gly Tyr Thr Asp Ser Ala Ala
65                  70                  75                  80

Gly Trp Leu Tyr Leu Gln Arg Tyr Val Lys Phe Thr Tyr Thr Ser Lys
                85                  90                  95

Arg Val Ser Ala Thr Glu Arg His Tyr Arg Ile Ser Gln Trp Glu Ser
            100                 105                 110

Ser Ala Ser Ser Ile Asp Glu Ser Pro Asp Val Ile Phe Asp Tyr Phe
        115                 120                 125

Met Arg Glu Met Ser Asp Ser His Asp Gly Leu Phe Leu Asn Ala Gln
    130                 135                 140

Lys Leu Asn Asp Lys Ala Ile Leu Leu Ser Ser Ile Asn Ser Pro Leu
145                 150                 155                 160

Trp Ile Cys Thr Leu Lys Ser Gly Ser Lys Leu Asp
                165                 170

<210> 55

<211> 182

<212> PRT

<213> Escherichia coli

<400> 55

Met Lys Ile Lys Val Ile Ala Leu Ala Thr Phe Val Ser Ala Val Phe
1               5                   10                  15

Ala Gly Ser Ala Met Ala Tyr Asp Gly Thr Ile Thr Phe Thr Gly Lys
            20                  25                  30

Val Val Ala Gln Thr Cys Thr Val Asn Thr Ser Asp Lys Asp Leu Ala
        35                  40                  45

Val Thr Leu Pro Thr Val Ala Thr Ser Ser Leu Lys Asp Asn Ala Ala
    50                  55                  60

Thr Ser Gly Leu Thr Pro Phe Ala Ile Arg Leu Thr Gly Cys Ala Thr
65                  70                  75                  80

Gly Met Asn Ser Ala Gln Asn Val Lys Ala Tyr Phe Glu Pro Ser Ser
                85                  90                  95

Asn Ile Asp Leu Ala Thr His Asn Leu Lys Asn Thr Ala Thr Pro Thr
            100                 105                 110

Lys Ala Asp Asn Val Gln Ile Gln Leu Leu Asn Ser Asn Gly Thr Ser
        115                 120                 125

Thr Ile Leu Leu Gly Glu Ala Asp Asn Gly Gln Asp Val Gln Ser Glu
    130                 135                 140

Thr Ile Gly Ser Asp Gly Ser Ala Thr Leu Arg Tyr Met Ala Gln Tyr
145                 150                 155                 160

Tyr Ala Thr Gly Gln Ser Thr Ala Gly Asp Val Lys Ala Thr Val His
                165                 170                 175

Tyr Thr Ile Ala Tyr Glu
            180

<210>  56

<211>  359

<212>  PRT

<213>  Escherichia coli


<400>  56

Met Lys Arg Ile Phe Phe Ile Pro Leu Phe Leu Ile Leu Leu Pro Lys
1               5                   10                  15

Leu Ala Val Ala Gly Pro Asp Asp Tyr Val Pro Ser Gln Ile Ala Val
            20                  25                  30

Asn Thr Ser Thr Leu Pro Gly Val Val Ile Gly Pro Ala Asp Ala His
        35                  40                  45

Thr Tyr Pro Arg Val Ile Gly Glu Leu Ala Gly Thr Ser Asn Gln Tyr
    50                  55                  60

Val Phe Asn Gly Gly Ala Ile Ala Leu Met Arg Gly Lys Phe Thr Pro
65                  70                  75                  80

Ala Leu Pro Lys Ile Gly Ser Ile Thr Val Tyr Phe Pro Ser Arg Lys
                85                  90                  95

Gln Arg Asp Ser Ser Asp Phe Asp Ile Tyr Asp Ile Gly Val Ser Gly
            100                 105                 110

Leu Gly Ile Ile Ile Gly Met Ala Gly Tyr Trp Pro Ala Thr Pro Leu
        115                 120                 125

Val Pro Ile Asn Ser Ser Gly Ile Tyr Ile Asp Pro Val Gly Ala Asn
    130                 135                 140

Thr Asn Pro Asn Thr Tyr Asn Gly Ala Thr Ala Ser Phe Gly Ala Arg
145                 150                 155                 160

Leu Phe Val Ala Phe Val Ala Thr Gly Arg Leu Pro Asn Gly Tyr Ile
                165                 170                 175

Thr Ile Pro Thr Arg Gln Leu Gly Thr Ile Leu Leu Glu Ala Lys Arg
            180                 185                 190

Thr Ser Leu Asn Asn Lys Gly Leu Thr Ala Pro Val Met Leu Asn Gly
        195                 200                 205

Gly Arg Ile Gln Val Gln Ser Gln Thr Cys Thr Met Gly Gln Lys Asn
    210                 215                 220

Tyr Val Val Pro Leu Asn Thr Val Tyr Gln Ser Gln Phe Thr Ser Leu
225                 230                 235                 240

Tyr Lys Glu Ile Gln Gly Gly Lys Ile Asp Ile His Leu Gln Cys Pro
                245                 250                 255

Asp Gly Ile Asp Val Tyr Ala Thr Leu Thr Asp Ala Ser Gln Pro Val
            260                 265                 270

Asn Arg Thr Asp Ile Leu Thr Leu Ser Ser Glu Ser Thr Ala Lys Gly
        275                 280                 285

Phe Gly Ile Arg Leu Tyr Lys Asp Ser Asp Val Thr Ala Ile Ser Tyr
    290                 295                 300

Gly Glu Asp Ser Pro Val Lys Gly Asn Gly Ser Gln Trp His Phe Ser
305                 310                 315                 320

Asp Tyr Arg Gly Glu Val Asn Pro His Ile Asn Leu Arg Ala Asn Tyr
                325                 330                 335

Ile Lys Ile Ala Asp Ala Thr Thr Pro Gly Ser Val Lys Ala Ile Ala
            340                 345                 350

Thr Ile Thr Phe Ser Tyr Gln
        355

<210>  57

<211>  844

<212>  PRT

<213>  Escherichia coli

<400>  57

Met Asn Ala Asn Asn Leu Ser Cys Leu Ile Tyr Cys Arg Cys Ser Leu
1               5                   10                  15

Leu Leu Phe Ala Ala Leu Gly Leu Thr Val Thr Asn His Ser Phe Ala
            20                  25                  30

Ala Glu Glu Ala Glu Phe Asp Ser Glu Phe Leu His Leu Asp Lys Gly
        35                  40                  45

Ile Asn Ala Ile Asp Ile Arg Arg Phe Ser His Gly Asn Pro Val Pro
    50                  55                  60

Glu Gly Arg Tyr Tyr Ser Asp Ile Tyr Val Asn Asn Val Trp Lys Gly
65                  70                  75                  80

Lys Ala Asp Leu Gln Tyr Leu Arg Thr Ala Asn Thr Gly Ala Pro Thr
                85                  90                  95

Leu Cys Leu Thr Pro Glu Leu Leu Ser Leu Ile Asp Leu Val Lys Asp
            100                 105                 110

Thr Met Ser Gly Asn Thr Ser Cys Phe Pro Ala Ser Thr Gly Leu Ser
        115                 120                 125

Ser Ala Arg Ile Asn Phe Asp Leu Ser Thr Leu Arg Leu Asn Ile Glu
    130                 135                 140

Ile Pro Gln Ala Leu Leu Asn Thr Arg Pro Arg Gly Tyr Ile Ser Pro
145                 150                 155                 160

Ala Gln Trp Gln Ser Gly Val Pro Ala Ala Phe Ile Asn Tyr Asp Ala
                165                 170                 175

Asn Tyr Tyr Gln Tyr Ser Ser Ser Gly Thr Ser Asn Glu Gln Thr Tyr
            180                 185                 190

Leu Gly Leu Lys Ala Gly Phe Asn Leu Trp Gly Trp Ala Leu Arg His
        195                 200                 205

Arg Gly Ser Glu Ser Trp Asn Asn Ser Tyr Pro Ala Gly Tyr Gln Asn
    210                 215                 220

Ile Glu Thr Ser Ile Met His Asp Leu Ala Pro Leu Arg Ala Gln Phe
225                 230                 235                 240

Thr Leu Gly Asp Phe Tyr Thr Asn Gly Glu Leu Met Asp Ser Leu Ser
                245                 250                 255

Leu Arg Gly Val Arg Leu Ala Ser Asp Glu Arg Met Leu Pro Gly Ser
            260                 265                 270

Leu Arg Gly Tyr Ala Pro Ala Val Arg Gly Ile Ala Asn Ser Asn Ala
        275                 280                 285

Lys Val Thr Ile Tyr Gln Asn Ala His Ile Leu Tyr Glu Thr Thr Val
    290                 295                 300

Pro Ala Gly Pro Phe Val Ile Asn Asp Leu Tyr Pro Ser Gly Tyr Ala
305                 310                 315                 320

Gly Asp Leu Leu Val Lys Ile Thr Glu Ser Asn Gly Gln Thr Arg Met
                325                 330                 335

Phe Thr Val Pro Phe Ala Ala Val Ala Gln Leu Ile Arg Pro Gly Phe
            340                 345                 350

Ser Arg Trp Gln Met Ser Val Gly Lys Tyr Arg Tyr Ala Asn Lys Thr
        355                 360                 365

Tyr Asn Asp Leu Ile Ala Gln Gly Thr Tyr Gln Tyr Gly Leu Thr Asn
    370                 375                 380

Asp Ile Thr Leu Asn Ser Gly Leu Thr Thr Ala Ser Gly Tyr Thr Ala
385                 390                 395                 400

Gly Leu Ala Gly Leu Ala Phe Asn Thr Pro Leu Gly Ala Ile Ala Ser
                405                 410                 415

Asp Ile Thr Leu Ser Arg Thr Ala Phe Arg Tyr Ser Gly Val Thr Arg
            420                 425                 430

Lys Gly Tyr Ser Leu His Ser Ser Tyr Ser Ile Asn Ile Pro Ala Ser
        435                 440                 445

Asn Thr Asn Ile Thr Leu Ala Ala Tyr Arg Tyr Ser Ser Lys Asp Phe
    450                 455                 460

Tyr His Leu Lys Asp Ala Leu Ser Ala Asn His Asn Ala Phe Ile Asp
465                 470                 475                 480

Asp Val Ser Val Lys Ser Thr Ala Phe Tyr Arg Pro Arg Asn Gln Phe
                485                 490                 495

Gln Ile Ser Ile Asn Gln Glu Leu Gly Glu Lys Trp Gly Gly Met Tyr
            500                 505                 510

Leu Thr Gly Thr Thr Tyr Asn Tyr Trp Gly His Lys Gly Ser Arg Asn
        515                 520                 525

Glu Tyr Gln Ile Gly Tyr Ser Asn Phe Trp Lys Gln Leu Gly Tyr Gln
    530                 535                 540

Ile Gly Leu Ser Gln Ser Arg Asp Asn Glu Gln Gln Arg Arg Asp Asp
545                 550                 555                 560

Arg Phe Tyr Ile Asn Phe Thr Leu Pro Leu Gly Gly Ser Val Gln Ser
                565                 570                 575

Pro Val Phe Ser Thr Val Leu Asn Tyr Ser Lys Glu Glu Lys Asn Ser
            580                 585                 590

Ile Gln Thr Ser Ile Ser Gly Thr Gly Gly Glu Asp Asn Gln Phe Ser
        595                 600                 605

Tyr Gly Ile Ser Gly Asn Ser Gln Glu Asn Gly Pro Ser Gly Tyr Ala
    610                 615                 620

Met Asn Gly Gly Tyr Arg Ser Pro Tyr Val Asn Ile Thr Thr Thr Val
625                 630                 635                 640

Gly His Asp Thr Gln Asn Asn Asn Gln Arg Ser Phe Gly Ala Ser Gly
                645                 650                 655

Ala Val Val Ala His Pro Tyr Gly Val Thr Leu Ser Asn Asp Leu Ser
            660                 665                 670

Asp Thr Phe Ala Ile Ile His Ala Glu Gly Ala Gln Gly Ala Val Ile
        675                 680                 685

Asn Asn Ala Ser Gly Ser Arg Leu Asp Phe Trp Gly Asn Gly Val Val
    690                 695                 700

Pro Tyr Val Thr Pro Tyr Glu Lys Asn Gln Ile Ser Ile Asp Pro Ser
705                 710                 715                 720

Asn Leu Asp Leu Asn Val Glu Leu Ser Ala Thr Glu Gln Glu Ile Ile
                725                 730                 735

Pro Arg Ala Asn Ser Ala Thr Leu Val Lys Phe Asp Thr Lys Thr Gly
            740                 745                 750

Arg Ser Leu Leu Phe Asp Ile Arg Met Ser Thr Gly Asn Pro Pro Pro
        755                 760                 765

Met Ala Ser Glu Val Leu Asp Glu His Gly Gln Leu Ala Gly Tyr Val
    770                 775                 780

Ala Gln Ala Gly Lys Val Phe Thr Arg Gly Leu Pro Glu Lys Gly His
785                 790                 795                 800

Leu Ser Val Val Trp Gly Pro Asp Asn Lys Asp Arg Cys Ser Phe Val
                805                 810                 815

Tyr His Val Ala His Asn Lys Asp Asp Met Gln Ser Gln Leu Val Pro
            820                 825                 830

Val Leu Cys Ile Gln His Pro Asn Gln Glu Lys Thr
        835                 840

<210>  58

<211>  277

<212>  PRT

<213>  Escherichia coli

<400>  58

Met Val Lys Cys His Thr Leu Ile Asn Arg Arg Asn Lys Cys Leu Leu
1               5                   10                  15

Ile Val Phe Ile Val Leu Ile Gly Trp Ile Ile Phe Arg Pro Lys Ala
            20                  25                  30

Tyr Thr Tyr Ser Leu Asn Asp Lys Glu Lys Glu Met Leu Ile Met Leu
        35                  40                  45

Ser Gln His Pro Glu Thr Arg Tyr Phe Gly Phe Tyr Ser Ile Glu Leu
    50                  55                  60

Pro Ala Asp Tyr Lys Pro Thr Gly Met Val Met Phe Ile Gln Gly Ser
65                  70                  75                  80

Ala Met Ile Pro Val Glu Thr Lys Leu Gln Tyr Tyr Pro Pro Phe Leu
                85                  90                  95

Gln Tyr Met Thr Arg Tyr Glu Ala Glu Leu Lys Asn Thr Ser Ala Leu
            100                 105                 110

Asp Pro Leu Asp Thr Pro Tyr Leu Lys Gln Val His Pro Leu Ser Pro
        115                 120                 125

Pro Met Asn Gly Val Ile Phe Glu Arg Met Lys Ala Lys Tyr Thr Pro
    130                 135                 140

Asp Phe Ala Arg Val Leu Asp Ala Trp Lys Trp Glu Asn Gly Val Thr
145                 150                 155                 160

Phe Ser Val Lys Ile Glu Ala Lys Asp Gly Arg Ala Thr Arg Tyr Asp
                165                 170                 175

Gly Ile Ser Lys Ile Ala Glu Tyr Ser Tyr Gly Tyr Asn Ile Pro Glu
            180                 185                 190

Lys Lys Val Gln Leu Leu Thr Ile Leu Ser Gly Leu Gln Pro Arg Ala
        195                 200                 205

Asp Asn Gln Pro Pro Ser Glu Asn Lys Leu Ala Ile Gln Tyr Ala Gln
    210                 215                 220

Val Asp Ala Ser Leu Leu Gly Glu Tyr Glu Leu Ser Val Asp Tyr Lys
225                 230                 235                 240

Asn Ser Asn Asn Ile Lys Ile Ser Leu Gln Thr Asp Asn Asn Ser Tyr
                245                 250                 255

Ile Asp Ser Leu Leu Asp Ile Arg Tyr Pro Ser Asn Gly Asn Arg Ala
            260                 265                 270

Trp Tyr Asn Ser Ile
        275

<210> 59

<211> 366

<212> PRT

<213> Escherichia coli

<400> 59

Met Leu Pro Glu Pro Val Tyr Arg Arg Trp Ile Ile Leu Leu Ile Ser
1               5                   10                  15

Met Leu Thr Val Gly Thr Leu Phe Ile Leu Ser Val Trp Asn Ser Ala
            20                  25                  30

Thr Tyr Trp Asp Ile Phe Ile Tyr Gly Val Leu Pro Met Leu Phe Leu
        35                  40                  45

Trp Leu Cys Leu Phe Gly Ile Ala Leu Asn Lys Tyr Glu Gln Ser Val
    50                  55                  60

Ala Ala Cys Ile Ser Trp Glu Ser Glu Arg Gln Gln Val Lys Gln Leu
65                  70                  75                  80

Trp Gln His Trp Ser Gln Lys Gln Leu Ala Ile Val Gly Asn Val Leu
                85                  90                  95

Phe Thr Pro Glu Glu Lys Gly Met Ser Val Leu Leu Gly Pro Gln Glu
            100                 105                 110

Glu Ile Pro Ala Tyr Pro Lys Lys Ala Arg Pro Leu Phe Ser Ala Ser
        115                 120                 125

Arg Tyr Ser Leu Ser Ser Ile Phe His Asp Ile His Gln Gln Leu Thr
    130                 135                 140

Gln Gln Phe Pro Asp Tyr Arg His Tyr Leu His Thr Ile Tyr Val Leu
145                 150                 155                 160

Gln Pro Glu Lys Trp Arg Gly Glu Thr Val Arg Gln Ala Ile Phe His
                165                 170                 175

Gln Trp Asp Leu Val Pro Glu Arg Thr Asn Thr Leu Asn Gln Ile Gln
            180                 185                 190

Ser Leu Tyr Asp Glu Arg Phe Asp Gly Leu Ile Leu Val Val Cys Leu
        195                 200                 205

Gln Asn Trp Pro Glu Asn Lys Pro Glu Asp Thr Ser Glu Leu Val Ser
    210                 215                 220

Ala Gln Leu Ile Ser Ser Ser Ser Phe Val Arg Gln His Gln Ile Pro
225                 230                 235                 240

Val Ile Ala Gly Leu Gly Arg Val Met Pro Leu Glu Pro Glu Glu Leu
                245                 250                 255

Glu His Asn Leu Asp Val Leu Phe Glu Tyr Asn Gln Leu Asp Asn Lys
            260                 265                 270

Gln Leu Gln His Val Trp Val Ser Gly Leu Asp Glu Gly Thr Ile Glu
        275                 280                 285

Asn Leu Met Gln Tyr Ala Glu Gln His Gln Trp Ser Leu Pro Lys Lys
    290                 295                 300

Arg Pro Leu His Met Ile Asp His Ser Phe Gly Pro Thr Gly Glu Phe
305                 310                 315                 320

Ile Phe Pro Val Ser Leu Ala Met Leu Ser Glu Ala Ala Lys Glu Thr
                325                 330                 335

Glu Gln Asn His Leu Ile Ile Tyr Gln Ser Ala Gln Tyr Ala Gln Lys
            340                 345                 350

Lys Ser Leu Cys Leu Ile Thr Arg Lys Leu Tyr Leu Arg Thr
        355                 360                 365

<210>  60

<211>  260

<212>  PRT

<213>  Escherichia coli


<400>  60

Met Leu Asn Arg Lys Leu Asn Ile Arg Leu Arg His Ser Leu Asn Ser
1               5                   10                  15

His Cys Ile Pro Ser Ile Ile Ile Asn Asn Thr Val Arg Ser Phe Gln
            20                  25                  30

Arg Ser Val Met Asn Thr Arg Ala Leu Phe Pro Leu Leu Phe Thr Val
        35                  40                  45

Ala Ser Phe Ser Ala Ser Ala Gly Asn Trp Ala Val Lys Asn Gly Trp
    50                  55                  60

Cys Gln Thr Met Thr Glu Asp Gly Gln Ala Leu Val Met Leu Lys Asn
65                  70                  75                  80

Gly Thr Ile Gly Ile Thr Gly Leu Met Gln Gly Cys Pro Asn Gly Val
                85                  90                  95

Gln Thr Leu Leu Gly Ser Arg Ile Ser Ile Asn Gly Asn Leu Ile Pro
            100                 105                 110

Thr Ser Gln Met Cys Asn Gln Gln Thr Gly Phe Arg Ala Val Glu Val
        115                 120                 125

Glu Ile Gly Gln Ala Pro Glu Met Val Lys Lys Ala Val His Ser Ile
    130                 135                 140

Ala Glu Arg Asp Val Ser Val Leu Gln Ala Phe Gly Val Arg Met Glu
145                 150                 155                 160

Phe Thr Arg Gly Asp Met Leu Lys Val Cys Pro Lys Phe Val Thr Ser
                165                 170                 175

Leu Ala Gly Phe Ser Pro Lys Gln Thr Thr Thr Ile Asn Lys Asp Ser
            180                 185                 190

Val Leu Gln Ala Ala Arg Gln Ala Tyr Ala Arg Glu Tyr Asp Glu Glu
        195                 200                 205

Thr Thr Glu Thr Ala Asp Phe Gly Ser Tyr Glu Val Lys Gly Asn Lys
    210                 215                 220

Val Glu Phe Glu Val Phe Asn Pro Glu Asp Arg Ala Tyr Asp Lys Val
225                 230                 235                 240

Thr Val Thr Val Gly Ala Asp Gly Asn Ala Thr Gly Ala Ser Val Glu
                245                 250                 255

Phe Ile Gly Lys
            260

<210>  61

<211>  385

<212>  PRT

<213>  Escherichia coli

<400> 61

Val Val Ile Ile Asn Ser Thr Ile Leu Ser Gly Ala Gly Ala Ile Pro
1               5                   10                  15

Ser Leu Thr Ser Leu Leu Pro Asp Ile Arg Lys Met Leu Leu Val Thr
            20                  25                  30

Asp Arg Asn Ile Ala Gln Leu Asp Gly Val Gln Gln Ile Arg Ala Leu
        35                  40                  45

Leu Glu Lys His Cys Pro Gln Val Asn Val Ile Asp Asn Val Pro Ala
    50                  55                  60

Glu Pro Thr His His Asp Val Arg Gln Leu Met Asp Ala Pro Gly Asp
65                  70                  75                  80

Ala Ser Phe Asp Val Val Val Gly Ile Gly Gly Gly Ser Val Leu Asp
                85                  90                  95

Val Ala Lys Leu Leu Ser Val Leu Cys His Pro Gln Ser Pro Gly Leu
            100                 105                 110

Asp Ala Leu Leu Ala Gly Glu Lys Pro Thr Gln Arg Val Gln Ser Trp
        115                 120                 125

Leu Ile Pro Thr Thr Ala Gly Thr Gly Ser Glu Ala Thr Pro Asn Ala
    130                 135                 140

Ile Leu Ala Ile Pro Glu Gln Ser Thr Lys Val Gly Ile Ile Ser Gln
145                 150                 155                 160

Val Leu Leu Pro Asp Tyr Val Ala Leu Phe Pro Glu Leu Thr Thr Ser
                165                 170                 175

Met Pro Ala His Ile Ala Ala Ser Thr Gly Ile Asp Ala Leu Cys His
            180                 185                 190

Leu Leu Glu Cys Phe Thr Ala Thr Val Ala Asn Pro Val Ser Asp Asn
        195                 200                 205

Ala Ala Leu Thr Gly Leu Ser Lys Leu Phe Arg His Ile Gln Pro Ala
    210                 215                 220

Val Asn Asp Pro Gln Asp Leu Arg Ala Lys Leu Glu Met Leu Trp Ala
225                 230                 235                 240

Ser Tyr Tyr Gly Gly Val Ala Ile Thr His Ala Gly Thr His Leu Val
                245                 250                 255

His Ala Leu Ser Tyr Pro Leu Gly Gly Lys Tyr His Leu Pro His Gly
            260                 265                 270

Val Ala Asn Ala Ile Leu Leu Ala Pro Cys Met Ala Phe Val Arg Pro
        275                 280                 285

Trp Ala Val Glu Lys Phe Ala Arg Val Trp Asp Cys Ile Pro Asp Ala
    290                 295                 300

Glu Thr Ala Leu Ser Ala Glu Glu Lys Ser His Ala Leu Val Thr Trp
305                 310                 315                 320

Leu Gln Ala Leu Val Asn Gln Leu Lys Leu Pro Asn Asn Leu Ala Ala
                325                 330                 335

Leu Gly Val Pro Pro Glu Asp Ile Ala Ser Leu Ser Glu Ala Ala Leu
            340                 345                 350

Asn Val Lys Arg Leu Met Asn Asn Val Pro Cys Gln Ile Asp Leu Gln
        355                 360                 365

Asp Val Gln Ala Ile Tyr Gln Thr Leu Phe Pro Gln His Pro Phe Lys
    370                 375                 380

Glu
385

<210>  62

<211>  105

<212>  PRT

<213>  Escherichia coli

<400>  62

Met Asn Ile Arg Lys Leu Phe Cys Pro Gly Asn Thr Pro Arg Ile Leu
1               5                   10                  15

Leu Phe Leu Phe Phe Phe Val Val Ser Ala Ile Thr Thr Ile Ala Cys
            20                  25                  30

Gly Tyr Thr Glu Lys Asn Ala Thr Gly Asn Val Leu Leu Leu Phe Leu
        35                  40                  45

Leu Leu Leu Leu Ala His Arg Asn Thr Leu Thr Ser Ile Thr Ala Leu
    50                  55                  60

Leu Phe Leu Phe Cys Cys Ala Leu Tyr Ala Pro Ala Gly Met Thr Tyr
65                  70                  75                  80

Gly Lys Ile Asn Asn Ser Phe Ile Val Ala Leu Leu Gln Thr Thr Thr
                85                  90                  95

Asp Glu Ala Ala Glu Phe Thr Gly Met
            100                 105

<210>  63

<211>  147

<212>  PRT

<213>  Escherichia coli

<400>  63

Met Asn Ile Gln Ala Ile Lys Glu Met Val Asn Leu Ile Cys Ser Phe
1               5                   10                  15

Leu Phe Ile Phe Phe Leu Ser Ser Ala Phe Val Ser Phe Gly Cys Tyr
            20                  25                  30

Ala Ile Tyr Glu Leu Phe Leu Trp Asn Asp Ile Ile Val Tyr Ser Trp
        35                  40                  45

Gly Tyr Ile Leu Ile Val Phe Leu Pro Phe Thr Leu Tyr Val Met Ser
    50                  55                  60

Phe Glu Ile Leu Phe Phe Ala Ile Ser Gly Arg Arg Leu Ser Lys Val
65                  70                  75                  80

Thr Met Val Arg Leu Trp Leu Ile Ile Lys Ile Ile Ile Ala Phe Ser
                85                  90                  95

Ile Cys Ala Val Leu Ile Phe Ser Ser Ile Tyr Lys Lys Glu Leu Leu
            100                 105                 110

Ser Arg Asn Tyr Ile Ala Cys Ser Gly Ile Pro Ser Gly Trp Met Pro
        115                 120                 125

Gly Leu Ala Thr Lys Tyr Val Lys Glu Lys Ser Leu Cys Glu Lys Asn
    130                 135                 140

Gly Asn Asn
145

<210> 64

<211> 178

<212> PRT

<213> Escherichia coli

<400> 64

Met Phe Pro Ile Arg Phe Lys Arg Pro Ala Leu Leu Cys Met Ala Met
1               5                   10                  15

Leu Thr Val Val Leu Ser Gly Cys Gly Leu Ile Gln Lys Val Val Asp
            20                  25                  30

Glu Ser Lys Ser Val Ala Ser Ala Val Phe Tyr Lys Gln Ile Lys Ile
        35                  40                  45

Leu His Leu Asp Phe Phe Ser Arg Ser Ala Leu Asn Thr Asp Ala Glu
    50                  55                  60

Asp Thr Pro Leu Ser Thr Met Val His Val Trp Gln Leu Lys Thr Arg
65                  70                  75                  80

Glu Asp Phe Asp Lys Ala Asp Tyr Asp Thr Leu Phe Met Gln Glu Glu
                85                  90                  95

Lys Thr Leu Glu Lys Asp Val Leu Ala Lys His Thr Val Trp Val Lys
            100                 105                 110

Pro Glu Gly Thr Ala Ser Leu Asn Val Pro Leu Asp Lys Glu Thr Gln
        115                 120                 125

Phe Val Ala Ile Ile Gly Gln Phe Tyr His Pro Asp Glu Lys Ser Asp
    130                 135                 140

Ser Trp Arg Leu Val Ile Lys Arg Asp Glu Leu Glu Ala Asp Lys Pro
145                 150                 155                 160

Arg Ser Ile Glu Leu Met Arg Ser Asp Leu Arg Leu Leu Pro Leu Lys
                165                 170                 175

Asp Lys

<210>  65

<211>  209

<212>  PRT

<213>  Escherichia coli

<400>  65

Met Phe Leu Lys Arg Lys Trp Tyr Tyr Ala Val Thr Thr Ser Val Val
1               5                   10                  15

Ile Thr Leu Cys Gly Gly Gly Tyr Tyr Met Tyr Arg Gln Glu Tyr Gln
            20                  25                  30

Met Val Val Thr Val Pro Thr Ala Asp Ala Asn Asp Pro Asn Trp Pro
        35                  40                  45

Asn Lys Arg Ile Gln Phe Asp Thr Ser Glu Trp Leu Gln Gln Leu Gln
    50                  55                  60

Tyr Ile Lys Ile Asp Asp His Tyr Ile Leu Asn Thr Gln Tyr Thr Pro
65                  70                  75                  80

Ile Ala Asn Leu Asp Asp Phe Gly Ile Thr Leu Lys Leu Gln Asn Ala
                85                  90                  95

Leu Asn Gly Ser Asp Lys Arg Leu Pro Ala Leu Tyr Gly Leu Ala Glu
            100                 105                 110

Met Asp Ala Gln Lys Phe Lys Asp Leu Met Arg Gly Lys Ile Lys Cys
        115                 120                 125

Glu Tyr Leu Arg Thr Thr Phe Asp Ala Glu Thr Leu Lys Pro Val Asn
    130                 135                 140

Asp Tyr Phe Leu Ile Ser Phe Thr Tyr Lys Asp Lys Trp Tyr Glu Phe
145                 150                 155                 160

Glu Thr Glu Arg Lys Ile Ser Lys Thr Ser Asp Asp Gly Tyr Phe Leu
                165                 170                 175

Trp Ala Phe Asp Asn Thr Val His Glu Ala Gly Tyr Trp His Asn Thr
            180                 185                 190

Asp Pro Ala Ala Tyr Ser Tyr Arg Asp Tyr Gln Asn Gly Lys Ala Val
        195                 200                 205

Lys

<210> 66

<211> 424

<212> PRT

<213> Escherichia coli

<400> 66

Met Asp Ile Trp Arg Gly His Ser Phe Leu Met Thr Ile Ser Ala Arg
1               5                   10                  15

Phe Arg Gln Tyr Val Phe Ser Leu Met Ser Ile Leu Leu Gln Glu Arg
            20                  25                  30

Lys Met Asn Ile Phe Thr Leu Ser Lys Ala Pro Leu Tyr Leu Leu Ile
        35                  40                  45

Ser Leu Phe Leu Pro Thr Met Ala Met Ala Ile Asp Pro Pro Glu Arg
    50                  55                  60

Glu Leu Ser Arg Phe Ala Leu Lys Thr Asn Tyr Leu Gln Ser Pro Asp
65                  70                  75                  80

Glu Gly Val Tyr Glu Leu Ala Phe Asp Asn Ala Ser Lys Lys Val Phe
                85                  90                  95

Ala Ala Val Thr Asp Arg Val Asn Arg Glu Ala Asn Lys Gly Tyr Leu
            100                 105                 110

Tyr Ser Phe Asn Ser Asp Ser Leu Lys Val Glu Asn Lys Tyr Thr Met
        115                 120                 125

Pro Tyr Arg Ala Phe Ser Leu Ala Ile Asn Gln Asp Lys His Gln Leu
    130                 135                 140

Tyr Ile Gly His Thr Gln Ser Ala Ser Leu Arg Ile Ser Met Phe Asp
145                 150                 155                 160

Thr Pro Thr Gly Lys Leu Val Arg Thr Ser Asp Arg Leu Ser Phe Lys
                165                 170                 175

Ala Ala Asn Ala Ala Asp Ser Arg Phe Glu His Phe Arg His Met Val
            180                 185                 190

Tyr Ser Gln Asp Ser Asp Thr Leu Phe Val Ser Tyr Ser Asn Met Leu
        195                 200                 205

Lys Thr Ala Glu Gly Met Lys Pro Leu His Lys Leu Leu Met Leu Asp
    210                 215                 220

Gly Thr Thr Leu Ala Leu Lys Gly Glu Val Lys Asp Ala Tyr Lys Gly
225                 230                 235                 240

Thr Ala Tyr Gly Leu Thr Met Asp Glu Lys Thr Gln Lys Ile Tyr Val
                245                 250                 255

Gly Gly Arg Asp Tyr Ile Asn Glu Ile Asp Ala Lys Asn Gln Thr Leu
            260                 265                 270

Leu Arg Thr Ile Pro Leu Lys Asp Pro Arg Pro Gln Ile Thr Ser Val
        275                 280                 285

Gln Asn Leu Ala Val Asp Ser Ala Ser Asp Arg Ala Phe Val Val Val
    290                 295                 300

Phe Asp His Asp Asp Arg Ser Gly Thr Lys Asp Gly Leu Tyr Ile Phe
305                 310                 315                 320

Asp Leu Arg Asp Gly Lys Gln Leu Gly Tyr Val His Thr Gly Ala Gly
                325                 330                 335

Ala Asn Ala Val Lys Tyr Asn Pro Lys Tyr Asn Glu Leu Tyr Val Thr
            340                 345                 350

Asn Phe Thr Ser Gly Thr Ile Ser Val Val Asp Ala Thr Lys Tyr Ser
        355                 360                 365

Ile Thr Arg Glu Phe Asn Met Pro Val Tyr Pro Asn Gln Met Val Leu
    370                 375                 380

Ser Asp Asp Met Asp Thr Leu Tyr Ile Gly Ile Lys Glu Gly Phe Asn
385                 390                 395                 400

Arg Asp Trp Asp Pro Asp Val Phe Val Glu Gly Ala Lys Glu Arg Ile
                405                 410                 415

Leu Ser Ile Asp Leu Lys Lys Ser
            420

<210>  67

<211>  489

<212>  DNA

<213>  Escherichia coli

<400>  67
atgaaactga aagctattat attggccacc ggtcttatta actgtattgt attttcagca      60

caggcagtgg atacgacgat tactgtgacg ggtaatgttt tgcaaagaac atgtaatgta     120

ccagggaatg tggatgtttc tttgggtaat ctgtatgtat cagactttcc caatgcagga     180

agtggatctc catgggttaa ttttgatctg tctctcaccg gatgccagaa tatgaatact     240

gttcgggcaa catttagtgg tactgcggat gggcagacat actatgcgaa tacagggaat     300

gctggcggta tcaagattga aattcaggac agggatggaa gtaatgcatc atatcacaat     360

ggtatgttca agacgcttaa tgtacaaaat aataatgcaa cctttaatct taaagcccgt     420

gcagtgagta aaggccaggt tactcctgga aatatcagtt ctgttataac cgtcacctat     480

acctatgcg                                                             489

<210>  68

<211>  2019

<212>  DNA

<213>  Escherichia coli

<400>  68
   atgaaaatga cacggcttta tcctctggcc ttggggggat tattgctccc cgccattgct      60
   aatgcccaga cttcacagca agacgaaagc acgctggtgg ttaccgccag taaacaatct     120
   tcccgctcgg catcagccaa caacgtctcg tctactgttg tcagcgcgcc ggaattaagc     180
   gacgccggcg tcaccgccag cgacaaactc cccagagtct tgcccgggct caatattgaa     240
   aatagcggca acatgctttt ttcgacgatc tcgctacgcg gcgtctcttc agcgcaggac     300
   ttctataacc ccgccgtcac cctgtatgtc gatggcgtcc ctcagctttc caccaacacc     360
   atccaggcgc ttaccgatgt gcaaagcgtg gagttgctgc gaggcccaca gggaacgtta     420
   tatggcaaaa gcgctcaggg cgggatcatc aacatcgtca cccagcagcc ggacagcacg     480
   ccgcgcggct atattgaagg cggcgtcagt agccgcgaca gttatcgaag taagttcaac     540
   ctgagcggcc ccattcagga tggcctgctg tacggcagcg tcaccctgtt acgccaggtt     600
   gatgacggcg acatgattaa ccccgcgacg ggaagcgatg acttaggcgg cacccgcgcc     660
   agcataggga atgtgaaact gcgtctggcg ccggacgatc agccctggga aatgggcttt     720
   gccgcctcac gcgaatgtac ccgcgccacc caggacgcct atgtgggatg gaatgatatt     780
   aagggccgta agctgtcgat cagcgatggt tcaccagacc cgtacatgcg gcgctgcact 840
   gacagccaga ccctgagtgg gaaatacacc accgatgact gggttttcaa cctgatcagc 900
   gcctggcagc agcagcatta ttcgcgcacc ttcccttccg gttcgttaat cgtcaatatg 960
   tctcagcgct ggaatcagga tgtgcaggag ctgcgcgctg caaccctggg cgatgcgcgt 1020
   accgttgata tggtgtttgg gctgtaccgg cagaacaccc gcgagaagtt aaattcagcc 1080
   tacgacatgc cgacaatgcc ttatttaagc agtaccggct ataccaccgc tgaaacgctg 1140
   gccgcataca gtgacctgac ctggcattta accgatcgtt ttgatatcgg cggcggcgtg 1200
   cgcttctcgc atgataaatc cagtacacaa tatcacggca gcatgctcgg caacccgttt 1260
   ggcgaccagg gtaagagcaa tgacgatcag gtgctcgggc agctatccgc aggctatatg 1320
   ctgaccgatg actggagagt gtatacccgt gtagcccagg gatataaacc ttccgggtac 1380
   aacatcgtgc ctactgcggg tcttgatgcc aaaccgttcg tcgccgagaa atccatcaac 1440
   tatgaacttg gcacccgcta cgaaaccgct gacgtcacgc tgcaagccgc gacgttttat 1500
   acccacacca aagacatgca gctttactct ggcccggtcg ggatgcagac attaagcaat 1560
   gcgggtaaag ccgacgccac cggcgttgag cttgaagcga agtggcggtt tgcgccaggc 1620
   tggtcatggg atatcaatgg caacgtgatc cgttccgaat tcaccaatga cagtgagttg 1680
   tatcacggta accgggtgcc gttcgtacca cgttatggcg cgggaagcag cgtgaacggc 1740
   gtgattgata cgcgctatgg cgcactgatg ccccgactgg cggttaatct ggtcggcccg 1800
   cattatttcg atggcgacaa ccagttgcgg caaggcacct atgccaccct ggacagcagc 1860
   ctgggctggc aggcgactga acggatgaac atttccgtct atgtcgataa cctgttcgac 1920
   cgtcgttacc gtacctatgg ctacatgaac ggcagcagcg ccgtcgcgca ggtcaatatg 1980
   ggtcgcaccg tcggtatcaa tacgcgaatt gatttcttc 2019

<210> 69

<211> 738

<212> DNA

<213> Escherichia coli

<400> 69
atgaataagg tttttgttgt ttcagtggtg gccgcagcct gtgtatttgc agtaaatgca 60

ggagcaaagg aaggtaaaag cggtttttat ctgaccggta aagccggtgc ctctgtgatg 120

tcactttcag accagcgttt cctgtcagga gatgaggaag aaacatcaaa gtataaaggc 180

ggcgatgacc atgatacggt attcagtggc ggtattgcgg tcggttatga tttttatccg 240

cagttcagta ttccggttcg tacagaactg gagttttacg ctcgtggaaa agctgattcg 300

aagtataacg tagataaaga cagctggtca ggtggttact ggcgtgatga cctgaagaat 360

gaggtgtcag tcaacacact aatgctgaat gcgtactatg acttccggaa tgacagcgca 420

ttcacaccat gggtatccgc agggattggc tacgccagaa ttcaccagaa aacaaccggt 480

atcagtacct gggattatga gtacggaagc agtggtcgcg aatcgttgtc acgttcaggc 540

tctgctgaca acttcgcatg gagccttggc gcgggtgtcc gctatgacgt aaccccggat 600

atcgctctgg acctcagcta tcgctatctt gatgcaggtg acagcagtgt gagttacaag 660

gacgagtggg gcgataaata taagtcagaa gttgatgtta aaagtcatga catcatgctt 720

ggtatgactt ataacttc 738

<210> 70

<211> 498

<212> DNA

<213> Escherichia coli

<400> 70
atgaaactga aagctattat attggccacc ggtcttatta actgtattgc attttcagca 60

caggcagtgg atacgacgat tactgttaca gggagggtat tgccacgtac ctgtaccatt 120

ggtaatggag gaaacccaaa cgccaccgtt gttttggata acgcttacac ttctgacctg 180

atagcagcca acagcacctc tcagtggaaa aatttttcgt tgacattgac gaattgtcag 240

aatgtaaaca atgttacttc atttggtgga accgcagaaa atacaaatta ttacagaaat 300

acaggggatg ctactaatat catggttgag ctacaggaac aaggtaatgg taataccccc 360

ttgaaagttg gttcaacaaa agttgttaca gtgagcaatg ggcaggcgac attcaatctt 420

aaagtccgtg ccgtaagcaa aggtaatgct ggtgcgggaa gtattaattc acaaattact 480

gtcacctata cctatgcg 498

<210> 71

<211> 3885

<212> DNA

<213> Escherichia coli

<400> 71
atgaataaaa tatactccct taaatatagt gctgccactg gcggactcat tgctgtttct 60

gaattagcga aaagagtttc tggtaaaaca aaccgaaaac ttgtagcaac aatgttgtct 120

ctggctgttg ccggtacagt aaatgcagca aatattgata tatcaaatgt atgggcgaga 180

gactatcttg atcttgcaca aaataaaggt attttccagc ccggagcaac agacgtaaca 240

atcactttaa aaaacggaga taaattctct ttccataatc tctcaattcc ggatttttct 300

ggtgcagcag cgagtggcgc agctaccgca ataggaggtt cttatagtgt tactgttgca 360

cataacaaaa agaaccctca ggccgcagaa acccaggttt acgctcagtc ttcttacagg 420

gttgttgaca gaagaaattc caatgatttt gagattcaga ggttaaataa atttgttgtg 480

gaaacagtag gtgccacccc ggcagagacc aaccctacaa catattctga tgcattagaa 540

cgctacggta tagtcacttc tgacggttca aaaaaaatca taggttttcg tgctggctct 600

ggaggaacat catttattaa tggtgaatcc aaaatctcaa caaattcagc atatagccat 660

gatctgttaa gtgctagtct atttgaggtc acccaatggg actcatacgg catgatgatt 720

tataaaaatg ataaaacatt tcgtaatctt gaaatattcg gagacagcgg ctctggagca 780

tacttatatg ataacaaact agaaaaatgg gtattagtcg gaacaaccca tggtattgcc 840

agcgttaatg gtgaccaact gacatggata acaaaataca atgataaact ggttagtgag 900

ttaaaagata cctatagtca taaaataaat ctgaatggca ataatgtaac cattaaaaac 960

acagatataa cattacacca aaacaatgca gataccactg gtactcaaga aaaaataact 1020

aaagacaaag atattgtgtt cacaaatggg ggagatgtcc tgtttaagga taatttggat 1080

tttggtagcg gtggtattat ctttgacgaa ggccatgaat ataacataaa cggtcaggga 1140

tttacattta aaggagcagg aattgatatc ggaaaagaaa gcattgtaaa ctggaatgca 1200

ttgtattcca gtgatgatgt tttacacaaa ataggccccg gtactctgaa tgttcaaaaa 1260

aaacaggggg caaatataaa gataggtgaa ggaaatgtta ttcttaatga agaaggaaca 1320

tttaacaata tataccttgc aagcggaaat ggtaaggtaa tactaaataa agataattcc 1380

cttggcaatg atcaatatgc ggggatattt tttactaaac gtggtggtac gctagattta 1440

aatggacaca atcagacttt tactagaatt gccgccactg acgatggaac aacaataact 1500

aactcagata caacgaaaga agccgttctg gcaatcaata acgaagactc ctacatatat 1560

catgggaaca taaatggcaa tataaaacta acgcacaata ttaattctca ggataagaaa 1620

actaatgcaa aattaattct ggatggtagt gtcaacacaa aaaatgatgt tgaagtcagt 1680

aatgccagtc ttaccatgca aggccatgca acagagcatg caatattcag aagctcagcg 1740

aatcattgct ccctggtatt tctttgtgga acggactggg tcaccgtttt gaaagaaaca 1800

gagagttcat ataataaaaa attcaattct gattacaaaa gtaataatca gcagacctca 1860

tttgatcagc ctgactggaa aaccggggtg tttaaatttg atacattaca cctgaacaat 1920

gctgactttt caatatcacg caatgccaat gttgaaggaa atatatcagc aaataaatca 1980

gctatcacaa tcggcgataa aaatgtttac attgataatc ttgcagggaa aaatattact 2040

aataatggtt ttgacttcaa acaaactatc agtactaatc tatccatagg agaaactaaa 2100

tttacaggtg gcatcactgc acataacagc caaatagcca taggtgatca agctgtagtt 2160
acacttaatg gtgcaacctt tctggataat actcctataa gtatagataa aggagcaaaa 2220
gttatagcac aaaattccat gttcacaaca aaaggtattg atatctccgg tgaactgact 2280
atgatgggaa tccctgaaca gaatagtaaa actgtaacgc cgggtctcca ctacgctgct 2340
gatggattca ggctgagtgg tggaaatgca aatttcattg ccagaaatat ggcatctgtc 2400
accggaaata tttatgctga tgatgcagca accattactc tgggacagcc tgaaactgaa 2460
acaccgacta tatcgtctgc ttatcaggca tgggcagaga ctcttttgta tggctttgat 2520
accgcttatc gaggcgcaat aacagccccc aaagctacag ttagcatgaa taatgcgatc 2580
tggcatctaa atagccagtc atcaattaat cgtctagaaa caaaagacag tatggtgcgt 2640
tttactggtg ataatgggaa gtttacaacc cttacagtga acaaccttac tatagatgac 2700
agtgcatttg tgctgcgtgc aaatctggcc caagcagatc agcttgttgt caataaatcg 2760
ttgtctggta aaaacaacct tctgttagtc gacttcattg agaaaaatgg aaacagcaac 2820
ggactgaata tcgatctggt cagcgcacca aaaggaactg cagtagatgt ctttaaagct 2880
acgactcgga gtattggctt cagtgatgta acaccggtta tcgagcaaaa gaacgataca 2940
gacaaagcaa catggactct gatcggctat aaatctgtgg ccaacgccga tgcggctaaa 3000
aaggcaacat tactgatgtc aggcggctat aaagccttcc ttgctgaggt caacaacctt 3060
aacaaacgta tgggtgatct gcgtgacatt aacggtgagt ccggtgcatg ggcccgaatc 3120
attagcggaa ccgggtctgc cggcggtgga ttcagtgaca actacaccca cgttcaggtc 3180
ggtgcggata acaaacatga actcgatggc cttgacctct tcaccggggt gaccatgacc 3240
tataccgaca gccatgcagg cagtgatgcc ttcagtggtg aaacgaagtc tgtgggtgcc 3300
ggtctctatg cctctgccat gtttgagtcc ggagcatata tcgacctcat cggtaagtac 3360
gttcaccatg acaacgagta taccgcaact ttcgccggcc ttggcaccag agactacagc 3420
tcccactcct ggtatgccgg tgcggaagtc ggttaccgtt accatgtaac tgactctgca 3480
tggattgagc cgcaggcgga acttgtttac ggtgctgtat ccgggaaaca gttctcctgg 3540
aaggaccagg gaatgaacct caccatgaag gataaggact ttaatccgct gattgggcgt 3600
accggtgttg atgtgggtaa atccttctcc ggtaaggact ggaaagtcac agcccgcgcc 3660
ggccttggct accagtttga cctgtttgcc aacggtgaaa ccgtactgcg tgatgcgtcc 3720
ggtgagaaac gtatcaaagg tgaaaaagac ggtcgtatgc tcatgaatgt tggtctcaac 3780
gccgaaattc gcgataatct tcgcttcggt cttgagtttg agaaatcggc atttggtaaa 3840
tacaacgtgg ataacgcgat caacgccaac ttccgttact ctttc 3885

<210> 72

<211> 426

<212>  DNA

<213>  Escherichia coli

<400>  72
   atgattaata ttcccagtcc caccgctgtt gttatggcgc tggtagccat cagcacgctt      60
   cccagcccta gcagggtaaa gcttatgcca tatcctccca gagcccacaa caccacaggt     120
   ttactgccag tacgggaaat ttgctttccc caccacgggg acgatggcag aaacagcatt     180
   gagccaagca tcagcagggc agcccataca gacagactca gatttgtctg tatgaccaga     240
   acagggagca caaccagcag accgttctgc ccgataccga gaagcccggc actgaacgca     300
   agtggccagc aggacagtgg tttttggggc gtatcttcga tcccaggtga cattttaatg      360
   tttcaactcc atgtattaat tgtgtttatt tgtaaaatta atttatctga caataacatt     420
   tcttat                                                                426

<210>  73

<211>  954

<212>  DNA

<213>  Escherichia coli

<400>  73
atgtatgccc gcgagtatcg ctcaacacgc ccgcataaag cgattttctt tcatctttct      60

tgcctcaccc ttatctgtag tgcgcaagtt tatgcgaagc cggatatgcg gccactgggg     120

ccgaatatag ccgataaagg ctccgtgttt taccatttca gcgccacctc tttcgactct     180

gtcgatggca cacgccatta tcgggtatgg acggccgtgc cgaatacaac cgcaccggca     240

tcgggttacc cgattttata tatgcttgac ggtaacgcag ttatggaccg cctggatgac     300

gaactgctca aacaattgtc agaaaaaaca ccgccagtga tcgtggctgt cgggtatcag     360

accaacctcc ctttcgatct caacagcagg gcttacgact atacgccagc agcagaaagc     420

agaaaaacag atctccactc agggcgtttt agccgtaaga gtggtggcag caacaacttc     480

cgccagttac tggaaacgcg tattgcccca aaagtggaac agggactgaa tatcgatcgg     540

caacgccgcg gcttatgggg gcactcctac ggcggcctct tcgtgctgga ttcctggctg     600

tcctcctctt acttccggtc gtactacagc gccagcccgt cgttgggcag aggttatgat     660

gctttgctaa gccgcgttac ggcggttgag cctctgcaat tctgcaccaa acacctggcg     720

ataatggaag gctcggcgac acagggtgat aaccgggaaa cgcatgctgt cggggtgctg      780

tcgaaaattc ataccaccct cactatactg aaagataaag gcgtcaatgc cgtattttgg     840

gatttcccca acctgggaca cgggccgatg ttcaatgcct cctttcgcca ggcactgtta 900

gatatcagtg gtgaaaacgc aaattacaca gcaggttgtc atgagttaag ccac 954

<210> 74

<211> 2175

<212> DNA

<213> Escherichia coli

<400> 74
atgagaatta acaaaatcct ctggtcgcta actgtgctcc tagttgggtt gaatagccag 60

gtatcagtag ccaaatactc cgacgatgat aatgacgaga ctctggtggt ggaagccacc 120

gctgagcagg tattaaaaca gcagccgggc gtgtcggtta ttaccagcga ggatattaaa 180

aagacccctc cggtaaacga cctttcagat attattcgta aaatgcctgg tgttaatctt 240

accggcaata gcgcctcggg cacacgcggt aataaccgcc agatcgatat tcgtggtatg 300

gggccggaaa acaccttaat tttaattgat ggtgtaccgg tgacgtcacg taactccgtg 360

cgttatagct ggcgtgggga gcgtgatacc cgcggtgaca ccaactgggt gccaccggaa 420

caggttgagc gtattgaagt gatccgcggc cctgcggcgg cgcgctacgg ttcgggggcc 480

gccggggggg tggtgaacat cattaccaaa cgtcccacca acgactggca cggttcgctg 540

tcgttataca ccaaccagcc ggaaagtagc gaagagggcg ctacgcgtcg cgccaatttc 600

agccttagtg ggcctctggc tggtgatgct cttaccacgc gtttgtatgg taacctgaat 660

aaaacggatg ctgacagttg ggatattaat tctccggtcg gtacgaaaaa cgcagccggg 720

catgaagggg tacgtaacaa agatattaac ggcgttgtct cgtggaaatt aaatccgcag 780

cagattctcg atttcgaagt cggatatagc cgccagggga atatctatgc gggcgatacg 840

cagaacagtt cttccagtgc agttaccgaa agcctggcaa aatccggcaa agagacgaac 900

cgcctgtacc gacagaatta tggcattacg cataatggta tctgggactg gggacaaagt 960

cgctttggtg tttattacga gaaaaccaat aatacccgca tgaatgaagg attatccggc 1020

ggtggtgaag gacgtatttt agcgggtgaa aagtttacga ccaatcgcct gagttcctgg 1080

cgaaccagcg gtgagcttaa tattcctttg aatgtgatgg ttgatcaaac gctgaccgtt 1140

ggtgcagagt ggaaccgcga taagctcgat gatccttcct ctaccagcct gacggtgaat 1200

gacagagata tcagcggtat ttctggctct gctgcggatc gcagcagtaa aaatcattct 1260

caaatcagtg cgctgtatat tgaagataac attgagccgg ttcctggcac gaatatcatt 1320

cccggcctgc gctttgatta tctcagcgac tccggcggga acttcagccc cagtctgaat 1380

ctttcgcagg aattgggcga ttatttcaaa gtcaaagcag gggttgcccg aacctttaaa 1440

gccccaaacc tgtatcaatc cagtgaaggc tatctgctct actcgaaagg caatggctgt 1500

ccaaaagata ttacatcagg cgggtgctac ctgatcggta ataaagatct cgatccggaa 1560

atcagcgtca ataaagaaat tggactggag ttcacctggg aagattacca cgcaagtgtg 1620

acctacttcc gcaatgatta ccagaataag atcgtggccg gggataacgt tatcgggcaa 1680

accgcttcag gcgcatatat cctcaagtgg cagaatggcg ggaaagctct ggtggacggt 1740

atcgaagcca gtatgtcttt cccactggtg aaagagcgtc tgaactggaa taccaatgcc 1800

acatggatga tcacttcgga gcaaaaagac accggtaatc ctctgtcggt catcccgaaa 1860

tatactatca ataactcgct taactggacc atcacccagg cgttttctgc cagcttcaac 1920

tggacgttat atggcagaca aaaaccgcgt actcatgcgg aaacccgcag tgaagatact 1980

ggcggtctgt caggtaaaga gctgggcgct tattcactgg tggggacgaa cttcaattac 2040

gatattaata aaaatctgcg tcttaatgtc ggcgtcagta atatcctcaa taaacagatc 2100

ttccgatctt ctgaaggggc gaatacctat aacgagccag gccgggctta ttatgccgga 2160

gttaccgcat cattc 2175

<210> 75

<211> 3042

<212> DNA

<213> Escherichia coli

<400> 75
atgggtaacc aatggcaaca aaaatatctt cttgagtaca atgagttggt atcaaatttc 60

ccttcacctg aaagagttgt cagcgattac attaagaatt gttttaaaac tgacttgccg 120

tggtttagtc ggattgatcc tgataatgct tatttcatct gcttttctca aaaccggagt 180

aatagcagat cttatactgg atgggatcat cttgggaaat ataaaacaga agtactgaca 240

ctcactcaag ccgctcttat taatattggt tatcgttttg atgtttttga tgatgcaaat 300

tcaagcacag gaatttataa aacaaagagt gcagatgtgt ttaacgaaga aaatgaagaa 360

aaaatgctcc cgtcggaata cctgcatttt ttacaaaagt gtgattttgc aggtgtttat 420

ggaaaaactc tgtcagatta ctggtcgaaa tactatgata aatttaagct tttactaaaa 480

aattattata tttcttctgc tttgtatctt tataaaaatg gagagcttga tgagcgtgaa 540

tataatttct ccatgaacgc cttaaatcgc agtgataata tatcactatt attctttgat 600

atttatggat attacgcatc tgatattttt gtagccaaaa ataatgataa ggtaatgctt 660

ttcattcctg gtgcaaaaaa acctttttta ttcaagaaga atatcgctga tttgcggctt 720

acccttaaag aacttattaa ggatagtgac aacaaacaat tactttccca acatttttca 780

ttatatagtc gtcaagatgg agtttcctat gcaggagtaa attctgttct acatgcaata 840
gaaaatgatg gtaattttaa tgagtcttac tttctgtatt ccaataagac acttagcaat 900
aaagatgttt ttgatgctat agctatttct gttaagaaac gcagtttcag tgatggtgat 960
atcgttataa aatcaaacag tgaagctcaa cgagactatg ctctgactat actccagacg 1020
attttatcaa tgacccctat atttgatatc gtagtcccgg aggtatctgt tccgcttgga 1080
ctggggatta ttacttccag tatggggatc agttttgatc aactgattaa tggtgatact 1140
tatgaagaac gtcgttctgc tatacctggt ttggcgacaa atgcagtatt gcttggtctg 1200
tcttttgcaa ttccactctt gattagtaag gcaggaataa accaggaggt acttagcagc 1260
gttataaata atgagggcag gactctgaat gaaacaaata tcgatatatt tttgaaggaa 1320
tatggaattg ctgaagatag tatatcctca actaatttgt tagacgttaa gcttaaaagt 1380
tccgggcagc atgtcaatat tgtaaagctt agtgatgaag ataatcaaat tgtcgctgta 1440
aaagggagtt ctctgagcgg catctactat gaagtggaca ttgaaacagg atatgagatt 1500
ttatcccgaa gaatttatcg taccgaatat aataatgaaa ttctctggac tcgaggtggt 1560
ggtctaaaag gggggcagcc atttgatttt gaaagtctca atattcctgt attttttaaa 1620
gatgaaccct attctgcagt gaccggatct ccgttatcat ttattaatga tgacagctca 1680
cttttatatc ctgatacaaa cccaaaatta ccgcaaccaa cgtcagaaat ggatattgtt 1740
aattatgtta agggttctgg aagctttggg gatagatttg taactttgat gagaggagct 1800
actgaggaag aagcatggaa tattgcctct tatcatacgg ctgggggaag tacagaagaa 1860
ttacacgaaa ttttgttagg tcagggccca cagtcaagct taggttttac tgaatatacc 1920
tcaaatgtta acagtgcaga tgcagcaagc agacgacact ttctggtagt tataaaagtg 1980
cacgtaaaat atatcaccaa taataatgtt tcatatgtta atcattgggc aattcctgat 2040
gaagccccgg ttgaagtact ggctgtggtt gacaggagat ttaattttcc tgagccatca 2100
acgcctcctg atatatcaac catacgtaaa ttgttatctc tacgatattt taaagaaagt 2160
atcgaaagca cctccaaatc taactttcag aaattaagtc gcggtaatat tgatgtgctt 2220
aaaggacggg gaagtatttc atcgacacgt cagcgtgcaa tctatccgta ttttgaagcc 2280
gctaatgctg atgagcaaca acctctcttt ttctacatca aaaaagatcg ctttgataac 2340
catggctatg atcagtattt ctatgataat acagtggggc taaatggtat tccaacattg 2400
aacacctata ctggggaaat tccatcagac tcatcttcac tcggctcaac ttattggaag 2460
aagtataatc ttactaatga aacaagcata attcgtgtgt caaattctgc tcgtggggcg 2520
aatggtatta aaatagcact tgaggaagtc caggagggta aaccagtaat cattacaagc 2580
ggaaatctaa gtggttgtac gacaattgtt gcccgaaaag aaggatatat ttataaggta 2640
catactggta caacaaaatc tttggctgga tttaccagta ctaccggggt gaaaaaagca 2700

gttgaagtac ttgagctact tacaaaagaa ccaatacctc gcgtggaggg aataatgagc 2760

aatgatttct tagtcgatta tctgtcggaa aattttgaag attcattaat aacttactca 2820

tcatctgaaa aaaaaccaga tagtcaaatc actattattc gtgataatgt ttctgttttc 2880

ccttacttcc ttgataatat acctgaacat ggctttggta catcggcgac tgtactggtg 2940

agagtggacg gcaatgttgt cgtaaggtct ctgtctgaga gttattctct gaatgcagat 3000

gcctccgaaa tatcggtatt gaaggtattt tcaaaaaaat tt 3042

<210> 76

<211> 1362

<212> DNA

<213> Escherichia coli

<400> 76
atggtggaca tgattaatga aagtgcacgg caaacgccag tcattgcaca aacggacgtt 60

ctggttatcg ggggcggtcc ggcaggatta tccgctgcca ttgcggcagg gcggttaggt 120

gccagaacca tgattgttga gcgctacggg tcgctaggcg gcgtattgac gcaggtcggg 180

gtagaaagtt ttgcctggta tcgtcatccg gggacggaag attgtgaagg gatctgtcgt 240

gagtatgaag gccgcgcacg agcgctgggt ttcacacgac cagaacctca gtcaattagc 300

gaagttatag atactgaagg atttaaagtt gtcgccgatc agatgattac ggaatctggc 360

gttgagccgt tatatcactc ctgggttgtg gacgtgatca aggacgggga tacgttatgc 420

ggtgttatcg tcgagaataa atcaggtcga ggggcaattc tggcgaaaag aatcgtcgat 480

tgcacggggg atgctgatat tgccgctcgt gcaggcgcgc cctggacgaa acggagcaag 540

gaccaactga tgggcgtcac cgtgatgttc agttgcgcag gtgttgatgt ggcacgcttt 600

aaccgttttg ttgcggaaga acttaagccg acctacgcgg attggggcaa aaactggacg 660

attcaaacca cgggtaaaga agacccgatg tttagcccgt atatggagga tatttttacc 720

cgcgcgcaac aggatggtgt gattccaggt gacgcccagg cgattgccgg aacctggtcg 780

accttttctg aaagcggtga ggctttccag atgaatatgg tgtacgcctt tggttttgac 840

tgtaccgatg tcttcgattt aaccaaagct gagattgccg gaaggcagca agcattatgg 900

gcaattgacg cactacgcca ctatgttccg ggctttgaaa atgtacggtt acgcaatttt 960

ggtgccacgc tggggacgcg tgaatcacgg cttattgagg gggaaatacg tattgctgat 1020

gattacgtcc ttaatcaggg gcgttgttcg gacagtgtag ggattttccc ggaatttatt 1080

gatggttccg gttatctcat tttgccaacg accgggcgtt tctttcagat cccttatggt 1140

tgtctggtgc cgcaaaaagt ggagaacctt ttggtcgccg gtcgctgtat ttccgcaggc 1200

gtagttgcac atacttctat gcgtaacatg atgtgttgtg ccgttaccgg tgaggccgca 1260

ggtactgccg ccgtggtttc gctacagcaa aattgcaccg tgcgtcaggt tgctatccct 1320

gatttgcaaa acacgctgca acagcagggc gttcgtctgg ca 1362

<210> 77

<211> 759

<212> DNA

<213> Escherichia coli

<400> 77
atgtctgcca aaagacgact tcttattgcg tgtaccttga taacagctat ctatcatttt 60

cctgcatatt cttcattaga atataaagga acctttggtt caataaatgc gggttatgca 120

gactggaaca gtggatttgt aaacactcac cgtggtgaag tatggaaagt gactgcggat 180

tttggggtaa attttaaaga agcagaattt tactcatttt atgaaagtaa tgtactcaat 240

catgctgtag cagggagaaa tcatacggtt tcagcaatga cgcatgtcag actctttgac 300

tctgatatga cattctttgg caaaatttat ggccaatggg ataactcatg gggtgacgat 360

ctggacatgt tttatggatt cggttacctc ggctggaacg gcgagtgggg cttttttaaa 420

ccgtatattg gattgcataa tcaatctggt gactacgtat cagctaaata tggtcaaacg 480

aatggttgga atggttatgt tgttggctgg acagcagtat taccatttac gttatttgac 540

gaaaaatttg ttttatctaa ctggaatgaa atagaactgg acaggaacga tgcttacacg 600

gagcagcaat ttggccggaa cgggttaaat ggcggtttaa ctattgcctg gaagttctat 660

cctcgctgga aagcaagtgt gacgtggcgt tatttcgata ataagctggg ctacgatggc 720

tttggcgatc aaatgattta tatgcttggt tatgatttc 759

<210> 78

<211> 1476

<212> DNA

<213> Escherichia coli

<400> 78
atggccagtt tgatcggcct tgcagtttgc acagggaatg cttttagtcc tgccttagcc 60

gcagaggcta aacaacctaa tttagtcatt attatggcgg atgatttagg ttatggcgat 120

ttagcaacat atggtcatca gatcgttaaa acacctaata tcgacaggct tgcccaggaa 180

ggggtcaaat ttactgacta ctatgccccc gctcctttaa gttcaccttc acgcgcaggg 240

ctattaaccg gccggatgcc atttcgtact ggaattcgct catggattcc ttcaggcaaa 300

gatgttgcct tagggcgtaa cgaactcacg attgctaatc tactcaaagc gcaagggtac 360

gacacggcaa tgatgggtaa gctgcatctg aatgcaggcg gcgatcgcac cgatcagcca 420

caagcacaag atatgggctt tgattactca ctggctaata cggcgggctt tgttaccgac 480

gccacgctgg ataacgctaa agaacgcccg cgttatggca tggtttaccc gacaggctgg 540

ctacgtaatg ggcaacccac tccacgagcc gataaaatga gcggtgagta tgtcagttcg 600

gaagtcgtca actggctgga taacaaaaag gacagcaagc ctttcttcct ctatgttgct 660

tttaccgaag tgcatagccc cctggcttcg cccaaaaaat acctcgacat gtactcacaa 720

tatatgagcg cgtatcagaa gcagcatcct gatttatttt atggcgactg ggcagacaaa 780

ccctggcgtg gtgtggggga atattatgcc aatatcagct atctggatgc acaggttgga 840

aaagtgctgg ataaaatcaa agcgatgggt gaagaagata acacaatcgt tatttttacc 900

agtgataacg gtccggtaac gcgtgaagcg cgcaaagtgt atgagctgaa tttggcaggg 960

gaaacggatg gattacgcgg tcgcaaggat aacctttggg aaggcggaat tcgtgttcca 1020

gccattatta aatatggtaa acatctacca cagggaatgg tttcagatac acccgtttat 1080

ggtctggact ggatgcctac tttagcgaaa atgatgaact tcaaattacc tacagaccgt 1140

actttcgatg gtgaatcgct ggttcctgtt cttgagcaaa aagcattgaa acgcgaaaag 1200

ccattaattt tcgggattga tatgccattc caggatgatc caaccgatga atgggcgatc 1260

cgtgatggtg actggaagat gattatcgat cgcaataata aaccgaaata tctctacaat 1320

ctgaaatctg atcgttatga aacacttaat ctgatcggta aaaaaccaga tattgaaaaa 1380

cagatgtatg gtaagttttt aaaatataaa actgatattg ataatgattc tctaatgaaa 1440

gccagaggtg ataaaccaga agcggtgacc tggggc 1476

<210> 79

<211> 954

<212> DNA

<213> Escherichia coli

<400> 79
gtgacaacaa ctatctgcgc tatgggcgaa ttgctggccg agtttttgtc ccgcaaccca 60

catcaaaaat tcactcagcc tggggagttt atcgggccat ttcccagcgg tgcgccagca 120

atttttgctg ctcaggtggc aaaactgtcc catcgggcca tcttctttgg atgtgttggt 180

aatgatgatt ttgcccgact cattatagag cgtctccgtc atgaaggtgt cattaccgat 240

gggatccatg ttatgaacaa tgccgtcaca ggtacggcgt tcgtgagtta tcaaaatccc 300

cagcagcggg atttcgtctt taatatccct aacagcgcct gcggtttgtt tactgccgag 360

cacattgata aggatctgct taaacagtgt aaccatctgc atattgtggg ctcatcgttg 420

ttctcatttc gcatgatcga tgtcatgcgt aaagcaataa cgacgatcaa atcggctggc 480

ggcaccgttt ctttcgatcc caatattcgc aaagagatgc tgagcattcc tgaaatggcg 540

caggctctcg attatttgat tgaatatacg gatattttta tccccagcga aagcgaactc 600

cctttcttcg cgcgtcacaa aaatctgtca gaggaacaga ttgttagcga tcttctccac 660

ggcggcgtaa aacatgtggc gataaaacgc gcccagcgtg gggccagcta ttacaagctt 720

aaaaacggta cattacacgc ccagcatgtt gcaggtcacg atatcgaaat tatcgatcca 780

acgggtgcag gcgactgctt tggcgcaacg tttatcactc ttttcttatc cggtttcccg 840

gcacacaagg cgctgcaata tgcaaatgcc agcggcgcgc tcgccgtaat gcggcaaggt 900

ccgatggaag ggatatcctc actggcagac attgaagact ttttgcagca gcac 954

<210> 80

<211> 513

<212> DNA

<213> Escherichia coli

<400> 80
atgaagatat tcattagttt atttttgttt ataatatcaa caaattcttt tgctgatgat 60

atcactcatg ccggagtggt tcgtattgaa gggttaatta ccgaaaaaac ctgcattatt 120

tctgatgagt caaaaaattt tacagttaat atgccagacg tacccagtag ttcggtaagg 180

agtgcagggg atgttactga aaaggtttat ttttccataa cgttaacccg ctgtggtagt 240

gatgttggca acgcgtatat aaagtttacc ggcaatacag tttctgaaga tgccagttta 300

tataagctgg aagatggctc ggtagagggg cttgcactta cgatttttga taagaacaaa 360

ggcagtatta gtaatgatgt taaaagcatg gttttttcac ttacatcatc agttgataat 420

atattgcatt tttttgcggc ttacaaagca ttaaaaaata atgtccaacc aggggatgca 480

aatgcgtcag tatcgtttat tgtcacctat gat 513

<210> 81

<211> 603

<212> DNA

<213> Escherichia coli

<400> 81

atgattaaat tccggcttta tattccccct gtaattctcg gttttgttat cgtaccatta 60

ttggtatggc cgacggttat tgccttagcc gtacttatat tcacgttaac ttttctggcg 120

gaaataatat tctcctttcc gctcctggtt gtgcgtattt ctcttcagga attacaactt 180

gagttattgg ttgtatatgc actttttttc agtgtaatgg gtggcatcgg ttggcaattc 240

tcccgcagaa cgcctcctga attaaaaaac aggctacatt gctggctggt cttttctccg 300

gtctatttct ggttaattct ctcgaatttc attctttata tttctccaga gaaatcagcg 360

ttgctggaaa atatccgaaa tttctttctg acatttgtct ggcttcccct gaatttttcc 420

cctttttggc cgcagccgtg gactgatttt gtcggcccga ttagtgccca gcttggtttt 480

gcgttgggat attattgcca gtggcgtagc aaaaatagaa gccataggaa gaagtggggc 540

gattgggtaa cgtgcttaag tttggcgatt ttagctctgg ggccgttatt caattattta 600

caa 603

<210> 82

<211> 702

<212> DNA

<213> Escherichia coli

<400> 82
atgaaattca atttatctaa tttatccgca gtattactgg catcaggtat gctgatgtct 60

actgcggtaa ccgcagcacc cggcgatgca acacaatttg gtggggcgga tactgactgg 120

agcaccgttg attatcccag gctcactgat atggatgaca acgttgattc aatggggggg 180

aaaatccgct ttactggccg tgtagtgaaa gctacctgta aggtcgcaac cgattcaaaa 240

cagattgaag ttgtcctgcc ggttgtgcct tccaaccttt tcactggtat cgacgtagaa 300

gcacaggggg cgagcaacca gaccgatttc aatattaatc tgaccgaatg tagcaataca 360

gatgatcaga aaattgagtt ccgttttacc ggtactgcag atagcgctaa taaaacgctc 420

gctaacgaag tagaaggatc aacggatgct gacaacagcg gcaatgcggg ggcgactggt 480

gtagggattc gaatttactc caaaggtacg acgaataatg gtctgattaa cctgaatacc 540

actgcggcag agggtagcgc ctccaccgcc gcttatacaa ttccaggaaa tgctacgacc 600

catgatttca gcgcggcctt tactgcaggt tatgctcaaa acggtagcac tgttgcacca 660

ggtgtagtta agtcaacagc aagttttgtt gtgctgtacg ag 702

<210> 83

<211> 1008

<212> DNA

<213>  Escherichia coli

<400>  83
atgcgtatac atacttattg gtatagaaga tatttcattt tattgattat tatattttca      60

aatgttcttt cttctattgc taatgctgaa gatatggggc gagaacgtgc atattgttat     120

ccgggttcac cgagtaataa tactacgcct gcatcctttt cttataattt tggtactata     180

gtggtttctg atgtcaacaa aaatgcgcct ggcactgtat tgccatcaca aatctggaag     240

gttggaacct ataaggctta ttgtaattct cttgatgatt atgaaattta cttcagtgct     300

gtctctggaa tagatccgtc tggtgccagt ggtgatcatc aagggagtga tgtatttatt     360

ccactcaccc atgaaatatc tgtctctact catataaaac tttataatca aaatggcaca     420

atgacagata aaattgtgcc attcgaaaat tataatacca attatccggg ggacagaagc     480

aaaccatcta attgggcatc aggtactgaa ggatatatta aaatcaggat tgataaaaaa     540

attatatctg atgtttcatt aagtaacgta ttattggtgt cattatatgt cagccagatc     600

cctaccgaac atggtcctat ccctgtcttt aatgcctaca taggaaactt aaatattcag     660

gttccgcaag gttgcactat taatgagggt acgagtttta ctgttaatat gccggatgtg     720

tgggccagtg aattgagccg ggctggtgcc ggagcgaagc ccgctggtgt tactcctgta     780

gcaacaacta ttccgattaa ttgtacgaat aaagatacag atgcggtaat gacgttggta     840

ttcgacggta acatttccgc cacacgtgat accaatggga aacaaagtat tattcaggca     900

caagataatc ctgatgttgg tattatgatt atggatagtc agcaaaactc cgtagattta     960

aatgccctgg caacatcagt aggcgttccg ttcagattgg tggaaaac                 1008

<210>  84

<211>  2592

<212>  DNA

<213>  Escherichia coli

<400>  84
atgaacctaa agctcaaaag atgcgaatat tggatggcgg cacaaaagca gatgaaacgg      60

gttgtgccgc ttcttctggt tattatgcct gcatgttcaa tcgcgggaat gcgctttaac     120

cctgcttttc tgtcgggtga tactgaagct gttgctgact tatcccgctt cgagaaaggg     180

atgacttatc ttcctggtag ctatgaagtc gaagtttggg tcaatgattc ccctttactc     240

tctcgtactg taacttttaa agcagacgat gagaatcaac tgattccctg cctttcactt     300

gctgacttat taagccttgg aattaacaaa aatgcgctgc cagagcaggc tttggcttca     360

tctgaaaata gttgccttga tttgcgtatc tggtttcccg atgtgcatta catgccggag 420
ctggatgcac agagacttaa actgaccttt ccacaggcga taataaaacg tgacgctcgc 480
ggatatattc caccagaaca gtgggataac ggtattacag cttttttgct gaattatgac 540
ttttctggta ataacgatcg tggtgattac tcttcaaata actattattt aaatcttcgc 600
gctgggatca atattggtgc atggcgtttt cgcgattatt caacctggag tcgtgggagt 660
aattcagcag gtaaactgga gcatatcagt agtacgttgc agcgcgttat tattcctttc 720
agaagtgaat taacgctagg agatacatgg tcatcatcag atgttttcga cagtgttagt 780
attcgtggca taaaactgga atctgacgaa aatatgttgc ccgatagtca aagtggtttc 840
gctcccacgg tgcgcggaat tgcgaaaagt cgcgctcagg taacaatcaa acagaatggt 900
tatgtcattt atcaaaccta tatgccgccg ggaccgtttg agattagcga tcttaacccg 960
acatcatctg cgggagatct ggaagttacc atcaaagagt ctgataattc agaaactgtc 1020
tataccgtac cttatgccgc tgtccccatc ctgcaacgag aaggtcattt aaaatattct 1080
actacggttg gccaatatcg aagcaatagc tataaccaga aaagtcctta tgtatttcag 1140
ggggaattaa tttggggttt accctgggat attacggctt atggtggggc acaattctct 1200
gaggattacc gggcgttggc gctcggcctt ggcctgaatc tgggtgtatt tggtgcaaca 1260
tcgtttgatg ttactcaggc taacagttcg cttgtggatg ggagcaaaca tcaagggcaa 1320
tcttatcgtt ttctttattc caaatcgtta gttcagacag gaacagcatt ccatattatt 1380
ggctatcgtt attcaaccca gggcttttac actttaagtg atacgacata ccaacaaatg 1440
tcagggactg ttgttgatcc aaaaacgtta gatgataaag attacgttta taactggaat 1500
gatttttata acttgcgtta tagcaaacgt ggaaaatttc aggctagtgt atcgcaacct 1560
ttcggtaact acgggtctat gtatttatcg gctagtcagc aaacatactg gaatactgat 1620
aaaaaagatt ctttatacca agttggttat aacaccagta ttaagggtat ctatctaaat 1680
gttgcgtgga attacagtaa atcaccaggg acaaatgcgg ataaaattgt ctcgctaaat 1740
gtctcattac ctataagtaa ttggttatct tccacgaatg atgggcgctc atcatcgaat 1800
gccatgactg caacgtatgg ttatagtcag gataaccacg gacaggtaaa ccaatatacg 1860
ggggtatctg gttctctgtt ggagcagcat aatctcagtt ataacataca acatggtttt 1920
gctaatcagg ataatagcag tagtggttct gttggtgtta attatcgtgg ggcatatggt 1980
tccttgaatt ccgcctacag ttacgataat gaaggtaatc aacaaataaa ctatggcatc 2040
agtggtgctc ttgttgtaca tgaaaatggt cttacgttga gtcaaccatt aggtgaaact 2100
aatgttttga taaaagcgcc tggagcgaat aatgtggatg ttcagcgggg gacaggaata 2160
tccactgact ggcgtggata tgcagttgtt ccttatgcaa cagaatatag acgtaataat 2220
atttcattag atcctatgtc aatgaatatg catactgaac tggatatcac ttccactgaa 2280

gttattccgg gaaaaggtgc gttagttcgt gcagagtttg ctgctcatat cggtattcgt 2340

ggtttgttca cagttcgtta tcgtaataaa tcagtcccat tcggtgctac agccagcgct 2400

cagattaaaa acagtagtca aattaccggg attgtcggcg ataatggaca actttatctc 2460

tcaggattgc ctttagaagg tgttattaat atccagtggg gagacggtgt tcagcaaaaa 2520

tgtcaggcta attacaagct ccctgaaaca gaactggata atcctgttag ctatgcaact 2580

ctggagtgcc gc 2592

<210> 85

<211> 507

<212> DNA

<213> Escherichia coli

<400> 85
atgggagcga tttatgttaa acgtttgatt ctgtcggtag cactgataat accgatagca 60

tccaatgctt ctgatgcttt gaaccagccg agcagtagtc taaatgatgg tgttgagact 120

ttttttattt cctgctttga tatgcctcag gaaacaacta ctgatatgga cgcttgtcag 180

agagttcagt tagctcaggt tagttgggtt aagaataagt attcggtggc cgccctgaat 240

cgtttgaaac aagacaacaa ggatgatcca cagcgtctgc aggaattaac tgcttctttt 300

aacgcggaaa gtgaagcttg gacagaatta attgagaaag cgtcaaagtc cgtccaggtt 360

gattatgtag gaggaactat agctggcact gcagttgcat cacgtcaaat tggtcttctg 420

gaattacaat cccacgatat ctgggagcac tggctacgat ctcgaggact caactcctcc 480

tcttttgcca gaaccaaagt tcaaatc 507

<210> 86

<211> 2139

<212> DNA

<213> Escherichia coli

<400> 86
atggctatgt tcacaccttc attctcagga ctcaaaggtc gggcgctctt ttcactgctt 60

tttgcggcac cgatgattca tgcaacagac tctgtaacga ccaaagatgg cgaaacaatc 120

actgttacag cagatgcaaa taccgcaact gaggcaaccg atggttatca acctctgagc 180

acctccacgg cgacattaac cgatatgccg atgctggata tcccgcaggt ggtcaatacg 240

gttagcgatc aggttctgga aaaccagaat gcgacaacgc tggatgaggc gctttataac 300

gtcagtaacg tggtacagac caatacatta ggcgggactc aggatgcttt tgtacgccgt 360

gggtttggcg caaaccggga tggctccatc atgaccaacg gtctgcgaac cgtacttcct 420

cgtagtttca acgccgcaac agagcgtgtg gaagtgctaa aaggcccggc ctccacgctg 480

tatggcattc tcgatcctgg cggactgatt aacgtcgtga ccaagcgccc ggaaaaaaca 540

ttccatggtt cggtttcagc cacctcctcc agttttggtg gcggcactgg gcaacttgat 600

atcacaggtc ccattgaagg cactcagctg gcgtatcgcc ttaccgggga agtgcaggat 660

gaagattact ggcgaaactt cggtaaagag cgcagtacat ttattgcccc gtcactcacc 720

tggtttggtg ataatgcaac agtaaccatg ctctattccc atcgggacta taaaactcca 780

ttcgatcgtg gaacgatttt cgaccttacg acgaaacagc ccgtaaacgt tgatcgaaaa 840

atacgttttg acgaaccgtt taatattaca gatggtcagt ccgatctggc gcaactcaac 900

gcagaatatc atctcaatag ccagtggaca gcgcgctttg attacagcta cagccaggat 960

aaatacagcg ataatcaggc gcgtgttacc gcgtatgatg caacgacagg aacactgaca 1020

cggcgtgttg atgcaactca gggatctacc cagcgtatgc atgctactcg tgcggatctg 1080

caagggaatg ttgatattgc cggattctat aatgagattc tgggtggggt gtcatatgaa 1140

tattatgatc ttctgcgtac agatatgatt cgctgtaaaa aagctaaaga tttcaatata 1200

tacaaccctg tttatggtaa taccagcaaa tgtacaacgg tttcggcgtc ggacagcgat 1260

cagacgatca aacaggagaa ctactcagct tatgcacagg acgcgctcta tctgaccgat 1320

aactggattg ccgtcgccgg gatccgctat cagtattaca cgcaatatgc gggtaaaggc 1380

cgtcctttta atgtcaatac tgacagccgc gatgaacaat ggacgcccaa actggggtta 1440

gtctacaaac tgacgccatc ggtatcctta tttgccaatt attcgcaaac atttatgccg 1500

cagtcgtcaa ttgccagcta cattggcgat cttccaccag aatcatctaa tgcttacgaa 1560

gtcggggcaa aattcgagct attcgatggt atcaccgcag atattgcgct gtttgatatc 1620

cataaacgta atgtgttgta taccgaaagt attggtgatg aaaccatcgc caaaacggca 1680

ggccgcgttc gttcaagagg ggtagaagtc gaccttgcgg gagcattaac tgaaaacatt 1740

aatatcattg ccagctacgg ctataccgat gcaaaggttc tggaagatcc tgattatgca 1800

gggaaaccat tgccgaatgt tcctcgtcat accggttcgc tattcctgac ctatgatatt 1860

cataacatgc caggcaataa cacactgacg tttggcggtg gcggacatgg tgtaagccgt 1920

cgttcggcaa ccaatggggc tgactattat ctgcctggct atttcgttgc cgatgccttc 1980

gccgcataca aaatgaaatt gcagtatccg gtcactctgc aattaaacgt caaaaacctg 2040

tttgataaaa cgtattacac ctcttccatc gccacaaata atctgggcaa ccagattggc 2100

gatccgcgtg aagtgcaatt cacggtgaaa atggaattt 2139

<210> 87

<211>  1818

<212>  DNA

<213>  Escherichia coli

<400>  87
atgaaaatat cgtggaatta tatatttaag aacaaatggc gatttcacat tacaagcatt      60

tcactttttc ttatcatgct cgcggtttca atcgcttttt tgcacttgcg ttttaatacc     120

ttgtccagta ccgataaaat gcggcttgaa atgtataagt ccacattata ttccaccatc     180

gagcaatttt atgttttacc ctatatgctc tcaacagacc atatcatccg tcaggcggta     240

attacgcctg acgatatgac gtccagcgaa ctcaatcaac gaattgcaca tttcaatact     300

caactcaaaa ccgcagcaat atttattctg gatacccaag gtaaggccat cgcttctagc     360

aactggcagg accccggcag ctatgtaggg caaaattata gctatcgccc ctattataaa     420

cacgccatgt ctggcttaaa tggacgcttt tacggtattg gtagcactac gaatacaccg     480

ggattcttcc tctctacaag tataaaagat aaaggaaaaa ttgtcggtgt tgtagtagta     540

aaaataagtc ttaatgaaat tgaaaaagca tgggccgaag gtcctgaaaa tattatcgtg     600

aatgatgaac atgggattat atttttaagt tcaaaatcgc catggcgaat gcgaacactg     660

caaccgttac ctgttcaggc aaaacaaaaa ctacaatcta cccgccaata tagtctcgac     720

aatcttttac cggcggatta ttatccctgt tataccgtga gcaattttac tttcctgaaa     780

gataaaaaag aacaactctg tttattcccg caatattata cgcaacaaat agccattcca     840

gaatttaact ggaaaatgac aattatggtc cccttagata acctgtactg gtcatgggct     900

atttcgttag tcattacact aattatttac ctgctgtttt tgttatttat taaatactgg     960

agaatgcgat ctcatgcaca acaattatta acacttgcga atgaaacatt agaaaaacag    1020

gttaaagagc gtacatctgc cctggaattg atcaatcaaa aattaataca ggagataaaa    1080

gagcgcagtc aagctgaaca agtattacaa attacgcgta gtgaactggc agagtccagc    1140

aaactggcgg cgcttggaca gatggcaacc gaaattgccc atgaacaaaa tcaaccgtta    1200

gccgccattc acgcacttac tgataacgcg cgtactatgc taaaaaaaga gatgtatccg    1260

caggttgaac agaatctgaa acatattatt tcagtgattg agcggatgac gcagctcatt    1320

tccgaactta aagcatttgc ctcgcgccat cgcgtaccta aaggttctgc cgatgtcatc    1380

aaagtgatgt atagcgccgt ggcgttactt aatcacagca tggagaaaaa taacattgag    1440

cgacgaataa aagccccatc catgccgtta tttgtcaatt gcgatgagct cggtcttgaa    1500

cagatattca gtaatttaat tagcaacgcc ttagattcta tggaaggtag ctcttacaaa    1560

cgactggata tcgccattcg ccaggcaaat aacaaagtta ttattaccat taaagacagc    1620

ggtggcggtt ttgcacctga agttgtcgat cgcatatttg aaccattttt taccactaaa 1680

cgtagaggaa tggggttggg actggcaata gtcagcgaaa ttgtccgaaa ttcgaacggc 1740

gcactccacg ccagtaatca tcctgaaggc ggcgcagtaa tgacattaac ctggcctgaa 1800

tggggagaag aacatgaa 1818

<210> 88

<211> 303

<212> DNA

<213> Escherichia coli

<400> 88
gtgcttacac cacaacattt acgttgtgtg ttaacatgta gcgatttact gactcttttg 60

agtggtaccg ttatgtctca aatgcccctc tattttctta atacccaaaa gaaactcact 120

gctcactatg aatggcttca aatcaacctg actgatacct acgaactagt taaaaggtta 180

atgccgattc cttcactgga cgtggtggtt aaagtaggga aacttgtcct cccggagaaa 240

gggcatcatg gtttttaccc tgaagctgga gttgtctata gaacagtagc tccagaaaat 300

cca 303

<210> 89

<211> 789

<212> DNA

<213> Escherichia coli

<400> 89
atgatgaaaa atacaggcta tatcttagct ctttgtctga cagcatcggg gcatgtccta 60

gcccatgatg tctggattac aggtaaacag gcagagaaca acgttaccgc agagattggt 120

tatggtcata atttcccctc aaaggggaca attcctgaca gaagggattt ctttgaaaat 180

ccccggcttt ataacgggaa agagacaata acactgaagc cagcgtccac ggattatgtc 240

tataaaactg agtctgcaag caaagataat ggttacgttc tgtcaacgta tatgaaaccg 300

ggatactggt cgagaacctc gtcaggatgg aaaccggtca gccgggaggg cagaaatgat 360

gtggcttact gtgaatttgt cactaaatat gcaaaatctt ttattcctgg tgaacagcag 420

atgccagcac aactctatca gtctccaaca gggcatgagc ttgaaatcat tccgttatcc 480

gatataagtc gtttcagtga aaatgtgaag ctgaaagttc tgtataaaac gtccccgctc 540

gccggagcta tcatggagct tgactcggtc agttatctga catcatcccg tcatactcat 600

gcagttgagc acaaacatcc tgttcataaa gcagaactca cctttgtaac taatgaggat 660

ggtatcgtca cagtaccttc tcttcatatc ggacagtggc tggcgaaagt ccaaaataag 720

aaaagttttc aggacaaaag cctgtgtgat gaaactgtcg atgtggcaac cttaagcttc 780

tcccgaaat 789

<210> 90

<211> 1134

<212> DNA

<213> Escherichia coli

<400> 90
atgggaaaaa taaaatattg gctaatagta ggatttatta tactttttgc gattttttac 60

attgctatta gtgacaggga ttctacgctt tctaggttga aatcagcagg tgaaaacgga 120

gatgtagaag ctcagtatgc tttggggctc atgtatttgt atggagaaat tctggatgtt 180

gattatcagc aggcaaagat ttggtatgaa aaagccgctg accaaaatga tccgcgtgcg 240

caggccaaac tcggtgtgat gtatgcaaat ggtctcgggg taaatcagga ttatcagcaa 300

tcaaaattat ggtatgaaaa ggcggctgcg caaaatgatg ttgatgcgca atttttgctt 360

ggggagatgt atgacgatgg tctcggggta agccaagact accagcatgc aaagatgtgg 420

tatgaaaaag cggctgctca aaatgatgag cgtgctcagg tcaatctcgc tgttctatac 480

gcaaagggta atggtgttga acaggattat cgacaggcca aaagctggta tgaaaaggct 540

gcagctcaaa atagtcctga tgcgcagttc gctcttggaa ttctgtatgc caatgctaat 600

ggtgtagagc aggactatca gcaggcaaaa gactggtatg agaaagcagc agaacaaaat 660

ttcgccaatg ctcagtttaa tcttggtatg ctctattaca aaggtgaggg tgttaaacaa 720

aactttcggc aagccagaga atggtttgaa aaagccgcat ctcaaaatca gccgaatgcc 780

caatataatt taggtcagat ttattactac ggtcagggtg tgactcagag ctatcgacag 840

gcgaaagact ggtttgaaaa agcggcagag aaaggtcatg tcgatgctca atataatctc 900

ggtgtaatat acgaaaatgg tgaaggtgtg agtcagaact atcaacaggc aaaggcttgg 960

tatgaaaagg cagcctcaca aaatgatgcg caggcgcagt tcgaacttgg cgttatgaat 1020

gaactgggtc agggtgaaag catagacctg aaacaagcaa gacattacta tgagcggtca 1080

tgtaataatg ggcttaagaa aggttgtgaa cggttaaaag agttattata caaa 1134

<210> 91

<211> 1962

<212> DNA

<213>  Escherichia coli

<400>  91
atgaatgtaa tcagaactgt catttgtaca ttaattatac ttccggtggg attacaggca      60

gcgaccagtc attcttctat ggttaaagat acaatcacca ttgtcgcgac aggaaatcag     120

aacacggtat ttgaaacgcc gtcgatggtc agtgtcgtca cgaatgacac accgtggagt     180

cagaatgcgg ttacatcggc cggcatgctg aaaggtgttg ccggtctcag ccagactggt     240

gcaggacgga ccaatggcca gacctttaat ttacgcggct atgacaaaag cggggtactt     300

gttcttgttg acggcgttcg ccaactcagt gacatggcaa aaagcagtgg cacttatctg     360

gatccggcac tcgtcaaacg tatcgaagtt gtccgcgggc caaactccag tctgtacggc     420

agtggcgggc tgggaggtgt agtggacttc agaactgccg atgcagcaga ttttcttccc     480

cccggagaga caaacggttt aagtctgtgg ggaaatatcg ccagtggtga ccacagcaca     540

ggctcggggc tcacctggtt tggtaaaact ggaaaaacag atgcgctcct ttctgtcatt     600

atgcgtaaaa gaggtaatat ctatcaaagt gatggtgagc acgcacctaa caaggaaaaa     660

cctgcagccc tgtttgcgaa aggctctgtc ggtataacag acagtaacaa agcaggtgcc     720

agcttgcgtc tctaccggaa taacaccact gaaccgggca attccactca gacacatggt     780

gacagcggcc tgcgtgacag aaaaacagta caaaatgacg tacagttctg gtaccagtac     840

gctcctgtgg ataacagcct catcaatgta aagtcaacgt tatatctcag tgatatcact     900

atcaagacaa acggtcacaa caaaacggca gaatggagaa acaacagaac ctccggtgtt     960

aatgttgtca acaggagtca tactctgatt tttccgggag cccatcagtt aagttatggc    1020

gctgaatatt accgtcagca gcagaagcca gaaggctctg ccacactata tccggaagga    1080

aacattgact ttacatcgtt gtatttccag gatgaaatga caatgaaaag ctacccggtt    1140

aacattatcg tcggttcccg ctatgaccgg tacaagagct tcaatccccg tgccggagaa    1200

ctgaaagccg aacgcctgtc ccaaggggcg gcgatttcag tctcaccgac agactggctg    1260

atgatgtacg gctccatatc ctctgcattc cgagcgccca caatggcaga aatgtacagg    1320

gatgatgtac atttttaccg caagggtaaa cccaattact gggttcctaa ccttaatctg    1380

aaaccagaaa ataacatcac ccgtgagatt ggcgcaggta ttcaactgga tggcctgctt    1440

acagacaatg accggctgca gttaaaaggc ggatatttcg gaacggatgc cagaaactat    1500

attgccacac gcgtggatat gaaacggatg cgttcttatt cttataatgt atcccgggcc    1560

cgtatctggg gatgggatat gcagggtaat taccagtctg attatgttga ctggatgctt    1620

tcttataacc ggacggaaag tatggatgcc agcagcaggg aatggctggg ctccggcaat    1680

cctgacacac ttatcagtga catcagcata cctgttggtc atagaggcgt ttatgccgga    1740

tggcgtgctg aactttcagc atcagccacg catgtgaaaa aaggcgatcc ccatcaggct 1800

ggttatacca tacattcctt ttcactgtct tataagcctg taagtgttaa aggctttgag 1860

gcgtcagtaa ctctggataa tgccttcaac aagcttgcca tgaatggcaa aggtgtgccg 1920

ctttcaggca gaactgtcag tctttatacc cgttatcagt gg 1962

<210> 92

<211> 4128

<212> DNA

<213> Escherichia coli

<400> 92
atgaataaaa tatacgctct aaaatattgt tatattacta acacagtaaa ggttgtctct 60

gaactagccc gaagggtatg taaagggagt acccgcagag gaaaaagact ttcagtactt 120

acctctctgg cactatctgc attactccca accgttgctg gtgcatcaac ggttggtggc 180

aacaatcctt accagacata ccgcgacttt gcagaaaaca aagggcagtt tcaggctggc 240

gcaacaaaca ttcctatttt taataataaa ggggaattag taggacatct tgataaagcg 300

cccatggttg attttagcag tgtgaatgta agctcaaatc ccggcgttgc aacattaatt 360

aacccgcaat atatagccag tgtaaaacat aataaaggat atcagagcgt cagcttcggt 420

gatggtcaga acagttacca tattgtggat cgtaatgaac acagttcatc tgatctccac 480

acaccaagac ttgataagct cgtaactgag gttgctccgg ctaccgtaac cagctcatca 540

acagctgata tattgaaccc ttcaaaatac tcggcattct acagggctgg ttcgggaagt 600

cagtatattc aggatagtca gggtaagcga cattgggtaa caggtgggta tggttatctg 660

acaggaggaa tactcccgac atcattcttt tatcacggct cagacggcat tcagctgtat 720

atggggggca acatacatga tcatagcatc ctgccctctt ttggagaggc cggcgacagt 780

ggttctccat tatttggctg gaatacggcc aaagggcagt gggaactggt cggtgtttac 840

tcgggagtag gaggggggac caatttgata tattctctta ttcctcagag ttttctctca 900

cagatctatt cagaggataa tgacgctccc gtctttttta atgcctcatc cggcgccccc 960

ctgcaatgga aatttgacag cagcaccggc actggctctc tgaaacaggg ttccgatgaa 1020

tatgccatgc acgggcaaaa aggttctgac ctgaacgcag gtaaaaatct gacattcctg 1080

ggacataatg gtcagattga cctggaaaac tctgtcacgc agggtgccgg ttcactgaca 1140

tttactgatg actacactgt caccacttca aacggaagta cctggaccgg ggccggtatt 1200

attgtggaca aggatgcctc cgtaaactgg caggttaatg gtgtgaaagg tgacaacctg 1260

cataaaatcg gcgaaggaac cctggttgta cagggaaccg gtgttaatga gggcggcctg 1320

aaagtcgggg atgggaccgt tgtcctcaat cagcaggctg acagttcagg acacgttcag 1380

gcattcagta gcgtgaatat tgccagcggc cgcccgacag tcgtgctggc agacaaccag 1440

caggttaatc cggacaatat atcctggggc taccgggggg gggttctgga tgttaacggg 1500

aatgacctga catttcataa gctgaatgcc gccgattatg gcgcaactct cggtaacagc 1560

agtgataaaa cggctaatat cactctggat tatcagacgc gtccggcaga cgtaaaagtt 1620

aatgaatggt catcatcaaa caggggaaca gtaggttcat tatatattta taataatccc 1680

tatactcata ccgtcgatta ttttatcctg aaaacaagta gttatggctg gttccctacc 1740

ggtcaggtca gtaacgagca ctgggaatat gtcggacatg accagaacag tgcacaggca 1800

ctgcttgcaa acagaattaa taataaaggg tatctgtatc atggcaagtt gctgggaaat 1860

attaatttct caaataaagc aaccccgggt acaaccggcg cattggttat ggacggctca 1920

gcgaatatgt ccggtacatt tactcaggaa aacggtcgtc tgaccattca gggccacccg 1980

gttatccatg cttcaacgtc tcagagtatt gcaaatacag tctcgtctct gggcgacaat 2040

tccgttctga cacagcccac ctcatttaca caggatgact gggagaacag gacgttcagc 2100

tttggttcgc tcgtgttaaa agatacagac tttggtctgg gccgcaatgc cacactgaac 2160

acaaccatcc aggcagataa ctccagcgtc acgctgggcg acagtcgggt atttatcgac 2220

aaaaaagatg gccagggaac agcatttacc cttgaagaag gcacatctgt tgcaactaaa 2280

gatgcagata aaagcgtctt caacggcacc gtcaacctgg ataatcagtc agtgctgaat 2340

atcaatgaga tattcaatgg cggaatacag gcgaacaaca gtaccgtgaa tatctcctca 2400

gacagtgccg ttctggagaa ctcaacgctg accagtaccg ccctgaatct gaacaaggga 2460

gcaaatgttc tggccagtca gagttttgtt tctgacggtc cggtgaatat ttctgatgcc 2520

accctgagtc tgaacagccg tcctgatgag gtatctcaca cacttttacc tgtatacgat 2580

tatgccggtt catggaacct gaagggagac gatgcccgcc tgaacgtggg gccgtacagt 2640

atgttgtcag gtaatatcaa tgttcaggat aaagggactg tcaccctcgg aggggaaggg 2700

gaactgagtc ctgacctgac tcttcagaat cagatgttgt acagcctgtt taacgggtac 2760

cgcaatacct ggagcgggag cctgaatgca ccggatgcca ccgtcagcat gacagacacc 2820

cagtggtcga tgaacggaaa ctccacggca ggaaatatga aacttaaccg gacaatagtc 2880

ggttttaacg ggggaacatc atcgttcacg acactgacaa cagataatct ggacgcggtt 2940

cagtcagcat ttgtcatgcg tacagacctt aacaaggcag acaaactggt gataaacaag 3000

tcggcaacag gtcatgacaa cagcatctgg gttaacttcc tgaaaaaacc ctctgacaag 3060

gacacgcttg atattccact ggtcagcgca cctgaagcga cagctgataa tctgttcagg 3120

gcatcaacac gggttgtggg attcagtgat gtcaccccca cccttagtgt cagaaaagag 3180

gacgggaaaa aagagtgggt cctcgatggt taccaggttg cacgtaacga cggccagggt 3240

aaggctgccg ccacattcat gcacatcagc tataacaact tcatcactga agttaacaac 3300

ctgaacaaac gcatgggcga tttgagggat attaacggcg aagccggtac gtgggtgcgt 3360

ctgctgaacg gttccggctc tgctgatggc ggtttcactg accactatac cctgctgcag 3420

atgggggctg accgtaagca cgaactggga agtatggacc tgtttaccgg cgtgatggcc 3480

acctacactg acacagatgc gtcagcaggc ctgtacagcg gtaaaacaaa atcatggggt 3540

ggtggtttct atgccagtgg tctgttccgg tccggcgctt actttgattt gattgccaaa 3600

tatattcaca atgaaaacaa atatgacctg aactttgccg gagctggtaa acagaacttc 3660

cgcagccatt cactgtatgc aggtgcagaa gtcggatacc gttatcatct gacagatacg 3720

acgtttgttg aacctcaggc ggaactggtc tggggaagac tgcagggcca aacatttaac 3780

tggaacgaca gtggaatgga tgtctcaatg cgtcgtaaca gcgttaatcc tctggtaggc 3840

agaaccggcg ttgtttccgg taaaaccttc agtggtaagg actggagtct gacagcccgt 3900

gccggcctgc attatgagtt cgatctgacg gacagtgctg acgttcacct gaaggatgca 3960

gcgggagaac atcagattaa tggcagaaaa gacggtcgta tgctttacgg tgtggggtta 4020

aatgcccggt ttggcgacaa tacgcgtctg gggctggaag ttgaacgctc tgcattcggt 4080

aaatacaaca cagatgatgc gataaacgct aatattcgtt attcattc 4128

<210> 93

<211> 1047

<212> DNA

<213> Escherichia coli

<400> 93
atgattacac tttttcgact actggcgatt ctttgccttt tttttaacgt ttcagctttt 60

gctgttgatt gctatcagga tgggtacaga ggaacaaccc tcataaatgg agatttacca 120

acgttcaaaa ttccagagaa tgcgcaacct gggcaaaaaa tttgggagag cggagatatt 180

aatatcacag tttattgtga caatgcacca ggatggtcaa gtaataaccc atcagaaaat 240

gtctatgcct ggatcaaatt gccccaaata aatagtgccg atatgttgaa taatccgtat 300

ttaacatttg gcgtgactta taatggtgta gattatgaag ggacaaatga aaaaattgat 360

actcatgcgt gcctggataa atatgaacaa tactataatg ggtattatca tgaccctgta 420

tgcaatggca gcactcttca aaaaaatgta acatttaacg cccattttcg cgtctatgta 480

aaattcaaaa gccgcccggc aggagatcag acggtaaact ttggcacagt caacgtgctg 540

caattcgacg gtgaaggcgg ggcgaacatg gcccccaacg cgaaaaattt acgctatgcg 600

attacggggt tagataatat ttcattcctt gactgtagtg tcgacgtccg catttccccg 660

gaaagtcaga tagtcaattt tgggcagatc gctgcgaatt ccattgcaac tttcccaccg 720

aaggcagcat tcagcgtttc taccataaaa gacattgcgt ctgattgtac cgaacagttt 780

gatgttgcaa ccagtttctt tacttcagat acattatatg acaatacgca tctggaaata 840

ggtaacggct tgctcatgcg aattactgat caaaaaacgc aagaagatat taaatttaac 900

cagttcaaat tatttagtac ttatattccc ggtcagagtg cggcaatggc aacccgcgat 960

taccaggccg aattaaccca aaaacctggt gaaccactcg tctatggccc atttcagaaa 1020

gacctgatag ttaaaatcaa ctaccac 1047

<210> 94

<211> 2520

<212> DNA

<213> Escherichia coli

<400> 94
atgaacaata aaaacacgtt ttcccgggat aagttatccc atgcaattaa aaatgccctg 60

tctggcgttg tgtgttccct actcttcgtt ttgccagtcc acgccgtaga attcaacgtc 120

gatatgattg acgcagaaga ccgtgagaat atcgacatct ctcgttttga gaaaaaaggc 180

tatatccccc ctggtagata cctcgttcgt gtgcaaataa ataaaaatat gttgccacaa 240

acgttaatac tggaatgggt aaaagccgat aatgaaagtg gttcgttact ctgcttaacc 300

aaagaaaatt tgactaattt cggtcttaat acggaattta ttgaatcatt gcaaaacata 360

gctggcagcg aatgtctcga tttaagccaa cgtcaggagt taacgacacg acttgataaa 420

gctacgatga tattatcgct aagtgttccc caggcatggt taaaatacca ggcaacaaac 480

tggacgccac cagagttttg ggataccggt atcaccgggt ttatccttga ttacaacgtg 540

tacgccagcc agtatgcccc acatcacgga gacagcaccc aaaacgtcag ctcctatggt 600

acgttaggct ttaacctcgg cgcatggcgc ttacgtagcg attaccaata taatcagaat 660

tttgctgatg gacgctcggt aaaccgcgac agcgaatttg cgcgaactta tctgtttcgc 720

cctatcccct cctggtcgtc aaaattcact atgggccagt acgacctgag ctccaatctt 780

tacgatacct tccactttac tggcgcatcg ctggaaagtg atgaaagcat gctgccgcca 840

gatttacagg gttatgcgcc acaaattacc ggcatcgcgc agaccaacgc gaaagtaact 900

gtggcacaaa atggtcgtgt actttatcaa accactgtcg cgccaggccc ttttactatt 960

tctgatttgg ggcaatcgtt tcaggggcag ctggatgtca cagtggaaga agaagatggc 1020

cgcaccagca ccttccaggt tggctccgca tccattccct atttaacccg taaagggcaa 1080

gtgcgctata aaacgtcact gggaaaaccg acatccgtcg ggcataacga tatcaataat 1140

ccctttttct ggacggcgga agcctcctgg ggctggctga acaatgtgtc gttgtatggt 1200

ggtggcatgt tcaccgctga tgattatcag gctatcacta ccggtattgg ctttaacctt 1260

aaccaattcg gttcgctttc ttttgatgtc actggagcag acgcgtcttt acagcaacaa 1320

aatagcggca atctgcgtgg ttacagctat cgcttcaact atgcaaagca tttcgaatcg 1380

acaggcagtc agattacctt cgcgggttat cgcttctcag ataaagatta cgtgtcgatg 1440

agtgagtacc tcagctcgcg taatggcgat gagtcaatcg ataatgaaaa agagagttat 1500

gtcatttcct tgaaccagta ctttgaaacg ctggaattaa actcttatct caacgttaca 1560

cgcaatactt attgggacag cgccagcaat accaactact ccgtatctgt aagcaaaaac 1620

tttgatattg gcgatttcaa aggtatatct gcatcgctgg cagtaagtcg aatccgctgg 1680

gatgacgacg aagagaatca atattacttc tctttctctc tacctttaca acaaaaccgc 1740

aacatctcct acagtatgca gcgaacggga agcagtaata cttcgcagat gatttcctgg 1800

tacgattcat cagatcgcaa caatatctgg aatatttcag cgtcggcaac ggacgacaat 1860

atacgtgatg gcgaaccaac actgcgcggc agctaccagc actattcgcc gtggggacgc 1920

ctgaacatta atggcagtgt acagccgaat cagtacaatt ctgttaccgc aggctggtac 1980

ggttcactta ccgctacacg tcatggtgtc gcccttcacg attatagcta tggcgataac 2040

gcccgcatga tggtcgatac cgatggcatc tccggcattg aaatcaactc taaccgtacc 2100

gttaccaacg ggctgggcat cgccgtgata ccttcgttat cgaactacac cacctccatg 2160

ttgcgggtga acaataacga tctgccagaa ggtgtcgatg tcgaaaactc ggttattcgt 2220

actacgctca cccagggtgc catcggctac gcaaaactga atgccaccac cggataccaa 2280

atcgtcggcg ttattcgtca ggaaaatggc cgcttccctc cactaggtgt gaatgtcacg 2340

gataaagcga caggtaaaga tgtgggcctg gtagcggaag atggcttcgt ttatctcagc 2400

ggtattcagg aaaacagtat tctgcattta acctggggtg ataatacctg tgaagtcacg 2460

ccgccaaacc aaagtaacat tagtgaaagc gcgataattt taccttgtaa aacagtcaaa 2520

<210> 95

<211> 507

<212> DNA

<213> Escherichia coli

<400> 95
ttgatgaaca caaaacagtc tgttgctcaa ctcgccgtac cgcaccgcaa gcgcctttca 60

tcaacgatgg tggtggcgct gttactttgt gtggttgctg gcgcggtgat gattaatgcc 120

gctgatttcc cagcaactgc cattgaaacg gatcccggtg caagtgcctt ccctaccttc 180

tatgcctgtg ccctgattgt gctcgctgtc ttgctggtga tacgcgatct tttgcaggca 240

aaaccagcct cttgcgccaa cgcacaggaa aaaccggcat tcaggaaaac agcaacagga 300

attgcggcaa ccgcgtttta tattgtggcg atgagctact gcggttatct cattactact 360

cctgttttcc tcatcgtcat tatgacgttg atgggctaca ggcgatgggt actcacaccg 420

ggtattgcgc tgctgttaac ggcaatcctc tggttgctgt ttgtcgaagc gttacaggtg 480

ccattgcctg tcggcacatt tttcgaa 507

<210> 96

<211> 933

<212> DNA

<213> Escherichia coli

<400> 96
atggtacttc ttgcaggcgc tgccctcagc attgcgcctg tacaggcagc ctcctaccca 60

accaaacaga tcgagttagt cgttccctac gctgccggag gcggtacgga tctggttgcc 120

cgtgcctttg ctgatgccgc caaaaaccat ttacccgtca gcatcggggt tatcaataaa 180

cctggcggag gcggtgctat cggcctgagt gaaatcgccg ctgcccgccc taacggttac 240

aaaattggtt taggcacggt tgaactgacc acccttccca gcctcggaat ggtgcgtttt 300

aaaaccagcg actttaaacc cattgcccgt ctgaatgcgg atccggctgc tatcacagtc 360

cgtgccgatg cgccgtggaa tagctatgaa gaatttatgg cttactccaa agcgaatccc 420

ggaaaagtac gcattggtaa ctcaggcacc ggagctatct ggcatctggc ggcagctgca 480

ctggaagaca aaacgggcac aaagttttct catgtcccgt atgacggcgc agcccctgcc 540

attacaggcc tgttaggcgg gcatattgaa gcggtttccg taagcccagg agaagttatc 600

aaccatgtga atggcggcaa gctgaagaca ctggtagtga tggcggatga gcgaatgaaa 660

accatgcctg acgtcccgac gttaaaagag aaaggcgttg atctctccat cggcacctgg 720

cgcggcctga ttgtgtcgca aaaaacgccg caggatgtgg tggatgttct ggcaaaggca 780

gcaaaagaga cggctgaaga gcctgcattc caggatgcac tgcaaaagtt gaatctcaac 840

tatgcatggc ttgacgctgc cagcttccag acccaaatca gcgaacagga aaagtacttt 900

gacgagttgc tgactcgcct gggcctgaaa aaa 933

<210> 97

<211> 2166

<212> DNA

<213> Escherichia coli

<400> 97
atgctgcgat ggaaacgctg tattattcta acatttatct ctggtgctgc tttcgcggcg 60
ccagagataa atgttaagca aaacgaatcg ttacctgatt taggtagcca ggcagcacaa 120
caggatgaac aaaccaacaa gggtaaatcg ctgaaagagc gcggagccga ttacgtcatc 180
aactccgcca cgcaagggtt tgaaaacttg acccctgagg cgctggaatc tcaggccaga 240
agctatctgc aaagtcaaat cacctcaacc gcacaatctt atattgaaga cacactctct 300
ccctacggta aggtccgttt gaacctctcc attggtcagg gcggcgatct ggatggcagt 360
tccatcgatt attttgttcc ctggtacgat aatcaaacca ctgtttattt cagccaattt 420
tctgcgcaac gaaaagaaga tcgtacgatc gggaatattg gccttggggt aaggtataat 480
tttgataaat atctattggg tggaaatata ttttatgatt atgactttac ccgtggacat 540
cgccgtttag gtttaggcgc cgaagcctgg acggattatt taaaattctc aggcaactat 600
tatcacccac tttctgactg gaaagactct gaagatttcg acttttatga agaacgccct 660
gcgcgcggtt gggatattcg tgccgaagtc tggttacctt cttatccgca actggggggc 720
aaaattgtct tcgagcaata ttacggcgat gaagtcgccc tttttggtac ggataatttg 780
gagaaagatc cctacgcggt aacgcttgga ctgaattatc aaccagtgcc gttactgaca 840
gttgggacgg actataaagc ggggaccgga gataacagtg atgtcagcat taatgccact 900
cttaattatc agttcggcgt tccgctaaaa gatcaattgg atagcgataa agtgaaagcg 960
gcgcactcgc tgatgggcag ccgtcttgat ttcgttgagc gtaataactt tattgttctg 1020
gaatacaaag aaaaagatcc gcttgatgtc accctgtggt tgaaagcgga tgccaccaac 1080
gagcaccctg agtgcgtcat taaggacact cccgaagcgg ccgtcggtct ggaaaaatgt 1140
aagtggacca ttaacgcact cattaatcat cattacaaaa tcgttgcggc ctcctggcag 1200
gcgaaaaaca atgccgcccg cacgctggtg atgccggtta tcaaagagaa tactctgaca 1260
gagggtaaca ataaccactg gaacctggtg ctgcctgcct ggcagtacag ttccgatcaa 1320
gccgaacaag aaaaactcaa tacctggcga gtacgtctgg cgctggaaga tgaaaagggc 1380
aaccgacaga actctggcgt ggtggaaatc accgttcagc aggaccgtaa aatagagttg 1440
attgttaata acatcgcgaa cccagaagag aacaaccaca gccacgaagc cagcgcacag 1500
gcagatggcg ttgatggtgt agtgatggat ctcgatgtaa ccgacagctt tggcgataac 1560
accgaccgca acggcgatgc gttgccggaa gataacctta cgcctcagct ttacgacgcg 1620
caggacaaac gagtgacgtt aaccaacaag ccctgctcga ccgataaccc ctgcgttttt 1680
attgccaaac aagataaaga aaagggcact gtcaccctct ccagtacctt acctggcacc 1740
tatcgctgga aagcaaaagc cgcgccctac gatgacagta actatgtgga tgtcactttc 1800

ctcggggcag aaattggtgg gctaaatgct tttatctatc gtgtgggggc ggctaaaccc 1860

agcaacctga taggtaaaga taaagaaccg ttgccgtcaa caacatttat cgatttgttt 1920

tatggcgcga caacaataaa gacggtgtct tccagcaggt cgaaaaacct gacgaagaga 1980

tggtgcagta cgactacaag tgggaattta ccggcaagag catcaatggt aagtgggtgc 2040

acaggcgaac actccaatga ggacattgtg attccggcca ctaaccgtga agcggcgcaa 2100

acctatggcg cacaagcggg agatggcttg cagggatacg gtttacgcgt gctgtatacc 2160

aaaaaa 2166

<210> 98

<211> 957

<212> DNA

<213> Escherichia coli

<400> 98
atgaagcagg ataaaagacg cggtctgacc cggatcgcat tagcgctggc actggcaggt 60

tattgtgtgg cacctgtggc gctggctgaa gacagcgcct gggtcgacag cggtgaaacc 120

aatattttcc aggggaccat tccgtggctc tattcggaag ggggaagtgc tacgacagat 180

gccgaccgtg taacgttgac ttctgatcta aaaggcgctc gcccgcaagg catgaaacgg 240

acaagcgttt ttactcgggt gataaatatt ggtgataccg aaggcgacgt ggatcttggt 300

ggattgggcg ataacgcgaa aactatcgat actatccgct ggatgagcta caaggatgcg 360

cagggggggg atccaaaaga gctggcaacg aaggtgacca gttacactct taccgatgcc 420

gaccgtggtc gctatatcgg tattgaaatt acgccaacca cgcagaccgg tacgccaaac 480

gtcgggactg cgctgcatct ttatgacgtt tctactgcca gcggcggcgg aagcgacagc 540

gataacgttg caccggggcc ggtggttaac cagaacctga aagtcgccat ctttgttgat 600

ggtaccagta tcaaccttat caacggtagc acaccaatcg aacttggcaa aacctacgtg 660

gccaaactgt actcggatga gaacaaaaat ggcaagtttg atgcgggtac cgatgctgac 720

gtcaccgcca attatgactt ccgttgggta ctttctggca gcagccaaca gcttggcact 780

tcgggtggca tcgttaactc aagcttcgat aataacaatt tggtcatccc tgcgaccaac 840

gacgaagcca gaaccaacct taacggccct gcgcgcgatg gaaaagaggc actttccatc 900

ccgaccaacg gcgacggggt acagggttac aaacttcaca ttatttacaa acacaaa 957

<210> 99

<211> 1887

<212> DNA

<213>  Escherichia coli

<400>  99
atgaagaaag tgctcactct ctcactactg gctctgtgtg tgtctcatag tgcagtagca      60

gcaaactata cgttcaataa cgataatatt gccctctcgt ttgatgatac aaactcgacg     120

attgtgctga aggaccgtag aactaaccat ccgatcacac cacaggaatt gttctttctg     180

acactaccgg atgagacaaa aatccacacc gcagatttca aaatcaagca catcaaaaaa     240

caggacaatg cgattgtcat cgactttacg cgcccagatt ttaacgtaac agtgcagttg     300

aaccttgtga agggaaaata tgccagcatc gactacacta ttgccgccgt tgggcaacca     360

cgagacgtcg ccaagattac cttcttcccg accaaaaaac agtttcaggc tccttacgta     420

gacggcgcaa tcactagctc accgatcatt gcggactcgt tctttatcct gccgaataaa     480

ccgatcgtga atacctacgc ctatgaagca acaaccaatc tcaacgtaga actgaaaact     540

ccaattcagc cagagacgcc ggttagcttt accacctggt tcggtacttt cccggaaacc     600

agccagttgc gacgcagtgt gaaccagttt attaatgccg tacgtccacg tccgtacaag     660

ccttatttgc attacaacag ttggatggat atcggctttt tcactccgta caccgaacag     720

gatgttctgg gacgcatgga cgaatggaac aaggaattca ttagcggccg cggagtggcg     780

ttagacgctt ttctgctgga cgatggctgg gacgatctta ccggacgctg gttatttggc     840

ccggcattca gcaacggttt tagcaaagta cgagagaaag ccgatagcct gcacagctcc     900

gttgggctat ggctttcacc gtgggggggt tacaataagc cgcagcgacg ttcgcgtttc     960

gcatgcaaaa gagtatgggt tcgaaaccgt ggacggcaag ctggcgcttt cgggagcgaa    1020

ctacttaaaa acttcaatga gcagatcatt aatcttatca aaaatgaaca cattacctcg    1080

tttaaactcg acggaatggg gaacgccagt tcacatataa agggtagccc gttcgcctcg    1140

gattttgatg cgtcaatagc tctgctgcac aatatgcgca gagcaaaccc gaatctattt    1200

atcaacctga ccaccggcac caacgccagc ccgtcctggt tgttctatgc tgattctatc    1260

tggcgtcagg gggatgatat aaacctgtat ggccccggca cgccggtgca gcagtggata    1320

acatatcgtg atgccgagac ataccgctct attgtacgta aaggcccgct attcccgctg    1380

aactcgctga tgtaccacgg gatagtcagc gccgagaatg cctattacgg gttagagaag    1440

gtgcaaacgg acagcgactt tgccgatcag gtctggagct acttcgcgac cggcacccag    1500

ctgcaggagc tgtatattac cccgtccatg ctgaacaagg tgaagtggga tacgctggcg    1560

aaggctgcaa aatggtcgaa ggaaaatgcc agcgtgctgg ttgataccca ctggattggc    1620

ggcgacccaa cggcgcttgc cgtgtacggc tgggcatcct ggagcaaaga caaagccatt    1680

ctcggtttgc gcaacccatc ggataagcca cagacctact atctggattt ggcgaaggat    1740

ttcgaaatac cggcaggaaa cgcggcgcag tttagtctga aagcggtata cggcagcaat 1800

aaaacagtgc ccgttgagta taaaaacgcg acggtgatta cgttgcagcc gctggaaacg 1860

ctggtgtttg aggcggtgac cattaac 1887

<210> 100

<211> 5334

<212> DNA

<213> Escherichia coli

<400> 100
atgaacaaaa tatttaaagt tatctggaat ccggcaacag gcagttacac cgttgccagc 60

gaaacggcga agagccgtgg taaaaaaagc gggcgcagta agctgttaat ttctgcactg 120

gttgcgggtg ggttgttgtc gtcgtttggg gcaagtgcag ataattacac tgggcagcca 180

actgattatg gcgatggctc agcaggtgac ggctgggttg ctatcggtaa aggggcaaaa 240

gcaaatacct ttatgaacac tagtggcgcg agtacagctt taggatatga cgcgatagcc 300

gaaggtgagt acagttctgc catcgggtca aaaacccttg caactggtgg agcatccatg 360

gcgttcgggg ttagtgcaaa agcaatgggt gacagaagtg tcgcgctagg tgcatcgtca 420

gtagcaaatg gcgatcgttc gatggctttt ggtcgttacg caaagacgaa tggttttaca 480

tctcttgcta ttggggactc ctcccttgcc gatggtgaaa aaactattgc gttaggaaat 540

acggctaaag cttacgaaat tatgagcatc gccctcggtg ataatgccaa tgcgtcaaaa 600

gagtatgcaa tggcgctggg agcaagtagc aaagctggcg gtgctgatag cctcgcattc 660

ggcagaaaat ctacagctaa tagcactggc tcactggcaa taggtgctga cagtagcagt 720

tcgaacgata acgccatcgc gatagggaac aaaacgcaag ccctgggagt gaattcgatg 780

gccctgggta atgcaagtca ggcatctggc gaatccagta ttgcattagg taacaccagt 840

gaagccagcg aacaaaatgc gattgcgctg gggcaaggta gcattgcaag caaagtgaac 900

tcaatcgcgt tgggaagtaa cagtttgtcc tcgggagaga atgccatcgc attgggagag 960

ggtagtgccg ctggtggcag caacagcctt gctttcggta gccagtccag ggcaaacggc 1020

aatgattctg tcgccatcgg tgtaggggct gcagcagcga ccgacaattc tgtcgctatc 1080

ggcgcaggat cgaccacaga tgcaagcaat acggtttcag ttggcaacag cgcaacaaaa 1140

cgcaaaattg ttaatatggc tgctggtgcc ataagcaaca ccagtaccga tgccatcaac 1200

ggctcacagc tttatacgat cagtgattca gtcgccaagc gactcggagg aggcgctact 1260

gtaggcagcg atggcaccgt aaccgcagta agctacgcgt tgagaagcgg aacctataat 1320

aacgtgggtg atgctctgtc aggaatcgac aataataccc tacaatggaa taaaaccgcg 1380

ggggcgttca gcgccaatca cggtgcaaat gccaccaaca aaatcactaa tgttgctaaa 1440
ggtacggttt ctgcaaccag caccgatgta gtaaacggct ctcaattgta cgacctgcag 1500
caggatgctc tgttgtggaa cggcacagca ttcagtgccg cacacggcac cgaagccacc 1560
agcaaaatca ctaacgtcac cgctggcaac ctgactgccg gcagcactga cgccgttaac 1620
ggctctcagc tcaaaaccac caacgacaac gtgacgacca acaccaccaa catcgccact 1680
aacaccacca atatcaccaa cctgactgac gctgttaacg gtctcggtga cgactccctg 1740
ctgtggaaca aagcagctgg cgcattcagc gccgcgcacg gcaccgaagc caccagcaaa 1800
atcaccaacg tcaccgctgg caacctgact gccggtagca ctgacgccgt taacggctcc 1860
cagctcaaaa ccaccaacga caacgtgacg accaacacca ccaacatcgc cactaacacc 1920
accaatatca ccaacctgac tgacgctgtt aacggtctcg gtgacgactc cctgctgtgg 1980
aacaaaacag ctggcgcatt cagcgccgcg cacggcactg acgccaccag caagatcacc 2040
aacgtcaccg ctggcaacct gactgccggc agcactgacg ccgttaacgg ctcccagctc 2100
aaaaccacca acgacaacgt gacgaccaac accaccaaca tcgccactaa caccaccaat 2160
atcaccaacc tgactgacgc tgttaacggt ctcggtgacg actccctgct gtggaacaaa 2220
acagctggcg cattcagcgc cgcgcacggc actgacgcca ccagcaagat caccaatgtc 2280
aaagccggtg acctgacagc tggcagcact gacgccgtta acggctctca gctcaaaacc 2340
accaacgata acgtgtcgac caacaccacc aacatcacca acctgactga cgctgttaac 2400
ggtctcggtg acgactccct gctgtggaac aaaacagctg gcgcattcag cgccgctcac 2460
ggcactgacg ccaccagcaa gatcaccaat gtcaaagccg gtgacctgac agctggcagc 2520
actgacgccg ttaacggctc ccagctcaaa accaccaacg ataacgtgtc gaccaacacc 2580
accaacatca ctaacctgac ggattccgtt ggcgacctta aggacgattc tctgctgtgg 2640
aacaaagcgg ctggcgcatt cagcgccgcg cacggtaccg aagctaccag caagatcacc 2700
aacttactgg ctggcaagat atcttctaac agcactgatg ccattaatgg ctcacaactt 2760
tatggcgtag cggattcatt tacgtcatat cttggtggtg gtgctgatat cagcgatacg 2820
ggtgtattaa gtgggccaac ctacactatt ggtggtactg actacactaa cgtcggtgat 2880
gctctggcag ccattaacac atcatttagc acatcactcg gcgacgccct actttgggat 2940
gcaaccgcag gcaaattcag cgccaaacac ggcattaata atgctcccag tgtaatcact 3000
gatgttgcaa acggtgcagt ctcgtccacc agcagcgacg ccattaacgg ttcacaactt 3060
tatggtgtta gtgactacat tgccgatgct ctgggcggga atgctgtggt gaacactgac 3120
ggcagtatca ctacaccaac ttatgccatc gctggcggca gttacaacaa cgtcggtgac 3180
gcgctggaag cgatcgatac cacgctggat gatgctctgc tgtgggatac aacagccaat 3240
ggcggtaacg gtgcatttag cgccgctcac gggaaagata aaactgccag tgtaatcact 3300

aacgtcgcta acggtgcagt ctctgccacc agcaacgatg ccattaatgg ctcacagctc 3360

tatagcacta ataagtacat cgctgatgcg ctgggtggtg atgcagaagt caacgctgac 3420

ggtactatca ctgcaccgac ttacaccatt gcaaataccg attacaacaa cgtcggtgaa 3480

gccctggatg cgctcgataa taacgcgctg ctgtgggatg aagacgcagg tgcctacaac 3540

gccagccatg atggcaatgc cagcaaaatc accaacgttg cggctggtga tctctccaca 3600

accagtaccg atgctgttaa cggttcccag ttaaacgcaa ccaatattct ggttacgcaa 3660

aatagccaaa tgattaacca gcttgctggt aacactagcg aaacctacat cgaggaaaac 3720

ggtgcgggta ttaactatgt acgtaccaac gacagcggct tagcgttcaa cgatgccagc 3780

gcttcaggta ttggcgctac agctgtaggt tataacgcag ttgcctctca tgccagcagt 3840

gtagccatcg gtcaggacag catcagcgaa gttgatacgg gtatcgctct gggtagcagt 3900

tccgtttcca gccgtgtaat agttaaaggg actcgtaaca ccagcgtatc ggaagaaggt 3960

gttgtgattg gttatgacac cacggatggc gaactgcttg gcgcgttgtc gattggtgat 4020

gacggtaaat atcgtcaaat catcaacgtc gcggatggtt ctgaagccca tgatgcggtc 4080

actgttcgcc agttgcaaaa cgccattggt gcagtcgcaa ccacaccaac caaatactat 4140

cacgccaact caacggctga agactcactg gcagtcggtg aagactcgct ggcaatgggc 4200

gcgaaaacca tcgttaatgg taatgcgggt attggtatcg gcctgaacac gctggttctg 4260

gctgatgcga tcaacggtat tgctatcggt tctaacgcac gcgcaaatca tgccgacagc 4320

attgcaatgg gtaatggttc tcagactacc cgtggtgcgc agaccaacta cactgcctac 4380

aacatggatg caccgcagaa ctctgtgggt gagttctctg tcggcagtga agacggtcaa 4440

cgtcagatca ccaacgtcgc agcaggttcg gcggataccg atgcggttaa cgtgggtcag 4500

ttgaaagtaa cggacgcgca ggtttcccag aatacccaga gcattactaa cctgaacact 4560

caggtcacta atctggatac tcgcgtgacc aatatcgaaa acggcattgg cgatatcgta 4620

accaccggta gcactaagta cttcaagacc aacaccgatg gcgcagatgc caacgcgcag 4680

ggtaaagaca gtgttgcgat tggttctggt tccattgctg ccgctgacaa cagcgtcgca 4740

ctgggcacgg gttccgtagc agacgaagaa aacaccatct ctgtgggttc ttctaccaac 4800

cagcgtcgta tcaccaacgt tgctgccggt gttaatgcca ccgatgcggt taacgtttcg 4860

caactgaagt cttctgaagc aggcggcgtt cgctacgaca ccaaagctga tggctctatc 4920

gactacagca acatcactct cggtggcggc aatagcggta cgactcgcat cagcaacgtt 4980

tctgctggcg tgaacaacaa cgacgcagtg aactatgcgc agttgaagca aagtgtgcag 5040

gaaacgaagc aatacaccga tcagcgcatg gttgagatgg ataacaaact gtccaaaact 5100

gaaagcaagc tgagtggtgg tatcgcttct gcaatggcaa tgaccggtct gccgcaggct 5160

tacacgccgg gtgccagcat ggcctctatt ggtggcggta cttacaacgg tgaatcggct 5220

gttgctttag gtgtgtcgat ggtgagcgcc aatggtcgtt gggtctacaa attacaaggt 5280

agtaccaata gccagggtga atactccgcc gcactcggtg ccggtattca gtgg 5334

<210> 101

<211> 681

<212> DNA

<213> Escherichia coli

<400> 101
atgaacctaa agaaaacact gttaagcgtg ttaatgatat tgcaactttg cttattggta 60

gggtgtgact atattgaaaa agcgagtaag gtcgacgatc tcgttacaca gcaagagttg 120

caaaaaagca aaattgaggc gcttgaaaaa caacaagaac tcgacaagcg caagatagaa 180

cactttgaaa aacaacaaac taccatcata aacagtacca aaacgctcgc tggtgtggtg 240

aaggcagtta aaaacaaaca ggacgaattt gtctttacag aatttaaccc ggcacaaacc 300

caatacttta ttttaaataa cggctctgtt ggtttggcag ggaaaatact gtctattgac 360

gcagtagaaa acggcagtgt tattcgtatt tcactggtta acttattaag tgttcctgta 420

tcaaatatgg gtttctacgc aacatggggg ggagaaaaac ccaccgacat caacgcatta 480

gcaaaatggc agcaattgct atttagtacc gcaatgaact cctccctgaa attattacca 540

ggtcaatggc aagacattaa tttgacgcta aaaggtgtct cgcccaacaa cctcaaatat 600

ctgaaattag ccatcaacat ggcaaatatt cagttcgacc gtcttcaacc tgctgaatct 660

ccacagcgga aaaacaaaaa a 681

<210> 102

<211> 3327

<212> DNA

<213> Escherichia coli

<400> 102
atgaaaagag ttgtgcgtct tttgggtgtg gggttactgc tccttgttgt gttgttgctc 60

attttgtttg ttctggctca gaccacaccg ctgatatcag cacaggatga gcatgctgtc 120

tggcttcgtc tgttgataac agcgattgtg atctgtttgc taagtatgtg catatttttc 180

ctcttttctt tccggcagaa cgaagcctcg acgatatcac tatacgctca accgactgat 240

ataaaggaaa taaatacgga gcagccgaac tatgcatcac tgctgacgat atatttacgc 300

gaccgctacg gtccgttctg gcggcgtaaa gtccgcctgc tgctggtgac cggcgagcct 360

gaacaggcag aagccatcgc gccggggctg accgggcaac actggctgga aggcgaccac 420
acggtgctga tatatggcgg caggccaaca gcggagcctg atgtcacact gctgaccgcc 480
ttaaaaaaac tgcgccgcag ccgtccgctg gacggcatca tctgggcgct gacagaagaa 540
cagagccgcc agacagcgca actcgacaaa ggctggcgcg gactgataaa cggcggtaag 600
cgactcggtt ttcaggctcc actctatttg tggcaggtct gtgacgacgg tgattatcag 660
accggacgcc ccctgcaaag cgtcggctgc ctgctgccgg aacgctgtac cccggaacaa 720
ctggctgtaa tgctggaagc agccgctgac ggaacagggc atgtcgcagc tactgaccga 780
taccgcatgt tttctgctgc gtctggctca tacccttgca gagcggggta ttgctcactg 840
gcagaccgtc ctgaaaccgc tgctggcagg cggcgcattt tcttccctgc gcctgcgcgg 900
cctgatgttc agcccgccgc ttgccgccgt gccggaggcc agcacctcat gcagtggctg 960
ccgtcaccgg tctgggcggg cgtgacggtg ataacgcgcg cgggcgcacg gtgggttttc 1020
ctgtggctgc gtaccgcact gatgtccgct gtctgcgtgc tggtgatatg gggggccgga 1080
atgacgacct cgttcttcgc caaccgcgct cttgttcagg aaaccggtat ccagacggca 1140
cgtgcgcttg atacccgcct gccgctggca gaacaactgg tggcgctgca taccctgcag 1200
ggcgaactgg aacgcctgca atatcgtatc cgcgaaggtg cgccgtggta tcagcgtttt 1260
ggccttgaac gtaaccaaca actgctcgcc gccgcttttc ccggctatgc gcaggcggca 1320
aaccggctgg tgcgcgacgt ggccgttgac catctgcaac agcaactgaa cgcctttgtc 1380
gccctgccgc ccaacagtcc tcagcgtacc gccaccggtg aacaacgcta taagcagctt 1440
aaggcattgc tgatgacttc ccgcccggaa aaggccgacg ctgccttttt cagtaccacg 1500
ctgatggcgg acggtctgcg ctacgagaat atcccggaag gtgtgcggca gagcgtgttg 1560
ccgtcactgc tgaccttctg gacggcgaac ctgccggaac acccgcagtg gaaaacatcg 1620
ccgccaccgg aactgaccgg cgcagtgcgt aaaatcctgc tgcgccagat tggtgtgcgt 1680
aatgccgaaa acaccctcta ccagaacgtg ctgcaacagg tgtcccgcaa ctacgccgat 1740
atgacgctgg cggacatgac cggggatacc ctcaccgaat ctcttttcag tacggaacag 1800
acggtgccgg ggatgttcac ccgtcaggcg tgggaaggac aggtcaggga agccatcgag 1860
caggtggtga cggcgcggcg cgaggaaatc gactgggtac tcagcgaccg ccagcaggat 1920
acctctgcgg atatctcgcc ggatacgctg cgtaaccgtc tcacctcacg ctactttacc 1980
gactttgccg gaagctggct ggcgtttctc aacagcattc actggaaaaa ggaagactcg 2040
ctctccggca ttctcgacca gctgacactg atggccgatg cccgtcagtc gccactgatt 2100
gcgctgacgg acaccctcgc gtggcaggcg gcgacaggca gggaaaaccg tggtctgtca 2160
gactcgctgg cgaaatcggc acaggaactg tttaacggca aggagaaaac gccgcagcaa 2220
tcccgtgaag gtgacgacgt gcctgtcggg ccgctggata aaaccttcac gccgctgctg 2280

cgtttgctgg gcgataaggc cggaggcggc gacagccagc tgagtctaca gacctacctc 2340

acccgcgtca cccgcgtgcg cctcaaactg caacaggtga ccaacgcccc cgacccgcag 2400

gagatgaccc aacaactggc gcagacggtc ttacagggta aaaccgttga cctcaccgac 2460

acccgcgact acggacggtt aatcgccgcc agtctgggcg aagaatggag tggcttcggt 2520

caggcgctgt tcgttcgccc ggtagagcag tcgtggcggc aggtgctgac gcctgcggcg 2580

gacagcctga accgccagtg gcagcgggcg attgtcagcc actggaatca ggacttcgct 2640

ggccgctatc cgttcaaagc ctcacagaac gatgcctccc tccccctgct ggcgcagtac 2700

ctgcgcgatg acgggcgcat caacctgttt atcgccgcca acctttccgg cgtgctgaaa 2760

cgagagggcc gctactgggt ggctgacgcc atgaacacgc aggggctgac ggtcaatccg 2820

gactttatcc gcgccctgaa ccgcctgcgc gacgtggccg ataccgcctt tgccagcggc 2880

gatgccggga tacattttga actgcgggca aaaccggcgc gtgacgtgat gaagacgcat 2940

ctggtgattg acgggcagga gctggaatat ttcaaccaga aagaacgctg gcagcgtttt 3000

aactggccgg atgaacagtg gcaacccggc gcatcgctaa gctggaccag cacacaggcg 3060

atggagcgca tactggcgga ttaccgggga agctggagtc ttattcgcct gctggaacag 3120

gcgcaggtga cgccggtgga cagcagcacc tttaaggtgg tgtggaaagc gcaggacggc 3180

ctgccgctga attacctgct acgggttgaa cagggtaaag ggccgctggc gctgctggag 3240

ctgaaaaact tccgcctgcc gggacaggtg tttctgaccg gaaaaagtat gaaggatgtg 3300

gaagagtatg ggaagacgc cgatgag 3327

<210> 103

<211> 534

<212> DNA

<213> Escherichia coli

<400> 103
atgtttccta ttcgttttaa acgtccggcg ttgctctgta tggcgatgct gacggttgtt 60

ctgagtggct gcggcctgat tcagaaagtg gtggatgaat cgaaaagcgt ggcctcagcc 120

gttttctaca aacaaatcaa aatactgcat ctcgatttct tctcccgcag cgccctgaat 180

acggatgcgg aagatacgcc gctttccacg atggtgcatg tctggcaact gaaaacccgc 240

gaagattttg acaaggcgga ttacgacacc ctgtttatgc aggaagagaa gacgctggag 300

aaggacgtac tggcaaaaca caccgtctgg gtaaaaccgg aaggcacggc atccctgaat 360

gtgccgctgg ataaagagac gcagtttgtc gccattattg ggcagtttta tcaccctgat 420

gaaaaaagcg acagctggcg tctggtgatc aaaagggacg aactggaggc cgacaagccg 480

cgctcgattg aactgatgag aagcgacctg cgactgctgc ctctcaagga taaa          534

<210>  104

<211>  840

<212>  DNA

<213>  Escherichia coli

<400>  104
atgatttcag ggggaaatat gttgaaagaa tggatgatat ttacgtgcag tttattgact      60

ctggctgggg cgtcactgcc cctcagtggc tgtatttcca gaggccagga gtctatatcc     120

gaaggggcgg catttggggc agggatcctg cgcgaaccgg gagcaacaaa aaaagccgac     180

acgaaagacc tcaatgtgcc accaccggtt tatggtccgc cgcaggtgat atttcgcatt     240

gatgacaacc gctatttcac gctagaaaat tatacccact gcgagaacgg gcagacgttt     300

tataataata aagcaaaaaa cattcatgtt aaaatattag acgcttcagg gtatttattt     360

aaaggccgct tattctggtt atcaacgcgt gatgattttc tggcctttcc tgccacgtta     420

aataccagac acgcttcctg tatggggtcg aataaaggct gtatgaatgc ggtcattgtc     480

actaccgatg gtggaaaaag acgcagtggt gtgccatacg gcagttatac ccagaatccg     540

accggtgcca cgagggatta tgacatgctg gtgatgaatg acggcttcta cctgcttaga     600

tatcgggggg gacagggcag atttagtccg gtgatactta gatggattct cagtactgaa     660

gatagctctg gtgttgtgcg ttcagaagat gcttatgaat tgttccgtcc cggagaagag     720

gtaccctcca ccggttttta taaaatcgac ctgtcacgtt tttatcccaa aaacaacgtt     780

atggaaatgc agtgtgacag gacgctggag ccagttcaac cttcagagag taaaattcaa     840

<210>  105

<211>  1503

<212>  DNA

<213>  Escherichia coli

<400>  105
atggaacacg ttagcattaa aacattatat catctcctgt gctgtatgct gctctttatt      60

tccgctatgt gcgctttggc gcaagaacat gagcctatcg gggcgcaaga tgagcgcctg     120

tcgacattaa ttcaccaacg gatgcaggag gccaaggtcc cagccctttc cgtaagtgtg     180

accattaagg gggtacgtca gcgatttgtc tacggtgttg ccgatgtggc tagtcagaaa     240

gcgaatactc tagacacagt ttacgagctg ggatcgatga gtaaggcgtt taccggactt     300

gtggtgcaaa tactgattca ggaaggcaga ctccggcaag gggatgatat cattacctat 360

ctgccggaaa tgcgcttgaa ttatcaggga aaacctgctt ccctgaccgt ggctgatttc 420

ctttatcata catcaggatt gcctttttca acactggctc ggctggaaaa ccctatgcct 480

gggagcgctg tggcacagca actgcgcaac gagaatctgc tgtttgcgcc gggtgcgaag 540

tttagctatg cctccgccaa ttatgatgtg ttgggcgcgg tgattgaaaa tgtgacggga 600

aaaaccttta cagaggtcat tgcggaacga ctcacgcagc cgctgggcat gtcggcgact 660

gtggcagtta agggggatga gattattgtc aacaaggcaa gcggctataa actgggattc 720

ggcaaacccg ttctgtttca tgcgcctctg gcccggaacc atgttcctgc cgcctatatc 780

catagcactc tgcctgatat ggaaatatgg atagacgcct ggttgcacag aaaggctttg 840

ccggcaacgc tgcgtgaggc gatgagtaac agttggcgtg gtaatagtga tgttccgctt 900

gccgcagaca atcgtatcct ctatgccagc ggttggttta tcgaccagaa tcaaggccct 960

tacatcagtc acggtgggca gaatccaaac ttttcttctt gcattgcgtt gcgaccggat 1020

cagcagattg gcattgttgc gctggcaaat atgaattcga atctgatact acagctttgc 1080

gcggatatcg ataattatct gcgcattggc aaatatgctg acggcgctgg tgatgcaatt 1140

acagccaccg ataccctttt cgtctacctc acgttgttgc tgtgtttttg gggggcggtg 1200

gttgtagtgc gcggtgcttt ccgtgtttat cgcgcaacgg cgcatggccc tggaaaacag 1260

cagaggttac gtttacgcgt acgtgactat atcatcgcct tggcggttcc tgggctcgtg 1320

gccgccatgc tctatgtcgc accgggtata ctatctccag gacttgactg gcgttttatc 1380

ttggtatggg gtccatcgag cgtgttggcg ataccgttcg gaattatcct gttagctttc 1440

gttctgacat taaatcatca aattaaacga attctattac acaacaagga gtgggacgat 1500

gag 1503

<210> 106

<211> 2046

<212> DNA

<213> Escherichia coli

<400> 106
atgaagaaca aatatatcat tgctccgggc attgccgtga tgtgttctgc agttatatca 60

tcaggttatg ccagttctga taaaaaagaa gatacgcttg ttgttactgc ctccgggttc 120

actcagcagc tcagaaatgc cccggccagt gtctcagtca ttacttcaga acaactgcaa 180

aaaaaaccgg tttcagatct ggtcgatgca gtaaaagatg ttgaagggat tagtatcact 240

ggtgggaatg aaaaaccgga tatcagtata cgtggtctaa gtggcgatta cacgctgatt 300

ctggtcgatg gacgacgtca gagcggtcgg gaatccagac caaacggcag cggcggtttt 360

gaagccggat ttatccctcc tgtggaagca attgaacgca ttgaagtgat ccgtggccct 420

atgtcttccc tgtatggttc tgatgccatc ggaggggtca ttaatatcat aaccaaacca 480

gttaataacc aaacatggga tggcgtactt ggacttgggg ggattattca ggaacatggg 540

aaatttggta actcaaccac aaatgacttc tatctgtcag gcccattgat taaggataaa 600

cttggtcttc agctatatgg aggaatgaac tatcgcaagg aagatagtat ctctcaggga 660

acaccggcaa aagataataa gaatataacg gcaacgctcc agtttactcc gactgaaagc 720

cagaagtttg tttttgaata tggaaaaaat aaccaggtgc atacattaac acctggtgag 780

tctctcgatg cctggactat gcggggaaat cttaaacaac caaacagtaa aagagaaacg 840

cataattcac gtagtcactg ggtagcagca tggaatgccc agggcgaaat actgcatcct 900

gaaattgctg tttatcagga gaaagttatt cgtgaggtta aatcaggtaa aaaagataaa 960

tataatcatt gggatcttaa ttacgagtca agaaaaccgg aaataaccaa cacaatcata 1020

gatgcaaaag tgacggcatt tctgccggaa aatgtactga ccatcggagg tcaatttcag 1080

catgcagagc tccgtgatga ctcagccacg ggtaaaaaaa cgacagaaac acagtctgtt 1140

tcaattaaac agaaagctgt ttttatagaa aatgaatatg cagcaacgga ttctctcgcc 1200

ctgactggag gactgcgtct cgataatcat gaaatctatg gcagttactg gaatccaaga 1260

ttgtacgctg tttataacct gaccgataat ctcacactca aaggggggat cgcaaaagca 1320

tttcgggctc cttcaattcg tgaggtgagt cctggatttg gaacactgac gcagggtggt 1380

gcctctatta tgtatggaaa cagggacctg aaaccggaga ccagtgtaac cgaagagatc 1440

ggtattattt atagtaatga tagtggtttt tcggcgagcg cgacgctgtt taatactgat 1500

tttaaaaata agttgaccag ttacgatata ggtacaaaag atccagtcac cgggttaaac 1560

acttttattt atgataatgt aggtgaggca aatatcagag gggtggagct tgcaactcag 1620

attcctgtgt atgataaatg gcatgtatct gcaaactata catttactga ctctcgtcga 1680

aaaagtgatg acgaaagtct caatggcaag tcgctgaaag gggaacctct ggaaagaact 1740

cccagacatg cagccaatgc aaaactggaa tgggattaca ctcaggatat tacattttat 1800

tcatctctga attatacggg aaaacaaatc tgggcagcac aaagaaatgg tgctaaggtt 1860

ccccgcgttc gtaatggatt cacatctatg gatattggtc taaattacca gattctgcca 1920

gacacgctga ttaattttgc cgttcttaac gtcacagaca gaaagagcga ggatatcgat 1980

accattgatg gtaactggca ggtcgatgaa ggacgccgtt attgggctaa tgtaagagta 2040

tccttc 2046

<210> 107

<211> 492

<212>  DNA

<213>  Escherichia coli

<400>  107
atggggttta gaaaaacaat aatcacttcg gtaggtttga tatttatttc attctctttt      60

gtggcaaagt gctctcaact caaaaatttg aataattact cagtgatgct ttgtggaaaa     120

gtgtcaaata atatcctgga tgatattggt ggttataaag aaagaaatat attaatgctg     180

cgagctataa aaaaaatcat aataatgaca atcgtaaata ttatattttt ctattccttt     240

caatcgactg cggatgaaat ggttttaata aaaaaatacg ggtttgggct tgagagagat     300

atcaaaggaa ggccattaat ttatcctatc gaaaattatg atgagtgtaa gaaaaaatgc     360

aatcatatga attatatagc ggatgtcaat gctcaattag ctatgagtaa aaaaaataac     420

aggatttttg ctaacataac ctttactaac aatagctcta ccacgtattt ttttctaaat     480

attatctacc ta                                                         492

<210>  108

<211>  654

<212>  DNA

<213>  Escherichia coli

<400>  108
atgaatcaaa ttaaagataa taaggtaatt atgaaaataa aaaatttaat atcagtcatt      60

ttactatcag gaggtattat ggggactgga ttgtactcga gcgataacca tcaaaaaatc     120

cgcagcaggt ttaatataca ggaatcatat tgtgccatta agactaatgg tgtccttgga     180

ttcagcaacc gaaaggatgt attgcgagaa aatggtgatt caaccggaac caccagttcc     240

agcactaatg ccatgatgct gatggaaaat ggtgaaaatg aaatcagtct ggagattggg     300

gcgttaaggt ggttttctga taaacctgcc agtaccgaag aacgagggca tttctcccaa     360

aaagcagggt gcagtctgga tttggttcgt tttgttaagc aggaagaaac catactttct     420

tcgataaagg tgaccatcaa ccagcaggga atacctgaag cgcagccaga cagcatgcat     480

cctgttatcc gaaaagagat tctggctgag caggcagaac ccggatttat tgatccagac     540

tattttaatg aaacttattt cccgaaaggg atgaaggtgt atcaatttac acaaaaggtc     600

tcggtggcgg ggcttcctga tgggcctgga cgcagtacgc cctttaccgg agca           654

<210>  109

<211>  8198

<212>  DNA

<213>  Escherichia coli

<400>  109
atgcatcagc ctcccgttcg cttcacttac cgcctgctga gttacctggt cagtgcgatt      60

atcgccgggc agccgttgtt accggctgtg ggggccgtca tcaccccaca aaacggggcc      120

ggaatggata aagcggcaaa tggtgtgccg gtcgtgaaca ttgccacgcc gaacggggcc      180

gggatttcgc ataaccggtt tacggattac aacgtcggga aggaagggct gattctcaat      240

aatgccaccg gtaagcttaa tccgacgcag cttggtggac tgatacagaa taacccgaac      300

ctgaaagcgg gcggggaagc gaagggtatc atcaacgaag tgaccggcgg taagcgttca      360

ctgctgcagg gctatacgga agtggccggc aaagcggcga atgtgatggt tgccaacccg      420

tatggtatca cctgtgacgg ctgtggcttt atcaacacgc cgcacgcgac gctcaccacg      480

ggcaaacctg tgatgaatgc cgacggcagc ctgcaggcgc tggaggtgac tgaaggcagt      540

atcaccatca atggcgcggg cctggacggc acccggagcg atgccgtatc cattattgcc      600

cgtgcaacgg aagtgaatgc cgcgcttcat gcgaaggatt taactgtcac tgcaggcgct      660

aaccgtgtaa ctgcagatgg tcgtgtcaga gccctgaagg gcgaaggtga tgtgccgaaa      720

gttgccgttg ataccggcgc tctcggtgga atgtacgcca ggcgtattca tctgacctcc      780

actgaaagtg gtgtcggggt taatcttggt aacctttatg cccgcgatgg cgatatcacc      840

ctggatgcca gcggcagact gactgtcaac aacagtctcg ccacgggggc cgtcactgca      900

aaaggtcagg gcgtcacctt aaccggcgac cataaagcgg gaggtaacct gagcgtcagc      960

agccggagag atatcgttct cagcaatgga acgcttaaca gcgacaagga cctcagcctg     1020

accgccggcg gcagaatcac tcaacagaat gaaaaactga ctgccggccg ggatgtaacg     1080

cttgccgcga aaaacatcac acaggatacc gccagccaga ttaacgcggc ccgcgatatc     1140

gtgactgtcg ccagtgacac gctgacaaca cagggacaga taaccgccgg gcagaatctc     1200

acggccagcg ccaccacgct gacgcaggac ggaatattgc tggcgaaaag tcatgcggga     1260

ctcaatgccg gtacgctgaa taacagtggc gccgttcagg gagctaccct gacgctcggc     1320

agtacaacgc tcagcaacag tggctccctg ctcagtggcg gtcccctgac catgaatacc     1380

cgcgacttta cccagagcgg ccgcactggc gcgaagggca aagtggatat catggccagt     1440

gggaaactga ccagtacagg tttgctggtg acgatgcact tggtgctgaa ggcgcaggat     1500

gtgacacaga acggtgtgct gtccggcggc aaagggctga cggtcagtgc gacgagctcc     1560

ggtaaaaaat cggtcaccca cagcgatgct gcgatgacgc tgaatgtgac aacagtggcg     1620

ctggacgggg aaaccagtgc cggtgacacc ctccgggttc aggcagacaa actgagtacc     1680

gcagcgggcg cacaacttca gagcggcaaa aatctcagca tcaacgccag agatgcacgt 1740

cttgcaggta cgcaggcagc acaacagacc atggtggtga acgccagtga aaagctcacc 1800

cacagcggga aaagcagtgc cccgtcgctc agcctcagtg cgccggaact gaccagcagc 1860

ggcgtacttg ttggttccgc cctgaataca cagtcacaga ccctgaccaa cagcggtctg 1920

ttgcaggggg aggcctcact caccgttaac acacagaggc ttgataatca gcagaacggc 1980

acgctgtaca gtgctgcaga cctgacgctg gatataccgg acatccgcaa cagcgggctt 2040

atcaccggtg ataatggttt aatgttaaat gctgtctccc tcagcaatcc gggaaaaatc 2100

atcgctgaca cgctgagcgt cagggcgacc acgctggatg gtgacggcct gttgcagggc 2160

gccggtgcac tggcgcttgc tggcgacacc ctctcacagg gtagtcacgg acgctggctg 2220

acggcggacg acctctccct ccggggcaaa acactgaata ccgcagggac cacgcaggga 2280

cagaatatca ccgtgcaggc ggacagatgg gcgaacagtg gttccgtgct ggcaaccggt 2340

aaccttactg cttcggcaac cggtcagttg accagtaccg gcgatatcat gagccagggt 2400

gacaccacgc tgaaagcagc caccacggac aaccggggca gtctgctttc ggccggcacg 2460

ctctcccttg atggaaattc actggataac cgcggcactg tccagggtaa ccatgtcacg 2520

attcgccaga acagtgtcac caacagtggc acgctcaccg ggatcgccgc actgacgctt 2580

gccgcccgta tggcatcccc tcaacctgcg ctgatgaata acggaggttc attgctgacc 2640

agcggcgatc tgacaatcac cgcaggcagt attaccagtt ccggacactg gcagggcaaa 2700

cgggtgctga tcaccgcaga cagtctggca aacagcgggg cgatccaggc ggctgacagc 2760

ctgactgcac gtctgacggg tgagctcgtc agcacagcgg gcagcaaagt cacctcgaac 2820

ggtgaaatgg cgctcagtgc actgaattta agcaacagcg gacaatggat tgcaaaaaat 2880

ctgaccctga aggcgaactc actgaccagt gcgggtgaca tcaccggtgt ggatactctc 2940

acgctcacgg tgaatcagac gctgaacaat caggcgaacg gaaaactgct cagtgcaggt 3000

gtgctgacgc tgaaggcaga cagtgtcaca aacgacggcc aattacaggg aaatgtcacc 3060

accatcacgg caggacaact cacaaacggc gggcatctgc agggcgaaac gctgacgctg 3120

acagcctccg gtggcgtgaa caaccgttcc ggtggtgttc tgatgagccg gaatgcactg 3180

aatgtcagta ctgcgaccct gagtaaccag agcacgatac agggtggagg cggggtttcc 3240

ctgaacgcca cagaccgtct gcagaacgac ggcaaaatcc tctccggcag taacctcacg 3300

ctgacggcgc aggtgctggc gaacaccggc agcggactgg tacaggctgc caccctgctg 3360

ctggatgtgg tgaatactgt caacggcgga cgcgtacttg ccaccggcag tgacgttaaa 3420

ggaaccacgc tgaataatac cggtacgctt cagggtgcga ctctggtgaa ttaccacaca 3480

ttcagcagcg gtaccctgct gggaacctcc gggcttggcg tcaagggcag ttcactgctg 3540

caaaatggta cagggcggct gtacagtgca ggcaacctgc tgcttgacgc tcaggacttc 3600

agtggtcagg ggcaggtggt ggccaccggt gatgtcacac tgaaactgat tgctgccctc 3660

acgaatcatg gtaccctggc cgcagggaaa accctttccg tcacgtcgca aaatgccatc 3720

accaacggcg gtgtcatgca gggtgatgcc atggtgctcg gtgccggaga ggcattcacc 3780

aacaatggac tgactgccgg taaaggcaac agtgttttca gcgcacagcg tcttttcctt 3840

aacgcaccgg gttcacttca gggcggtggc gatgtgagtc tgaacagccg gagtgatatc 3900

accatcagtg gttttaccgg cacggcaggc agtctgacaa tgaatgtggc cggtaccctg 3960

ctgaacagtg cgctgattta tgcggggaat aacctgaagc tgtttacaga ccgtctgcat 4020

aaccagcatg gtgatatcct ggccggcaac agtctgtggg tacagaagga tgcttccggc 4080

ggtgcaaaca cagagattat caataattcc gggaatattg agacgcatca gggcgatatt 4140

gttgtaagaa ccgggcatct tctgaaccag cgggagggat tttctgccac aacaacaacc 4200

cggactaacc cctcatccat tcagggaatg ggaaatgctc tggttgatat tcccctttcc 4260

cttcttcctg acggcagcta tggctatttc acccgtgaag ttgaaaatca gcacggtacg 4320

ccctgcaacg ggcacggggc atgcaatatc acaatggata cgctttatta ttacgcgccg 4380

tttgctgaca gtgccacaca gcgctttctc agcagccaga acatcacaac agtaaccggt 4440

gctgataatc cggcaggccg cattgcgtca gggcgtaatc tttctgctga ggctgaacga 4500

ctggaaaacc gggcgtcatt tatcctggcg aatggggata tcgcactctc gggcagagag 4560

ttaagcaatc agagctggca gacggggaca gagaatgaat atctggtata ccgctacgac 4620

ccgaaaacgt tttacggtag ctatgcaaca ggctctctgg ataaactgcc cctgctgtca 4680

ccggaatttg aaaacaatac catcagattt tcactggatg gccgggaaaa agattacacg 4740

cccggtaaga cgtattattc cgttattcag gcgggcgggg atgttaagac ccgttttacc 4800

agcagtatca ataacggaac aaccactgca catgcaggta gtgtcagtcc ggtggtctct 4860

gcacctgtac tgaatacgtt aagtcagcag accggcggag acagtctgac acagacagcg 4920

ctgcagcagt atgagccggt ggtggttggc tctccgcaat ggcacgatga actggcaggt 4980

gccctgaaaa atattgccgg aggttcgcca ctgaccggtc agaccggtat cagtgatgac 5040

tggccactgc cttccggcaa caatggatac ctggttccgt ccacggaccc ggacagtccg 5100

tatctgatta cggtgaaccc gaaactggat ggtctcggac aggtggacag ccatttgttt 5160

gccggactgt atgagcttct tggagcgaag ccgggtcagg cgccacgtga aacggctccg 5220

tcgtataccg atgaaaaaca gtttctgggc tcatcgtatt ttcttgaccg cctcggcctg 5280

aaaccggaaa aagattatcg tttcctgggg gatgcggtct ttgatacccg gtatgtcagt 5340

aacgcggtgc tgagccggac gggttcacgt tatctcaacg gactgggttc agacacggaa 5400

cagatgcggt atctgatgga taacgcggcc agacaacaga aaggactggg attagagttt 5460

ggtgtggcgc tgacagctga acagattgct cagcttgacg gcagcatact gtggtgggag 5520

tcagtcacca tcaacggaca aacagtcatg gtcccgaaac tgtatctgtc gccggaagat 5580
atcaccctgc ataacggcag cgttatcagc gggaacaacg tgcaacttgc gggcggcaat 5640
atcaccaaca gcggcggcag catcaacgca cagaacgacc tctcgctcga cagttccggc 5700
tatatcgaca acctgaatgc ggggctgata agcgcgggcg gtagcctgga cctgagcgcc 5760
atcggggata tcagcaatat cagctcagtc atcagcggta aaaccgtaca actggaaagc 5820
gtgagtggca acatcagcaa tatcacccgg cgtcagcaat ggaatgcggg cagtgacagc 5880
caatatggtg gtgtgcatct cagcggtacg gacaccggtc cggttgcgac cattaaaggc 5940
actgattcac tttcgctgga tgcagggaaa aacattgata ttaccggggc aacggtctcg 6000
tccggtggag accttggaat gtctgcgggt aatgatatca acattgccgc aaacctgata 6060
agtgggagca aaagtcagtc cggtttctgg cacactgatg acaacagttc atcatccacc 6120
acctcacagg gcagcagcat cagcgccggc ggtaacctgg cgatggctgc aggccataat 6180
ctggatgtca cggcatcctc tgtttctgcc gggcacagcg ccctgctttc ttgcaggtca 6240
cgacctagtc ttgaatgcag tcagggaaaa gcaaaaacaa gtcgcaacgg caggtcagaa 6300
agtcatgaaa gccacgcagc tgtgtccacg gtgacagcgg gcgataactt cctccttgtt 6360
gccggtcgtg atattgccag tcaggctgcc ggtatggctg cggaaaataa cgtggtcatc 6420
cggggcggac gtgatgtgaa cctggtggca gagtctgccg gcgcaggcga cagctatacg 6480
tcgaagaaaa agaaagagat taacgagaca gtccgtcagc agggaacgga aatcgccagc 6540
ggtggtgaca ccaccgtcaa cgcaggacgg gatatcaccg ctgttgcgtc atccgttacc 6600
gcaaccggca atatcagcgt gaatgccggt cgtgatgttg ccctgaccac ggcgacagaa 6660
agtgactatc actatctgga aacgaagaaa aaaagcggag gttttctcag taagaaaacc 6720
acccgcacca tcagtgagga cagtgccacc cgtgaagcag gctccctgct gtcggggaac 6780
cgcgtgaccg ttaacgccgg tgataacctg acggtagagg gttcggatgt ggtggctgac 6840
cgggatgtgt cactggcggc gggtaaccat gttgatgttc ttgctgccac cagtacagat 6900
acgtcctggc gctttaagga aacgaagaaa tccggtctga tgggtaccgg cggtattggt 6960
ttcaccattg gcagcagtaa gacaacgcac gaccggcgcg aggccgggac aacgcagagt 7020
cagagtgcca gcaccatcgg ctccactgcc ggtaatgtca gtattaccgc gggcaaacag 7080
gctcatatca gcggttcgga tgtgattgcg aaccgggata tcagcattac cggtgacagt 7140
gtggtggttg acccggggca tgaccgtcgt actgtggacg aaaaatttga gcagaagaaa 7200
agcgggctga cggttgccct ttccggcacg gtgggcagtg ccatcaataa tgcggttacc 7260
agtgcacagg agacgaagga gagcagtgac agccgtctga aagccctgca ggccacaaag 7320
acagcgctgt ctggtgtgca ggccggacag gctgcgacaa tggcctccgc aaccggtgac 7380
ccgaatgcgg gagtcagcct gtcgctcacc acccagaaat cgaaatcaca acaacattct 7440

gaaagtgaca cagtatccgg cagtacgctg aatgccggga ataatctgtc tgttgtcgca 7500

accggcaaaa acaggggcga taaccgcgga gatattgtga ttgcaggaag ccagcttaag 7560

gccggtggta acacaagcct ggatgccgcg aatgatattc tgttgagtgg cgccgcaaac 7620

acacaaaaaa caacgggcag gaacagcagc agtggcggtg gcgtgggtgt cagtatcggt 7680

gcaggtaaag gtgccggtat cagcgccttt gccagcgtta atgcggcaaa aggcagggag 7740

aaaggtaacg gtactactac cgacaaaacc gtcaccatca acagtggtcg ggatacggta 7800

ctgaacggtg ctcaggtcaa cggcaacagg attatcgccg atgtgggcca cgacctgctg 7860

ataagcagcc agcaggacac cagtaagtac gacagtaaac agaccagcgt ggctgccggc 7920

ggcagtttta cctttggctc catgaccggc tcaggttaca tcgctgcctc ccgggataag 7980

atgaagagcc gctttgactc cgttgctgaa caaaccggaa tgtttgcccg ggtgatggtg 8040

gcttcgacat cacagtgggt aaacataccc aactggatgg tgcggtcatt gcctcactgc 8100

cacaccggag aaaaaccacc tggataccgg acgctgggtt tagtgacttt acaacgaagc 8160

gggattataa agtcaagtca caggtggaat cagtctga 8198

<210> 110

<211> 963

<212> DNA

<213> Escherichia coli

<400> 110
atgatgttga agaaaacgat atttatatta acgttattct ctggcaacgt aattgctgca 60

actgtagaat taggttttga aaatgagcaa tataattatg cttatcgttc tgcagatgtc 120

ttcatgccgt atattaagag taatttcaac cctgttactg attctgcttt gaatgtgtca 180

ctcacctata tgtatcagga tcaatatggg aaaaaacata aaaaaacatc tgaggacaga 240

tttaaaacca atcgcgatcg catagagctc tatcttaaag gttatacttt aaatagggga 300

gcatattctt tttctccttc cgcaggtttc cgttatgagt catgggatgt aaactacgat 360

aatccgaaaa agcaggataa gtggaaactg gaactacgct tttatcctaa tatgacttat 420

aaactcaatg accagttaag cctatatatg aatggttttg ttgcccctgt attttttaaa 480

acacaacaag agtcgagaaa agataacaat tatgtaaagg gtaagttagg ggcgaaacgt 540

tataacaacg attattatca ggaactccag attctgggtg tcagatataa atttaataat 600

gataatacgc tctgggcatc agtctataat gaaagaaaat ataatcaaca ttcctcaaaa 660

tatgatcgct ggcaattgcg tggaggctat gattttaaag ttacagagga gtttgttttg 720

agtccattca taagatatga cctctcttat agagaaaaaa acctcgaaag cacaagtaat 780

aatggtttat caaaaaataa taaagaaatt cgaactggag ccagcttttc ctataaaatt 840

atcccttctg taaaactggt aggagaaata tacaggcaaa caaccaacat tgaaaactat 900

tatggagagc attctgaaga caaaaaccgc atgttctaca aacttggtat aaacaaaaca 960

ttt 963

<210> 111

<211> 1761

<212> DNA

<213> Escherichia coli

<400> 111
atgcagcacc ggcagaaaaa cattctgacg aaaacgtccc ttttatcccg tgcgttgtct 60

gtcccctgtt gtgatatgtt ccggcgcggc tctccgtgga tatgctatct ctccctctcc 120

gttttttctg gttgtttcat ccccgcattt tcgtctccgg cagccatgct gtctccgggt 180

gaccgcagtg caattcagca gcaacagcag cagttgctgg atgaaaacca gcgtcagcgt 240

gatgcgctgg agcgcccgct gaccatcacg ccgtctccgg aaacgtctgc cggtactgaa 300

ggtccctgct ttacggtgtc aagcattgtt gtcagtgggg ccacccgact gacgtctgca 360

gaaaccgaca gactggtgcc gtgggtgaat cagtgtctga atatcacggg gctgaccgcg 420

gtcacggatg ccgtgacgga cggctatata cgccggggat atatcaccag ccgggccttt 480

ctgacagagc aggacctttc agggggcgta ctgcacataa cggtcatgga aggcaggctg 540

cagcaaatcc gggcggaagg cgctgacctt cctgcccgca ccctgaagat ggttttcccg 600

ggaatggagg ggaaggttct gaacctgcgg gatattgagc aggggatgga gcagattaat 660

cgtctgcgta cggagccggt acagattgaa atatcgcccg gtgaccgtga gggatggtcg 720

gtggtgacac tgacggcatt gccggaatgg cctgtcacag ggagcgtggg catcgacaac 780

agcgggcaga agagtaccgg tacggggcag ttaaatggtg tcctttcctt taataatcct 840

ctggggctgg ctgacaactg gtttgtcagc gggggacgga gcagtgactt ttcggtgtca 900

catgatgcga ggaattttgc cgccggtgtc agtctgccgt atggctatac cctggtggat 960

tacacgtatt catggagtga ctacctcagc accattgata accggggctg gcggtggcgt 1020

tccacgggag acctgcagac tcaccggctg ggactgtcgc atgtcctgtt ccgtaacggg 1080

gacatgaaga cagcactgac cggaggtctg cagcaccgca ttattcacaa ttatctggat 1140

gatgttctgc ttcagggcag cagccgtaaa ctcacttcat tttctgtcgg gctgaatcac 1200

acccacaagt ttctgggggg ggtcggaaca ctgaatccgg tattcacacg ggggatgccc 1260

tggttcggcg cagaaagcga ccacgggaaa aggggagacc tgcccgtaaa tcagttccgg 1320

aaatggtcgg tgagtgccag ttttcagcgc cccgtcacgg acagggtgtg gtggctgacc 1380

agcgcttatg cccagtggtc accggaccgt cttcatggtg tggaacaact gagcctcggg 1440

ggtgagagtt cagtgcgtgg ctttaaggat cagtatatct ccggtaataa cggcggttat 1500

ctgcggaatg agctgtcctg gtctctgttc tccctgccat atgtgggaac tgtccgtgca 1560

gtggctgcac tggacggcgg ctggctgcac tctgacagcg atgacccgta ctcgtccggc 1620

acgctgtggg gtgctgctgc cgggctcagc accaccagtg gccatgtttc cggttcgttc 1680

actgccggac tgcctctggt ttacccggac tggcttgccc ctgaccatct cacggtttac 1740

tggcgcgttg ccgtcgcgtt t 1761

<210> 112

<211> 2220

<212> DNA

<213> Escherichia coli

<400> 112
atgaataagc acacactatt actgactgtt ctttttctga atttgatttg tactcccgtt 60

tttgctcaaa actggcaggt ggcgacgttt ggtcagtcta cggatctcaa cttttcatcg 120

ctgatagatt cggccaagat cggacggaat aatgcctggc ttgcaggaaa caataatttt 180

cttgaagctg gaaaatttta cactttacca acagattttt ttattgaaag ccgtggggga 240

aaaattgcta actcccatga cggtatgacc gtcttttata ctattgttcc ggttactcag 300

acattccgac tggaggctga tttgacatta gaacagattg gtccggaggt gaatggaaaa 360

tcaccagcgg gacaggaggg agctggattg tttgtcagag atattatcgg tcctcagcga 420

caggaacctc agtcagctgg aacagaagaa tatccccagg cctctaatat attgatgaat 480

gcctttatta cacagaataa aaagaatgat aacttagtac agattacttc aattgttcgt 540

gaaggagtaa taaaaacatg gggtaatgaa ggtattacaa ttaagaaaca gccgatcatt 600

gagaatataa actttacgca aaaaagaaat attcatatga cgatcgagcg actaccagag 660

aagttcatcc tgaccgcttt tgataccgat cgtaaagaaa atcagtcatg gcaattttct 720

gattactcag gctttatgaa tcaactggat aataatagtt tagctattgg tttttttgcc 780

gcacgaaatg cgaaactaag ggtgaaaaat gcatcattta aaccgggcaa gccactggtt 840

gattacaaac aattaacttc acgtcaattc agtcgtgtcc ggcataaagc ccctgaactt 900

tttcttgctt cacctcaatc cgttgtaaga aactcaacaa ctcttcaatt tttggccaat 960

caggctggaa tagtcagtat tgataatgat aagcagacta agcaggtgca ggcgggtgaa 1020

ctggtacagt ttccagttac tttgcaaaaa aaacataatg acttcaccgt caactttaac 1080

gttgatggga atatatcaaa aaaagctata cgcatagagc aggttaaatc aaacctgact 1140

gatccttatg agatttacgt atgtagtgat tgtcgacagg gggccagagg cagcaaaaat 1200

gaccctgtag atttacagac agccgtaaaa tttgtcgcac ccggcggtaa tatatacctt 1260

aacgatggtc aatatcatgg aattacctta gatcgggaat taagtggaat acctggcaag 1320

tataaaacaa tttctgccat taatccacat aaagccattt ttataaacaa gacattcaat 1380

ctggatgcaa gttactggca tctaaaatcc gtggtctttg acggcaatgt ggataatgga 1440

aataataaac cagcatattt gcgtatagct ggtagctata atattattga gcatgtgata 1500

gccagaaata atgatgatac gggaatttct atttcagcga aagataaaaa ccgttttttc 1560

tggccagctc ataacttagt tttaaactca gattcatata ataatcttga tttatccggg 1620

attaatgccg atggttttgc tgcaaaatta ggtgtcggac cgggaaacat ttttcgagga 1680

tgcattgcac ataataatgc agatgatggt tgggacctat ttaacaaaat tgaagatggt 1740

ccaaatgcat ctgttactat tgagaattct gtagcctatg aaaatggcct gccatacaat 1800

aaagcggata tcctaaaagg gagtattggc aatggcggtg aaggtcaacc cagtaaatca 1860

caagttatta attccattgc tattaataat aatatggatg gattcactga taattttaat 1920

actgggtcat tgatagttag aaataatata gcaatgaaca atgcacgcta taattatatt 1980

ttaagaacta acccatataa attcccatca tctatccttt ttgataataa ttattcaatc 2040

agagatgatt gggaaaataa aataaaagac ttcttaggtg atacagttaa cagtgtgaat 2100

tataaattgc ttgtttcaca tgaaacagga ccggtacaaa aagatttatt tttcacacga 2160

gatgatagtg gaaatattat ctatcctgat ttttttctta atatcattaa taaatttaat 2220

<210> 113

<211> 408

<212> DNA

<213> Escherichia coli

<400> 113
atgaaaactt ttatcaaaac tttactcgtt gctgtaacta ttctgttctc tgtcttcgct 60

acggcgaaac aagtaaaact gccaaacaac atcaaatacg ttaatactac agaggcgttt 120

tcctgtactg agattgacgg tatgaattgc cagacgaaga atccgtttaa ctataaagat 180

aacagctatg ttttcgtgct tgaacgtggt ggtgcctggt gttacgacta cactgtctcg 240

gtacttaacc tgaaaaccgg gaaagcacag atgctcgaat acaaagacaa ccagctgtgc 300

tcaggtagca acaaaccgtt cttcgaaatc aaaaatggcg taccgacggt aggagtcatc 360

gacacatccg gaaaacctgt cgttgtggct ctggacaaac ttaaaacc 408

<210> 114

<211> 675

<212> DNA

<213> Escherichia coli

<400> 114
atgcaattac ctgtaaagtt attaatgagc cttatatctc tggtcagcgt tattgcacgt 60

gccgggaaat ataaaaatta catccgggat gaaataaaat actggcgata tacatcatac 120

aagggggggg aatttccgga aggtttcact gatgagaaat tttccagcgc catttacaac 180

ggaagaatat ttacaatgaa acgtttacat accctgatgt tatttctggc ggttctgttt 240

actggcttta acgtggaagc agcgagcgtg aaacaagcgc tcagctgcga cccaaacgcc 300

cgggctgaac aacctggagc gtgtccaaca acgtacgagt tgtacgaagg tgacgctgcc 360

tacaaagctg cgcttgacaa agcattaaaa ccggtcggac tgagcggcat gttcggtaaa 420

ggcgggtata tggatggccc tggcggaaac gtaacgccag taaccattaa cggtacagtc 480

tggctccagg gcgacggttg caaagccaat acctgcggct gggactttat cgtaacactc 540

tataacccaa aaacccatga agtcgttggc taccgctact ttggtttaga tgacccggcc 600

tacctggttt ggttcggcga aattggcgtg catgaattcg cgtatctggt gaaaaactac 660

gtagctgcgg ttaac 675

<210> 115

<211> 2163

<212> DNA

<213> Escherichia coli

<400> 115
atgaaaactc aaataacttt cgctgcgctt ttgccagcat tagcgtcttt cataccgctt 60

catgctcatg cctcgtctac ttctgaagat gaaatgattg tcacgggcaa caccgccgcc 120

gacaccaccg attctgccgc cggtgccggt ttcaaaacga acgatataga tgtcggcccg 180

ctgggaacga aatcctggat cgaaacacca tattccagca ccactgttac taaagagatg 240

attgaaaatc agcaggcgca aagcgtcagc gagatgctga aatactctcc cagtacgcaa 300

atgcaggcgc gcggtggaat ggatgtcggg cgtccgcaaa gtcgggggat gcagggcagc 360

gtggtggcca acagccgtct ggacgggctg aatatcgttt caacaaccgc gtttccggtg 420

gaaatgcttg agcgcatgga tgtgcttaac agtttgaccg gcgcgctgta cggcccggcg 480

agcccagcag ggcagtttaa tttcgtggcg aagcgcccaa ccgaagagac gctgcgtaaa 540

gtgacgctgg gctatcaaag ccgcagtgcg tttaccggcc atgccgatct gggtggccat 600

tttgatgaaa acaaacggtt tggctatcgc gtgaacctgc ttgatcagga aggggaaggc 660

aatgtggatg acagcacgct gcgtcgcaaa ctcgtttccg ttgcgctcga ctggaatatt 720

cagccgggca ctcagctaca gctcgacgcc agccattacg aatttatcca gaaaggctat 780

gtcggtagct ttaactatgg gccgaacgtc aaactgccgt ctgcgccgaa tccgaaggac 840

aaaaatctgg cgctcagcac tgcgggcaac gacctcacta ccgataccat cagcactcgc 900

ctgatccact actttaacga cgactggtcc atgaacgctg gcgtgggctg gcagcaggct 960

gaccgcgcga tgcgtagtgt ttccagtaaa atactcaaca atcagggcga tatctctcgt 1020

tcgatgaagg attccaccgc tgccggacgt tttcgcgtcc tgagcaacac cgccgggctg 1080

aatggtcata ttgataccgg ctctatcggc cacgatctgt cactttctac cacgggatat 1140

gtctggtcgc tttatagtgc caaaggaaca ggttccagct atagctgggg tacaacaaat 1200

atgtatcacc cggatgcgat agatgagcag ggcgatggca aaatccgcac cggcgggccg 1260

cgataccgct ccagcgtaaa tactcagcag agcgttacgc tcggcgatac ggtgacattt 1320

acgccgcagt ggtcggcaat gttctatctc agccagagct ggctgcagac taaaaactac 1380

gataagcacg gtaatcaaac gaaccaggtt gatgaaaatg gtttaagtcc gaacgccgcg 1440

ctgatgtata aaattacccc taacacaatg gcctacgtta gctatgccga ttcgctggag 1500

cagggcggta ccgcaccgac ggatgagagc gtaaaaaatg ccggtcaaac gctaaacccg 1560

tatcgcagca agcagtatga agtggggcta aaatcggaca tcggcgagat gaatctaggc 1620

gccgcgctgt tccgactgga acgtccgttt gcctatcttg atacggataa cgtgtataaa 1680

gagcagggta accaggttaa caacggcctt gagttaaccg ctgccgggaa tgtgtggcag 1740

gggctgaata tttacagcgg cgtgaccttc ctcgacccga aactgaaaga tacggcgaat 1800

gcctcaacca gcaataaaca ggttgtcggc gtgccgaaag tgcaggccaa tctgttggcg 1860

gaatacagtt tgccgtccat accggaatgg gtttacagcg ctaacgtcca ttatacgggc 1920

aaacgcgcgg cgaacgatac caacacctct tacgccagca gctataccac atgggatttg 1980

ggaacgcgtt acaccacgaa agtgagcaac gtcccaacca ctttccgcgt ggtggtaaac 2040

aacgtgtttg ataaacatta ctgggcttct atcttcccat cgggtaccga tggcgataac 2100

ggttccccaa gtgcgtttat cggcggcggc cgcgaagtgc gtgcatccgt caccttcgat 2160

ttc 2163

<210> 116

<211> 2007

<212> DNA

<213>  Escherichia coli

<400>  116
atgaaaaaca taacgctgtg gcagcgttta agacaggtca gtatcagtac cagcttacgt      60
tgcgcatttc tgatgggggc acttctgacc ctgattgtca gtagtgtcag tctgtattca     120
tggcatgaac aaagctcaca aattcgttac tcgctggata agtattttcc ccgtattcac     180
tctgctttcc ttattgaagg gaacctgaat ctggtggtag accagctaaa tgaatttttg     240
caggctccca acaccacggt gcgattgcaa cttcgtaccc agattattca gcatctcgac     300
accatagaac ggcttagtag gggactgtca tcccgggaac gccaacaact gacggtcatt     360
ttgcaggaca gtcgatcact gttatccgag ttggatcgtg cgctttacaa catgttttta     420
ctacgggaaa aggtgagtga gctatcagcg cggattgact ggttacacga tgattttact     480
accgagctta attctttagt gcaggatttc acctggcagc agggaacgct gctggatcaa     540
atcgcctccc gacagggcga tacggcgcaa tacctgaagc gatctcgtga agtgcaaaat     600
gaacagcagc aggtttatac cctggcacgc attgaaaatc agattgttga cgatctgcgt     660
gacagactca atgagctcaa atcaggacgt gatgacgaca tacaggtgga aactcatctc     720
cgttattttg aaaatctgaa aaaaacggca gatgaaaata tacgtatgct ggatgactgg     780
cctggcacca ttaccctgag gcagaccatc gatgaattgc tggatatggg aatcgtaaaa     840
aacaaaatgc cggatacgat gcgtgaatat gtcgccgccc aaaaagcctt agaggatgcc     900
agtcgcacca gggaagcgac acagggtcgc ttcagaacgt tactggaagc gcagcttggc     960
agtactcatc aacaaatgca gatgtttaat caacgaatgg aacaaattgt tcacgttagc    1020
ggtgggctga tcctggtggc gacagcactg gcgttactgc ttgcatgggt attcaaccat    1080
tattttatcc gctcacggtt ggtgaaacgc tttaccctac tgaatcaggc cgttgtgcaa    1140
attggtctgg gaggcacgga aacgactatt ccagtttatg ggaatgatga actggggaga    1200
attgcaggat tattacgcca tactctcggc caactcaatg tgcaaaaaca gcaacttgaa    1260
caagaaatta ccgatcgtaa ggtgatagaa gccgatctgc gtgccaccca ggacgaactg    1320
attcagacag caaagttggc ggtagtcggg caaacgatga ccacgctggc ccacgagatc    1380
aatcagccgc taaatgcgct gtcaatgtat ctgtttacag cccgcagggc cattgaacag    1440
acccagaaag aacaggccag catgatgctt ggtaaagccg aaggggtgat tagtcgtatt    1500
gacgccatta tccgttcact acggcagttt acccggcgcg ccgaactgga aacatcactc    1560
catgccgttg atttagcaca gatgttcagt gcggcctggg aacttctggc catgcgtcat    1620
cgctctctgc aagctacgct tgttctgccg caaggtacag ccacagtttc aggtgatgag    1680
gtcagaaccc agcaggtact ggttaacgta ctggcgaatg cgcttgatgt ttgtgggcaa    1740

ggcgctgtca ttaccgttaa ctggcaaatg cagggtaaaa cgctgaacgt attcattggc 1800

gataatggcc cgggctggcc tgaggcattg ttgccttcgt tattgaagcc gtttaccacc 1860

agtaaagaag taggactggg tattggtctt tcaatttgtg tgtcgttgat ggagcaaatg 1920

aaaggggaat tgcggctggc atcaacgatg accaggaatg cctgtgtggt actgcaattc 1980

agactaacgg atgtggaaga tgctaag 2007

<210> 117

<211> 2259

<212> DNA

<213> Escherichia coli

<400> 117
atgaacgtta taaaactggc tatcggctca ggcatattat tgctcagctg cggtgcttac 60

tcacaatcca tcagtgaaaa aactaattcc gacaaaaaag gagcggcaga attcagtccg 120

ctcagcgttt ctgtcgggaa gacgaccagt gagcaggaag ctctcgagaa aacaggcgcg 180

accagttccc ggacaacgga caaaaacctg caatcacttg acgcaacagt gcgtagtatg 240

cctggtactt atactcaaat agatcctggt cagggagcaa tcagtgtgaa tattcgaggc 300

atgagcggat ttggtcgtgt aaacactatg gtcgatggta ttacccagag tttttacgga 360

acctctacct ccggaacaac gacgcatggt tcaactaaca atatggctgg cgtacttata 420

gatcctaact tactggtagc agttgatgtt acacgcggtg acagcagtgg ctctgaaggg 480

atcaacgccc ttgccggtag tgcaaatatg cgtactattg gcgttgacga tgtaatattt 540

aacggtaata catatggcct tcgttcacgt ttctctgtcg gtagtaatgg gctgggacgc 600

agcggaatga tcgcccttgg tggaaaaagc gacgctttta cggatacggg aagcattggc 660

gttatggctg ctgtgagcgg cagttctgtg tactctaatt tctcaaatgg ttctggaatt 720

aacagcaaag agtttggtta tgataaatat atgaagcaga accccaaatc ccaactgtat 780

aaaatggata tcagaccaga cgaatttaac agcttcgaac tttccgctcg aacctatgaa 840

aataaattta cacgtcgtga tataaccagt gacgactatt acattaaata tcattacacc 900

cctttttctg aattaattga ctttaacgta acggccagta ccagtcgcgg taatcaaaag 960

tatcgtgatg gctcgctgta tactttctac aaaacctcag cgcaaaatcg ttctgacgcg 1020

ctggatatca acaataccag ccggttcact gtcgcggaca atgacctgga gtttatgctg 1080

ggcagcaaac tgatgcgtac ccgctatgac cggaccattc actcagcggc gggcgacccg 1140

aaagcgaatc aggaatcgat cgagaacaat ccgttcgcac cctccggcca gcaggatatt 1200

tcagcgctgt ataccgggct gaaggttacg cgcggcatct gggaggcaga tttcaatctc 1260

aactacacac gtaacaggat cacagggtac aagcccgcct gcgattcacg cgttatctgc 1320

gtgccacagg gtagctacga tattgacgat aaagagggtg gcttcaaccc ttcagttcag 1380

ctttctgctc aggtaacacc atggcttcag ccgttcattg gctacagcaa atccatgcgc 1440

gccccgaaca tccaggagat gttcttctct aattcaggag gcgcatccat gaacccattc 1500

ctgaagcctg aacgtgcaga aacctggcag gcgggtttta acattgatac cagagattta 1560

ctggtcgaac aggatgccct gcgctttaag gctctggcgt accgcagcag gatccagaac 1620

tacatctaca gcgagtctta tctggtttgt tctggaggtc gtaaatgcag tctgcctgag 1680

gtgattggca atggctggga gggcattagc gatgaataca gcgacaatat gtacatctac 1740

gttaactcgg caagcgacgt tatcgcaaag ggcttcgaac tggagatgga ttatgatgca 1800

ggttttgctt ttggccgact ctctttcagc cagcagcaaa cagaccagcc aacctccatc 1860

gccagcaccc actttggcgc aggggatata accgaactgc ccagaaaata catgacgctg 1920

gatactggtg ttcgcttctt cgataacgcg ttgaccctgg gcactatcat aaaatacaca 1980

ggcaaggctc gtcgcctgtc gcctgatttt gagcaggacg aacataccgg cgcaataatc 2040

aaacaggatt tgccgcagat cccaacgatt atcgatctct atggtactta cgagtacaac 2100

cgcaacctga cactgaaact ttcggtacaa aacctgatga acagagatta ttcggaggcg 2160

ctgaataagc tcaacatgat gccaggtctt ggtgacgaga cccacccagc caattccgcg 2220

cgtggcagaa catggatatt tggcggggac attcgtttc 2259

<210> 118

<211> 399

<212> DNA

<213> Escherichia coli

<400> 118
atgtcttcga aaacaaaatg ctggctatgg atgttactgg tcatcctttc tgaaacctct 60

gcaacatcca cacttaaaat gttcgataac agtgagggga tgacaaaaac gctgctgctg 120

gccctaatcg tcgtactgta ttgcatttgt tactactcgc tttcacgggc agtaaaagat 180

atccccgttg gtctggctta cgccacatgg tccggtactg gcattttgat ggtttcaacc 240

cttgggattt tattttacgg tcaacacccg gataccgccg ccattattgg tatggtcatc 300

atagccagcg gtattatcat tatgaatctg ttctcaaaaa tgggcagtga agaggcggaa 360

gaaactccag ttaccaacct cgataaaaaa atcgctaac 399

<210> 119

<211> 858

<212>  DNA

<213>  Escherichia coli

<400>  119
atgtatataa aaaagcactg gatagcttta tccattctat taataccttg cattggaaac      60

gctcaggaaa ttaaaattga tgaaagctgg ttacatcaaa gcttgaatgt cattggtcgc     120

acagactctc gctttggccc aagactgact aacgacctct accctgaata tactgtagca     180

ggaagaaaag actggtttga tttttatggt tatgttgatc taccgaaatt ctttggcgtc     240

ggcagtcact atgatgttgg gatctgggat gagggctcac cactatttac ggaaatagaa     300

cctcggtttt ccattgacaa attgaccgga ttaaatcttg cgttcggccc atttaaagaa     360

tggttcattg caaacaacta tgtctatgat atgggtgaca accagtcatc ccggcaaagt     420

acatggtata tggggcttgg tacagatatc gacacgggtc taccaattaa gctttctgcc     480

aatatatacg ccaagtatca gtggcaaaac tatggtgccg ctaatgaaaa tgaatgggac     540

ggatatcgat tcaaaataaa atatagcatc cctcttacaa atttattcgg aggacgattg     600

gtatacaata gttttactaa ctttgatttt ggctccgatc ttgcggacaa gtcacacaat     660

aataaacgaa ccagtaatgc tattgcttca agccatatcc tttcccttct atatgaacac     720

tggaaatttg catttacact acgttatttt cacaacggtg gacaatggaa tgcgggagag     780

aaggttaact tcggagatgg tccatttgaa ttaaaaaata caggatgggg aacctatact     840

actattggtt atcaattt                                                   858

<210>  120

<211>  516

<212>  DNA

<213>  Escherichia coli

<400>  120
atgagaatcg caccgcgtac cttctttgct atttccgccc tggcgtttat tgtcgcctcc      60

ggatttagtt tctggcggtt gtcccctgct gaaaatacag ggattatgag ttgttcaaca     120

aaaggcatca tgcgttttga gaatatggaa aaggagaacg ttaacggtaa tattcacttt     180

aactttggca gccagggtaa aggttcgatg gtgctcgaag gctacacgga ctctgccgct     240

ggctggctgt acctgcaacg ctatgtcaaa tttacctata ccagtaaacg tgtttccgcc     300

acggaacgcc attaccgcat cagccagtgg gaatccagcg cctcatcgat agatgaatca     360

ccagatgtga tttttgacta ctttatgcgt gaaatgtctg acagccatga cgggctgttc     420

ctcaacgccc agaagctgaa cgataaagcg attttgctca gttctattaa ttcaccgctt 480

tggatctgta cccttaaatc tggcagcaaa ttagac 516

<210> 121

<211> 546

<212> DNA

<213> Escherichia coli

<400> 121
atgaaaataa aagttatagc attggctaca tttgtttctg ctgtgtttgc tggttcagct 60

atggcctatg acggaacaat tacgtttacc ggtaaagttg tagctcagac ctgcacagtt 120

aatacaagcg acaaagactt agcagtaact ttacccactg ttgccacttc atctctaaaa 180

gacaatgctg ctacgtcagg gctgacaccc tttgccattc gtttaactgg ttgtgcaact 240

ggtatgaata gtgctcagaa tgttaaagcg tactttgagc cttcaagtaa cattgactta 300

gctacacata atttaaaaaa tactgctact ccaactaaag cggataatgt acagattcag 360

ttgctaaata gtaatggaac ttcaactatt cttttggggg aagcggataa tgggcaagat 420

gtccagtctg agacaatcgg atctgatgga agtgccacat tgcgttatat ggcccagtat 480

tatgcaacag gacaatctac cgcaggggat gtaaaagcga cggtccatta taccattgcc 540

tacgaa 546

<210> 122

<211> 1077

<212> DNA

<213> Escherichia coli

<400> 122
atgaaaagaa tctttttcat accattgttt ttaattttac tccctaagct ggcggtagcg 60

ggtccggatg attatgtgcc ttcgcagata gcggttaata catccacatt gccaggtgtt 120

gtgattggtc ctgctgatgc ccatacctat ccccgggtga taggagagct ggcgggaaca 180

agtaaccagt atgtttttaa tggcggtgcc atcgctctga tgcgtggaaa gtttacaccc 240

gcactgccta aaattggaag tattacggta tactttccat caaggaaaca gcgtgattca 300

tctgattttg atatctatga tattggtgta tccggactgg gtattattat tggcatggcg 360

ggctattggc ccgcaacgcc tctggtcccc ataaatagct caggtatata tattgaccct 420

gtaggtgcca atacaaaccc caatacttat aacggtgcga cagcaagctt cggagctcgt 480

ttgtttgttg cttttgtcgc aacgggaaga ttacccaatg gatatataac aatacccacc 540

aggcagcttg gtactatttt gttggaagca aaacgtacaa gtttaaataa taaaggactg 600

acagcacctg ttatgttaaa tggtgggcgc attcaggtac agagtcagac atgtaccatg 660

gggcaaaaaa actatgtggt gccattaaat accgtatatc aatcacagtt cacatctttg 720

tataaagaaa tacagggagg taaaattgac atacacctac aatgcccgga tggaattgat 780

gtttatgcta cattgacaga tgcatcacag ccagtgaaca gaacagatat attgacctta 840

agcagtgaat ccactgcaaa aggatttggc atcaggctat ataaagacag tgatgtaact 900

gccatcagct atggtgaaga ctcccctgtg aaaggaaatg gcagtcaatg gcacttctcc 960

gattacaggg gagaggtaaa tccacatatc aatttaagag ccaattatat aaaaattgct 1020

gatgcaacta cacctggaag tgtgaaggct attgcaacta ttactttctc atatcaa 1077

<210> 123

<211> 2532

<212> DNA

<213> Escherichia coli

<400> 123
   atgaacgcta ataatctgtc atgcctgatt tactgtcgtt gttctcttct gctttttgct 60
   gcattagggt taacagtaac aaaccattca tttgctgctg aagaggctga gtttgattct 120
   gagtttttgc atttggataa agggataaat gctattgata tccgccgctt tagtcatggt 180
   aaccctgtgc ctgagggcag gtattattct gatatttatg ttaataatgt atggaagggg 240
   aaggctgatt tgcagtattt acgtactgcc aataccggtg ctccgacgtt atgcctgacg 300
   cctgagctgc tttcattgat tgatttagtc aaagatacta tgtcgggaaa cacctcctgc 360
   tttccggcgt caacagggct ttcttcagcc agaattaatt ttgacttatc gactttaagg 420
   ttgaatatcg aaatccctca ggcactgctg aatacacgtc caagaggata tatttcccct 480
   gctcagtggc aaagtggtgt tcctgcagca tttataaact atgatgctaa ctattaccag 540
   tatagctctt ccgggacgag taacgaacag acttatctgg gattaaaagc tggattcaat 600
   ttgtggggat gggctttgcg ccaccgtggc agtgagagct ggaataatag ctatcctgcc 660
   ggatatcaga atatagaaac aagtataatg catgaccttg ccccattgag agcacaattc 720
   acattagggg atttttatac gaatggtgag ctaatggata gcctcagttt gcggggagtc 780
   aggttagcat cggatgaacg aatgttaccc ggctctttac gtggctatgc tcctgctgtc 840
   cggggaatag ctaacagtaa tgctaaagta accatttatc aaaatgctca tatcctctat 900
   gaaacgacgg tgccagccgg accatttgtc atcaatgatt tatatcccag tggatatgct 960
   ggtgaccttc tcgttaagat aacagagtct aatggccaga cacgaatgtt cacggttcct 1020
   tttgcggccg ttgctcaact cattcgtccc ggatttagtc gctggcaaat gtcagtggga 1080
   aagtatcgtt atgcgaataa aacatataat gatttaatag cacaaggcac ctatcaatac 1140
   ggcctgacga atgatattac tttaaacagt ggtcttacca cagcttcagg atatacagcg 1200
   gggttagctg gcctggcctt taatacccct ctgggtgcta tagcatctga cattacattg 1260
   tccagaacag cattcaggta ttccggtgta acgcgtaaag gttatagtct gcactcaagt 1320
   tatagcatca atattccagc ctcaaacaca aatataactc tggcggctta tcgttattca 1380
   tcaaaagatt tttatcatct gaaggatgcg ctatcagcta atcacaacgc gtttattgat 1440
   gatgtttctg taaaaagtac agcgttttat cgtcccagga atcaattcca gatttcaatc 1500
   aaccaggaat taggtgaaaa atggggtggg atgtatttaa caggaacaac ctataattac 1560
   tggggacata aaggaagtcg taatgaatac cagattgggt acagcaactt ctggaaacaa 1620
   ctcggctatc aaattggatt gtctcagtca agagataatg agcaacaacg ccgtgatgac 1680
   agattttata ttaattttac tctccctctg ggaggaagtg ttcaaagccc ggtgttttcc 1740
   actgttttaa attatagcaa agaagagaaa aatagtattc agacatcaat tagtggtact 1800
   ggcggggagg ataatcagtt ctcttatggt atttcaggaa acagccagga aaacgggcct 1860
   tccggttatg caatgaatgg gggttatcgt tcaccttatg taaatataac cacaacagtc 1920
   gggcatgata ctcagaataa taatcaaagg tcatttggtg cgtcgggagc ggtggtcgca 1980
   cacccctatg gagtgacatt gagtaatgac ctgagtgata cttttgccat tatccatgct 2040
   gaaggagctc agggggctgt catcaataat gcctctggta gtcgtctgga tttttgggga 2100
   aatggtgttg ttccttatgt tacaccctat gagaaaaatc aaattagcat cgatccctcc 2160
   aatttagatt tgaatgttga attatcggcg acggagcagg aaatcattcc tcgtgctaat 2220
   agcgccacgt tagtgaaatt tgacactaaa acaggaagaa gtctgttatt tgatattcgt 2280
   atgtctactg gcaatccccc tccaatggct tctgaagttc tggatgaaca tggacagttg 2340
   gccggatatg tcgctcaggc cgggaaggta tttaccaggg gactccctga aaaaggtcat 2400
   ctcagcgttg tatggggacc agataataaa gacagatgtt catttgtata tcatgttgca 2460
   cacaataaag atgatatgca atctcagctc gttcctgttc tgtgtataca gcaccctaat 2520
   caggaaaaaa ca 2532

<210> 124

<211> 831

<212> DNA

<213> Escherichia coli

<400>  124
atggtaaaat gtcatactct gattaaccgt agaaataaat gtctgctgat tgtttttata      60

gtccttattg gatggattat attcagacct aaagcatata cttattcact aaatgataaa     120

gaaaaagaga tgctcataat gttatcacaa catcctgaaa ctcggtactt tggattttat     180

tccatagaac ttccggctga ttacaaacca acaggaatgg ttatgttcat acaaggatcg     240

gcgatgatcc ctgtagaaac aaagctacaa tattatcctc cttttctgca atatatgaca     300

cgatatgagg cagaactaaa aaacacctca gcattagatc cactggatac gccttatttg     360

aagcaagttc acccactaag tccacctatg aatggagtca tttttgaacg aatgaaagcg     420

aaatacaccc cagattttgc acgagtattg gatgcatgga aatgggaaaa tggcgttacg     480

ttttcagtaa aaatagaagc taaagatggt agagcaaccc gctatgatgg aattagtaag     540

attgccgaat acagttatgg atataatatt ccagaaaaaa aagtacagtt acttactatt     600

ctttcaggac tacaacctcg tgcagataac caacccccat cagaaaataa attggcgata     660

caatatgcac aggttgacgc ttcactactt ggagagtatg aattatctgt agattataaa     720

aatagcaata atattaaaat aagtttgcag acggataata atagttatat tgactcatta     780

ttagatataa gatatccgag taatggaaac agagcatggt ataactctat a              831

<210>  125

<211>  1098

<212>  DNA

<213>  Escherichia coli

<400>  125
atgctacctg agcctgttta tcgacgctgg attatattat taatatctat gttaacagtt      60

ggtactctgt ttattttatc ggtctggaat tctgcgacat actgggatat ttttatttat     120

ggcgttctgc caatgctgtt tctttggcta tgtttgtttg gtattgcgct gaacaaatat     180

gaacaatccg ttgcagcctg tataagttgg gagtctgaaa gacaacaagt taaacaactc     240

tggcaacact ggagccaaaa acaactggca atagttggga atgttctttt tacaccggaa     300

gaaaaaggca tgagtgtttt actggggcca caggaagaga tccctgcata tcctaaaaag     360

gcacgaccgt tattctctgc atcccgttat tctctttcgt ctatattcca tgatattcac     420

cagcaactga cacaacaatt tcctgattat cgtcattatc tacatactat ctacgtatta     480

cagcctgaga aatggcgtgg agaaaccgtg agacaggcta ttttccatca atgggactta     540

gtacctgaac ggaccaatac tcttaatcaa atccagtctc tttatgatga aagatttgac     600

ggtctaattc tggttgtttg tttacaaaac tggccggaga ataaacctga agatacgagt     660

gaactggtat cagcacagct tatctcctca tcgtcatttg tacggcagca ccagataccc 720

gttattgctg gtctggggcg tgtaatgcca ttagaacccg aggagttgga gcataatctg 780

gatgtgttat ttgaatataa ccaattggat aacaaacaac tacagcatgt ctgggtctct 840

ggtttagatg agggaacgat agaaaacctt atgcagtatg ctgaacaaca tcaatggtca 900

cttcctaaaa aacggcccct acacatgatt gatcattcct ttggccctac aggagagttt 960

atttttcctg tctctctggc aatgctgtca gaggctgcca aagaaactga acaaaatcat 1020

ttaattatct atcagtcagc acagtatgct cagaaaaaga gcctttgcct gattacccgg 1080

aagctttatt taaggaca 1098

<210> 126

<211> 780

<212> DNA

<213> Escherichia coli

<400> 126
atgttgaaca gaaaactaaa tatacggcta cgtcattccc tgaacagtca ctgcatacct 60

tccatcatta tcaataacac cgtacgttca tttcagaggt cagtcatgaa taccagagct 120

ctttttcccc tgctgttcac tgtggcatca ttctccgcct ccgccggcaa ctgggctgtc 180

aaaaacggct ggtgtcagac catgacggaa gatggtcagg cgctggtaat gctgaaaaat 240

ggcacgattg gtattaccgg cctgatgcag ggatgcccga atggtgtaca gacgctcctg 300

ggcagccgta tcagtattaa cggtaacctg atccccacat cacaaatgtg taatcagcag 360

acgggattca gggctgttga ggtggaaatc ggacaggcgc cggaaatggt caaaaaagcc 420

gttcactcca tagcagagcg tgatgtgtcc gttttacagg catttggtgt acgaatggaa 480

ttcacccgcg gtgatatgct gaaggtctgt ccgaaatttg tcacatcact tgccggtttt 540

tccccgaaac agacgaccac tattaataaa gattccgtcc tgcaggctgc ccggcaggca 600

tacgcccggg aatatgacga ggaaacaaca gaaaccgctg attttggctc ttacgaagta 660

aaaggcaata aggttgagtt tgaagtattc aatcctgaag accgtgcgta cgacaaagtg 720

accgtcacgg ttggtgctga cggtaatgcc accggcgcca gcgttgaatt tatcggaaaa 780

<210> 127

<211> 1155

<212> DNA

<213> Escherichia coli

<400> 127
gtggtaatta tcaatagcac gatactgagc ggcgcaggcg ctatcccttc cctgacgtcg 60

ctcttacccg acatcagaaa aatgctgctg gtcactgacc gtaatattgc gcagctcgac 120

ggtgtgcagc agattcgcgc cttactggaa aagcactgcc cgcaggttaa cgttatcgat 180

aatgtgcccg cagagcccac gcatcatgat gtgcgccagc taatggatgc ccctggcgat 240

gcctcttttg atgtggtggt cgggatcggc ggtggcagcg tgttggatgt ggcgaagctg 300

ctatcggtgc tttgccatcc acaatcaccg gggctggatg cgctgcttgc gggtgaaaaa 360

ccgactcagc gggtgcaatc atggttgatt cctacaaccg ccggaaccgg ctcagaagcc 420

acgccgaatg cgattctggc aatccctgag caaagcacga aggtgggtat tatttcccag 480

gtgctgttac cagactatgt ggcgcttttc ccggaactga ccaccagcat gcccgcgcat 540

attgcggcgt ccacgggcat tgatgctctt tgccacttac tggagtgttt taccgcgacc 600

gtggcaaatc cggtcagcga taacgcggcg ctgactgggt taagtaaact tttccggcac 660

attcaacccg ccgtgaacga tcctcaggat ctgcgcgcaa aactggaaat gctgtgggcg 720

tcttactatg gcggcgtagc gataacccat gcgggcacgc atctcgttca tgcgctctcc 780

tacccgttag gtggcaaata tcatctgccg catggcgtcg cgaatgccat cttgctggcg 840

ccgtgcatgg cgtttgttcg cccctgggcg gtcgagaaat ttgcccgggt ctgggattgc 900

attcccgatg cggaaaccgc cctgagcgcg gaagaaaaat ctcatgccct ggtgacctgg 960

ttacaggcat tagtcaatca actcaagcta cccaacaatc tcgcggctct cggcgtaccg 1020

ccagaggata ttgcctctct gagcgaggcg gcactgaacg tgaagcgcct tatgaacaat 1080

gtgccgtgcc aaattgatct acaggacgta caggccattt accaaacact gtttccgcaa 1140

catccattta aggag 1155

<210> 128

<211> 315

<212> DNA

<213> Escherichia coli

<400> 128
atgaatatca gaaaactgtt ttgtccggga aacacccccc ggattttatt gtttttattc 60

ttttttgttg tttctgcaat aaccacaatt gcatgcggat acactgagaa gaatgcaaca 120

ggaaatgtgc tgcttctgtt tctccttctg ctccttgcac acagaaatac cctcacatcc 180

attacagcgc tgttatttct gttctgttgt gcactgtatg cgcctgccgg tatgacgtac 240

ggtaaaatca acaacagttt tattgtcgcg ttgttgcaga ccacaactga tgaggcagcg 300

gagtttaccg ggatg 315

<210> 129

<211> 441

<212> DNA

<213> Escherichia coli

<400> 129
atgaatattc aggcaataaa agaaatggta aatttaattt gtagtttttt atttatattc 60

tttctgtcct cggcttttgt ttcttttggg tgttatgcta tttatgaatt gtttttatgg 120

aatgatatta ttgtatatag ctggggatat atattaattg tctttttacc tttcacatta 180

tatgtaatgt cgtttgagat tttgtttttt gctattagtg ggcgacgatt gtctaaagta 240

acaatggtgc gcctttggtt gataattaaa attattattg ctttctctat ttgcgcagtg 300

ttgatttttt cttcaattta caaaaaagaa ttattatcta gaaattatat tgcttgtagt 360

ggtatcccgt ctgggtggat gccgggtctg gcaacgaaat acgttaaaga aaaatcatta 420

tgcgaaaaaa atggcaataa t 441

<210> 130

<211> 534

<212> DNA

<213> Escherichia coli

<400> 130
atgtttccta ttcgttttaa acgtccggcg ttgctctgta tggcgatgct gacggttgtt 60

ctgagtggct gcgggctgat tcagaaagtg gtggatgaat cgaaaagcgt ggcctcagcc 120

gttttctaca aacaaatcaa aatactgcat ctcgatttct tctcccgcag cgccctgaat 180

acggatgcgg aagatacgcc gctttccacg atggtgcatg tctggcaact gaaaacccgc 240

gaagattttg acaaggcgga ttacgacacc ctgtttatgc aggaagagaa gacgctggag 300

aaggacgtac tggcaaaaca caccgtctgg gtaaaaccgg aaggcacggc atccctgaat 360

gtgccgctgg ataaagagac gcagtttgtc gccattattg ggcagtttta tcaccctgat 420

gaaaaaagcg acagctggcg tctggtgatc aaaagggacg aactggaggc cgacaagccg 480

cgctcgattg aactgatgag aagcgacctg cgactgctgc ctctcaagga taaa 534

<210> 131

<211> 627

<212>  DNA

<213>  Escherichia coli

<400>  131
atgttcttaa aaagaaaatg gtattacgca gtgacgacat ctgtcgtcat tactttgtgt      60

ggtggaggat attatatgta caggcaagaa tatcagatgg ttgtcactgt accaactgct     120

gacgcgaacg atcccaactg gccaaataaa aggatacagt ttgataccag cgaatggcta     180

cagcaacttc aatatattaa aatagatgat cattatatat tgaatactca atatactcca     240

attgctaatt tggatgactt tggtattaca ttaaaattac agaacgcatt aaatgggtcg     300

gataaaagac ttcctgcact atatggcctt gctgagatgg atgctcagaa atttaaagac     360

ctgatgcgcg gtaaaattaa atgtgaatat ctgaggacga catttgatgc ggaaacatta     420

aagcctgtca atgattattt ccttatttct tttacttata aagataagtg gtatgaattt     480

gagacagaaa gaaaaatatc taaaacaagt gatgatgggt attttttgtg ggcatttgat     540

aatactgtcc acgaagcagg ctattggcat aacacagatc cggctgcgta ttcctataga     600

gattaccaga atggtaaggc tgtgaaa                                         627

<210>  132

<211>  1272

<212>  DNA

<213>  Escherichia coli

<400>  132
atggatattt ggcggggaca ttcgtttctg atgacaattt ccgctaggtt cagacaatac      60

gttttctctc ttatgtcaat tttattgcag gaacgaaaaa tgaatatttt cactttatcc     120

aaagcaccgc tatacctgtt aatttcacta tttttaccca cgatggccat ggctatcgat     180

ccacctgaac gcgaactttc gcgatttgcc ctgaaaacga attaccttca gtcccctgat     240

gaaggcgtct atgaactggc gtttgataat gccagtaaaa aggtgtttgc agcagtcacc     300

gatcgtgtaa atcgtgaagc caataaaggc tatctgtatt cgtttaattc agattcgctg     360

aaagtcgaaa ataaatacac gatgccatac cgggcatttt cgctggcgat aaatcaggat     420

aaacatcagc tctatatcgg acacacccag tcagcgtccc tgcgtatcag tatgtttgac     480

accccaaccg gcaaactggt aagaaccagc gacaggttaa gttttaaagc ggcaaacgct     540

gcagattcgc gttttgagca ttttcgccat atggtttaca gccaggattc cgataccctg     600

tttgtgagtt atagcaatat gctgaaaacg gccgagggca tgaagcctct gcataagctg     660

ttaatgctcg acgggacgac gcttgcctta aaaggcgagg ttaaggatgc ttacaaaggt 720

acagcgtatg gtctgacgat ggatgaaaaa acacagaaaa tctacgttgg cggaagagat 780

tacatcaacg aaattgatgc gaaaaatcag acgctgctgc gtaccatccc gttgaaagat 840

ccgagaccac aaatcacaag tgtgcagaat ctggcggtgg actccgcttc tgaccgtgcc 900

tttgtggtgg tattcgacca tgacgatcgt tccggtacaa aagatggact ctatattttt 960

gacttacgcg acggtaaaca gcttggctat gtgcacacag gagccggagc taacgcggtg 1020

aaatacaatc cgaaatataa cgaactgtat gtcaccaact tcactagcgg caccatcagc 1080

gtagtggatg ccaccaaata cagcatcacc cgtgaattta acatgccggt ctacccaaac 1140

cagatggtgt tgtcggacga tatggatacc ctttacattg gcatcaaaga aggctttaac 1200

cgcgattggg atcctgatgt gtttgtggaa ggagctaaag aacgtattct gagcattgat 1260

ttgaaaaagt cg 1272

<210> 133

<211> 163

<212> PRT

<213> Escherichia coli

<400> 133

Met Ala Ile Pro Ala Tyr Leu Trp Leu Lys Asp Asp Gly Gly Ala Asp
1               5                   10                  15

Ile Lys Gly Ser Val Asp Val Gln Gly Arg Glu Gly Ser Ile Glu Val
            20                  25                  30

Val Ala Leu Asp His Asp Val Tyr Ile Pro Thr Asp Asn Asn Thr Gly
        35                  40                  45

Lys Leu Thr Gly Thr Arg Thr His Lys Pro Phe Thr Phe Thr Lys Glu
    50                  55                  60

Ile Asp Ala Ser Ser Pro Tyr Leu Tyr Lys Ala Val Thr Thr Gly Gln
65                  70                  75                  80

Thr Leu Lys Thr Ala Glu Phe Lys Phe Tyr Arg Ile Asn Asp Ala Gly
                85                  90                  95

Gln Glu Val Glu Tyr Phe Asn Ile Thr Leu Asp Asn Val Lys Leu Val
            100                 105                 110

Arg Val Ala Pro Leu Met His Asp Ile Lys Asp Pro Ser Arg Glu Lys

115                 120                 125

His Asn His Leu Glu Arg Ile Glu Phe Arg Tyr Glu Lys Ile Thr Trp
    130                 135                 140

Thr Tyr Lys Asp Gly Asn Ile Ile His Ser Asp Ser Trp Asn Glu Arg
145                 150                 155                 160

Pro Ser Ala

<210>  134

<211>  550

<212>  PRT

<213>  Escherichia coli

<400>  134

Val Arg Asn Thr Leu Lys Gln Ala Ile Val Leu Trp Gly Met Val Leu
1               5                   10                  15

Leu Leu Val Leu Trp Ser Val Phe Ile Ser Pro Ser Gly Val Leu Arg
            20                  25                  30

Trp Ala Gly Ala Ala Ala Ile Val Leu Ala Val Ala Ala Leu Leu Ile
        35                  40                  45

Tyr Arg Arg Arg Gln Ala Trp Thr Glu Met Thr Gly Asp Ala Gly Leu
    50                  55                  60

Ser Ser Leu Pro Pro Glu Thr Tyr Arg Gln Pro Val Val Leu Val Cys
65                  70                  75                  80

Gly Gly Leu Ser Ala His Leu Ser Thr Asp Ser Pro Val Arg Gln Val
                85                  90                  95

Ser Glu Gly Leu Tyr Leu His Val Pro Asp Glu Glu Gln Leu Val Ala
            100                 105                 110

Gln Val Glu Arg Leu Leu Thr Leu Arg Pro Ala Trp Ala Ser Gln Leu
        115                 120                 125

Ala Val Ala Tyr Thr Ile Met Pro Gly Ile His Arg Asp Val Ala Val
    130                 135                 140

Leu Ala Gly Arg Leu Arg Arg Phe Ala His Ser Met Ala Thr Val Arg

145                 150                 155                 160

Arg Arg Ala Gly Val Asn Val Pro Trp Leu Leu Trp Ser Gly Leu Ser
                165                 170                 175

Gly Ser Pro Leu Pro Glu Arg Ala Ser Ser Pro Trp Phe Ile Cys Thr
            180                 185                 190

Gly Gly Glu Val Gln Val Ala Thr Ser Thr Glu Thr Thr Met Pro Ala
        195                 200                 205

Gln Trp Ile Ala Gln Ser Gly Val Gln Glu Arg Ser Gln Arg Leu Cys
    210                 215                 220

Tyr Leu Leu Lys Ala Glu Ser Leu Met Gln Trp Leu Asn Leu Asn Val
225                 230                 235                 240

Leu Thr Ala Leu Asn Gly Pro Glu Ala Lys Cys Pro Pro Leu Ala Met
                245                 250                 255

Thr Val Gly Leu Val Pro Ser Leu Pro Ala Val Asp Asn Asn Leu Trp
            260                 265                 270

Gln Leu Trp Ile Thr Ala Arg Thr Gly Leu Thr Pro Asp Ile Ala Asp
        275                 280                 285

Thr Gly Thr Asp Asp Ala Leu Pro Phe Pro Asp Ala Leu Leu Arg Gln
    290                 295                 300

Leu Pro Arg Gln Ser Gly Phe Thr Pro Leu Arg Arg Ala Cys Val Thr
305                 310                 315                 320

Met Leu Gly Val Thr Thr Val Ala Gly Ile Ala Ala Leu Cys Leu Ser
                325                 330                 335

Ala Thr Ala Asn Arg Gln Leu Leu Arg Gln Val Gly Asp Asp Leu His
            340                 345                 350

Arg Phe Tyr Ala Val Pro Val Glu Glu Phe Ile Thr Lys Ala Arg His
        355                 360                 365

Leu Ser Val Leu Lys Asp Asp Ala Thr Met Leu Asp Gly Tyr Tyr Arg
    370                 375                 380

Glu Gly Glu Pro Leu Arg Leu Gly Leu Gly Leu Tyr Pro Gly Glu Arg
385                 390                 395                 400

Ile Arg Gln Pro Val Leu Arg Ala Ile Arg Asp Trp Arg Pro Pro Glu

405                    410                    415

Gln Lys Met Glu Val Thr Ala Ser Leu Gln Val Gln Thr Val Arg Leu
            420                    425                    430

Asp Ser Met Ser Leu Phe Asp Val Gly Gln Ala Arg Leu Lys Asp Gly
        435                    440                    445

Ser Thr Lys Val Leu Val Asp Ala Leu Val Asn Ile Arg Ala Lys Pro
    450                    455                    460

Gly Trp Leu Ile Leu Val Ala Gly Tyr Thr Asp Ala Thr Gly Asp Glu
465                    470                    475                    480

Lys Ser Asn Gln Gln Leu Ser Leu Arg Arg Ala Glu Ala Val Arg Asn
                485                    490                    495

Trp Met Leu Gln Thr Ser Asp Ile Pro Ala Thr Cys Phe Ala Val Gln
            500                    505                    510

Gly Leu Gly Glu Ser Gln Pro Ala Ala Thr Asn Asp Thr Pro Gln Gly
        515                    520                    525

Arg Ala Val Asn Arg Arg Val Glu Ile Ser Leu Val Pro Arg Ser Asp
    530                    535                    540

Ala Cys Gln Asp Val Lys
545                    550

<210>  135

<211>  194

<212>  PRT

<213>  Escherichia coli

<400>  135

Met Ile Lys Ser Thr Phe Trp Arg Ala Leu Ala Leu Thr Ala Thr Leu
1               5                   10                  15

Ile Leu Thr Gly Cys Ser His Ser Gln Pro Glu Gln Glu Gly Arg Pro
            20                  25                  30

Gln Ala Trp Leu Gln Pro Gly Thr Leu Ile Thr Leu Pro Ala Pro Gly
        35                  40                  45

Ile Ser Pro Ala Val Asn Ser Gln Gln Leu Leu Thr Gly Ser Phe Asn

50                  55                  60

Gly Lys Thr Gln Ser Leu Leu Val Met Leu Asn Ala Glu Asp Gln Lys
65                  70                  75                  80

Ile Thr Leu Ala Gly Leu Ser Ser Val Gly Ile Arg Leu Phe Leu Val
                85                  90                  95

Thr Tyr Asp Ala Lys Gly Leu Arg Ala Glu Gln Ser Ile Val Val Pro
            100                 105                 110

Gln Leu Pro Pro Ala Ser Gln Val Leu Ala Asp Val Met Leu Ser His
        115                 120                 125

Trp Pro Ile Ser Ala Trp Gln Pro Gln Leu Pro Thr Gly Trp Thr Leu
    130                 135                 140

Arg Asp Asn Gly Asp Lys Arg Glu Leu Arg Asn Ala Ser Gly Lys Leu
145                 150                 155                 160

Val Thr Glu Ile Thr Tyr Leu Asn Arg Gln Gly Lys Arg Val Pro Ile
                165                 170                 175

Ser Ile Glu Gln His Val Phe Lys Tyr His Ile Thr Ile Gln Tyr Leu
            180                 185                 190

Gly Asp

<210>  136

<211>  129

<212>  PRT

<213>  Escherichia coli

<400>  136

Met Lys Arg Tyr Ile Lys Trp Phe Ala Ile Thr Ile Phe Ile Ser Met
1               5                   10                  15

Leu Ser Ala Cys Val Arg Thr Ala Pro Val Gln Gln Ile Ser Thr Thr
            20                  25                  30

Val Ser Val Gly His Thr Gln Glu Gln Val Lys Asn Ala Ile Leu Lys
        35                  40                  45

Ala Gly Ala Gln Arg Lys Trp Ile Met Thr Gln Val Ser Pro Gly Val

    50                  55                  60

Ile Lys Ala Arg Tyr Gln Thr Arg Asn His Val Ala Glu Val Arg Ile
65                  70                  75                  80

Thr Tyr Thr Ala Thr Tyr Tyr Asn Ile Lys Tyr Asp Ser Ser Leu Asn
                85                  90                  95

Leu Gln Ala Ser Asp Gly Lys Ile His Lys Asn Tyr Asn Arg Trp Val
            100                 105                 110

Arg Asn Leu Asp Lys Asp Ile Gln Val Asn Leu Ser Thr Gly Ala Thr
        115                 120                 125

Leu

<210> 137

<211> 415

<212> PRT

<213> Escherichia coli

<400> 137

Met Lys Arg Lys His Leu Leu Leu Leu Leu Leu Phe Ser Phe Ser Thr
1               5                   10                  15

Asn Ser Ala Pro Leu Tyr Ser Leu Ile Arg Glu Ala Val Met His Asp
            20                  25                  30

Pro Ile Val Met Glu Ala Arg Ala Glu Leu Thr Ser Ala Gln Ser Arg
        35                  40                  45

Ile Glu Gln Ala Ser Ser Ala His Trp Pro Val Val Thr Ala Thr Gly
    50                  55                  60

Ser Lys Leu Leu Ser Gln Ser His Arg Tyr Ser Tyr Asp Tyr Asp Thr
65                  70                  75                  80

Glu Asp Ile Leu Pro Gly Ile Arg Gly Glu Val Asn Ile Phe Ala Ser
                85                  90                  95

Gly Ala Ile Glu Ala Asp Val Arg Arg Ser Glu Ser Glu Ala Glu Tyr
            100                 105                 110

Tyr His Tyr Lys Met Glu Glu Thr Lys Glu Glu Thr Ile His Ser Phe

115                 120                 125

Val Ser Leu Tyr Leu Asp Ala Leu Arg Glu Lys Gln Ser Ile Ala Val
    130                 135                 140

Leu Glu Gln Ser Leu Ser Arg His Asn Ala Ile Leu Asn Asp Leu Asn
145                 150                 155                 160

Thr Ile Ser Ile His Asp Thr Gly Arg Glu Ser Glu Leu Val Gln Ala
                165                 170                 175

Glu Ala Arg Arg Leu Met Val Arg Gln Gln Ile Asn Ser Arg Ser Arg
            180                 185                 190

Val Leu Lys Thr Thr Leu Gly Lys Leu Ser Thr Trp Thr Lys Asn Pro
        195                 200                 205

Val Thr Glu Ala Asp Leu Glu Asn Pro Phe Ser Arg Met Thr Glu Ala
    210                 215                 220

Lys Leu Leu Thr Asp Phe Thr Gln Ala Pro Gln Lys Gly Asn Pro Ser
225                 230                 235                 240

Trp Leu Ala Ser Gln Ala Asp Val Glu Ser Lys Lys Ala Ala Leu Lys
                245                 250                 255

Ala Gln Glu Leu Ala Arg Tyr Pro Arg Val Asp Leu Thr Gly Ser Val
            260                 265                 270

Thr Arg Asp Asp Gln Gln Ile Gly Val Asn Leu Ser Trp Asp Leu Phe
        275                 280                 285

Asn Arg Asn Ala Ser Tyr Gly Val Thr Glu Lys Ala Ala Gln Ile Val
    290                 295                 300

Ala Ala Thr Gly Arg Leu Asp Ser Val Ala Arg Met Ile Asp Glu Thr
305                 310                 315                 320

Gly Arg Leu Ser Leu Ile Thr Val Arg Gln Ser Arg Gly Glu Met Glu
                325                 330                 335

Thr Leu Arg Arg Gln Glu Gln Ala Ser Ala Arg Val Val Asp Phe Tyr
            340                 345                 350

Arg Leu Gln Phe Gln Val Ala Arg Lys Thr Leu Ile Glu Leu Leu Asn
        355                 360                 365

Ala Glu Asn Glu Leu Tyr Ser Val Gly Leu Ser Arg Val Gln Thr Glu

370                 375                 380

Asp Gln Met Leu His Gly Met Leu Asp Tyr Leu Tyr Ser Gln Gly Met
385                 390                 395                 400

Leu Leu Lys Trp Ser Gly Val Asn Leu Ser Gly Glu Glu Glu Lys
                405                 410                 415

<210>  138

<211>  201

<212>  PRT

<213>  Escherichia coli

<400>  138

Met Lys Phe Leu Pro Leu Leu Ala Leu Leu Ile Ser Pro Phe Val Ser
1               5                   10                  15

Ala Leu Thr Leu Asp Asp Leu Gln Gln Arg Phe Thr Glu Gln Pro Val
            20                  25                  30

Ile Arg Ala His Phe Asp Gln Thr Arg Thr Ile Lys Asp Leu Pro Gln
        35                  40                  45

Pro Leu Arg Ser Gln Gly Gln Met Leu Ile Ala Arg Asp Gln Gly Leu
    50                  55                  60

Leu Trp Asp Gln Thr Ser Pro Phe Pro Met Gln Leu Leu Leu Asp Asp
65                  70                  75                  80

Lys Arg Met Val Gln Val Ile Asn Gly Gln Pro Pro Gln Ile Ile Thr
                85                  90                  95

Ala Glu Asn Asn Pro Gln Met Phe Gln Phe Asn His Leu Leu Arg Ala
            100                 105                 110

Leu Phe Gln Ala Asp Arg Lys Val Leu Glu Gln Asn Phe Arg Val Glu
        115                 120                 125

Phe Ala Asp Lys Gly Glu Gly Arg Trp Thr Leu Arg Leu Thr Pro Thr
    130                 135                 140

Thr Thr Pro Leu Asp Lys Ile Phe Asn Thr Ile Asp Leu Ala Gly Lys
145                 150                 155                 160

Thr Tyr Leu Glu Ser Ile Gln Leu Asn Asp Lys Gln Gly Asp Arg Thr

                165                 170                 175

Asp Ile Ala Leu Thr Gln His Gln Leu Thr Pro Ala Gln Leu Thr Asp
            180                 185                 190

Asp Glu His Gln Arg Phe Ala Ala Gln
        195                 200

<210> 139

<211> 770

<212> PRT

<213> Escherichia coli

<400> 139

Met Glu Asn Phe Phe Met Lys Asn Ser Lys Val Phe Tyr Arg Ser Ala
1               5                   10                  15

Leu Ala Thr Ala Ile Val Met Ala Leu Ser Ala Pro Ala Phe Ala Thr
            20                  25                  30

Asp Ser Thr Val Ser Thr Asp Pro Val Thr Leu Asn Thr Glu Lys Thr
        35                  40                  45

Thr Leu Asp Gln Asp Val Val Ile Asn Gly Asp Asn Lys Ile Thr Ala
    50                  55                  60

Val Thr Ile Glu Thr Ser Asp Ser Asp Lys Asp Leu Asn Val Thr Phe
65                  70                  75                  80

Gly Gly His Asp Ile Thr Ala Ala Ser Thr Val Asn Gln Asp Phe Val
                85                  90                  95

Glu Gly Val Lys Val Ser Gly Asn Lys Asn Val Val Ile Asn Ala Thr
            100                 105                 110

Asp Ser Thr Ile Thr Ala Gln Gly Glu Gly Thr Tyr Val Arg Thr Ala
        115                 120                 125

Met Val Ile Asp Ser Thr Gly Asp Val Val Val Asn Gly Gly Asn Phe
    130                 135                 140

Val Ala Lys Asn Glu Lys Gly Ser Ala Thr Gly Ile Ser Leu Glu Ala
145                 150                 155                 160

Thr Thr Gly Asn Asn Leu Thr Leu Asn Gly Thr Thr Ile Asn Ala Gln

                165                 170                 175

Gly Asn Lys Ser Tyr Ser Asn Gly Ser Thr Ala Ile Phe Ala Gln Lys
            180                 185                 190

Gly Asn Leu Leu Gln Gly Phe Asp Gly Asp Ala Thr Asp Asn Ile Thr
        195                 200                 205

Leu Ala Asp Ser Asn Ile Ile Asn Gly Gly Ile Glu Thr Ile Val Thr
    210                 215                 220

Ala Gly Asn Lys Thr Gly Ile His Thr Val Asn Leu Asn Ile Lys Asp
225                 230                 235                 240

Gly Ser Val Ile Gly Ala Ala Asn Asn Lys Gln Thr Ile Tyr Ala Ser
                245                 250                 255

Ala Ser Ala Gln Gly Ala Gly Ser Ala Thr Gln Asn Leu Asn Leu Ser
            260                 265                 270

Val Ala Asp Ser Thr Ile Tyr Ser Asp Val Leu Ala Leu Ser Glu Ser
        275                 280                 285

Glu Asn Ser Ala Ser Thr Thr Thr Asn Val Asn Met Asn Val Ala Arg
    290                 295                 300

Ser Tyr Trp Glu Gly Asn Ala Tyr Thr Phe Asn Ser Gly Asp Lys Ala
305                 310                 315                 320

Gly Ser Asp Leu Asp Ile Asn Leu Ser Asp Ser Ser Val Trp Lys Gly
                325                 330                 335

Lys Val Ser Gly Ala Gly Asp Ala Ser Val Ser Leu Gln Asn Gly Ser
            340                 345                 350

Val Trp Asn Val Thr Gly Ser Ser Thr Val Asp Ala Leu Ala Val Lys
        355                 360                 365

Asp Ser Thr Val Asn Ile Thr Lys Ala Thr Val Asn Thr Gly Thr Phe
    370                 375                 380

Ala Ser Gln Asn Gly Thr Leu Ile Val Asp Ala Ser Ser Glu Asn Thr
385                 390                 395                 400

Leu Asp Ile Ser Gly Lys Ala Ser Gly Asp Leu Arg Val Tyr Ser Ala
                405                 410                 415

Gly Ser Leu Asp Leu Ile Asn Glu Gln Thr Ala Phe Ile Ser Thr Gly

            420                 425                 430

Lys Asp Ser Thr Leu Lys Ala Thr Gly Thr Thr Glu Gly Gly Leu Tyr
        435                 440                 445

Gln Tyr Asp Leu Thr Gln Gly Ala Asp Gly Asn Phe Tyr Phe Val Lys
    450                 455                 460

Asn Thr His Lys Ala Ser Asn Ala Ser Ser Val Ile Gln Ala Met Ala
465                 470                 475                 480

Ala Ala Pro Ala Asn Val Ala Asn Leu Gln Ala Asp Thr Leu Ser Ala
                485                 490                 495

Arg Gln Asp Ala Val Arg Leu Ser Glu Asn Asp Lys Gly Gly Val Trp
            500                 505                 510

Ile Gln Tyr Phe Gly Gly Lys Gln Lys His Thr Thr Ala Gly Asn Ala
        515                 520                 525

Ser Tyr Asp Leu Asp Val Asn Gly Val Met Leu Gly Gly Asp Thr Arg
    530                 535                 540

Phe Met Thr Glu Asp Gly Ser Trp Leu Ala Gly Val Ala Met Ser Ser
545                 550                 555                 560

Ala Lys Gly Asp Met Thr Thr Met Gln Ser Lys Gly Asp Thr Glu Gly
                565                 570                 575

Tyr Ser Phe His Ala Tyr Leu Ser Arg Gln Tyr Asn Asn Gly Ile Phe
            580                 585                 590

Ile Asp Thr Ala Ala Gln Phe Gly His Tyr Ser Asn Thr Ala Asp Val
        595                 600                 605

Arg Leu Met Asn Gly Gly Gly Thr Ile Lys Ala Asp Phe Asn Thr Asn
    610                 615                 620

Gly Phe Gly Ala Met Val Lys Gly Gly Tyr Thr Trp Lys Asp Gly Asn
625                 630                 635                 640

Gly Leu Phe Ile Gln Pro Tyr Ala Lys Leu Ser Ala Leu Thr Leu Glu
                645                 650                 655

Gly Val Asp Tyr Gln Leu Asn Gly Val Asp Val His Ser Asp Ser Tyr
            660                 665                 670

Asn Ser Val Leu Gly Glu Ala Gly Thr Arg Val Gly Tyr Asp Phe Ala

675                 680                 685

Val Gly Asn Ala Thr Val Lys Pro Tyr Leu Asn Leu Ala Ala Leu Asn
    690                 695                 700

Glu Phe Ser Asp Gly Asn Lys Val Arg Leu Gly Asp Glu Ser Val Asn
705                 710                 715                 720

Ala Ser Ile Asp Gly Ala Ala Phe Arg Val Gly Ala Gly Val Gln Ala
                725                 730                 735

Asp Ile Thr Lys Asn Met Gly Ala Tyr Ala Ser Leu Asp Tyr Thr Lys
            740                 745                 750

Gly Asp Asp Ile Glu Asn Pro Leu Gln Gly Val Val Gly Ile Asn Val
        755                 760                 765

Thr Trp
    770

<210>  140

<211>  660

<212>  PRT

<213>  Escherichia coli

<400>  140

Met Ser Arg Pro Gln Phe Thr Ser Leu Arg Leu Ser Leu Leu Ala Leu
1               5                   10                  15

Ala Val Ser Ala Thr Leu Pro Thr Phe Ala Phe Ala Thr Glu Thr Met
            20                  25                  30

Thr Val Thr Ala Thr Gly Asn Ala Arg Ser Ser Phe Glu Ala Pro Met
        35                  40                  45

Met Val Ser Val Ile Asp Thr Ser Ala Pro Glu Asn Gln Thr Ala Thr
    50                  55                  60

Ser Ala Thr Asp Leu Leu Arg His Val Pro Gly Ile Thr Leu Asp Gly
65                  70                  75                  80

Thr Gly Arg Thr Asn Gly Gln Asp Val Asn Met Arg Gly Tyr Asp His
                85                  90                  95

Arg Gly Val Leu Val Leu Val Asp Gly Val Arg Gln Gly Thr Asp Thr

            100                 105                 110

.

Gly His Leu Asn Gly Thr Phe Leu Asp Pro Ala Leu Ile Lys Arg Val
115 120 125

Glu Ile Val Arg Gly Pro Ser Ala Leu Leu Tyr Gly Ser Gly Ala Leu
130 135 140

Gly Gly Val Ile Ser Tyr Asp Thr Val Asp Ala Lys Asp Leu Leu Gln
145 150 155 160

Glu Gly Gln Ser Ser Gly Phe Arg Val Phe Gly Thr Gly Gly Thr Gly
165 170 175

Asp His Ser Leu Gly Leu Gly Ala Ser Ala Phe Gly Arg Thr Glu Asn
180 185 190

Leu Asp Gly Ile Val Ala Trp Ser Ser Arg Asp Arg Gly Asp Leu Arg
195 200 205

Gln Ser Asn Gly Glu Thr Ala Pro Asn Asp Glu Ser Ile Asn Asn Met
210 215 220

Leu Ala Lys Gly Thr Trp Gln Ile Asp Ser Ala Gln Ser Leu Ser Gly
225 230 235 240

Leu Val Arg Tyr Tyr Asn Asn Asp Ala Arg Glu Pro Lys Asn Pro Gln
245 250 255

Thr Val Glu Ala Ser Asp Ser Ser Asn Pro Met Val Asp Arg Ser Thr
260 265 270

Ile Gln Arg Asp Ala Gln Leu Ser Tyr Lys Leu Ala Pro Gln Gly Asn
275 280 285

Asp Trp Leu Asn Ala Asp Ala Lys Ile Tyr Trp Ser Glu Val Arg Ile
290 295 300

Asn Ala Gln Asn Thr Gly Ser Ser Gly Glu Tyr Arg Glu Gln Ile Thr
305 310 315 320

Lys Gly Ala Arg Leu Glu Asn Arg Ser Thr Leu Phe Ala Asp Ser Phe
325 330 335

Ala Ser His Leu Leu Thr Tyr Gly Gly Glu Tyr Tyr Arg Gln Glu Gln
340 345 350

His Pro Gly Gly Ala Thr Thr Gly Phe Pro Gln Ala Lys Ile Asp Phe

355 360 365

Ser Ser Gly Trp Leu Gln Asp Glu Ile Thr Leu Arg Asp Leu Pro Ile
370 375 380

Thr Leu Leu Gly Gly Thr Arg Tyr Asp Ser Tyr Arg Gly Ser Ser Asp
385 390 395 400

Gly Tyr Lys Asp Val Asp Ala Asp Lys Trp Ser Ser Arg Ala Gly Met
405 410 415

Thr Ile Asn Pro Thr Asn Trp Leu Met Leu Phe Gly Ser Tyr Ala Gln
420 425 430

Ala Phe Arg Ala Pro Thr Met Gly Glu Met Tyr Asn Asp Ser Lys His
435 440 445

Phe Ser Ile Gly Arg Phe Tyr Thr Asn Tyr Trp Val Pro Asn Pro Asn
450 455 460

Leu Arg Pro Glu Thr Asn Glu Thr Gln Glu Tyr Gly Phe Gly Leu Arg
465 470 475 480

Phe Asp Asp Leu Met Leu Ser Asn Asp Ala Leu Glu Phe Lys Ala Ser
485 490 495

Tyr Phe Asp Thr Lys Ala Lys Asp Tyr Ile Ser Thr Thr Val Asp Phe
500 505 510

Ala Ala Ala Thr Thr Met Ser Tyr Asn Val Pro Asn Ala Lys Ile Trp
515 520 525

Gly Trp Asp Val Met Thr Lys Tyr Thr Thr Asp Leu Phe Ser Leu Asp
530 535 540

Val Ala Tyr Asn Arg Thr Arg Gly Lys Asp Thr Asp Thr Gly Glu Tyr
545 550 555 560

Ile Ser Ser Ile Asn Pro Asp Thr Val Thr Ser Thr Leu Asn Ile Pro
565 570 575

Ile Ala His Ser Gly Phe Ser Val Gly Trp Val Gly Thr Phe Ala Asp
580 585 590

Arg Ser Thr His Ile Ser Ser Ser Tyr Ser Lys Gln Pro Gly Tyr Gly
595 600 605

Val Asn Asp Phe Tyr Val Ser Tyr Gln Gly Gln Gln Ala Leu Lys Gly

610                 615                 620

Met Thr Thr Thr Leu Val Leu Gly Asn Ala Phe Asp Lys Glu Tyr Trp
625                 630                 635                 640

Ser Pro Gln Gly Ile Pro Gln Asp Gly Arg Asn Gly Lys Ile Phe Val
                645                 650                 655

Ser Tyr Gln Trp
            660

<210>  141

<211>  719

<212>  PRT

<213>  Escherichia coli

<400>  141

Met Arg Asp Glu Met Leu Tyr Asn Ile Pro Cys Arg Ile Tyr Ile Leu
1               5                   10                  15

Ser Thr Leu Ser Leu Cys Ile Ser Gly Ile Val Ser Thr Ala Thr Ala
            20                  25                  30

Thr Ser Ser Glu Thr Lys Ile Ser Asn Glu Glu Thr Leu Val Val Thr
        35                  40                  45

Thr Asn Arg Ser Ala Ser Asn Leu Trp Glu Ser Pro Ala Thr Ile Gln
    50                  55                  60

Val Ile Asp Gln Gln Thr Leu Gln Asn Ser Thr Asn Ala Ser Ile Ala
65                  70                  75                  80

Asp Asn Leu Gln Asp Ile Pro Gly Val Glu Ile Thr Asp Asn Ser Leu
                85                  90                  95

Ala Gly Arg Lys Gln Ile Arg Ile Arg Gly Glu Ala Ser Ser Arg Val
            100                 105                 110

Leu Ile Leu Ile Asp Gly Gln Glu Val Thr Tyr Gln Arg Ala Gly Asp
        115                 120                 125

Asn Tyr Gly Val Gly Leu Leu Ile Asp Glu Ser Ala Leu Glu Arg Val
    130                 135                 140

Glu Val Val Lys Gly Pro Tyr Ser Val Leu Tyr Gly Ser Gln Ala Ile

145                 150                 155                 160

Gly Gly Ile Val Asn Phe Ile Thr Lys Lys Gly Gly Asp Lys Leu Ala
                165                 170                 175

Ser Gly Val Val Lys Ala Val Tyr Asn Ser Ala Thr Ala Gly Trp Glu
            180                 185                 190

Glu Ser Ile Ala Val Gln Gly Ser Ile Gly Gly Phe Asp Tyr Arg Ile
        195                 200                 205

Asn Gly Ser Tyr Ser Asp Gln Gly Asn Arg Asp Thr Pro Asp Gly Arg
    210                 215                 220

Leu Pro Asn Thr Asn Tyr Arg Asn Asn Ser Gln Gly Val Trp Leu Gly
225                 230                 235                 240

Tyr Asn Ser Gly Asn His Arg Phe Gly Leu Ser Leu Asp Arg Tyr Arg
                245                 250                 255

Leu Ala Thr Gln Thr Tyr Tyr Glu Asp Pro Asp Gly Ser Tyr Glu Ala
            260                 265                 270

Phe Ser Val Lys Ile Pro Lys Leu Glu Arg Glu Lys Val Gly Val Phe
        275                 280                 285

Tyr Asp Thr Asp Val Asp Gly Asp Tyr Leu Lys Lys Ile His Phe Asp
    290                 295                 300

Ala Tyr Glu Gln Thr Ile Gln Arg Gln Phe Ala Asn Glu Val Lys Thr
305                 310                 315                 320

Thr Gln Pro Val Pro Ser Pro Met Ile Gln Ala Leu Thr Val His Asn
                325                 330                 335

Lys Thr Asp Thr His Asp Lys Gln Tyr Thr Gln Ala Val Thr Leu Gln
            340                 345                 350

Ser His Phe Ser Leu Pro Ala Asn Asn Glu Leu Val Thr Gly Ala Gln
        355                 360                 365

Tyr Lys Gln Asp Arg Val Ser Gln Arg Ser Gly Gly Met Thr Ser Ser
    370                 375                 380

Lys Ser Leu Thr Gly Phe Ile Asn Lys Glu Thr Arg Thr Arg Ser Tyr
385                 390                 395                 400

Tyr Glu Ser Glu Gln Ser Thr Val Ser Leu Phe Ala Gln Asn Asp Trp

                405                 410                 415

Arg Phe Ala Asp His Trp Thr Trp Thr Met Gly Val Arg Gln Tyr Trp
            420                 425                 430

Leu Ser Ser Lys Leu Thr Arg Gly Asp Gly Val Ser Tyr Thr Ala Gly
        435                 440                 445

Ile Ile Ser Asp Thr Ser Leu Ala Arg Glu Ser Ala Ser Asp His Glu
    450                 455                 460

Met Val Thr Ser Thr Ser Leu Arg Tyr Ser Gly Phe Asp Asn Leu Glu
465                 470                 475                 480

Leu Arg Ala Ala Phe Ala Gln Gly Tyr Val Phe Pro Thr Leu Ser Gln
                485                 490                 495

Leu Phe Met Gln Thr Ser Ala Gly Gly Ser Val Thr Tyr Gly Asn Pro
            500                 505                 510

Asp Leu Lys Ala Glu His Ser Asn Asn Phe Glu Leu Gly Ala Arg Tyr
        515                 520                 525

Asn Gly Asn Thr Trp Leu Ile Asp Ser Ala Val Tyr Tyr Ser Glu Ala
    530                 535                 540

Lys Asp Tyr Ile Ala Ser Leu Ile Cys Asp Gly Ser Ile Val Cys Asn
545                 550                 555                 560

Gly Asn Thr Asn Ser Ser Arg Ser Ser Tyr Tyr Tyr Tyr Asp Asn Ile
                565                 570                 575

Asp Arg Ala Lys Thr Trp Gly Leu Glu Ile Ser Ala Glu Tyr Asn Gly
            580                 585                 590

Trp Val Phe Ser Pro Tyr Ile Ser Gly Asn Leu Ile Arg Arg Gln Tyr
        595                 600                 605

Glu Thr Ser Thr Leu Lys Thr Thr Asn Thr Gly Glu Pro Ala Ile Asn
    610                 615                 620

Gly Arg Ile Gly Leu Lys His Thr Leu Val Met Gly Gln Ala Asn Ile
625                 630                 635                 640

Ile Ser Asp Val Phe Ile Arg Ala Ala Ser Ser Ala Lys Asp Asp Ser
                645                 650                 655

Asn Gly Thr Glu Thr Asn Val Pro Gly Trp Ala Thr Leu Asn Phe Ala

            660                 665                 670

Val Asn Thr Glu Phe Gly Asn Glu Asp Gln Ser Arg Ile Asn Leu Ala
        675                 680                 685

Leu Asn Asn Leu Thr Asp Lys Arg Tyr Arg Thr Ala His Glu Thr Ile
    690                 695                 700

Pro Ala Ala Gly Phe Asn Ala Ala Ile Gly Phe Val Trp Asn Phe
705                 710                 715

<210> 142

<211> 199

<212> PRT

<213> Escherichia coli

<400> 142

Met Arg Lys Val Cys Ala Val Ile Leu Ser Ala Ala Ile Cys Leu Ser
1               5                   10                  15

Val Ser Gly Ala Pro Ala Trp Ala Ser Glu His Gln Ser Thr Leu Ser
            20                  25                  30

Ala Gly Tyr Leu His Ala Arg Thr Asn Ala Pro Gly Ser Asp Asn Leu
        35                  40                  45

Asn Gly Ile Asn Val Lys Tyr Arg Tyr Glu Phe Thr Asp Ala Leu Gly
    50                  55                  60

Leu Ile Thr Ser Phe Ser Tyr Ala Asn Ala Glu Asp Glu Gln Lys Thr
65                  70                  75                  80

His Tyr Ser Asp Thr Arg Trp His Glu Asp Ser Val Arg Asn Arg Trp
                85                  90                  95

Phe Ser Val Met Ala Gly Pro Ser Val Arg Val Asn Glu Trp Phe Ser
            100                 105                 110

Ala Tyr Ser Met Ala Gly Val Ala Tyr Ser Arg Val Ser Thr Phe Ser
        115                 120                 125

Gly Asp Tyr Leu Arg Val Thr Asp Asn Lys Gly Lys Thr His Asp Val
    130                 135                 140

Leu Thr Gly Ser Asp Asp Gly Arg His Ser Asn Thr Ser Leu Ala Trp

145                 150                 155                 160

Gly Ala Gly Val Gln Phe Asn Pro Thr Glu Ser Val Thr Ile Asp Leu
                165                 170                 175

Ala Tyr Glu Gly Ser Gly Ser Gly Asp Trp Arg Thr Asp Ala Phe Ile
            180                 185                 190

Val Gly Ile Gly Tyr Arg Phe
        195

<210>  143

<211>  456

<212>  PRT

<213>  Escherichia coli

<400>  143

Met Lys Lys Ser Thr Leu Ser Leu Ala Ile Gly Leu Leu Leu Ala Cys
1               5                   10                  15

Ser Thr Gly Met Ala Lys Thr Gln His Leu Thr Leu Glu Gln Arg Leu
            20                  25                  30

Glu Ala Ala Glu Met Arg Ala Ala Lys Ala Glu Gly Gln Val Lys Gln
        35                  40                  45

Leu Gln Thr Gln Gln Ala Ala Glu Ile Arg Glu Ile Lys Thr Ala Gln
    50                  55                  60

Gly Asn Thr Pro Val Asn Gly Gln Ser Thr Thr Glu Ser Glu Lys Lys
65                  70                  75                  80

Asn Ala Thr Pro Pro Asn Leu Leu Leu Ser Gly Tyr Gly Asp Leu Lys
                85                  90                  95

Ile Tyr Gly Asp Val Glu Phe Asn Met Asp Ala Glu Ser Asn His Gly
            100                 105                 110

Leu Leu Ala Met Thr Asn Ala Asp Val Asn Ser Asp Pro Thr Asn Glu
        115                 120                 125

Trp Asn Leu Asn Gly Arg Ile Leu Leu Gly Phe Asp Gly Met Arg Lys
    130                 135                 140

Leu Asp Asn Gly Tyr Phe Ala Gly Phe Ser Ala Gln Pro Leu Gly Asp

145                 150                 155                 160

Met His Gly Ser Val Asn Ile Asp Asp Ala Val Phe Phe Phe Gly Lys
                165                 170                 175

Glu Asn Asp Trp Lys Val Lys Val Gly Arg Phe Glu Ala Tyr Asp Met
            180                 185                 190

Phe Pro Leu Asn Gln Asp Thr Phe Val Glu His Ser Gly Asn Thr Ala
        195                 200                 205

Asn Asp Leu Tyr Asp Asp Gly Ser Gly Tyr Ile Tyr Met Met Lys Glu
    210                 215                 220

Gly Arg Gly Arg Ser Asn Ala Gly Gly Asn Phe Leu Val Ser Lys Gln
225                 230                 235                 240

Leu Asp Asn Trp Tyr Phe Glu Leu Asn Thr Leu Leu Glu Asp Gly Thr
                245                 250                 255

Ser Leu Tyr Asn Asp Gly Asn Tyr His Gly Arg Asp Met Glu Gln Gln
            260                 265                 270

Lys Asn Val Ala Tyr Leu Arg Pro Val Ile Ala Trp Ser Pro Thr Glu
        275                 280                 285

Glu Phe Thr Val Ser Ala Ala Met Glu Ala Asn Val Val Asn Asn Ala
    290                 295                 300

Tyr Gly Tyr Thr Asp Ser Lys Gly Asn Phe Val Asp Gln Ser Asp Arg
305                 310                 315                 320

Thr Gly Tyr Gly Met Ser Met Thr Trp Asn Gly Leu Lys Thr Asp Pro
                325                 330                 335

Glu Asn Gly Ile Val Val Asn Leu Asn Thr Ala Tyr Leu Asp Ala Asn
            340                 345                 350

Asn Glu Lys Asp Phe Thr Ala Gly Ile Asn Ala Leu Trp Lys Arg Phe
        355                 360                 365

Glu Leu Gly Tyr Ile Tyr Ala His Asn Lys Ile Asp Glu Phe Ser Gly
    370                 375                 380

Val Val Cys Asp Asn Asp Cys Trp Ile Asp Asp Glu Gly Thr Tyr Asn
385                 390                 395                 400

Ile His Thr Ile His Ala Ser Tyr Gln Phe Ala Asn Val Met Asp Met

                405                 410                 415

Glu Asn Phe Asn Ile Tyr Leu Gly Thr Tyr Tyr Ser Ile Leu Asp Ser
            420                 425                 430

Asp Gly Asp Lys Ile His Gly Asp Asp Ser Asp Asp Arg Tyr Gly Ala
        435                 440                 445

Arg Val Arg Phe Lys Tyr Phe Phe
    450                 455

<210> 144

<211> 174

<212> PRT

<213> Escherichia coli

<400> 144

Met Asn Gly Lys Ala Phe Leu Ala Cys Val Leu Met Ser Val Val Leu
1               5                   10                  15

Thr Gly Cys Glu Thr Ala Lys Lys Ile Ser Gln Val Ile Arg Asn Pro
            20                  25                  30

Asp Ile Gln Val Gly Lys Leu Met Asp Gln Ser Thr Glu Leu Thr Val
        35                  40                  45

Thr Leu Leu Thr Glu Pro Asp Ser Asn Leu Thr Ala Asp Gly Glu Ala
    50                  55                  60

Ala Pro Val Asp Val Gln Leu Val Tyr Leu Ser Asp Asp Ser Lys Phe
65                  70                  75                  80

His Ala Ala Asp Tyr Asp Gln Val Ala Thr Thr Ala Leu Pro Asp Val
                85                  90                  95

Leu Gly Lys Asn Tyr Ile Asp His Gln Asp Phe Asn Leu Leu Pro Asp
            100                 105                 110

Thr Val Lys Thr Leu Pro Pro Ile Lys Leu Asp Glu Lys Thr Gly Tyr
        115                 120                 125

Ile Gly Val Ile Ala Tyr Phe Ser Asp Asp Gln Ala Thr Glu Trp Lys
    130                 135                 140

Gln Ile Glu Ser Val Glu Ser Ile Gly His His Tyr Arg Leu Leu Val

145                 150                 155                 160

His Ile Arg Ala Ser Ala Ile Glu Met Lys Lys Glu Glu Asn
                165                 170

<210>  145

<211>  1144

<212>  PRT

<213>  Escherichia coli

<400>  145

Leu Thr Leu Ala Trp Ile Phe Leu Leu Val Trp Ile Trp Trp Gln Gly
1               5                   10                  15

Pro Lys Trp Thr Leu Tyr Glu Gln His Trp Leu Ala Pro Leu Ala Asn
            20                  25                  30

Arg Trp Leu Ala Thr Ala Val Trp Gly Leu Ile Ala Leu Val Trp Leu
        35                  40                  45

Thr Trp Arg Val Met Lys Arg Leu Gln Lys Leu Glu Lys Gln Gln Lys
    50                  55                  60

Gln Gln Arg Glu Glu Glu Lys Asp Pro Leu Thr Val Glu Leu His Arg
65                  70                  75                  80

Gln Gln Gln Tyr Leu Asp His Trp Leu Leu Arg Leu Arg Arg His Leu
                85                  90                  95

Asp Asn Arg Arg Tyr Leu Trp Gln Leu Pro Trp Tyr Met Val Ile Gly
            100                 105                 110

Pro Ala Gly Ser Gly Lys Ser Thr Leu Leu Arg Glu Gly Phe Pro Ser
        115                 120                 125

Asp Ile Val Tyr Thr Pro Glu Ser Ile Arg Gly Val Glu Tyr His Pro
    130                 135                 140

Leu Ile Thr Pro Arg Val Gly Asn Gln Ala Val Ile Phe Asp Val Asp
145                 150                 155                 160

Gly Val Leu Thr Thr Pro Gly Gly Asp Asp Leu Leu Arg Arg Arg Leu
                165                 170                 175

Arg Glu His Trp Leu Gly Trp Leu Met Gln Thr Arg Ala Arg Gln Pro

            180                 185                 190

Leu Asn Gly Leu Ile Leu Thr Leu Asp Leu Pro Asp Leu Leu Thr Ala
        195                 200                 205

Asp Lys Ser Arg Arg Glu Thr Leu Val Gln Asn Leu Arg Gln Gln Leu
    210                 215                 220

Gln Glu Ile Arg Gln Ser Leu His Cys Arg Leu Pro Val Tyr Val Val
225                 230                 235                 240

Leu Thr Arg Leu Asp Leu Leu Asn Gly Phe Ala Ala Leu Phe His Ser
                245                 250                 255

Leu Asp Lys Lys Asp Arg Asp Ala Ile Leu Gly Val Thr Phe Thr Arg
            260                 265                 270

Arg Ala His Glu Ser Asp Gly Trp Arg Ser Glu Leu Gly Ala Phe Trp
        275                 280                 285

Gln Thr Trp Val Gln Gln Val Asn Leu Ala Leu Ser Asp Leu Val Leu
    290                 295                 300

Ala Gln Thr Gly Ala Ala Pro Arg Ser Ala Val Phe Ser Phe Ser Arg
305                 310                 315                 320

Gln Met Gln Gly Thr Gly Glu Ile Val Thr Ala Leu Leu Ala Ala Leu
                325                 330                 335

Leu Asp Gly Glu Asn Met Asp Val Met Leu Arg Gly Val Trp Leu Thr
            340                 345                 350

Ser Ser Leu Gln Arg Gly Gln Val Asp Asp Ile Phe Thr Gln Ser Ala
        355                 360                 365

Ala Arg Gln Tyr Gly Leu Gly Asn Ser Ser Leu Ala Thr Trp Pro Leu
    370                 375                 380

Val Glu Thr Thr Pro Tyr Phe Thr Arg Arg Leu Phe Pro Glu Val Leu
385                 390                 395                 400

Leu Ala Glu Pro Asn Leu Ala Gly Glu Asn Ser Val Trp Leu Asn Ser
                405                 410                 415

Ser Arg Arg Arg Leu Thr Ala Phe Ser Thr Cys Gly Ala Ala Leu Ala
            420                 425                 430

Ala Leu Met Val Gly Ser Trp His His Tyr Tyr Asn Gln Asn Trp Gln

435                 440                 445

Ser Gly Val Asn Val Leu Ala Gln Ala Lys Ala Phe Met Asp Val Pro
    450                 455                 460

Pro Pro Gln Gly Thr Asp Glu Phe Gly Asn Leu Gln Leu Pro Leu Leu
465                 470                 475                 480

Asn Pro Val Arg Asp Ala Thr Leu Ala Tyr Gly Asp Tyr Arg Asp His
                485                 490                 495

Gly Phe Leu Ala Asp Met Gly Leu Tyr Gln Gly Ala Arg Val Gly Pro
            500                 505                 510

Tyr Val Glu Gln Thr Tyr Ile Gln Leu Leu Glu Gln Arg Tyr Leu Pro
        515                 520                 525

Ser Leu Met Asn Gly Leu Ile Arg Asp Leu Asn Ile Ala Pro Pro Glu
    530                 535                 540

Ser Glu Glu Lys Leu Ala Val Leu Arg Val Val Arg Met Met Glu Asp
545                 550                 555                 560

Lys Ser Gly Arg Asn Asn Glu Ala Val Lys Gln Tyr Met Ala Arg Arg
                565                 570                 575

Trp Ser Asn Glu Phe His Gly Gln Arg Asp Ile Gln Ala Gln Leu Met
            580                 585                 590

Val His Leu Asp Tyr Ala Leu Glu His Thr Asp Trp His Ala Gln Arg
        595                 600                 605

Gln Ser Ser Asp Ser Asp Ala Val Ser Arg Trp Thr Pro Tyr Asp Lys
    610                 615                 620

Pro Ile Ile Asn Ala Gln Gln Glu Leu Ser Lys Leu Pro Ile Tyr Gln
625                 630                 635                 640

Arg Val Tyr Gln Thr Leu Arg Thr Lys Ala Leu Ser Val Leu Pro Ala
                645                 650                 655

Asp Leu Asn Leu Arg Asp Gln Val Gly Pro Thr Phe Asp Asn Val Phe
            660                 665                 670

Val Ala Gly Asn Asp Glu Lys Leu Val Ile Pro Gln Phe Leu Thr Arg
        675                 680                 685

Tyr Gly Leu Gln Ser Tyr Phe Val Lys Gln Arg Glu Gly Leu Val Glu

690                 695                 700

Leu Thr Ala Leu Asp Ser Trp Val Leu Asn Leu Thr Gln Ser Val Ala
705                 710                 715                 720

Tyr Ser Glu Ala Asp Arg Glu Glu Ile Gln Arg His Ile Thr Glu Gln
                725                 730                 735

Tyr Ile Ser Asp Tyr Thr Ala Thr Trp Arg Ala Gly Met Asp Asn Leu
            740                 745                 750

Asn Val Arg Asp Tyr Glu Ala Met Ser Ala Leu Thr Asp Ala Leu Glu
        755                 760                 765

Gln Ile Ile Ser Gly Asp Gln Pro Phe Gln Arg Ala Leu Thr Ala Leu
    770                 775                 780

Arg Asp Asn Thr His Ala Leu Thr Leu Ser Gly Lys Leu Asp Asp Lys
785                 790                 795                 800

Ala Arg Glu Ala Ala Ile Asn Glu Met Asp Tyr Arg Leu Leu Ser Arg
                805                 810                 815

Leu Gly His Glu Phe Ala Pro Glu Asn Ser Ala Leu Glu Glu Gln Lys
            820                 825                 830

Asp Lys Ala Ser Thr Leu Gln Ala Val Tyr Gln Gln Leu Thr Glu Leu
        835                 840                 845

His Arg Tyr Leu Leu Ala Ile Gln Asn Ser Pro Val Pro Gly Lys Ser
    850                 855                 860

Ala Leu Lys Ala Val Gln Leu Arg Leu Asp Gln Asn Ser Ser Asp Pro
865                 870                 875                 880

Ile Phe Ala Thr Arg Gln Met Ala Lys Thr Leu Pro Ala Pro Leu Asn
                885                 890                 895

Arg Trp Val Gly Lys Leu Ala Asp Gln Ala Trp His Val Val Met Val
            900                 905                 910

Glu Ala Val Arg Tyr Met Glu Val Asp Trp Arg Asp Asn Val Val Lys
        915                 920                 925

Pro Phe Asn Glu Gln Leu Ala Asp Asn Tyr Pro Phe Asn Pro Arg Ala
    930                 935                 940

Thr Gln Asp Ala Ser Leu Asp Ser Phe Glu Arg Phe Phe Lys Pro Asp

945                 950                 955                 960

Gly Ile Leu Asp Asn Phe Tyr Lys Asn Asn Leu Arg Leu Phe Leu Glu
                965                 970                 975

Asn Asp Leu Thr Phe Gly Asp Asp Gly Arg Val Leu Ile Arg Glu Asp
            980                 985                 990

Ile Arg Gln Gln Leu Asp Thr Ala  Gln Lys Ile Arg Asp  Ile Phe Phe
        995                 1000                1005

Ser Gln  Gln Asn Gly Leu Gly  Ala Gln Phe Ala Val  Glu Thr Val
    1010                 1015                 1020

Ser Leu  Ser Gly Asn Lys Arg  Arg Ser Val Leu Asn  Leu Asp Gly
    1025                 1030                 1035

Gln Leu  Val Asp Tyr Ser Gln  Gly Arg Asn Tyr Thr  Ala His Leu
    1040                 1045                 1050

Val Trp  Pro Asn Asn Met Arg  Glu Gly Asn Glu Ser  Lys Leu Thr
    1055                 1060                 1065

Leu Ile  Gly Thr Ser Gly Arg  Ala Pro Arg Ser Ile  Ala Phe Ser
    1070                 1075                 1080

Gly Pro  Trp Ala Gln Phe Arg  Leu Phe Gly Ala Gly  Gln Leu Thr
    1085                 1090                 1095

Asn Val  Thr Ser Asp Thr Phe  Asn Val Arg Phe Asn  Val Asp Gly
    1100                 1105                 1110

Gly Ala  Met Val Tyr Gln Val  His Val Asp Thr Glu  Asp Asn Pro
    1115                 1120                 1125

Phe Thr  Gly Gly Leu Phe Ser  Leu Phe Arg Leu Pro  Asp Thr Leu
    1130                 1135                 1140

Tyr

<210>  146

<211>  489

<212>  DNA

<213>  Escherichia coli

<400> 146
   atggctattc ctgcttatct ctggctgaaa gatgacggcg gcgcggatat caaaggttcc 60
   gtggacgttc aggggcgcga aggtagcatc gaagtggtgg cgctggatca cgatgtgtac 120
   atcccgaccg acaataacac cggcaaactg accggtaccc gtactcacaa gccttttacg 180
   tttaccaaag aaatcgatgc gtccagcccg tatctctaca aagctgtgac caccggacag 240
   accctgaaaa cggcagaatt taagttttac cgcatcaacg atgccggtca ggaagtggag 300
   tacttcaaca tcacgcttga taacgtcaag ctggtcagag tcgctccgct tatgcacgac 360
   atcaaggatc cttccagaga gaagcataac cacctggaac gtattgagtt ccgctacgag 420
   aaaatcacct ggacttacaa agacggcaac atcattcatt ccgactcgtg gaatgagcgt 480
   ccttccgcc 489

<210> 147

<211> 1650

<212> DNA

<213> Escherichia coli

<400> 147
gtgaggaaca cgctgaaaca ggccatcgtg ctgtggggaa tggtgttact gctggtgctg 60

tggtcagtgt ttatcagtcc gtctggcgtg ctgagatggg ccggtgcggc ggctatcgtt 120

ctggcggttg ccgcgttgtt gatttatcgg cgcaggcagg cgtggacgga gatgaccggc 180

gatgccgggt tgtcatcgct gccgccggaa acctaccgac agccggtagt gctggtctgt 240

ggcggtctgt cggcgcacct gtccactgac agcccggtcc gccaggtttc agaagggctg 300

tatctgcatg ttcctgatga agaacagctt gtggcgcagg tggagcgatt gctgaccctt 360

cgcccggcgt gggcatcgca gcttgccgtg gcgtatacca tcatgcccgg catacaccgg 420

gatgtggcgg ttctggccgg acggctgcga cggttcgccc acagtatggc gacggtgcgt 480

cgtcgggcag gcgtaaacgt cccctggctt ctctggagcg ggctgtccgg ctcgccgttg 540

ccggaaagag cgagttcacc gtggtttatc tgtaccggcg gcgaagttca ggtagcaaca 600

tccacagaga ccaccatgcc cgcgcagtgg attgcacaat ccggcgtaca ggagcgcagt 660

cagcgactct gttacctgct gaaagctgaa agcctgatgc agtggctgaa tcttaatgtg 720

ctgacggcac tgaacggccc ggaggcgaaa tgtccaccac tggcgatgac cgtggggctg 780

gtcccctcgt tgcctgcggt ggataacaac ctgtggcagt tgtggatcac cgccagaacc 840

ggcctgacgc cggatatcgc ggacaccggc acagacgatg cgctgccatt cccggatgcc 900

ctgttacggc agttgccgcg tcagtcgggc tttaccccgc tgcgacgagc ctgcgtgacc 960

atgctgggcg tcaccaccgt ggcgggtatc gccgcgctgt gcctgtcagc cacggcaaat 1020

cgccagttat tacggcaggt cggtgacgat ctgcaccggt tttatgccgt cccggtggag 1080

gaatttatca ccaaagcccg tcacctgtcg gtgctgaaag acgatgcgac catgctcgat 1140

gggtattacc gggaaggaga acccctgcgc ctcggtctgg ggttataccc cggcgaacgc 1200

atccgccagc cggtattacg cgccattcgc gactggcgtc cgcctgaaca aaaaatggag 1260

gtgacggctt cgcttcaggt tcagaccgtg cgtcttgaca gtatgtcgct gtttgacgtc 1320

ggacaggccc gcctgaaaga cggctcgaca aaagtgctgg tggacgcact ggtgaacatc 1380

cgggcaaaac cgggctggct gatcctcgtg gccggatata ccgatgccac cggcgatgaa 1440

aaaagcaatc agcagttatc gctgcggcgt gccgaagcgg tgcgcaactg gatgctgcag 1500

accagcgaca tcccggccac ctgttttgcc gtacagggac tgggcgagag ccagcctgcg 1560

gcgaccaacg acacgccaca gggccgggca gtcaaccggc gtgtcgaaat cagtcttgtt 1620

ccgcgttctg acgcctgtca ggacgtgaaa 1650

<210> 148

<211> 582

<212> DNA

<213> Escherichia coli

<400> 148
atgatcaaat ccacattctg gcgagcgctc gccctgaccg ctacgcttat cctcactggc 60

tgtagccact cgcaaccgga acaggaaggc cgcccgcagg cgtggctgca acctggtacg 120

ctcatcacgc tgcctgcgcc ggggatttca cccgcagtca attcccagca actgttgacc 180

ggcagcttca acggcaaaac ccagtctctg ctagtgatgc ttaatgccga agatcagaaa 240

atcacccttg ccgggctgtc gtcggtcggc attcgcctgt ttctggtgac ctacgatgca 300

aaagggctac gcgccgagca atccatcgtc gtcccacagt taccgcccgc aagtcaggta 360

ctggctgacg tgatgctcag ccactggccg attagcgcct ggcaaccgca acttcccaca 420

ggctggacgc ttcgcgacaa cggcgacaaa cgcgagctgc gtaacgccag cggcaaactg 480

gtcacggaaa tcacctatct gaatcgccag ggaaaacgcg tgccaatcag cattgagcag 540

catgtcttta aataccacat caccattcaa tacttaggtg ac 582

<210> 149

<211> 387

<212> DNA

<213> Escherichia coli

<400> 149
atgaaacgtt atataaaatg gtttgccatc acaattttta tcagtatgtt gagtgcctgt 60

gtccgtacgg ccccagtgca acagataagc accactgtca gtgtgggtca tactcaggag 120

caggttaaaa atgccatttt gaaagcaggt gcgcagcgca agtggattat gacgcaagtg 180

tcccctggag ttattaaagc tcgctatcaa acacgaaatc acgttgcaga ggttcgtatt 240

acatatacag ctacctacta taacatcaaa tatgacagta gcctgaatct gcaggcttct 300

gatggaaaaa ttcataaaaa ctataaccgc tgggtgcgta acctggataa agatatacag 360

gttaacttat ctacaggagc aacgtta 387

<210> 150

<211> 1245

<212> DNA

<213> Escherichia coli

<400> 150
atgaagcgta aacatttgtt attattattg ttgttttcat tttccactaa cagtgcgcct 60

ctttactcct taattaggga ggcagttatg cacgatccca tagtaatgga agcccgggcg 120

gagttaactt cggcacaatc ccgcatagag caggcaagct ctgcacattg gccagttgtc 180

acagctacag gaagtaaact cctttcacaa agtcaccgtt attcctacga ttatgacact 240

gaagatattt tacccggtat tcgtggtgaa gtgaatatat ttgcttcagg ggctattgag 300

gcggatgtgc gtcggagtga gtcagaagcc gaatattatc attataaaat ggaagaaaca 360

aaagaggaaa caattcactc ttttgtttca ttatatcttg atgcactcag ggaaaaacaa 420

tccattgcgg tacttgaaca gagcctttcc cggcataacg caattcttaa tgacctgaat 480

accatcagta ttcatgatac cgggcgggag tctgagcttg ttcaggccga agccagaagg 540

ttgatggttc ggcagcagat aaattctagg agcagagtac ttaaaaccac gctgggaaaa 600

ctgtccactt ggacaaaaaa tccggtaacc gaagctgatc ttgaaaatcc tttttctagg 660

atgacagagg ccaaattatt aactgatttt acacaggctc cacagaaagg taacccgtcg 720

tggcttgcca gccaagctga tgttgagagt aaaaaagcgg cactgaaagc acaggagctt 780

gcccggtacc ctcgggtgga tttaacgggg tctgtaaccc gggatgacca gcagataggg 840

gtcaatctgt cttgggacct ctttaaccgt aatgccagtt atggtgttac agaaaaagct 900

gcgcaaatag tggcagctac cggacgactg gactctgtcg cccgaatgat tgatgaaacc 960

gggcgattat ctctgataac agtcagacaa agtcgagggg aaatggaaac gctcagacgt 1020

caggaacagg cttcagccag agttgtggac ttttatcgtc ttcagtttca ggtggcaaga 1080

aaaacactga ttgaattact gaatgctgaa aacgaactgt acagtgtcgg actctcccgg 1140

gttcagacgg aggatcagat gctccacggt atgctggatt atctgtattc ccagggaatg 1200

ctcctgaaat ggagcggagt gaatctttct ggtgaagaag aaaaa 1245

<210> 151

<211> 603

<212> DNA

<213> Escherichia coli

<400> 151
atgaaatttt taccgctgct ggcgctgctg attagcccgt ttgtgagcgc cctgaccctg 60

gacgatcttc agcaacgctt taccgaacaa ccggtgatcc gcgcccattt tgatcaaacc 120

cggacgatta aagatctgcc gcagccgctg cgatctcagg gtcagatgtt gatcgcccgc 180

gaccaggggt tattgtggga tcaaacctca ccgttcccca tgcagctatt gctggatgat 240

aaacgcatgg tgcaggtgat caacggtcag ccgccgcaaa tcatcacggc agaaaacaac 300

ccgcagatgt tccagtttaa ccacctgctg cgcgcgctgt tccaggccga tcgcaaagtg 360

ctggaacaaa acttccgcgt cgaatttgct gacaaaggcg aaggccgctg gacgctgcgc 420

ctgacgccga ccaccacgcc gctggataaa attttcaaca ccatcgatct cgccgggaaa 480

acctatctgg agagcattca acttaatgat aaacagggcg atcgcaccga tattgctctt 540

acccaacatc aactgacgcc agcgcaactg accgatgacg aacaccaacg ttttgccgcc 600

cag 603

<210> 152

<211> 2295

<212> DNA

<213> Escherichia coli

<400> 152
atgaaaaaca gtaaggtatt ttaccgcagc gcattagcga cagctattgt tatggctctt 60

tctgcaccag cattcgctac tgatagcacg gtatcaactg atccggttac gctgaataca 120

gagaagacga ctctggatca agatgttgtt attaacggtg ataacaagat tacagccgta 180

acaattgaaa cgtcagattc agataaagac cttaatgtta cttttggcgg tcacgatatt 240

accgccgcat caacggtaaa ccaagatttc gttgaaggtg taaaagttag tggtaacaaa 300

aatgttgtga ttaatgctac agactccacc atcacagctc aaggtgaagg cacctatgtc 360

cggactgcaa tggtcattga ttcaactggc gatgttgttg ttaatggcgg taatttcgtt 420

gcaaaaaatg aaaaaggtag tgcgacaggg atatctctgg aagcgaccac gggaaataat 480

ttaacgctca atggtacaac cataaatgct caaggtaata agagttacag caacggctct 540

acggcaattt ttgctcaaaa gggtaatttg ttgcagggtt ttgacggtga tgcaaccgac 600

aacatcaccc ttgctgactc aaatattatt aatggcggga ttgaaacaat agttactgcc 660

gggaataaga cgggaattca tacagtcaac ctgaatatta aggatggctc agtaattggg 720

gcggctaata ataaacaaac aatttatgcc tctgcttcgg cacaaggcgc aggttcagca 780

acgcaaaatt taaatttgtc tgttgctgat tcaaccatct actctgatgt cctggccctt 840

tctgaaagcg agaattcagc cagtaccaca acaaatgtaa atatgaacgt tgcccgctct 900

tactgggaag gtaatgctta taccttcaat agcggcgata aagcgggtag tgatctggat 960

ataaatcttt ccgatagttc agtctggaaa ggcaaagttt caggggcagg agatgccagt 1020

gtatctctgc aaaacgggtc tgtctggaat gttacgggtt cctcaactgt tgatgctctg 1080

gcagtaaaag acagtacggt taatatcacg aaggctacag tcaatactgg cacgtttgct 1140

tctcaaaacg gcactctgat tgttgatgcc tcttctgaaa acactctgga tatcagcggt 1200

aaagcgagcg gtgacttgcg tgtttacagt gcgggttcat tggatcttat caatgaacaa 1260

acggcattta tttctaccgg caaagacagc actctaaaag ccacaggcac aacggaaggt 1320

ggtctgtatc aatatgacct gacacaggga gctgatggta actttatatt cgtaaaaaac 1380

acgcataaag catccaacgc cagctccgtg attcaggcaa tggcagctgc tccggctaac 1440

gtcgctaatc tgcaggctga cacgctctcc gcccgtcagg atgctgtccg tctgagcgaa 1500

aatgacaagg gtggcgtatg gattcagtac tttggcggta aacagaaaca taccaccgcg 1560

ggaaatgcat cctatgacct ggatgtaaat ggtgtaatgc tgggtggtga tacccgcttc 1620

atgactgaag atggtagctg gctggccggt gtggcgatgt cttctgcgaa aggtgacatg 1680

actaccatgc agagcaaagg tgacactgaa ggttacagct tccacgctta cctgagccgc 1740

cagtataaca acggtatctt cattgatact gctgcacagt ttggtcacta cagcaacacg 1800

gcagatgttc gcctgatgaa tggtggcggt accatcaaag ctgactttaa caccaatggt 1860

tttggtgcga tggttaaagg cggttacaca tggaaagacg gtaatggcct gtttattcag 1920

ccatatgcca aactgtctgc tctgactctg gaaggtgtgg attatcaact caacggcgtg 1980

gacgttcatt ctgacagcta taactctgtg ctgggtgagg ccggtacgcg cgtgggttat 2040

gacttcgctg tgggcaacgc gaccgttaaa ccttatctga atctggccgc actgaacgaa 2100

ttctctgatg gcaacaaagt ccgtctgggt gatgagtctg tcaatgccag cattgacggt 2160

gcagcattcc gcgtgggtgc aggtgtacaa gctgatatca ccaaaaacat gggagcatat 2220

gcaagccttg actacaccaa aggtgacgac attgagaacc cgctacaggg tgtagttggt 2280

atcaatgtga cctgg 2295

<210> 153

<211> 1980

<212> DNA

<213> Escherichia coli

<400> 153
atgtcacgtc cgcaatttac ctcgttgcgt ttgagtttgt tggctttggc tgtttctgcc 60

accttgccaa cgtttgcttt tgctactgaa accatgaccg ttacggcaac ggggaatgca 120

cgtagttcct tcgaagcgcc tatgatggtc agcgttatcg acacttccgc tcctgaaaat 180

caaactgcta cttcagccac tgatttgctg cgtcatgttc ctggaattac tcttgatggt 240

accggacgaa ccaacggtca ggatgtaaat atgcgtggct atgatcatcg cggcgtgctg 300

gttcttgtcg atggtgttcg ccagggaacg gataccggac acctgaatgg cacttttctc 360

gatccggcgc tgatcaagcg tgttgagatt gttcgcggac cttcagcatt actgtatggc 420

agtggcgcgc tgggtggagt gatctcctac gatacggtcg atgcaaaaga tttattgcag 480

gaaggacaaa gcagtggttt tcgtgtcttt ggtactggcg gcacggggga ccatagcctg 540

ggattaggcg cgagcgcgtt tgggcgaact gaaaatctgg atggtattgt ggcctggtcc 600

agtcgcgatc ggggtgattt acgccagagc aatggtgaaa ccgcgccgaa tgacgagtcc 660

attaataaca tgctggcgaa agggacctgg caaattgatt cagcccagtc tctgagcggt 720

ttagtgcgtt actacaacaa cgacgcgcgt gaaccaaaaa atccgcagac cgttgaagct 780

tctgatagca gcaacccgat ggtcgatcgt tcaacaattc aacgcgatgc gcagctttct 840

tataaactcg ccccgcaggg taacgactgg ttaaatgcag atgcaaaaat ttactggtcg 900

gaagtccgta ttaatgcgca aaacacgggg agttcaggcg agtatcgtga acagataaca 960

aaaggagcaa ggctggagaa ccgttccact ctatttgccg acagtttcgc ttctcactta 1020

ctgacatatg gcggtgagta ttatcgtcag gaacaacatc cgggtggcgc gacgacgggc 1080

ttcccgcaag caaaaatcga ttttagctct ggttggctac aagatgagat caccttacgc 1140

gatctgccga ttaccctgct tggcggaacc cgctatgaca gttatcgcgg tagcagcgac 1200

ggctacaaag atgttgatgc cgacaaatgg tcatctcgtg cggggatgac tatcaacccg 1260

accaactggc tgatgttatt tggctcatat gctcaggcat tccgcgcccc gacgatgggc 1320

gaaatgtata acgattctaa acacttctcg attggtcgct tctataccaa ctattgggtg 1380

ccaaacccga acttacgtcc ggaaactaac gaaactcagg agtacggttt tgggctgcgt 1440

tttgatgacc tgatgttgtc caatgatgct ctggaattta aagccagcta ctttgatacc 1500

aaagcgaaag attatatctc cacgaccgtc gatttcgcgg cggcgacaac tatgtcgtat 1560

aacgtcccga acgccaaaat ctggggctgg gatgtgatga cgaaatatac cactgatctg 1620

tttagccttg atgtggccta taaccgtacc cgcggcaaag acaccgatac cggggaatat 1680

atctccagca ttaacccgga taccgttacc agtaccctga atattccgat cgctcacagc 1740

ggcttctctg ttggttgggt cggtacgttt gccgatcgct caacacatat cagcagcagc 1800

tacagcaaac aacctggcta tggtgtgaat gatttctacg tcagttatca agggcagcag 1860

gcgctcaaag gcatgaccac tactctggta ttgggcaacg ccttcgataa agagtactgg 1920

tcgccgcaag gcatcccaca ggatggtcgt aacggaaaaa ttttcgtgag ttatcaatgg 1980

<210> 154

<211> 2157

<212> DNA

<213> Escherichia coli

<400> 154
   atgagggatg aaatgttata taatatacct tgtcgaattt atatcctttc cactctgtca 60
   ttatgcattt ctgggatagt ttctactgca accgcaactt cttcagaaac aaaaatcagc 120
   aacgaagaga cgctcgtcgt gaccacgaat cgttcggcaa gcaacctttg ggaaagcccg 180
   gcgactatac aggttattga ccaacaaaca ttgcagaact ccaccaatgc ctccatagcc 240
   gataatttgc aggacatccc cggagtagag ataacagaca actccttggc aggccgtaaa 300
   caaatccgca ttcgtggcga agcatcctcc cgtgttttaa ttctcattga tggtcaggag 360
   gtaacttatc agcgcgccgg agataattat ggtgtgggac tgttgataga tgagtctgcg 420
   ctggagcgtg ttgaggtagt gaaaggtcca tattccgtac tgtacggttc acaggcaatt 480
   ggcggtattg ttaacttcat aaccaaaaag ggaggtgaca aacttgcatc tggagttgtg 540
   aaagctgttt ataattccgc aacagcaggc tgggaagaat caatcgcggt ccaggggagc 600
   atcggtggat ttgattatcg catcaacggt agttattctg atcagggcaa tcgtgatacg 660
   ccggatggac gtctgccgaa taccaactat cgtaacaata gtcagggtgt atggttgggt 720
   tataactccg gaaaccatcg ttttggcctc tcgcttgatc gctacagact cgcgacgcaa 780
   acttactatg aggatccaga cggaagctat gaggcattta gtgtcaaaat acctaaactt 840
   gaacgagaga aagttggggt attctatgac acagacgtgg acggtgacta tctaaaaaaa 900
   attcatttcg acgcgtatga gcagaccatc cagcgccaat ttgccaacga agtaaaaacg 960
   acacagcctg ttcccagtcc gatgattcag gctctgaccg ttcataacaa gactgacacc 1020
   catgataagc aatacactca ggcggtcaca ttgcagagtc acttttcgct gcctgctaat 1080
   aatgaacttg ttaccggtgc acagtacaaa caagacaggg tcagccaaag gtccggtggc 1140
   atgacctcaa gcaaatctct gaccggcttc attaataagg aaacacgaac tcgctcctat 1200
   tatgagtcag agcaaagtac agtctcacta ttcgcacaaa atgactggcg attcgccgat 1260
   cactggacat ggacaatggg agttcgccaa tactggcttt cttcaaagtt gacgcgtggt 1320
   gacggagtat catataccgc aggcattata agcgatacct ctcttgccag agagtctgcg 1380
   agtgatcacg aaatggtaac atctacaagc ctgcgctatt caggtttcga taacttggag 1440
   ttacgcgctg cgttcgcgca aggctacgta tttcccacac tctcccagct ttttatgcag 1500
   acatctgcgg gcggcagtgt cacatacgga aatcctgatc ttaaggctga acactccaat 1560
   aactttgaat taggtgcacg atataatggt aatacgtggc tgattgacag cgcagtttac 1620
   tactcagaag ctaaagatta tattgcaagt ctgatctgtg atggcagtat agtttgcaat 1680
   ggtaacacca actcctcccg tagtagctac tattattatg acaatattga tcgggcaaaa 1740
   acatggggac tggaaataag cgcggaatat aatggctggg ttttctcgcc atatatcagt 1800
   ggcaatttaa ttcgtcggca atatgaaact tcaacattaa aaacaactaa tacaggagaa 1860
   ccagcgataa acggacgtat agggctgaaa catactcttg tgatgggtca ggccaacata 1920
   atctctgatg tttttattcg tgctgcctct agtgcaaaag atgacagtaa cggtaccgaa 1980
   acaaatgttc cgggctgggc cactctcaac tttgcagtaa atacagaatt cggtaacgag 2040
   gatcagtccc ggattaacct agcactcaat aacctgacag acaaacgcta ccgtacagca 2100
   catgaaacta ttcctgcagc aggttttaat gcagctatag gttttgtatg gaatttc 2157

<210> 155

<211> 600

<212> DNA

<213> Escherichia coli

<400> 155
atgcgtaaag tttgtgcagt cattttgtcc gcagccatct gtctgtccgt atccggtgcg 60

cctgcatggg cgtctgaaca tcagtccaca ctgagcgcgg ggtatcttca tgcccgtacg 120

aacgctcccg gcagcgataa tctgaacggg attaacgtga aataccgtta tgagtttacg 180

gacgcgctgg ggctgattac gtccttcagt tatgccaatg ctgaggatga gcaaaaaacg 240

cactacagcg atacccgctg gcatgaagat tccgtgcgta accgctggtt cagcgtgatg 300

gcggggccgt ctgtacgcgt gaatgaatgg ttcagcgcgt attcgatggc gggtgtggct 360

tacagccgtg tgtcgacttt ctccggggat tatctccgcg taactgacaa caaggggaaa 420

acgcacgatg tgctgaccgg aagtgatgac ggtcgccaca gcaacacgtc tctggcgtgg 480

ggggctggcg tgcagtttaa cccgaccgaa tccgtgacca ttgaccttgc ttatgaaggt 540

tccggtagtg gcgactggcg aacggatgca tttattgttg gtatcggata ccgtttctga 600

<210> 156

<211> 1368

<212> DNA

<213> Escherichia coli

<400> 156
atgaaaaaat cgacattatc tttagccatc ggtttattat tggcatgtag taccggtatg 60

gcaaaaacac agcatttaac gctggaacaa cgcctggaag cggcagaaat gcgggcagca 120

aaagcagagg ggcaggttaa acagcttcag acacaacaag ccgccgagat ccgcgaaatt 180

aaaaccgcac agggcaacac gccggtaaac ggtcaatcaa cgacggagtc agagaagaaa 240

aacgccaccc cgcctaatct cctgctttca gggtatggcg atttaaaaat ctacggtgac 300

gtagaattta atatggatgc ggaaagtaat catggcctgc tggcaatgac caacgctgat 360

gtgaatagcg atcccactaa tgaatggaat ctcaatggtc gtattctgtt aggttttgat 420

ggtatgcgaa aactggataa tggctatttc gctgggttct ccgcacaacc gctgggggat 480

atgcacggtt cagtaaatat cgatgatgcg gttttcttct ttggcaaaga aaacgactgg 540

aaggtcaaag taggccgttt tgaagcctac gatatgttcc cgctgaatca ggataccttt 600

gttgaacatt ccggtaatac tgcgaacgat ctttatgacg atggcagcgg ttatatctat 660

atgatgaaag agggccgcgg acgttctaac gctggcggta atttcctcgt cagcaaacaa 720

ctcgataact ggtattttga attaaacacg ttactggaag acggaacatc tttatataac 780

gacggtaatt atcatggacg cgatatggaa cagcagaaaa atgttgctta tctgcgtccg 840

gtaattgcct ggtcgccgac ggaagaattc accgtttccg cagcgatgga agcgaatgtg 900

gtaaataatg cttatggtta taccgatagc aagggtaatt ttgtcgatca gtccgatcgt 960

accggttatg gcatgagtat gacctggaat ggcctgaaaa ccgatccgga aaatggcatc 1020

gtggttaatc ttaataccgc ctatttagat gctaataatg aaaaagattt cacggcaggg 1080

attaacgcgc tgtggaaacg tttcgagctg ggttatatct atgcacataa taagattgat 1140

gaatttagtg gcgtggtttg tgataacgat tgctggattg atgatgaagg aacatacaac 1200

attcacacca ttcatgcgtc ttatcagttc gctaatgtga tggatatgga gaactttaat 1260

atttacctcg gcacgtatta ctccattctg gatagcgacg gcgataagat acacggcgac 1320

gatagtgatg accgttacgg cgcacgcgtt cgctttaaat acttcttc 1368

<210> 157

<211> 522

<212> DNA

<213> Escherichia coli

<400> 157
atgaacggca aagcgtttct ggcctgcgtt ctgatgagcg tcgtattaac tggctgtgaa 60

acagcgaaaa aaatcagcca ggtgatccgc aatccggata ttcaggtcgg aaagctgatg 120

gatcagtcaa ccgagctgac cgtcacgctg ctgaccgagc cggacagcaa cctgacggcg 180

gatggcgaag ccgcgccggt ggatgtccag ttggtttatc tgagcgacga ctcaaaattc 240

catgccgccg actacgacca ggttgccacc accgcgctgc ccgacgtgct ggggaaaaac 300

tatatcgatc accaggactt caacctgttg ccggataccg taaaaacact gccgccgatc 360

aagttggatg agaaaaccgg ttatatcggt gtcattgcct atttttcaga cgaccaggcc 420

acagaatgga aacaaattga gtcggtagaa agtatcggcc accactatcg cctgctggtg 480

catatccgcg ccagtgcgat tgagatgaaa aaagaggaaa ac 522

<210> 158

<211> 3432

<212> DNA

<213> Escherichia coli

<400> 158
ctgacgctgg catggatttt tctgctggtg tggatctggt ggcagggtcc aaaatggacg 60

ctctatgagc agcactggct ggctccgctg gcaaaccgct ggctggcgac cgccgtctgg 120

ggacttatcg ctctggtctg gctcacctgg cgggtgatga agcgtctgca aaagctggaa 180

aaacagcaga aacagcagcg ggaggaagaa aaagatccgt tgaccgtgga actccaccgc 240

cagcagcaat atctggatca ctggctgctg cgcctgcgcc gccatctgga taaccgccgt 300

tatctgtggc agttgccgtg gtatatggtc attggtcctg cgggtagcgg caaaagcacg 360

ctgctgcgcg agggctttcc gtctgacatt gtttacacgc cggaaagcat ccggggtgtg 420

gaataccacc cgctgatcac accgcgagtg ggcaaccagg cggtaatttt cgatgttgac 480

ggcgtactga ccactcccgg cggggatgat ctgctccgcc gccgcctgcg cgaacactgg 540

ctgggctggc tgatgcaaac gcgcgctcgc cagccgctca acggtcttat cctgacgctc 600

gatcttcccg atctgctgac ggcggataaa tcccgccgtg agacactggt acaaaatttg 660

cgccagcaac ttcaggagat ccgtcagagc ctgcactgcc gtctgcccgt ttacgtggtg 720
ctgacacggc tggatctgct gaacggcttt gccgcgctgt tccattcact ggataaaaaa 780
gaccgcgatg cgatcctcgg cgtcacattt acccgccgcg cccatgaaag tgacggctgg 840
cgcagcgaac tgggggcttt ctggcagacg tgggtacaac aggtgaacct ggcgctgtcg 900
gatctggtgc tcgcacaaac cggtgctgct ccccgcagcg ctgtgttcag cttctcccgt 960
cagatgcagg gaacaggaga aatcgtcacc gcactgctcg ccgcattgct ggacggtgag 1020
aacatggatg taatgctgcg tggcgtctgg ctcacatcct cgctacagcg tggccaggtg 1080
gatgatattt tcacgcagtc cgccgcccgc cagtacggac tgggtaacag ctcgctggca 1140
acctggcctc tggtggagac gacgccgtat tttactcgcc gcctcttccc ggaagtcctg 1200
ctggctgagc cgaacctggc gggtgaaaac agcgtctggc tgaacagctc ccggcgcagg 1260
ctgaccgcct tttccacctg tggcgcggca ctggcggcat tgatggtcgg aagctggcac 1320
cattattaca atcagaactg gcagtctggc gttaacgtac tggcacaagc taaagccttt 1380
atggacgtac caccaccgca gggaacggat gaattcggca atctgcaatt gccattgctt 1440
aacccggtac gcgatgccac cctggcctat ggtgattatc gcgatcacgg ttttctggcg 1500
gatatgggat tgtaccaggg cgcccgcgta gggccgtatg tggagcaaac ctacattcag 1560
cttcttgagc agcgttatct cccctcgtta atgaacggcc tgatccggga tctaaacatt 1620
gccccgccag agagcgaaga aaagctcgct gtgctgcgcg tagtgcgcat gatggaagac 1680
aaaagtgggc gcaacaacga ggcggtaaaa cagtacatgg cacggcgctg gagcaatgaa 1740
tttcacggcc agcgcgatat tcaggcgcaa ctgatggtgc atctggacta tgcgctggag 1800
cacaccgact ggcacgcgca gcgccaaagc agcgacagcg atgctgtcag ccgctggacc 1860
ccctatgata aaccgatcat taatgcgcag caggaactga gcaagctgcc catataccag 1920
cgtgtctacc agaccctgcg caccaaagca ttaagcgtgt tgcccgccga tttgaatttg 1980
cgcgaccagg ttggtcccac cttcgacaac gtgttcgtcg ccggtaatga tgaaaaactg 2040
gtgatcccgc agttcctcac ccgctatgga ctgcaaagct attttgtcaa acagcgtgag 2100
ggcctcgttg agctgaccgc gctggattcg tgggtactga acctgacgca aagcgtcgcc 2160
tacagcgagg ccgaccgtga agagatccag cgccatatca ccgaacagta catcagtgac 2220
tataccgcca cctggcgtgc cggaatggat aacctcaacg tccgtgacta tgaggccatg 2280
tcggcgctga ccgacgcgct ggagcagatt atcagcggcg atcagccatt ccagcgtgcg 2340
ctgacggcgc tgcgcgataa tacccacgcg ctgacgctct ccggcaaact ggatgataag 2400
gcgagggaag cggcgataaa tgagatggat taccgcctgt tatcccggct ggggcatgag 2460
ttcgcaccgg aaaacagcgc actggaggag caaaaggaca aggcgagtac gctacaggcc 2520
gtgtaccagc aactgaccga gctgcaccgt tacctgctgg cgatccagaa ctcgccagtg 2580

ccggggaaat cggcgctgaa agcagtacag ctacggctgg atcaaaacag cagcgatcca 2640

atcttcgcca cccgtcagat ggcaaaaacc ctgcctgcgc ctcttaaccg ctgggtaggt 2700

aagctcgcgg atcaggcctg gcatgtggtg atggtggaag ccgttcgtta catggaagtg 2760

gactggcgcg acaatgtagt gaaacccttc aacgagcagc ttgccgataa ctatccgttt 2820

aatccgcgcg ccacacagga tgcctcactg gattcgtttg aacgtttctt taaaccggat 2880

ggcattctgg acaatttcta caagaacaac ctgcgcctgt tccttgaaaa cgatctgacc 2940

tttggcgacg acggcagagt gttaatccgt gaagatatcc ggcagcaact ggataccgcg 3000

cagaaaatcc gcgacatctt cttcagccag cagaacgggc tgggcgcaca gtttgccgtg 3060

gaaaccgtat cgctttccgg caataagcgg cgcagcgtac ttaacctgga cggccagtta 3120

gtggactaca gccagggacg caactacacc gcccatctgg tctggccgaa caacatgcgt 3180

gaaggcaatg aaagcaagct gacgctgatt ggcaccagcg gcagagcacc gcgcagtatc 3240

gcgttcagtg gaccgtgggc gcagttccgc ctgttcggcg cgggccagtt gaccaatgtg 3300

accagtgaca cctttaacgt gcgctttaac gtggacggcg gcgcaatggt ttaccaggtg 3360

catgtggata ccgaagataa cccgttcacc ggcggtctgt tcagcctgtt ccgtttaccg 3420

gatacgttgt at 3432

<210> 159

<211> 725

<212> PRT

<213> Escherichia coli

<400> 159

Met Arg Ile Asn Lys Ile Leu Trp Ser Leu Thr Val Leu Leu Val Gly
1               5                   10                  15

Leu Asn Ser Gln Val Ser Val Ala Lys Tyr Ser Asp Asp Asp Asn Asp
            20                  25                  30

Glu Thr Leu Val Val Glu Ala Thr Ala Glu Gln Val Leu Lys Gln Gln
        35                  40                  45

Pro Gly Val Ser Val Ile Thr Ser Glu Asp Ile Lys Lys Thr Pro Pro
    50                  55                  60

Val Asn Asp Leu Ser Asp Ile Ile Arg Lys Met Pro Gly Val Asn Leu
65                  70                  75                  80

Thr Gly Asn Ser Ala Ser Gly Thr Arg Gly Asn Asn Arg Gln Ile Asp

                85                  90                  95

Ile Arg Gly Met Gly Pro Glu Asn Thr Leu Ile Leu Ile Asp Gly Val
            100                 105                 110

Pro Val Thr Ser Arg Asn Ser Val Arg Tyr Ser Trp Arg Gly Glu Arg
        115                 120                 125

Asp Thr Arg Gly Asp Thr Asn Trp Val Pro Pro Glu Gln Val Glu Arg
    130                 135                 140

Ile Glu Val Ile Arg Gly Pro Ala Ala Ala Arg Tyr Gly Ser Gly Ala
145                 150                 155                 160

Ala Gly Gly Val Val Asn Ile Ile Thr Lys Arg Pro Thr Asn Asp Trp
                165                 170                 175

His Gly Ser Leu Ser Leu Tyr Thr Asn Gln Pro Glu Ser Ser Glu Glu
            180                 185                 190

Gly Ala Thr Arg Arg Ala Asn Phe Ser Leu Ser Gly Pro Leu Ala Gly
        195                 200                 205

Asp Ala Leu Thr Thr Arg Leu Tyr Gly Asn Leu Asn Lys Thr Asp Ala
    210                 215                 220

Asp Ser Trp Asp Ile Asn Ser Pro Val Gly Thr Lys Asn Ala Ala Gly
225                 230                 235                 240

His Glu Gly Val Arg Asn Lys Asp Ile Asn Gly Val Val Ser Trp Lys
                245                 250                 255

Leu Asn Pro Gln Gln Ile Leu Asp Phe Glu Val Gly Tyr Ser Arg Gln
            260                 265                 270

Gly Asn Ile Tyr Ala Gly Asp Thr Gln Asn Ser Ser Ser Ser Ala Val
        275                 280                 285

Thr Glu Ser Leu Ala Lys Ser Gly Lys Glu Thr Asn Arg Leu Tyr Arg
    290                 295                 300

Gln Asn Tyr Gly Ile Thr His Asn Gly Ile Trp Asp Trp Gly Gln Ser
305                 310                 315                 320

Arg Phe Gly Val Tyr Tyr Glu Lys Thr Asn Asn Thr Arg Met Asn Glu
                325                 330                 335

Gly Leu Ser Gly Gly Gly Glu Gly Arg Ile Leu Ala Gly Glu Lys Phe

            340                 345                 350

Thr Thr Asn Arg Leu Ser Ser Trp Arg Thr Ser Gly Glu Leu Asn Ile
        355                 360                 365

Pro Leu Asn Val Met Val Asp Gln Thr Leu Thr Val Gly Ala Glu Trp
    370                 375                 380

Asn Arg Asp Lys Leu Asp Asp Pro Ser Ser Thr Ser Leu Thr Val Asn
385                 390                 395                 400

Asp Arg Asp Ile Ser Gly Ile Ser Gly Ser Ala Ala Asp Arg Ser Ser
                405                 410                 415

Lys Asn His Ser Gln Ile Ser Ala Leu Tyr Ile Glu Asp Asn Ile Glu
            420                 425                 430

Pro Val Pro Gly Thr Asn Ile Ile Pro Gly Leu Arg Phe Asp Tyr Leu
        435                 440                 445

Ser Asp Ser Gly Gly Asn Phe Ser Pro Ser Leu Asn Leu Ser Gln Glu
    450                 455                 460

Leu Gly Asp Tyr Phe Lys Val Lys Ala Gly Val Ala Arg Thr Phe Lys
465                 470                 475                 480

Ala Pro Asn Leu Tyr Gln Ser Ser Glu Gly Tyr Leu Leu Tyr Ser Lys
                485                 490                 495

Gly Asn Gly Cys Pro Lys Asp Ile Thr Ser Gly Gly Cys Tyr Leu Ile
            500                 505                 510

Gly Asn Lys Asp Leu Asp Pro Glu Ile Ser Val Asn Lys Glu Ile Gly
        515                 520                 525

Leu Glu Phe Thr Trp Glu Asp Tyr His Ala Ser Val Thr Tyr Phe Arg
    530                 535                 540

Asn Asp Tyr Gln Asn Lys Ile Val Ala Gly Asp Asn Val Ile Gly Gln
545                 550                 555                 560

Thr Ala Ser Gly Ala Tyr Ile Leu Lys Trp Gln Asn Gly Gly Lys Ala
                565                 570                 575

Leu Val Asp Gly Ile Glu Ala Ser Met Ser Phe Pro Leu Val Lys Glu
            580                 585                 590

Arg Leu Asn Trp Asn Thr Asn Ala Thr Trp Met Ile Thr Ser Glu Gln

595                 600                 605

Lys Asp Thr Gly Asn Pro Leu Ser Val Ile Pro Lys Tyr Thr Ile Asn
    610                 615                 620

Asn Ser Leu Asn Trp Thr Ile Thr Gln Ala Phe Ser Ala Ser Phe Asn
625                 630                 635                 640

Trp Thr Leu Tyr Gly Arg Gln Lys Pro Arg Thr His Ala Glu Thr Arg
                645                 650                 655

Ser Glu Asp Thr Gly Gly Leu Ser Gly Lys Glu Leu Gly Ala Tyr Ser
            660                 665                 670

Leu Val Gly Thr Asn Phe Asn Tyr Asp Ile Asn Lys Asn Leu Arg Leu
        675                 680                 685

Asn Val Gly Val Ser Asn Ile Leu Asn Lys Gln Ile Phe Arg Ser Ser
    690                 695                 700

Glu Gly Ala Asn Thr Tyr Asn Glu Pro Gly Arg Ala Tyr Tyr Ala Gly
705                 710                 715                 720

Val Thr Ala Ser Phe
                725

<210>  160

<211>  2175

<212>  DNA

<213>  Escherichia coli

<400>  160
   atgagaatta acaaaatcct ctggtcgcta actgtgctcc tagttgggtt gaatagccag      60
   gtatcagtag ccaaatactc cgacgatgat aatgacgaga ctctggtggt ggaagccacc     120
   gctgagcagg tattaaaaca gcagccgggc gtgtcggtta ttaccagcga ggatattaaa     180
   aagacccctc cggtaaacga cctttcagat attattcgta aaatgcctgg tgttaatctt     240
   accggcaata gcgcctcggg cacacgcggt aataaccgcc agatcgatat tcgtggtatg     300
   gggccggaaa acaccttaat tttaattgat ggtgtaccgg tgacgtcacg taactccgtg     360
   cgttatagct ggcgtgggga gcgtgatacc cgcggtgaca ccaactgggt gccaccggaa     420
   caggttgagc gtattgaagt gatccgcggc cctgcggcgg cgcgctacgg ttcgggggcc     480
   gccggggggg tggtgaacat cattaccaaa cgtcccacca acgactggca cggttcgctg     540
   tcgttataca ccaaccagcc ggaaagtagc gaagagggcg ctacgcgtcg cgccaatttc 600
   agccttagtg ggcctctggc tggtgatgct cttaccacgc gtttgtatgg taacctgaat 660
   aaaacggatg ctgacagttg ggatattaat tctccggtcg gtacgaaaaa cgcagccggg 720
   catgaagggg tacgtaacaa agatattaac ggcgttgtct cgtggaaatt aaatccgcag 780
   cagattctcg atttcgaagt cggatatagc cgccagggga atatctatgc gggcgatacg 840
   cagaacagtt cttccagtgc agttaccgaa agcctggcaa aatccggcaa agagacgaac 900
   cgcctgtacc gacagaatta tggcattacg cataatggta tctgggactg gggacaaagt 960
   cgctttggtg tttattacga gaaaaccaat aatacccgca tgaatgaagg attatccggc 1020
   ggtggtgaag gacgtatttt agcgggtgaa aagtttacga ccaatcgcct gagttcctgg 1080
   cgaaccagcg gtgagcttaa tattcctttg aatgtgatgg ttgatcaaac gctgaccgtt 1140
   ggtgcagagt ggaaccgcga taagctcgat gatccttcct ctaccagcct gacggtgaat 1200
   gacagagata tcagcggtat ttctggctct gctgcggatc gcagcagtaa aaatcattct 1260
   caaatcagtg cgctgtatat tgaagataac attgagccgg ttcctggcac gaatatcatt 1320
   cccggcctgc gctttgatta tctcagcgac tccggcggga acttcagccc cagtctgaat 1380
   ctttcgcagg aattgggcga ttatttcaaa gtcaaagcag gggttgcccg aacctttaaa 1440
   gccccaaacc tgtatcaatc cagtgaaggc tatctgctct actcgaaagg caatggctgt 1500
   ccaaaagata ttacatcagg cgggtgctac ctgatcggta ataaagatct cgatccggaa 1560
   atcagcgtca ataaagaaat tggactggag ttcacctggg aagattacca cgcaagtgtg 1620
   acctacttcc gcaatgatta ccagaataag atcgtggccg gggataacgt tatcgggcaa 1680
   accgcttcag gcgcatatat cctcaagtgg cagaatggcg ggaaagctct ggtggacggt 1740
   atcgaagcca gtatgtcttt cccactggtg aaagagcgtc tgaactggaa taccaatgcc 1800
   acatggatga tcacttcgga gcaaaaagac accggtaatc ctctgtcggt catcccgaaa 1860
   tatactatca ataactcgct taactggacc atcacccagg cgttttctgc cagcttcaac 1920
   tggacgttat atggcagaca aaaaccgcgt actcatgcgg aaacccgcag tgaagatact 1980
   ggcggtctgt caggtaaaga gctgggcgct tattcactgg tggggacgaa cttcaattac 2040
   gatattaata aaaatctgcg tcttaatgtc ggcgtcagta atatcctcaa taaacagatc 2100
   ttccgatctt ctgaaggggc gaatacctat aacgagccag gccgggctta ttatgccgga 2160
   gttaccgcat cattc 2175